# EUROPEAN PATENT SPECIFICATION

(11) **EP 1 343 771 B1**
(45) Date of publication and mention of the grant of the patent: **12.04.2006**
(21) Application number: 01271107.3
(22) Date of filing: 07.12.2001
(51) Int. Cl.: C07D 271/06, C07D 263/32, C07D 263/48, C07D 413/04, C07D 413/06, C07D 413/12, C07D 417/06, A61K 31/421, A61K 31/422, A61K 31/4245, A61P 17/00, C07C 311/06, C07C 233/48, C07C 259/14, C07D 205/04

(54) **3-OX(ADI)AZOLYLPROPANOHYDROXAMIC ACIDS USEFUL AS PROCOLLAGEN C-PROTEINASE INHIBITORS**
ZUR VERWENDUNG ALS PROCOLLAGEN-C-PROTEINASE-INHIBITOREN GEEIGNETE 3-OX(ADI)AZOLYLPROPANOHYDROXAMSÄUREN
ACIDES 3-OX(ADI)AZOLYLPROPANOHYDROXAMIQUES UTILES EN TANT QU'INHIBITEURS DE LA PROCOLLAGENE C-PROTEINASE

(30) Priority: 21.12.2000 GB 0031321
(43) Date of publication of application: 17.09.2003
(73) Proprietor: Pfizer Limited, Sandwich, Kent CT13 9NJ (GB); PFIZER INC., New York, N.Y. 10017 (US)
(72) Inventor: DATTA, Usa, Pfizer Global Research and Development, Sandwich, Kent CT13 9NJ (GB); FISH, Paul, Vincent, Sandwich, Kent CT13 9NJ (GB); JAMES, Kim, Pfizer Global Research and Development, Sandwich, Kent CT13 9NJ (GB); WHITLOCK, Gavin, Alistair, Sandwich, Kent CT13 9NJ (GB)
(74) Representative: Motion, Keith Robert
(86) International application number: PCT/IB2001/002360
(87) International publication number: WO 2002/050046

(56) References cited:
- WO-A-01/47901
- WO-A-95/23790
- FRAY M J ET AL: "Application of Epimerisation-Free Amide Coupling Conditions to the Synthesis of Matrix Metalloprotease Inhibitor Intermediates" TETRAHEDRON, vol. 54, no. 45, 5 November 1998 (1998-11-05), pages 13825-13832, XP004139896
- FLOYD C D ET AL: "Rapid synthesis of matrix metalloproteinase inhibitors via Ugi four-component condensation" SYNLETT, no. 6, June 1998 (1998-06), pages 637-639, XP002163187
- YAMAMOTO M ET AL: "Inhibition of membrane-type 1 matrix metalloproteinase by hydroxamate inhibitors: an examination of the subsite pocket" JOURNAL OF MEDICINAL CHEMISTRY, vol. 41, no. 8, 9 April 1998 (1998-04-09), pages 1209-1217, XP000867897 ISSN: 0022-2623 cited in the application
- DANKWARDT S M ET AL: "Solid-phase synthesis of di- and tripeptidic hydroxamic acids as inhibitors of procollagen C-proteinase" BIOORGANIC & MEDICINAL CHEMISTRY LETTERS, vol. 10, no. 22, 20 November 2000 (2000-11-20), pages 2513-2516, XP004224252 ISSN: 0960-894X cited in the application
- FRAY M J ET AL: "Selectivity of inhibition of matrix metalloproteases MMP-3 and MMP-2 by succinyl hydroxamates and their carboxylic acid analogues is dependent on P3' group chirality" BIOORGANIC & MEDICINAL CHEMISTRY LETTERS, vol. 11, no. 4, 26 February 2001 (2001-02-26), pages 567-570, XP004230061

## Description

This invention relates to a certain class of compounds, and the pharmaceutically acceptable salts and solvates thereof, which inhibit Procollagen C-proteinase ("PCP"). They are therefore useful in the treatment of mammals having conditions alleviable by inhibition of PCP. Especially of interest is an antiscarring treatment for wounds.

Fibrotic tissues, including dermal scars, are characterised by excessive accumulation of extracellular matrix, mainly collagen type I. It is thought that inhibition of collagen deposition will reduce formation of scar tissue. Collagen is secreted as the precursor, procollagen, which is transformed into the insoluble collagen by cleavage of the C-temninal propeptide by PCP. PCP is a zinc-dependent metalloprotease which is secreted from TGF-p-activated fibroblasts belonging to the subfamily of astacin-like proteases and able to cleave the C-terminal peptide of types I, II and III procollagens. Furthermore, data suggest that PCP activates lysyl oxidase, an enzyme essential for the formation of covalent cross-links which stabilise the fibrous form of collagen. Therefore, inhibition of PCP may not only reduce collagen deposition but may also make collagen more accessible for degradation.

Collagen is integral to, among other things, the proper formation of connective tissue. Thus, the over- or under-production of collagen or the production of abnormal collagen (including incorrectly processed collagen) has been linked with numerous connective tissue diseases and disorders. Mounting evidence suggests that PCP is an essential key enzyme for the proper maturation of collagen (see for example International Patent Application publication number WO 97/05865).

The present invention relates to substances capable of inhibiting PCP activity in order to regulate, modulate and/or reduce collagen formation and deposition. More specifically, the invention relates to the use of compounds and pharmaceutical compositions thereof for the treatment of various conditions relating to production of collagen.

At present more than nineteen types of collogens have been identified. These collagens, including fibrillar collagen types I, II, III are synthesized as procollagen precursor molecules which contain amino- and carboxy-terminal peptide extensions. These peptide extensions, referred to as "pro-regions," are designated as N- and C- propeptides, respectively. The pro-regions are typically cleaved upon secretion of the procollagen triple helical precursor molecule from the cell to yield a mature triple helical collagen molecule. Upon cleavage, the "mature" collagen molecule is capable of association, for example, into highly structured collagen fibers. See e.g., Fessler and Fessler, 1978, Annu. Rev. Biochem. 47:129-162; Bornstein and Traub, 1979, in: The Proteins (eds. Neurath, H. and Hill, R.H.), Academic Press, New York, pp. 412-632; Kivirikko et al., 1984, in: Extracellur Matrix Biochemistry (eds. Piez, K.A. and Reddi. A.H.), Elsevier Science Publishing Co., Inc., New York, pp. 83-118; Prockop and Kivirikko, 1984, N. Engl, J. Med. 311:376-383; Kuhn, 1987, in: Structure and Function of Collagen Types (eds. Mayne, R. and Burgeson, R.E.). Academic Press, Inc., Orlando, Florida, pp. 1-42.

An array of conditions has been associated with the inappropriate or unregulated production of collagen, including pathological fibrosis or scarring, including endocardial sclerosis, idiopathic interstitial fibrosis, interstitial pulmonary fibrosis, perimuscular fibrosis, Symmers' fibrosis, pericentral fibrosis, hepatitis, dermatofibroma, cirrhosis such as billary cirrhosis and alcoholic cirrhosis, acute pulmonary fibrosis, idiopathic pulmonary fibrosis, acute respiratory distress syndrome, kidney fibrosis/glomerulonephritis, kidney fibrosis/diabetic nephropathy, scleroderma/systemic, scleroderma/local, keloids, hypertrophic scars, severe joint adhesions/arthritis, myelofibrosis, corneal scarring, cystic fibrosis, muscular dystrophy (duchenne's), cardiac fibrosis, muscular fibrosis/retinal separation, esophageal stricture and Pyronie's disease. Further fibrotic disorders may be induced or initiated by surgery, including scar revision/plastic surgeries, glaucoma, cataract fibrosis, corneal scarring, joint adhesions, graft vs. host disease, tendon surgery, nerve entrapment, dupuytren's contracture, OB/GYN adhesions/fibrosis, pelvic adhesions, peridural fibrosis, restenosis. Other conditions where collagen plays a key role include burns, and chronic dermal ulcers which sometimes have a build-up of periulcer fibrosed material around them. Fibrosis of lung tissue is also observed in patients suffering from chronic obstructive airways disease (COAD) and asthma. One strategy for the treatment of these diseases and conditions is to inhibit the overproduction and/or deposition and/or unregulation of collagen. Thus, identification and isolation of molecules which control, inhibit and/or modulate the production and deposition of collagen are of major medical interest.

Recent evidence suggests that PCP is the essential key enzyme that catalyzes the cleavage of the Procollagen C-propeptide. This has been demonstrated in fibrillar collagens, including type I, type II, and type III collagen.

PCP was first observed in the culture media of human and mouse fibroblasts (Goldberg et al., 1975, Cell 4:45-50; Kessler and Goldberg, 1978, Anal. Biochem. 86:463-469), and chick tendon fibroblasts (Duskin et al., 1978, Arch. Biochem. Biophys. 185:326-332; Leung et al., 1979, J.

Biol, Chem. 254:224-232). An acidic proteinase which removes the C-terminal propeptides from type I procollagen has also been identified (Davidson et al., 1979, Eur. J. Biochem. 100:551).

A partially purified protein having PCP activity was obtained from chick calvaria in 1982. Njieha et al., 1982, Biochemistry 23:757-764. In 1985, chicken PCP was isolated, purified and characterized from conditioned media of chick embryo tendons. Hojima et al., 1985, J. Biol. Chem. 260:15996-16003. Murine PCP has been subsequently purified from media of cultured mouse fibroblasts. Kessler et al., 1986, Collagen Relat. Res. 6:249-266; Kessler and Adar, 1989, Eur. J. Biochem. 186:115-121. Finally, the cDNA encoding human PCP has been identified, as set forth in the above-referenced articles and references disclosed therein.

Experiments conducted with these purified forms of chick and mouse PCP have indicated that the enzyme is instrumental in the formation of functional collagen fibers. Fertala et al., 1994, J. Biol. Chem. 269:11584.

As a consequence of the enzyme's apparent importance to collagen production, scientists have identified a number of PCP inhibitors. See e.g., Hojima et al., supra. For example, several metal chelators have demonstrated activity as PCP inhibitors. Likewise, chymostatin and pepstatin A were found to be relatively strong inhibitors of PCP. Additionally, α₂-Macroglobuline, ovostatin, and fetal bovine serum appear to at least partially inhibit PCP activity.

Dithiothreitol, SDS, concanavalin A, Zn²⁺, Cu²⁺, and Cd²⁺ are similarly reported to be inhibitory at tow concentrations. Likewise, some reducing agents, several amino acids, phosphate, and ammonium sulfate were inhibitory at concentrations of 1-10mM. Further, the enzyme was shown to be inhibited by the basic amino acids lysine and arginine (Leung et al., supra; Ryhänen et al., 1982, Arch. Biochem. Biophys. 215:230-235). Finally, high concentrations of NaCl or Tris-HCl buffer were found to inhibit PCP's activity. For example, it is reported that, with 0.2, 0.3, and 0.5M NaCl, the activity of PCP was reduced 66, 38, and 25%, respectively, of that observed with the standard assay concentration of 0.15M. Tris-HCl buffer in a concentration of 0.2-0.5M markedly inhibited activity (Hojima et al., supra). PCP activity and its inhibition have been determined using a wide array of assays. See e.g., Kessler and Goldberg, 1978, Anal. Biochem. 86:463; Njieha et al., 1982, Biochemistry 21:757-764. As articulated in numerous publications, the enzyme is difficult to isolate by conventional biochemical means and the identity of the cDNA sequence encoding such enzyme was not known until reported in the above references and related patent applications.

In view of its essential role in the formation and maturation of collagen PCP appears to be an ideal target for the treatment of disorders associated with the inappropriate or unregulated production and maturation of collagen.
The identification of effective compounds which specifically inhibit the activity of PCP to regulate and modulate abnormal or inappropriate collagen production is therefore desirable and the object of this invention.

Matrix metalloproteases (MMPs) constitute a family of structurally similar zinc-containing metalloproteases, which are involved in the remodelling, repair and degradation of extracellular matrix proteins, both as part of normal physiological processes and in pathological conditions.

Another important function of certain MMPs is to activate other enzymes, including other MMPs, by cleaving the pro-domain from their protease domain. Thus, certain MMPs act to regulate the activities of other MMPs, so that over-production in one MMP may lead to excessive proteolysis of extracellular matrix by another, e.g. MMP-14 activates pro-MMP-2

During the healing of normal and chronic wounds, MMP-1 is expressed by migrating keratinocytes at the wound edges (U.K. Saarialho-Kere, S.O. Kovacs, A.P. Pentland, J. Clin. Invest. 1993, 92, 2858-66). There is evidence which suggests MMP-1 is required for keratinocyte migration on a collagen type I matrix in vitro, and is completely inhibited by the presence of the non-selective MMP inhibitor SC44463 ((N4-hydroxy)-N1-[(1S)-2-(4-methoxyphenyl)methyl-1-((1R)-methylamino)carbonyl)]-(2R)-2-(2-rnethylpropyl)butanediamide) (B.K. Pilcher, J.A. Dumin, B.D. Sudbeck, S.M. Krane, H.G. Welgus, W.C. Parks, J. Cell Biol., 1997, 137, 1-13). Keratinocyte migration in vivo is essential for effective wound healing to occur.

MMP-2 and MMP-9 appear to play important roles in wound healing during the extended remodelling phase and the onset of re-epithelialisation, respectively (M.S. Agren, Brit. J. Dermatology, 1994, 131, 634-40; T. Salo, M. Mäkänen, M. Kylmänieml, Lab. Invest., 1994, 70, 176-82). The potent, non-selective MMP inhibitor BB94 ((2S,3R)-5-methyl-3-{[(1S)-1-(methylcarbamoyl)-2-phenylethyl]carbamoyl}-2-[(2-thienylthio)methyl]hexanohydroxamic acid, batimastat), inhibits endothelial cell invasion of basement membrane, thereby inhibiting angiogenesis (G. Tarboletti, A. Garofalo, D. Belotti, T. Drudis, P. Borsotti, E. Scanziani, P.D. Brown, R. Giavazzi, J. Natl. Cancer Inst., 1995, 87, 293-8). There is evidence that this process requires active MMP-2 and/or 9.

Thus PCP inhibitors which significantly inhibit MMPs 1 and/or 2 and/or 9 would be expected to impair wound healing. MMP-14 is responsible for the activation of MMP-2, and thus inhibition of MMP-14 might also result in impaired wound healing.

For recent reviews of MMPs, see Zask et al, Current Pharmaceutical Design, 1996, 2, 624-661; Beckett, Exp. Opin. Ther. Patents, 1996, 6, 1305-1315; and Beckett et al, Drug Discovery Today, vol 1(no.1), 1996,16-26.

Alternative names for various MMPs and substrates acted on by these are shown in the table below (Zask et al, supra).

| Enzyme | Other names | Preferred substrates |
|---|---|---|
| MMP-1 | Collagenase-1, interstitial collagenase | Collagens I, II, III, VII, X, gelatins |
| MMP-2 | Gelatinase A, 72kDa gelatinase | Gelatins, collagens IV, V, VII, X, elastin, fibronectin; activates pro-MMP-13 |
| MMP-3 | Stromelysin-1 | Proteoglycans, laminin, fibronectin, gelatins. |
| MMP-7 | Pump, Matrilysin | Proteoglycans, laminin, fibronectin, gelatins, collagen IV, elastin, activates pro-MMP-1 and -2 . |
| MMP-8 | Collagenase-2, neutrophil collagenase | Collagens I, II, III |
| MMP-9 | Gelatinase B, 92 kDa gelatinase | Gelatins, collagens IV, V, elastin |
| MMP-12 | Macrophage metalloelastase | Elastin, collagen IV, fibronectin, activates pro-MMP-2 & 3. |
| MMP-13 | Collagenase-3 | Collagens I, II, III, gelatins |
| MMP-14 | MT-MMP-1 | Activates pro-MMP-2 & 13, gelatins |
| MMP-15 | MT-MMP-2 | unknown |
| MMP-16 | MT-MMP-3 | Activates pro-MMP-2 |
| MMP-17 | MT-MMP-4 | unknown |

International Patent Application publication number WO 01/47901 discloses a number of 3-heterocyclylpropanohydroxamic acid PCP inhibitors.

International Patent Application publication number WO 95/23790 discloses a number of imidazole-substituted hydroxamic acid derivatives as MMP inhibitors, including collagenase inhibitors.

Yamamoto *et al*, in J.Med.Chem. (1998) 41, 1209 discloses a series of hydroxamic acid derivatives as MMP-1 inhibitors.

Dankwardt *et al*, in Bioorg.Med.Chem Letters (2000) 10,2513 discloses various peptidic hydroxamic acid inhibitors of PCP.

According to one aspect of the present invention, there are provided compounds of formula (I): 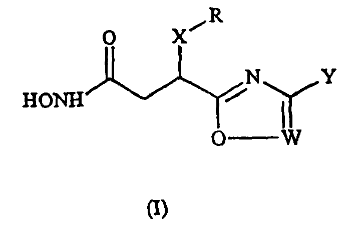 wherein:
X is C₁₋₆ alkylene or C₂₋₆ alkenylene, each of which is optionally substituted by one or more fluorine atoms;
R is aryl or C₃₋₆ cycloalkyl optionally substituted by one or more fluorine atoms;
W is N or CZ;
Y is
   (a) NR¹R³,
   (b) C₁₋₄ alkyl substituted by NR¹R² or by a 4- to 7-membered N-heterocycle attached via the N-atom, which heterocycle optionally contains 1 or 2 further ring hetero-atoms independently selected from N, O and S, and which heterocycle is optionally substituted by one or more substituents Independently selected from R², =O, OH, SO₂(C₁₋₄ alkyl) and/or C(O)ₚ(C₁₋₄ alkyl) groups,
      or (c) a 4- to 7-membered saturated or partially or fully unsaturated N-heterocycle, which heterocycle optionally contains 1 or 2 further ring hetero-atoms independently selected from N, O and S, and which heterocycle is optionally substituted by one or more substituents independently selected from R², =O, OH, SO₂(C₁₋₄ alkyl) and/or C(O)ₚ(C₁₋₄ alkyl) groups,
Z is H or C₁₋₄ alkyl,
   or when W is CZ, Y is H or C₁₋₄ alkyl, and Z is
   (a) NR¹R³,
   (b) C₁₋₄ alkyl substituted by NR¹R² or by a 4- to 7-membered N-heterocycle attached via the N-atom, which heterocycle optionally contains 1 or 2 further ring hetero-atoms independently selected from N, O and S, and which heterocycle is optionally substituted by one or more substituents independently selected from R², =O, OH, SO₂(C₁₋₄ alkyl) and/or C(O)ₚ(C₁₋₄ alkyl) groups,
      or (c) a 4- to 7-membered saturated or partially or fully unsaturated N-heterocycle attached via the N-atom, which heterocycle optionally contains 1 or 2 further ring hetero-atoms independently selected from N, O and S, and which heterocycle is optionally substituted by one or more substituents independently selected from R², =O, OH, SO₂(C₁₋₄ alkyl) and/or C(O)ₚ(C₁₋₄ alkyl) groups,
R² is H,
   C₁₋₆ alkyl (optionally substituted by one or more substituents independently selected from OH,
   C₁₋₄ alkoxy, C(O)ₚ(C₁₋₄ alkyl, aryl, heteroaryl, or NR¹R³), CONR¹R³ or NR¹R³),
   SO₂(C₁₋₄ alkyl, aryl, heteroaryl or NR¹R³),
   C(O)ₚ(C₁₋₄ alkyl optionally substituted by C₁₋₄ alkoxy or NR¹R³),
   C(O)ₚ(C₃₋₇ cycloalkyl),
   C(O)ₚ(aryl),
   C(O)ₚ(heteroaryl),
   CONR¹R³,
   C₃₋₇ cycloalkyl optionally substituted by one or more substituents independently selected from OH and C₁₋₄ alkoxy,
   a 4- to 7-membered saturated or partially or fully unsaturated heterocycle, which heterocycle ring contains up to 3 ring hetero-atoms independently selected from N, O and S, and which heterocycle is optionally substituted by one or more substituents independently selected from
   R³, =O, OH, SO₂(C₁₋₄ alkyl) and/or C(O)ₚ(C₁₋₄ alkyl) groups,
   or aryl,
R¹ and R³ are each independently selected from H and C₁₋₄ alkyl optionally substituted by OH, NR⁴R⁵ or by C₁₋₄ alkoxy,
R⁴ and R⁶ are each independently selected from H and C₁₋₄ alkyl,
p is 1 or 2,
"aryl" is phenyl optionally substituted by one or more substituents independently selected from R³, OH, SO₂(C₁₋₄ alkyl) and/or C(O)ₚ(C₁₋₄ alkyl) groups,
"heteroaryl" is a 5- to 7-membered aromatic heterocycle with 1, 2 or 3 ring hetero-atoms independently selected from N, O and S, and which ring is optionally substituted by one or more substituents independently selected from R³, =O, OH, SO₂(C₁₋₄ alkyl) and/or C(O)ₚ(C₁₋₄ alkyl) groups,
   and the pharmaceutically acceptable salts and solvates (including hydrates) thereof.

"Alkyl", "alkylene", "alkoxy", "alkanoyl", and "alkenylene" groups, including in groups incorporating said moieties, may be straight chain or branched where the number of carbon atoms allows.

Halogen is taken to mean fluorine, chlorine, bromine or iodine.

Pharmaceutically-acceptable salts are well known to those skilled In the art, and for example include those mentioned in the art cited above, and by Berge et al, in J.Pharm.Sci., 66, 1-19 (1977). Suitable acid addition salts are formed from acids which form non-toxic salts and include the hydrochloride, hydrobromide, hydrolodide, nitrate, sulphate, bisulphate, phosphate, hydrogenphosphate, acetate, trifluoroacetate, gluconate, lactate, salicylate, citrate, tartrate, ascorbate, succinate, maleate, fumarate, gluconate, formate, benzoate, methanesulphonate, ethanesulphonate, benzenesulphonate, pamoate, camsylate, and p-toluenesulphonate salts.

Pharmaceutically acceptable base addition salts are well known to those skilled in the art, and for example include those mentioned in the art cited above, and can be formed from bases which form non-toxic salts and include the aluminium, calcium, lithium, magnesium, potassium, sodium and zinc salts, and salts of non-toxic amines such as diethanolamine.

Certain of the compounds of formula (I) may exist in one or more zwitterionic forms. Certain of the compounds of formula (I) may exist in one or more tautomeric forms. Certain of the compounds of formula (I), their salts, solvates, etc. may exist in one or more polymorphic forms. It is to be understood that the compounds of formula (I) include all such zwitterions, tautomers and polymorphs.

The compounds of formula (I), their salts, hydrates, etc. can exhibit isotopic variation, e.g. forms with enriched ²H, ³H, ¹³C, ¹⁴C, ¹⁵N, ¹⁸O, etc. may be prepared, for example by suitable variation of the synthetic methods described herein using methods and reagents known in the art or routine modification thereof. All such isotopic variants are included in the scope of the invention.

Certain of the compounds of the formula (I) may exist as geometric isomers. The compounds of the formula (I) may possess one or more asymmetric centres, apart from the specified centres in formula (I), and so exist in two or more stereoisomeric forms. The present invention indudes all the individual stereoisomers and geometric isomers of the compounds of formula (I) and mixtures thereof.

Preferably the compounds of formula (I) have the following stereochemistry (IA):

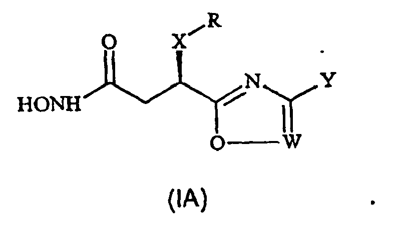

Preferably W is N.

Preferably X is a linear C₂₋₆alkylene moiety optionally substituted by one or more fluorine atoms. More preferably X is propylene.

Preferably R is C₃₋₈ cycloalkyl optionally substituted by one or more fluorine atoms.
More preferably R is cyclobutyl, cyclopentyl or cyclohexyl optionally substituted by one or more fluorine atoms.
Most preferably R is cyclohexyl.

Preferably Y is C₁₋₄ alkyl substituted by NR¹R², or Y is a 4- to 7-membered saturated or partially or fully unsaturated N-heterocycle, which heterocycle optionally contains 1 or 2 further ring hetero-atoms independently selected from N, O and S, and which heterocycle is optionally substituted by one or more substituents independently selected from R², =O, OH, SO₂(C₁₋₄ alkyl) and/or C(O)ₚ(C₁₋₄ alkyl) groups.
More preferably Y is CH₂ substituted by NR¹R², or Y is a 6-membered saturated or partially or fully unsaturated N-heterocycle, and which heterocycle is optionally substituted by one or more substituents independently selected from R², =O, OH, SO₂(C₁₋₄ alkyl) and/or C(O)ₚ(C₁₋₄ alkyl) groups.
Yet more preferably Y is CH₂N(H or CH₃)(SO₂(C₁₋₄ alkyl, aryl, heteroaryl or NR¹R³)), or Y is a 6-membered saturated or partially or fully unsaturated N-heterocycle, and which heterocycle is optionally substituted by SO₂(C₁₋₄ alkyl) and/or C(O)ₚ(C₁₋₄ alkyl) groups.
Further more preferably Y is CH₂NHSO₂(C₁₋₄ alkyl), or Y is a 6-membered saturated N-heterocycle, and which heterocycle is optionally substituted by SO₂(C₁₋₄ alkyl) or C(O)ₚ(C₁₋₄ alkyl).
Most preferably Y is CH₂NHSO₂CH₃ or methylsulphonylpiperidinyl.

Preferably Z is H or CH₃

A preferred group of compound of formula (I) are those wherein each substituent is as specified in the Examples below.

Another preferred group are the compounds of the Examples below and the salts or solvates thereof.

A further aspect of the invention is a PCP inhibitor of formula (I) which is selective against MMP-1 and/or MMP-2 and/or MMP-9 and/or MMP-14.
A further aspect of the invention is a PCP inhibitor of formula (I) which is selective against MMP-1 and/or MMP-2 and/or MMP-9 and/or MMP-14 for use in medicine.
Further related to this aspect of the invention is the use of a PCP inhibitor of formula (I) which is selective against MMP-1 and/or MMP-2 and/or MMP-9 and/or MMP-14 in the manufacture of an antiscarring medicament.
Further related to this aspect of the invention is a compound of formula (I) for use in a method of treating a condition mediated by PCP and in which MMP-1 and/or MMP-2 and/or MMP-9 and/or MMP-14 have a beneficial effect, with an effective amount of PCP inhibitor of formula (I) which is selective against MMP-1 and/or MMP-2 and/or MMP-9 and/or MMP-14, an example of such a condition being a wound.

Preferably the PCP inhibitor of formula (I) mentioned in this aspect of the invention is selective against at least MMP-1, MMP-2 and MMP-9.
Most preferably the said PCP inhibitor of formula (I) is selective against MMP-1, MMP-2, MMP-9, and MMP-14.
Preferably the said selective PCP inhibitor of formula (I) has an IC₅₀ vs. PCP of 0.5µM or lower, and selectivities vs. MMP-2 and MMP-9 of at least 30-fold, in the tests described herein. Preferably the selective PCP inhibitor of formula (I) has an IC₅₀ vs. PCP of 0.1 µM or lower, and selectivities vs. MMP-1, MMP-2, MMP-9 and MMP-14 of at least 300-fold, in the tests described herein.

Another aspect of the invention is a substance of formula (I) described herein, including the salts and solvates thereof, for use in medicine.

Another aspect of the invention is the use of the substances of formula (I) described herein, including the salts and solvates thereof, in the manufacture of a medicament for treatment of a PCP-mediated condition (e.g. an antiscarring medicament).

Another aspect of the invention is a pharmaceutical composition comprising a compound of formula (I), salts thereof and solvates thereof and a pharmaceutically acceptable diluent, carrier or adjuvant.

Another aspect of the invention is the combination of a compound of formula (I), or a salt, or solvate thereof, with one or more other active agent useful in treating wounds, such as:
(I) a growth factor such as TGF-β-3 (Renovo), IGF-1 (Genentech), IGF-1 complex (Celtrix), KGF-2 or FGF-10 (Sumitomo), DWP-401/EGF (Daewoong) or SNK-863 (Sanwa Kagaku Kenkyusho);
(ii) a growth factor agonist such as Noggin (Regeneron);
(iii) a growth factor antibody/antisense material, such as those to: TGF-β-1 or 2 (Renovo, CaT), PDGF (II Yang) or CTGF (Fibrogen);
(iv) a hormone such as DHEAS (Pharmadigm). ConXn / Relaxin (Connetics);
(v) an antibody to adhesion compounds such as ICAM-1 (Boehringer);
(vi) a MMP beneficial to healing of wounds, such as Collagenase ABC (BioSpecifics);
(vii) a barrier such as ADCON (Gliatech);
(viii) skin products such as artificial skin systems such as those based on DermaGraft (Advanced Tissue Sciences Inc.), INTEGRA Artificial Skin (Integra Life Sciences Holding Corp.), cell cultures such as Apligraf / Graftskin (Novartis), those developed by Cell Genesys Inc., AlloDerm (LifeCell) or matrix formulation products such as Argidene gel (Tellos Pharmaceuticals Inc.);
(ix) a uPA inhibitor such as those disclosed in patent applications WO 99/20608, WO 00/05214 and EP 1 044 967; and/or
(x) a MMP-3 inhibitor such as those disclosed in patent applications WO99/35124,WO 99/29667, EP 0 931 788 and WO 00/74681.

Yet another aspect of the invention is a compound of formula (I) for use in a method of treatment of a condition mediated by PCP comprising administration of a therapeutically-effective amount of a substance according to the above definitions.

It is to be appreciated that reference to treatment includes prophylaxis as well as the alleviation of established symptoms of PCP-mediated conditions and diseases.

The invention further provides Methods for the production of compounds of the invention, which are described below and in the Examples and Preparations. The skilled man will appreciate that the compounds of the invention could be made by methods other than those specifically described herein, by adaptation of the methods herein described in the sections below and/or adaptation thereof, for example by methods known in the art. Suitable guides to synthesis, functional group transformations, use of protecting groups, etc. are, for example, "Comprehensive Organic Transformations" by RC Larock, VCH Publishers Inc. (1989), "Advanced Organic Chemistry" by J March, Wiley Interscience (1985), "Designing Organic Synthesis" by S Warren, Wiley Interscience (1978), "Organic Synthesis - The Disconnection Approach" by S Warren, Wiley Interscience (1982), "Guidebook to Organic Synthesis" by RK Mackie and DM Smith, Longman (1982), "Protective Groups in Organic Synthesis" by TW Greene and PGM Wuts, John Wiley and Sons Inc. (1999), and PJ Kocienski, in "Protecting Groups", Georg Thieme Verlag (1994), and any updated versions of said standard works.

In the Methods below, unless otherwise specified, the substituents are as defined above with reference to the compounds of formula (I) above.

The compounds of formula (I) where W is N can be prepared according to the chemistry outlined in the schemes below.

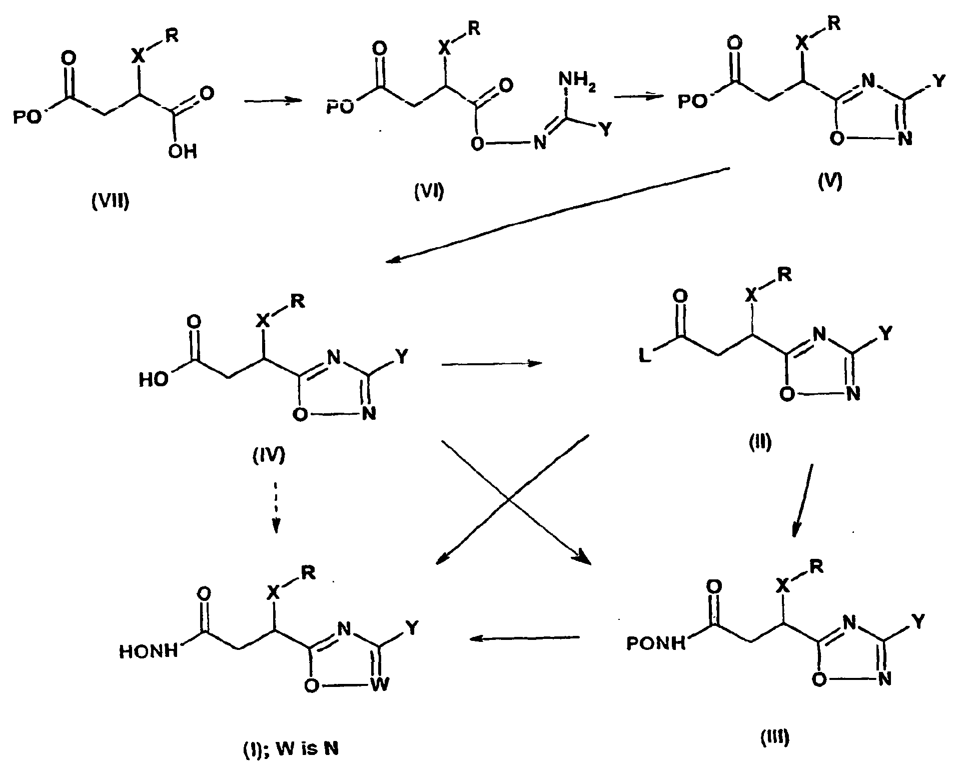

The hydroxamic acid compounds of formula (I) where W is N can be made by reaction of the corresponding activated acid derivative of formula (II), where L⁻ is a suitable leaving group, with hydroxylamine.

Suitable leaving groups are generally those which would leave in a more efficient manner than the hydroxide of the parent acid (IV), in a nucleophilic substitution reaction, such as a halide, C₁₋₄ alkoxide optionally substituted by halogen, an alkylsulphonate such as methylsulphonate or mesylsulphonate, an arylsulphonate such as p-tosylsulphonate. Other suitable leaving groups are familiar to those working in the field of amino acid coupling.

Such compounds of formula (II) may be made via standard chemistry from the corresponding acid (IV). Compounds of formula (II) where L is a leaving group such as Cl, Br, l, OCO(C₁₋₄ alkyl optionally substituted by one or more halogen), mesylate, tosylate, and the like, can be made from the corresponding compound of formula (II) where L is OH by conventional methods, using an acid-activating agent including methods typified in e.g. Examples 1-6, etc.

A coupling agent such as a diimide coupling agent, e.g. carbonyldiimidazole or N,N'-dicyclohexylcarbodiimide, may be used to convert compounds of formula (IV) to compounds of formula (I) (see e.g. Examples 7,19), under standard conditions with the agents mentioned previously, e.g. via intermediates of formulae (II) and/or (III).

The hydroxylamine used in this reaction can be suitably generated in situ by treatment of a hydroxylamine salt such as the hydrochloride salt with a suitable base such as triethylamine. Suitably the reaction is carried out in a polar solvent such as DMF.
This reaction, leaving groups, solvents, reagents, etc. are exemplified below in the relevant Examples below.

Alternatively the compounds of formula (I) may be made from a NHO-protected compound of formula (III), where P is a suitable O-protecting group, by suitable deprotection.
Suitable O-protecting groups can be found in the text by Greene and Wuts, supra, and include trialkylsilyl (such as trimethylsilyl), benzyl, etc. Compounds of formula (III) can be made in an analogous manner to the compounds of formula (I) from the compounds of formula (II), using a protected hydroxylamine PONH₂ or a suitable salt thereof in place of hydroxylamine itself or the hydroxylamine salt.
The deprotection method is determined by the protective group used, as is well known in the art (see e.g. Greene and Wuts, supra). E.g. benzyl groups may be hydrogenated, suitably using a catalytic transfer hydrogenation method. The reagents and conditions for reaction (III) -> (I) are typified in certain of the Examples below, such as where a protected hydroxylamine reagent (e.g. O-trimethylsilylhydroxylamine) can be used (e.g. Examples 1-6, etc.), where conveniently the deprotection is carried out in the same vessel as the previous step.

Other methods of making hydroxamic acids (I) are known and may be used, e.g. those mentioned in the text by J.March, supra, chapters 0-54, 0-57 and 6-4, and relevant references therein.

Acids of formula (IV) may be made by deprotection of the O-protected species of formula (V). Suitable O-protecting groups can be found in the chapter on O-protection in the book by Greene and Wuts, supra, and include C₁₋₄ alkoxy such as t-butoxy (as typified in Preparations 4, 7, 9, 11, 13, 15, etc.), benzyloxy, trialkylsilyloxy such as trimethylsilyloxy, etc..
The deprotection method is determined by the protective group used, as is well known in the art (see Greene and Wuts, supra). E.g. benzyl groups may be removed by hydrogenation, suitably using a catalytic transfer hydrogenation method, t-butyl groups may be removed by treatment with an acid in a suitable solvent, such as trifluoroacetic acid in dioxan, hydrochloric acid in toluene, etc.

Compounds of formula (V), e.g. where P is a t-butoxy can be made for example by condensation reaction of a corresponding compound of formula (VI), for example by heating to elevated temperature in an inert solvent such as in xylene at about 130°C, this reaction being typified by Preparation 3, 32, etc. below.

Compounds of formula (VI) can be made for example by coupling an acid of formula (VII) with a reagent of formula C(NH₂)(Y)=NOH, which is available via literature methods or adaptation thereof in a conventional manner (see e.g. Preparation 1), such as typified in methods described herein (e.g. see Preparation 2, etc.). Typically the condensation is carried out by adding a solution of the acid (VII) in a suitable inert solvent such as 1,4-dioxane or dichloromethane or the like, to a suitable agent such as 1-hydroxybenzotriazole hydrate, followed by addition of a suitable coupling agent such as a carbodiimide coupling agent, e.g. N,N'-dicyclohexylcarbodiimide, then treatment with the reagent C(NH₂)(Y)=NOH. Suitably the coupling is carried out at ambient temperature.

Compounds of formula (VII), and salts thereof which can generate the acid in situ on acidification, can be made by hydrogenation of the corresonding itaconate derivative, which in turn can be made by conventional methods such as the Stobbe condensation (see e.g. Preparation 168).
Compounds of formula (VII) where R is cyclohexyl can be made by catalytic hydrogenation of the corresponding compound of formula (I) where R is phenyl, using suitable catalysts and conditions, such as those mentioned in Preparation 168 (in part).

The compounds of formula (I), where W is CZ, can be prepared according to the chemistry outlined in the scheme below:

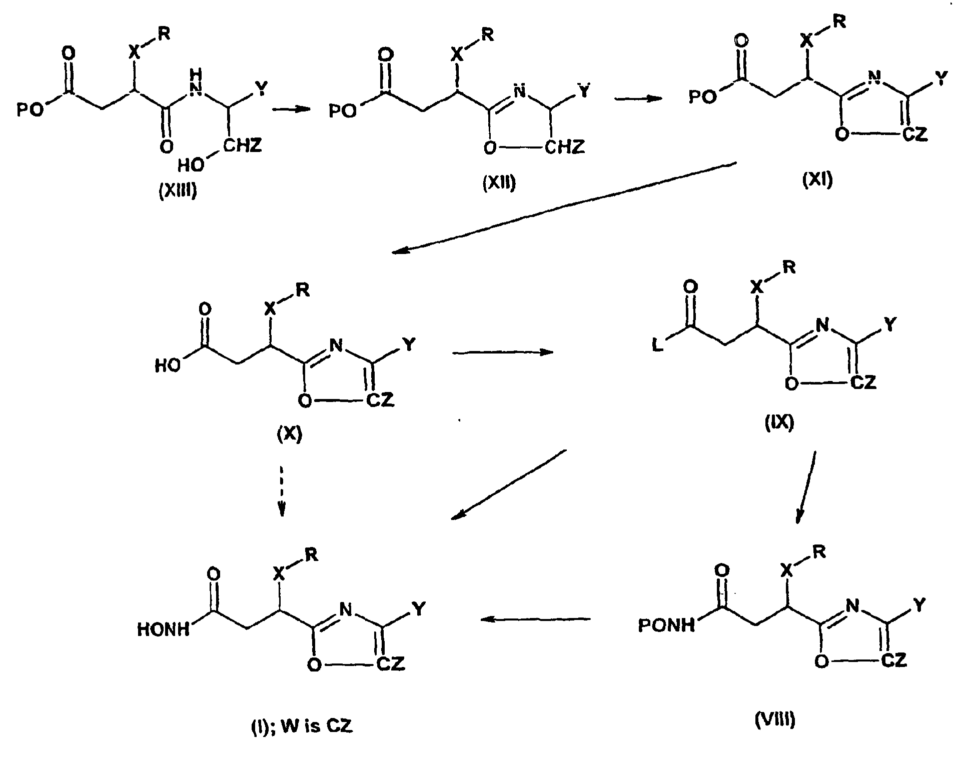

The hydroxamic acid compounds of formula (I) where W is CZ can be made by reaction of the corresponding activated acid derivative of formula (IX), where L⁻ is a suitable leaving group, with hydroxylamine. The leaving groups, reagents, etc. are the same as those mentioned above in relation to the corresponding compounds of formula (I) where W is N.

Such compounds of formula (IX) may be made via standard chemistry from the corresponding acid (X) using the same or similar chemistry to that outlined above in relation to the corresponding compounds of formula (II) where W is N (supra).

Alternatively the compounds of formula (I) may be made from a NHO-protected compound of formula (VIII), where P is a suitable O-protecting group, by suitable deprotection. Suitable O-protecting groups can be found in the text by Greene and Wuts, supra, and include trialkylsilyl (such as trimethylsilyl), benzyl, etc.

Compounds of formula (VIII) can be made in an analogous manner to the compounds of formula (II) from the compounds of formula (II), using a protected hydroxylamine PONH₂ or a suitable salt thereof in place of hydroxylamine itself or the hydroxylamine salt.
The deprotection method is determined by the protective group used, as is well known in the art. E.g. benzyl groups may be hydrogenated, suitably using a catalytic transfer hydrogenation method. The reagents and conditions for reaction (VIII) -> (I) are typified in Examples 68-72 below.

Other methods of making hydroxamic acids (I) are known and may be used, e.g. those mentioned in the text by J.March, supra, chapters 0-54, 0-57 and 6-4, and relevant references therein.

Acids of formula (X) may be made by deprotection of the O-protected species of formula (XI). Suitable O-protecting groups can be found in the chapter on O-protection in the book by Greene and Wuts, supra, and include C₁₋₄ alkoxy such as t-butoxy (as typified by the conditions mentioned in Preparation 4, etc.), benzyloxy, trialkylsilyloxy such as trimethylsilyloxy, etc..
The deprotection method is determined by the protective group used, as is well known in the art (see Greene and Wuts, supra). E.g. benzyl groups may be removed by hydrogenation, suitably using a catalytic transfer hydrogenation method, t-butyl groups may be removed by treatment with an acid such as trifluoroacetic acid, etc.

Compounds of formula (XI), e.g. where P is a t-butoxy group can be made for example by oxidation of a compound of formula (XII). Suitably the oxidation is carried out using copper (II) bromide with hexamethylenetetramine and a base such as DBU.

Compounds of formula (XII) may be made by condensation of compounds of formula (XIII), for example by treatment of the compound of formula (XIII) with s suitable agent such as Burgess Reagent, in an anhydrous solvent such as THF.

Compounds of formula (XIII) may be made by condensation of the acid of formula (II) above with an agent of formula NH₂CH(Y)CH(Z)OH. Compounds of formula NH₂CH(Y)CH(Z)OH are available commercially, from the literature or by routine modification thereof.

Alternatively, compounds of formula (XI) may be made by condensation of compounds of formula (XIV) (see for example, Preparation 173): 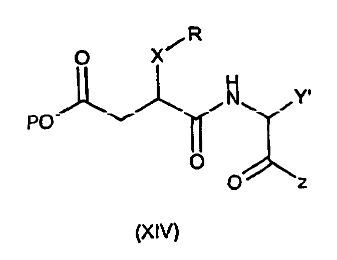 where Y' is a precursor moiety which can undergo functional group transformation to give Y as defined earlier to give compounds of formula (XI).

Certain other of the intermediates mentioned earlier (en route to compounds of formula (I) where W is N or CZ) can incorporate a Y' moiety instead of the Y moiety, which Y' moiety can undergo suitable functional group transformations at a suitable juncture at some point in the synthetic sequence to give intermediates or final compounds of formula (I) where the Y group is present as defined above.

Some specific routes to compounds of formula (I) (where X is propylene and R is cyclohexyl) are outlined in the following schemes, and with reference to certain Examples mentioned below, as further illustrations of the methods of preparation. As the skilled reader will recognise immediately, in these schemes the "Y" substituent in formula (I) above is designated in a different manner. The chemistry mentioned below can readily be extrapolated to give certain other compounds of formula (I) as defined above.

In the scheme below, W is N, and the Y substituent is represented as R:

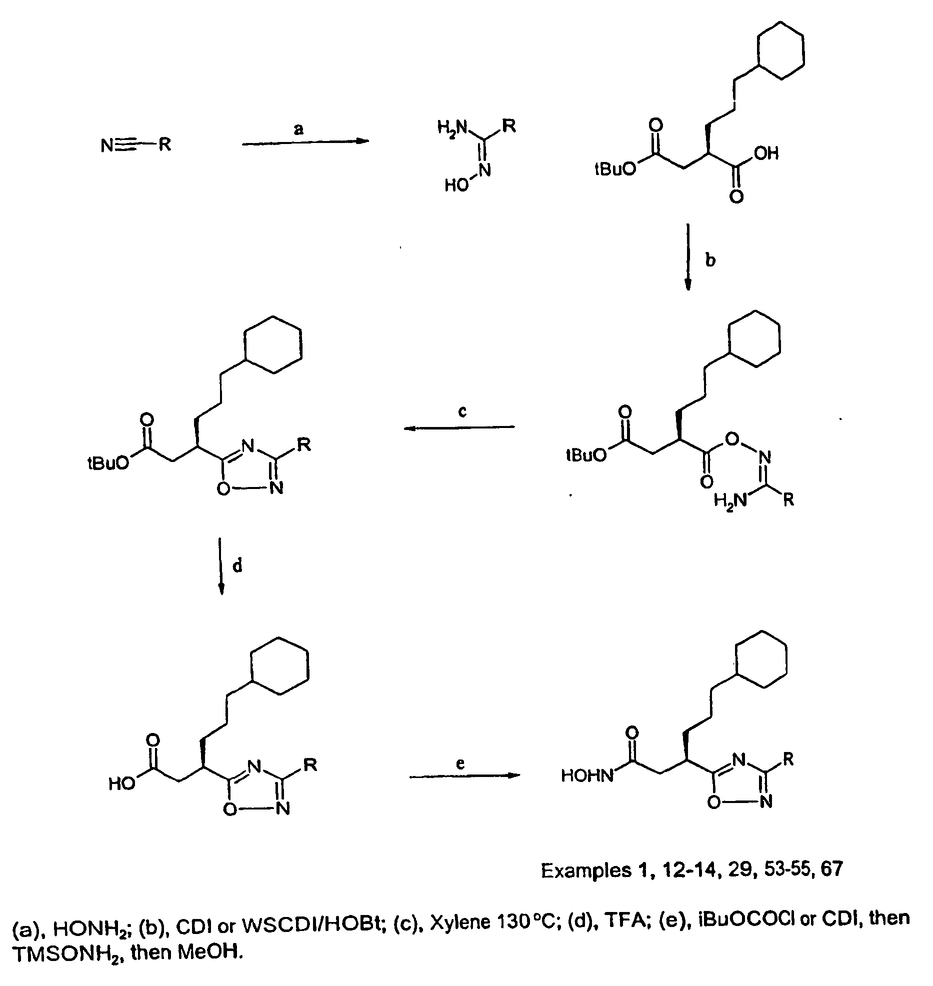

The scheme below illustraies synthesis of certain compounds where W is N and Y is an amine moiety (NB The functional group interconversions could equally be applied to the corresponding oxazoles (i.e. where W is CZ)):

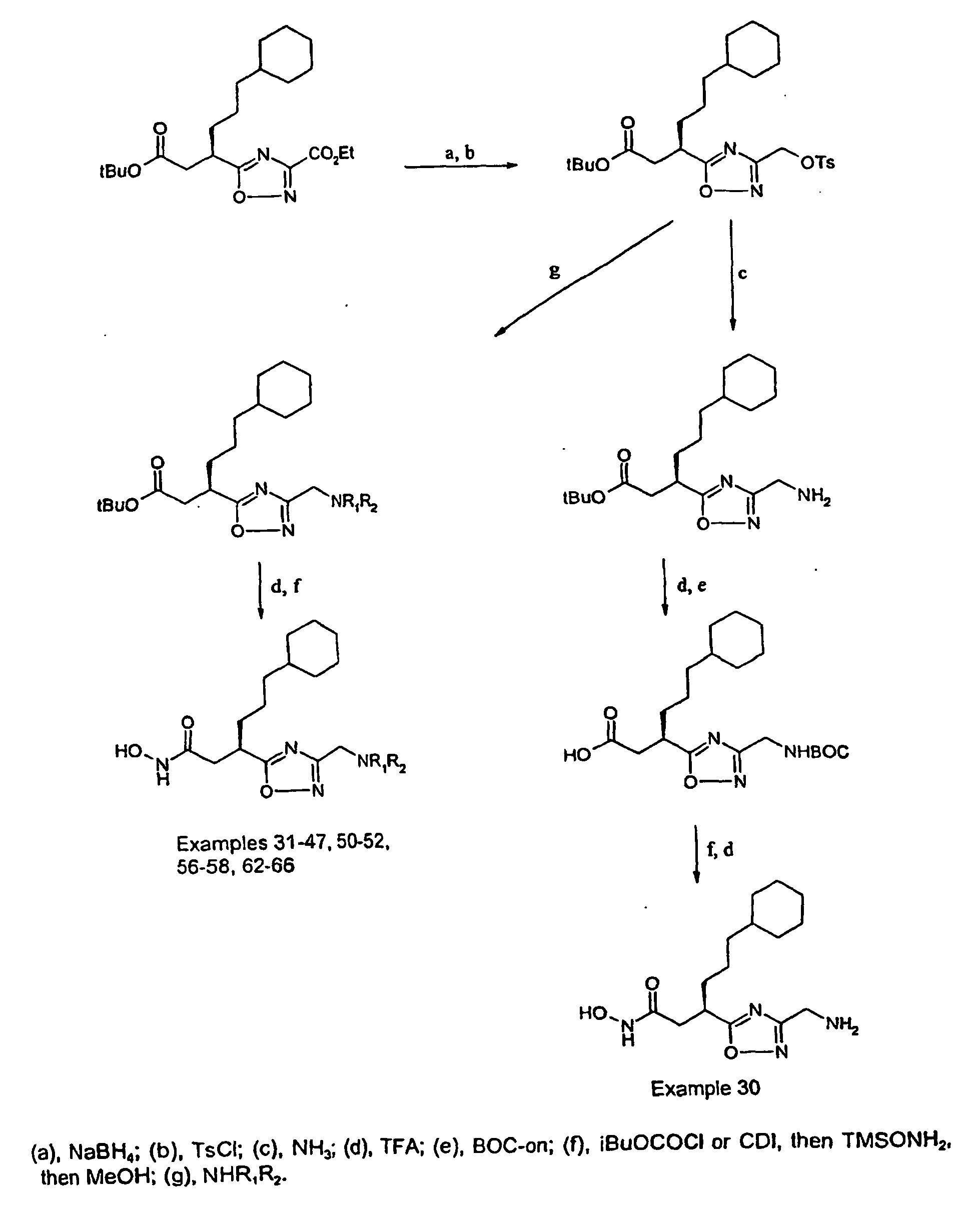

The scheme below represents a general route to compounds where W is N and Y incorporates a certain sulfonamide, amide, or urea moiety (NB again this chemistry will be equally applicable to the corresponding oxazole compounds, i.e. where W is CZ):

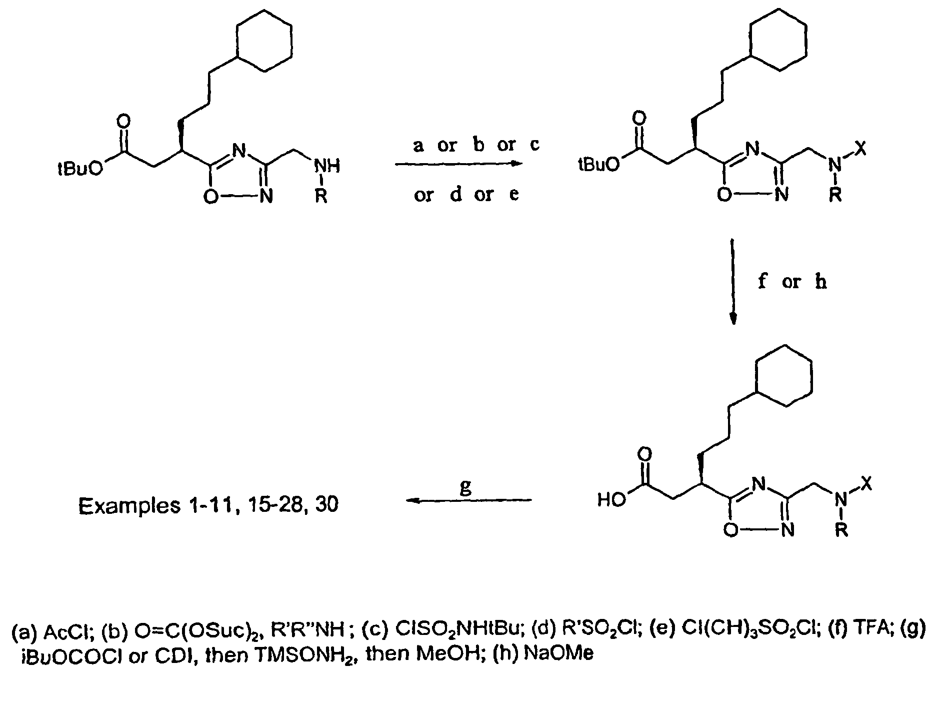

The scheme below represents a general route to compounds where W is N and Y incorporates certain amide moieties (NB again this chemistry will be equally applicable to the corresponding oxazole compounds, i.e. where W is CZ):

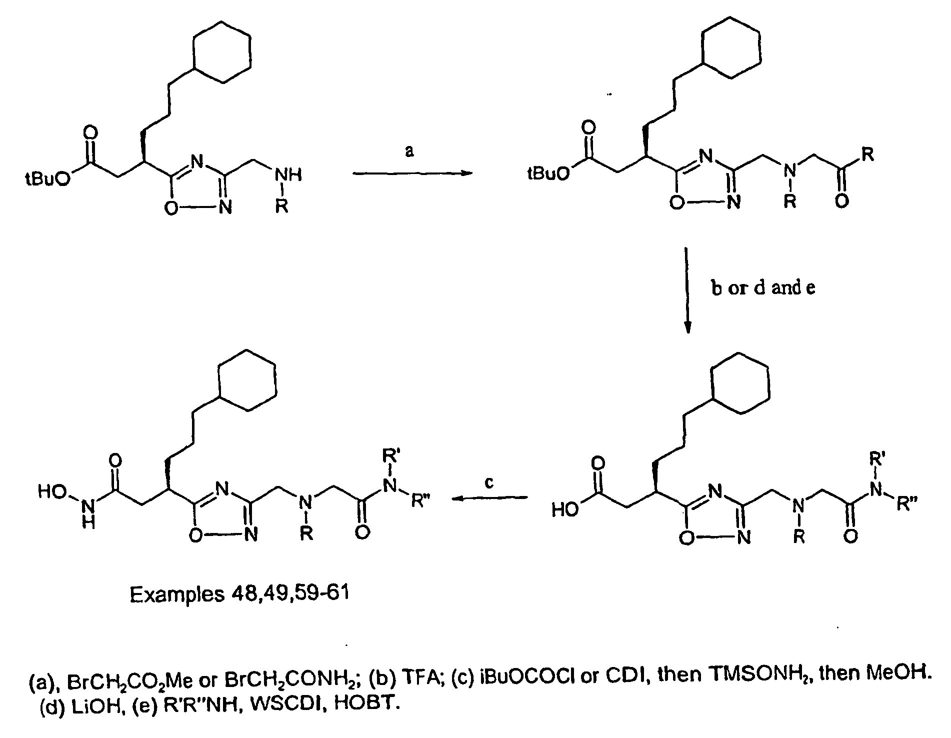

The scheme below represents a general route to compounds where W is CZ and Y incorporates an amine moiety (NB the latter stages of this chemistry will be equally applicable to the corresponding oxadiazole compounds, i.e. where W is N):

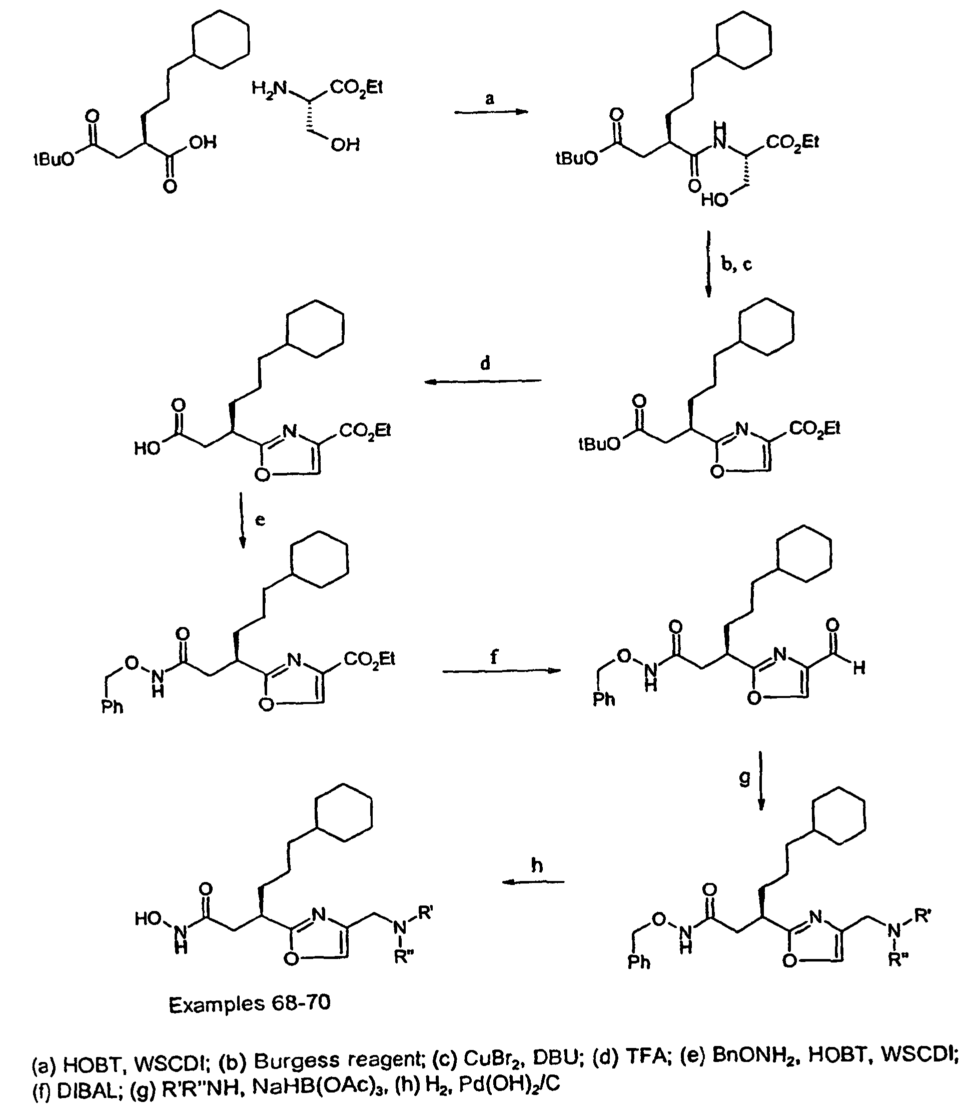

Similar types of chemistry were used used in the preparation of the compounds of Examples 71 and 72.

It will be apparent to those skilled in the art that other protection and subsequent deprotection regimes during synthesis of a compound of the invention may be achieved by conventional techniques, for example as described in the volumes by Greene and Wuts, and Kocienski, *supra*.

Where desired or necessary the compound of formula (I) is converted into a pharmaceutically acceptable salt thereof. A pharmaceutically acceptable salt of a compound of formula (I) may be conveniently be prepared by mixing together solutions of a compound of formula (I) and the desired acid or base, as appropriate. The salt may be precipitated from solution and collected by filtration, or may be collected by other means such as by evaporation of the solvent.

Certain compounds of the invention may be interconverted into certain other compounds of the invention by methods mentioned in the Examples and Preparations, and well-known methods from the literature.

Compounds of the invention are available by either the methods described herein in the Methods, Examples and Preparations or suitable adaptation thereof using methods known in the art. It is to be understood that the synthetic transformation methods mentioned herein may be carried out in various different sequences in order that the desired compounds can be efficiently assembled. The skilled chemist will exercise his judgement and skill as to the most efficient sequence of reactions for synthesis of a given target compound.

The compounds, salts and solvates of the invention may be separated and purified by conventional methods.

Separation of diastereomers may be achieved by conventional techniques, e.g. by fractional crystallisation, chromatography or H.P.L.C. of a stereoisomeric mixture of a compound of formula (I) or a suitable salt or derivative thereof. An individual enantiomer of a compound of formula (I) may also be prepared from a corresponding optically pure intermediate or by resolution, such as by H.P.L.C. of the corresponding racemate using a suitable chiral support or by fractional crystallisation of the diastereomeric salts formed by reaction of the corresponding racemate with a suitably optically active acid or base. In certain cases preferential crystallisation of one of the enantiomers can occur from a solution of a mixture of enantiomers, thus enriching the remaining solution in the other enantiomer.

For human use, the compounds of formula (I) or their salts can be administered alone, but will generally be administered in admixture with a pharmaceutically acceptable diluent or carrier selected with regard to the intended route of administration and standard pharmaceutical practice.

For example, they can be administered orally, including bucally and sublingually. The compounds or salts can be injected parenterally, for example, intravenously, intradermally intramuscularly or subcutaneously. They can be administered topically and/or transdermally. The compound or salt could also be administered intraocularly for ophthalmic use. For certain uses, vaginal, rectal and nasal (e.g. by inhalation of a dry powder or aerosol) administration would be suitable.

Formulations may be of immediate-, delayed-, modified-, sustained-, pulsed- or controlled-release type, and may be sterile or preserved or self-preserving.

Suitable formulations for oral administration would include tablets containing such excipients as starch or lactose, capsules or ovules either alone or in admixture with excipients, or in the form of elixirs, solutions or suspensions containing flavouring or colouring agents. The compound or salt could be incorporated into capsules or tablets for targetting the colon or duodenum via delayed dissolution of said capsules or tablets for a particular time following oral administration. Dissolution could be controlled by susceptibility of the formulation to bacteria found in the duodenum or colon, so that no substantial dissolution takes places before reaching the target area of the gastrointestinal tract.

The compounds or salts can be administered parenterally, by injection for example, intravenously, intradermally intramuscularly or subcutaneously. For parenteral administration, they are best used in the form of a sterile, preserved or self-preserving aqueous solution or suspension which may contain other substances, for example, enough salt or glucose to make the solution isotonic with blood. The compounds or salts may be used in combination with cyclodextrins. By injection we include standard injection techniques, and also needlefree injector or implant injection techniques.

The compounds or salts can be administered topically, and/or transdermally, in the form of sterile or preserved or self-preserving creams, gels, suspensions, lotions, solutions, sponges, fibres, microemulsions, films, ointments, dusting powders, sprays, foams, mousses, drug-incorporated dressings, skin patches, ointments such as petrolatum or white soft paraffin based ointments or via a skin patch or other device. They could be administered directly onto a wound. The compounds or salts may be delivered using iontophoresis, electroporation, phonophoresis and sonophoresis.They could be incorporated into a coated suture. For example they can be incorporated into a lotion or cream consisting of an aqueous or oily emulsion of mineral oils; sorbitan monostearate; polysorbate 60; cetyl esters wax; cetearyl alcohol; 2-octyldodecanol; benzyl alcohol; water; polyethylene glycols and/or liquid paraffin, or they can be incorporated into a suitable ointment consisting of one or more of the following - mineral oil; liquid petrolatum; white petrolatum; propylene glycol; polyoxyethylene polyoxypropylene compound; emulsifying wax and water, or as a gel such as a hydrogel with cellulose or polyacrylate derivatives or other viscosity modifiers, or as a dry powder or liquid spray or aerosol with butane/propane, HFA, CFC, CO₂ or other suitable propellant, optionally also including a lubricant such as sorbitan trioleate, or as a drug-incorporated dressing either as a tulle dressing, with white soft paraffin or polyethylene glycols impregnated gauze dressings or with a gel such as a hydrogel, hydrocolloid, alginate or film dressings. The compound can be dissolved or suspended in, for example, water, appropriate buffers, alcohols (e.g. ethanol, isopropyl alcohol, benzyl alcohol), glycols (e.g. propylene glycol, glycerol, polyethylene glycols), diethylene glycol ethers, poloxamers (polyoxyethylene polyoxypropylene compounds), isopropylmyristate, isopropylpalmitate, fixed oils (e.g. castor oil), mineral oil, silicone oils (e.g. dimeticone), other synthetic mono-/di-/triglycerides, fatty acids, liquid petrolatum, white petrolatum, liquid paraffin, emulsifying wax, sorbitan monostearate, polysorbates, cetostearyl alcohol, cetyl alcohol, 2-octyldodecanol or combinations thereof. Suitable viscosity modifiers, for example cellulose or polyacrylate derivatives can also be Incorporated.
Penetration enhancers may also be used.

The compound or salt could also be administered intraocularly for ophthalmic use e.g. in a lens implant, ointment, micronised suspension, absorbable gel sponges, implants, particulate or vesicular systems such as niosomes or liposomes, or as an eye drop with appropriate buffers, viscosity modifiers (e.g. cellulose or polyacrylate derivatives), preservatives (e.g. benzalkonium chloride (BZK)) and agents to adjust tonicity (e.g. sodium chloride), The compound may be used in combination with cyclodextrins. In addition, compounds may be delivered by lontophoresis. Such formulation techniques are well-known in the art.

For certain uses, vaginal, rectal and nasal (e.g. by inhalation of a dry powder or aerosol) administration would be suitable.

All such formulations may also contain auxiliaries such as appropriate stabilisers, preservatives such as antioxidants and chelating agents, flavours, colourings, cyclodextrins, etc. Such auxiliaries are well-known in the formulation art and the skilled person will be able to choose from such auxiliaries based on the properties of the compound or salt to be administered, the administration route, etc.

For oral and parenteral administration to human patients, the daily dosage level of the compounds of formula (I) or their salts will be from 0.001 to 20, preferably from 0.01 to 20, more preferably from 0.1 to 10, and most preferably from 0.5 to 5 mg/kg with respect to the active compound (in single or divided doses). Thus tablets or capsules of the compounds will contain from 0.1 to 500, preferably from 50 to 200, mg of active compound for administration singly or two or more at a time as appropriate.

For topical administration to human patients with acute/surgical wounds or scars, the daily dosage level of the compounds, in suspension or other formulation, could be from 0.01 to 50mg/ml, preferably from 0.3 to 30 mg/ml.

For treatment of a wound, the dosage will vary with the size of the wound, whether or not the wound is open or closed or partially closed, and whether or not the skin is intact.

The physician in any event will determine the actual dosage which will be most suitable for a an individual patient and it will vary with the condition to be treated, the age, weight and response of the particular patient, as well as the efficacy of the drug compound. The above dosages are exemplary of the average case; there can of course be individual instances where higher or lower dosage ranges are merited, and such are within the scope of this invention.

### Biological Test Methods

### PCP Inhibition

In order to determine potency of PCP inhibitors a fluorogenic PCP cleavage assay was used. This assay is based on the template of Beekman et al. (FEBS Letters (1996), 390: 221-225) using a fluorogenic substrate. The substrate (Dabcyl-Arg-Tyr-Tyr-Arg-Ala-Asp-Asp-Ala-Asn-Val-Glu(EDANS)-NH₂) contains the cleavage site of human PCP (Hojima et al., J Biol Chem (1985), 260: 15996-16003). Human PCP has been purified from supematant of stable transfected CHO cells using hydrophobic interaction column followed by Superdex 200 gel filtration. 4 µg total protein of this enzyme preparation was incubated with various concentrations of the substance to be tested and 3x 10⁻⁶ M substrate in assay buffer (50 mM Tris-Base, pH 7.6 containing 150 mM NaCl, 5 mM CaCl₂, 1µM ZnCl₂ and 0.01 % Brij 35). The assay was performed in 96-well black fluorimeter plates and fluorescence was read continuously in a fluorimeter over 2.5 hours (λₑₓ = 340 nm, λₑₘ = 485 nm) at a constant 37°C with shaking. Release of the fluorogenic signal was in linear correlation to PCP activity. Reading of the mean velocity from 30 min after start of experiment until 2.5 hours was calculated by the Biolise software. IC₅₀ values were calculated by plotting % inhibition values against compound concentration using Tessela add in for Excel spreadsheet. Example compounds were tested and were found to have IC₅₀ values vs. PCP of 1µM or less.

### MMP Inhibition

The ability of compounds to inhibit the cleavage of fluorogenic peptides by MMPs 1, 2, 9, and 14 is described below.
The assays for MMPs 2, 9, and 14 are based upon the original protocol described by Knight et al. (Fed.Euro.Biochem.Soc., 296 (3), 263-266; 1992) with the slight modifications given below.

### Inhibition of MMP-1

### (i) Enzyme Preparation

Catalytic domain MMP-1 was prepared at Pfizer Central Research. A stock solution of MMP-1 (1 µM) was activated by the addition of aminophenylmercuric acetate (APMA), at a final concentration of 1mM, for 20 minutes at 37°C. MMP-1 was then diluted in Tris-HCl assay buffer (50mM Tris, 200mM NaCl, 5mM CaCl₂, 20µM ZnSO₄, 0.05% Brij 35) pH 7.5 to a concentration of 10nM. The final concentration of enzyme used in the assay was 1nM.

### (ii) Substrate

The fluorogenic substrate used in this assay was Onp-Pro- -cyclohexyl-Ala-Gly-Cys(Me)-His-Ala-Lys(N-Me-AJa)-NH₂ as originally described by Bickett et al (Anal. Biochem, 212, 58-64, 1993). The final substrate concentration used In the assay was 10µM.

### (iii) Determination of Enzyme Inhibition

Test compounds were dissolved In dimethyl sulphoxide and diluted with assay buffer so that no more than 1% dimethyl sulphoxide was present. Test compound and enzyme were added to each well of a 96 well plate and allowed to equilibrate for 15 minutes at 37°C in an orbital shaker prior to the addition of substrate. Plates were then incubated for 1 hour at 37°C prior to determination of fluorescence (substrate cleavage) using a fluorimeter (Fluostar; BMG LabTechnologies, Aylesbury, UK) at an excitation wavelength of 355nm and emission wavelength of 440nm. The potency of inhibitors was measured from the amount of substrate cleavage obtained using a range of test compound concentrations, and, from the resulting dose-response curve, an IC₅₀ value (the concentration of inhibitor required to inhibit 50% of the enzyme activity) was calculated.

### Inhibition of MMP-2 and MMP-9

### (i) Enzyme Preparation

Catalytic domain MMP-2 and MMP-9 were prepared at Pfizer Central Research. A stock solution of MMP-2 / MMP-9 (1 M) was activated by the addition of aminophenylmercuric acetate (APMA). For MMP-2 and MMP-9, a final concentration of 1mM APMA was added, followed by incubation for 1 hour at 37°C. The enzymes were then diluted in Tris-HCl assay buffer (100mM Tris, 100mM NaCl, 10mM CaCl₂ and 0.16% Brij 35, pH 7.5), to a concentration of 10nM. The final concentration of enzyme used in the assays was 1nM.

### (ii) Substrate

The fluorogenic substrate used in this screen was Mca-Arg-Pro-Lys-Pro-Tyr-Ala-Nva-Trp-Met-Lys(Dnp)-NH₂ (Bachem Ltd, Essex, UK) as originally described by Nagase et al (J.Biol.Chem., 269(33), 20952-20957, 1994). This substrate was selected because it has a balanced hydrolysis rate against MMPs 2 and 9 (k_{cal} / kₘ of 54,000, 59,400 and 55,300 s⁻¹ M⁻¹ respectively). The final substrate concentration used in the assay was 5µM.

### (iii) Determination of Enzyme Inhibition

Test compounds were dissolved in dimethyl sulphoxide and diluted with test buffer solution (as above) so that no more than 1% dimethyl sulphoxide was present. Test compound and enzyme were added to each well of a 96 well plate and allowed to equilibrate for 15 minutes at 37°C in an orbital shaker prior to the addition of substrate. Plates were then incubated for 1 hour at 37°C prior to determination of fluorescence using a fluorimeter (Fluostar; BMG LabTechnologies, Aylesbury, UK) at an excitation wavelength of 328nm and emission wavelength of 393nm. The potency of inhibitors was measured from the amount of substrate cleavage obtained using a range of test compound concentrations, and, from the resulting dose-response curve, an IC₅₀ value (the concentration of inhibitor required to inhibit 50% of the enzyme activity) was calculated.

### Inhibition of MMP-14

### (i) Enzyme Preparation

Catalytic domain MMP-14 was purchased from Prof. Tschesche, Department of Biochemistry, Faculty of Chemistry, University of Bielefeld, Germany. A 10 M enzyme stock solution was activated for 20 minutes at 25°C following the addition of 5 g/ml of trypsin (Sigma, Dorset, UK). The trypsin activity was then neutralised by the addition of 50 g/ml of soyabean trypsin inhibitor (Sigma, Dorset, UK), prior to dilution of this enzyme stock solution in Tris-HCl assay buffer (100mM Tris, 100mM NaCl, 10mM CaCl₂ and 0.16% Brij 35, pH 7.5) to a concentration of 10nM. The final concentration of enzyme used in the assay was 1 nM.

### (ii) Substrate

The fluorogenic substrate used in this screen was Mca-Pro-Leu-Gly-Leu-Dpa-Ala-Arg-NH₂ (Bachem Ltd, Essex, UK) as described by Will et al (J.Biol.Chem., 271(29), 17119-17123, 1996). The final substrate concentration used in the assay was 10µM.

Determination of enzyme inhibition by test compounds was performed in the same manner as described for MMPs-2 and -9 above.

All the compounds of the Examples had IC₅₀ values vs PCP of 0.5µM or less.
The compounds of Examples 6, 17, and 72 had IC₅₀ values vs PCP of 107nM, 98nM and 37nM respectively.

The compounds of the invention are illustrated by the Examples below.

### EXAMPLES AND PREPARATIONS

Melting points were determined using open glass capillary tubes and a Gallenkamp melting point apparatus and are uncorrected. Nuclear magnetic resonance (NMR) data were obtained using Varian Unity Inova-400, Varian Unity Inova-300 or Bruker AC300 spectrometers and are quoted in parts per million from tetramethylsilane. Mass spectral (MS) data were obtained on a Finnigan Mat. TSQ 7000 or a Fisons Instruments Trio 1000. The calculated and observed ions quoted refer to the isotopic composition of lowest mass. Infra red (IR) spectra were measured using a Nicolet Magna 550 Fourier transform infra-red spectrometer. Flash chromatography refers to column chromatography on silica gel (Kieselgel 60, 230-400 mesh, from E. Merck, Darmstadt. Kleselgel 60 F₂₅₄ plates from E. Merck were used for TLC, and compounds were visualised using UV light, 5% aqueous potassium permanagate or Dragendorff's reagent (oversprayed with aqueous sodium nitrite). Thermal analyses by Differential Scanning Calorimetry (DSC) and ThermoGravimetric Analysis (TGA) were obtained using Perkin Elmer DSC7 and TGA7.

Moisture sorption characteristics were recorded using Surface Measurement Systems Ltd. Automated Water Sorption Analyser DVS 1. Water content was determined on a Mitsubishi CA100 (Coulometric Karl Fisher Titrator). Powder X-ray diffraction (PXRD) pattern was determined using a Siemens D5000 powder X-ray diffractometer fitted with an automatic sample changer, a theta-theta goniometer, automatic beam divergence slits, a secondary monochromator and a scintillation counter. Other measurements were taken using standard equipment. Hexane refers to a mixture of hexanes (hplc grade) b.p. 65-70°C. "Ether" and "Et₂O" refers to diethyl ether. Acetic acid refers to glacial acetic acid. 1-Hydroxy-7-aza-1H-1,2,3-benzotriazole (HOAt). "HOBt" is 1-hydroxy-1H-1,2,3-benzotriazole. N-[(dimethylamino)-1H-1,2,3-triazolo[4,5-b]pyridin-1-ylmethylene]-N-methylmethaninium hexafluorophosphate N-oxide (HATU) and 7-azabenzotriazol-1-yloxytris(pyrrolidino)phosphonium hexafluorophosphate (PyAOP) were purchased from PerSeptive Biosystems U.K. Ltd. "DIPE" refers to diisopropyl ether. Reverse-phase silica gel for flash chromatography was obtained from Fluka (Fluka 100, C₁₈, 40-63µ). "DCM" is dichloromethane. "THF" is tetrahydrofuran. "WSCDI" is 1-(3-dimethylaminopropyl)-3-ethylcarbodiimide hydrochloride. "CDI" is carbonyldiimidazole. "EtOAc" is ethyl acetate. "MeOH" is methanol. "DMSO" is dimethylsulphoxide. "ACE-Cl" is 1-chloroethyl chloroformate. "NMM" is N-methylmorpholine. "Pentane" refers to High Performance Liquid Chromatography (HPLC) grade n-pentane (b.pt.35-37°C). Nomenclature has been allocated using the commercially available ACD program. Standard abbreviations are used throughout, e.g. "Me" is methyl, "Et" is ethyl, "Pr" is propyl, "Ph" is phenyl, etc.
^{a}HPLC autopurification performed using 2 columns - Phenomonex LUNA C8 150x21.2mm, 10µm and Phenomonex MAGELLEN C18 150x21.2mm, 5µm, eluting with a gradient system of organic solvent [ammonium acetate (aq) 100mM : acetonitrile (1 : 9)] : aqueous solvent [ammonium acetate (aq) 100mM : acetonitrile (9 :1)]
^{b} HPLC autopurification performed using 2 columns - Phenomonex LUNA C8 150x21.2mm, 10µm and Phenomonex MAGELLEN C18 150x21.2mm, 5µm, eluting with a gradient system of organic solvent (acetonitrile) : aqueous solvent (0.1 % aqueous trifluoroacetic acid)

### Example 1:

### (3R)-6-cyclohexyl-N-hydroxy-3-(3-{[(methylsulfonyl)amino]methyl}-1,2,4-oxadiazol-5-yl)hexanamide

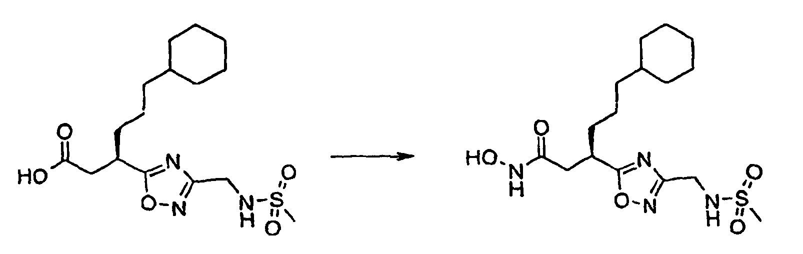

METHOD A. A solution of (3*R*)-6-cyclohexyl-3-(3-{[(methylsulfonyl)amino]methyl}-1,2,4-oxadiazol-5-yl)hexanoic acid (preparation 4) (6.80g, 18.2mmol) and 2,6-lutidine (2.3ml, 20.0mmol) in THF (100ml) at 0°C, under a nitrogen atmosphere was treated with *i*-butyl chloroformate (2.6ml, 20.0mmol) and stirred at 0°C for 1.5hours. *o*-(trimethylsilyl)hydroxylamine (4.9ml, 40.0mmol) was added and the reaction mixture was stirred for 18 hours warming to room temperature over this time. MeOH was added and the reaction mixture stirred for a further 2 hours. The solvent was removed under reduced pressure. The yellow solid was dissolved in EtOAc (400ml), washed with dilute HCl (150ml 2M HCl + 200ml H₂O) and brine, dried over MgSO₄ and filtered. The solvent was remove under reduced pressure. The solid was recrystallised from hot EtOAc to afford the title compound as a white solid (4.74g, 67%).

METHOD B. COI (7.6g, 47mmol) was added portionwise over 2 min to a solution of (3*R*)-6-cyclohexyl-3-(3-{[(methylsulfonyl)amino]methyl}-1,2,4-oxadiazol-5-yl)hexanoic acid (preparation 4) (14.0g, 42mmol) in THF (160ml), at ambient temperature, under a nitrogen. The mixture was stirred at ambient temperature for 30 min and then cooled to 0°C. Aqueous hydroxylamine (50%, 13.1ml, 0.21 mol) was added between 0 and 5°C and the reaction mixture was stirred for 18 hours warming to room temperature over this time. Aqueous citric acid solution (10%, 160ml) was added and the mixture was extracted with ethyl acetate (2x160ml). The combined organic fractions were washed with demineralised water (160ml) and then concentrated *in vacuo* to a colourless solid. Ethyl acetate (64ml) was added and the mixture was stirred at ambient temeprature for 1 hour and then filtered. The residue was dried *in vacuo* at 45°C to afford the title compound as an off-white solid (14.2g, 85%).
Mpt : 115-116°C
¹H nmr : (d₆DMSO) 0.80 (2H, m), 1.00-1.15 (8H, m), 1.50-1.70 (8H, m), 2.50-2.55 (2H, obs), 2.89 (3H, s), 3.41 (1H, m), 4.24 (2H, d), 7.63 (1H, brs), 8.62 (1H, brs), 10.38 (1H, brs)
MS : 411 (MNa⁺)
CHN : Found: C49.42%; H7.40%; N14.48%; C₁₆H₂₈N₄O₅S requires C49.47%; H7.26%; N14.42%

METHOD C (Alternative Synthesis): Ethyl chloroformate (0.11 ml, 1.2mmol) was added to a stirred solution of (3*R*)-6-cyclohexyl-3-(3-{[(methylsulfonyl)amino]methyl}-1,2,4-oxadiazol-5-yl)hexanoic acid (preparation 4) (0.40g, 1.1mmol) in THF (10m1) and ether (10ml) at 0 °C under a nitrogen atmosphere. The reaction was stirred at 0 °C for 40 minutes then aqueous hydroxylamine (80 µl of 50% solution by weight, 1.2 mmol) was added. The reaction was left to warm to room temperature overnight. The solvents were removed under reduced pressure, then the residue was dissolved in EtOAc (20ml) and washed with water (20ml) and brine (20 ml). The organic layer was dried (MgSO₄) and filtered. The solvent was removed under reduced pressure. The residue was triturated with pentane, the solid was collected by filtration to give a white solid (410 mg, 98%). HPLC analysis indicated 95.5% was the title compound, 2% starting material and 2.5% other unidentified products.

### Example 2:

### (3R)-6-cyclohexyl-N-hydroxy-3-(3-{[methyl(methylsulfonyl)amino]methyl}-1,2,4-oxadiazol-5-yl)hexanamide

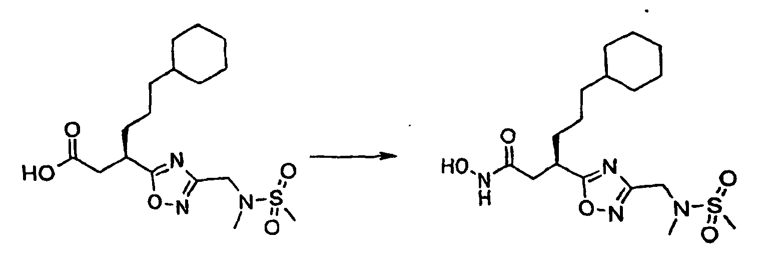

Method is the same as for Example 1 using (3*R*)-6-cyclohexyl-3-(3-{[methyl(methylsulfonyl)amino]methyl}-1,2,4-oxadiazol-5-yl)hexanoic acid (preparaton 7) (225mg, 0.58mmol) as starting material.
Purification: The crude material was purified on a silica column eluting with a solvent gradient of DCM : MeOH (100 : 0) gradually changing to (97 : 3) to yield the title compound as sticky colourless gum (180mg, 77%)
¹H nmr: (d₆DMSO) 0.80 (2H, m), 1.05-1.30 (8H, m), 1.50-1.70 (7H, m), 2.52 (2H, obs), 2.80 (3H, s), 2.93 (3H, s), 3.42 (1H, m), 4.44 (1H, m), 8.62 (1H, brs), 10.38 (1H, brs).
MS : 425 (MNa⁺)
CHN: Found: C50.87%; H7.69%; N13.47%; C₁₇H₃₀N₄O₅S. 0.1H₂O requires C50.50%; H7.53%; N13.86%

### Example 3:

### (3R)-6-cyclohexyl-3-(3-{[(ethylsulfonyl)amino]methyl}-1,2,4-oxadiazol-5-yl)-N-hydroxyhexanamide

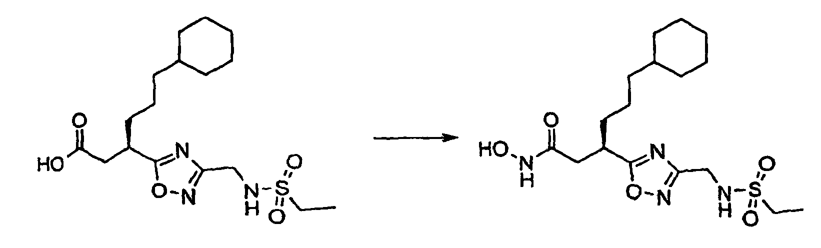

Method same is for Example 1 using (3*R*)-6-cyclohexyl-3-(3-{[(ethylsulfonyl)amino]methyl}-1,2,4-oxadiazol-5-yl)hexanoic acid (preparation 9) (143mg, 0.37mmol) as starting material.
Purification: Crude material was purified on a silica column eluting with a solvent gradient of DCM : MeOH (100 : 0) gradually changing to (95: 5) to afford the title compound as a sticky yellow gum (103mg, 69%).
¹H nmr: (d₆DMSO) 0.80 (2H, m), 1.00-1.25 (11H, m), 1.55-1.70 (7H, m), 2.52 (2H, obs), 3.00 (2H, q), 3.41 (1H, m), 4.24 (2H, d), 7.67 (1H, brs), 10.38 (1H, brs).
MS: 403 (MH⁺)
Accurate mass : Found 425.1828 (MNa⁺), Calculated C₁₇H₃₀N₄O₆S 425.1829 (MNa⁺)

### Example 4:

### (3R)-6-cyclohexyl-N-hydroxy-3-(3-{[(isopropylsulfonyl)amino]methyl}-1,2,4-oxadiazol-5-yl)hexanamide

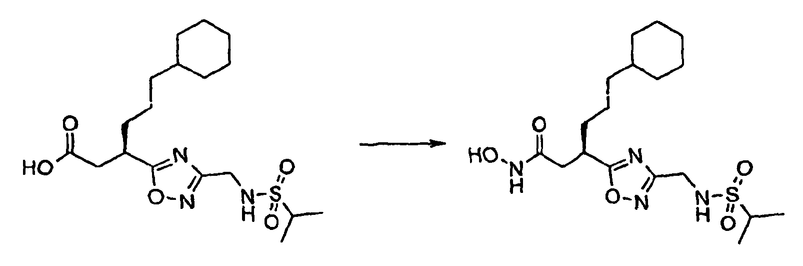

Method same as for example 1 using (3*R*)-6-cyclohexyl-3-(3-{[(isopropylsulfonyl)amino]methyl}-1,2,4-oxadiazol-5-yl)hexanoic acid (prepapration 11) (88mg, 0.22mmol) as starting material. Purification: The crude material was purified on a silica column eluting with a solvent gradient of DCM : MeOH (100 : 0) gradually changing to (95 : 5) to afford the title compound as an orange gum (76mg, 83%).
¹H nmr : (CD₃OD) 0.80-0.90 (2H, m), 1.10-1.30 (14H, m+d). 1.60-1.80 (7H, m), 2.50 (1H, dd), 2.60 (1H, dd), 3.20 (1H, m), 3.50 (1H, m), 4.35 (2H, s).
MS : 439 (MNa⁺)
CHN : Found: C50.61%; H7.72%; N12.83%: C₁₈H₃₂N₄O₅S. 0.3 H₂O. 0.1 DCM requires C50.51%; H7.68%; N13.02%

### Example 5:

### (3R)-6-cyclohexyl-N-hydroxy-3-(3-{[(phenylsulfonyl)amino]methyl}-1,2,4-oxadiazol-5-yl)hexanamide

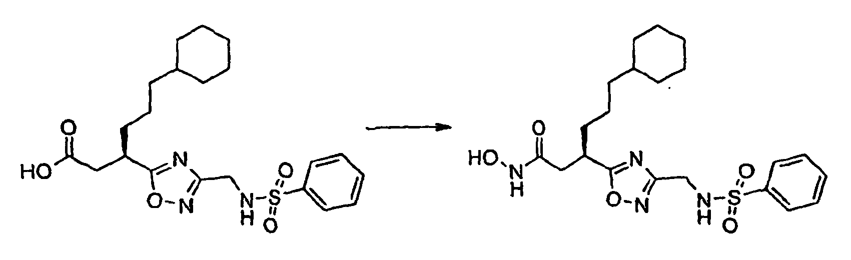

Method same as for example 1 using (3*R*)-6-cyclohexyl-3-(3-{[(phenylsulfonyl)amino]methyl}-1,2,4-oxadiazol-5-yl)hexanoic acid (preparation 13) (149mg, 0.34mmol) as starting material.

Purification: The crude material was purified on a silica column eluting with a solvent gradient of DCM : MeOH (100 : 0) gradually changing to (95 : 5) to afford the title compound as a colourless gum (116mg, 75%).
¹H nmr : (d₆DMSO) 0.80 (2H, m), 1.00-1.20 (8H, m), 1.50-1.70 (7H, m), 2.38 (1H, dd), 2.52 (1H, obs), 3.37 (1H, m), 4.11 (2H, s), 7.48-7.62 (3H, m). 7.78 (2H, d), 8.25 (1H, brs), 8.62 (1H, brs), 10.38 (1H, brs).
MS : 473 (MNa⁺)
Accurate mass: Found 451.2004 (MH⁺), Calculated C₂₁H₃₀N₄O₆S 451.2010 (MH⁺)

### Example 6:

### (3R)-6-cyclohexyl-N-hydroxy-3-(3-{[(2-pyridinylsulfonyl)amino]methyl}-1,2,4-oxadiazol-5-yl)hexanamide

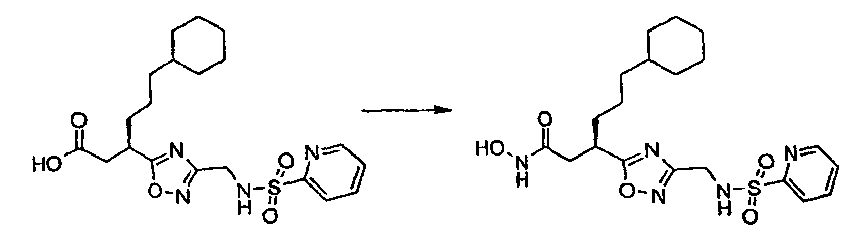

Method same as for example 1 except using NMM as the base and (3R)-6-cyclohexyl-3-(3-{[(2-pyridinylsulfonyl)amino]methyl}-1,2,4-oxadiazol-5-yl)hexanoic acid (preparation 15) (231mg, 0.53mmol) as the starting material.
Purification: Crude material was purified on a silica column eluting with Et₂O : MeOH (19 : 1) to afford the title compound as a white foam (36mg).
¹H nmr : (d₆DMSO) 0.80 (2H, m), 1.05-1.25 (8H, m), 1.50-1.70 (7H, m), 2.38 (2H, m), 3.37 (1H, m), 4.28 (2H, d), 7.60 (1H, m), 7.88 (1H, d), 8.03 (1H, t), 8.44 (1H, brs), 8.62 (2H, brd), 10.38 (1H, brs).
MS : 474 (MNa⁺)

### Example 7:

### (3R)-6-cyclohexyl-N-hydroxy-3-(3-{[(3-pyridinylsulfonyl)amino]methyl}-1,2,4-oxadiazol-5-yl)hexanamide

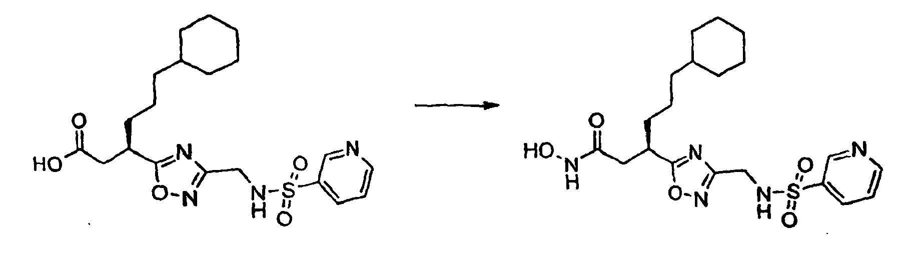

A solution of (3*R*)-6-cyclohexyl-3-(3-{[(3-pyridinylsulfonyl)amino]methyl}-1,2,4-oxadiazol-5-yl)hexanoic acid (preparation 17) (167mg, 0.33mmol) DCM (6ml) under a nitrogen atmosphere was treated with CDI (80mg, 0.49mmol) and stirred at room temperature for 1 hour. *o-*(trimethylsilyl)hydroxylamine (121µl, 0.99mmol) was added and the reaction mixture was stirred for 18 hours. MeOH (3ml) was added and the reaction mixture stirred for a further 2 hours. The solvent was removed under reduced pressure. The residue was dissolved in EtOAc, washed with H₂O and brine, dried over MgSO₄ and filtered. The solvent was removed under reduced pressure. The crude material was purified on a silica column eluting with a solvent gradient of DCM : MeOH (100 : 0) gradually changing to (95 : 5) to afford the title compound as a white solid (100mg, 67%).
¹H nmr: (d₆DMSO) 0.80 (2H, m), 1.00-1.30 (8H, m), 1.50-1.70 (7H, m), 2.38 (2H, brm), 3.35 (1H, m), 4.21 (2H, s), 7.59 (1H, m), 8.11 (1H, d), 8.63 (1H, brs), 8.77 (1H, d), 8.90 (1H, s).
MS : 450 (M-H)
Accurate mass : Found 452.1947 (MH⁺), Calculated C₂₀H₂₉N₅O₅S 452.1962

### Example 8:

### (3R)-6-cyclohexyl-N-hydroxy-3-[3-({[(1-methyl-1H-imidazol-4-yl)sulfonyl]amino}methyl)-1,2,4-oxadiazol-5-yl]hexanamide

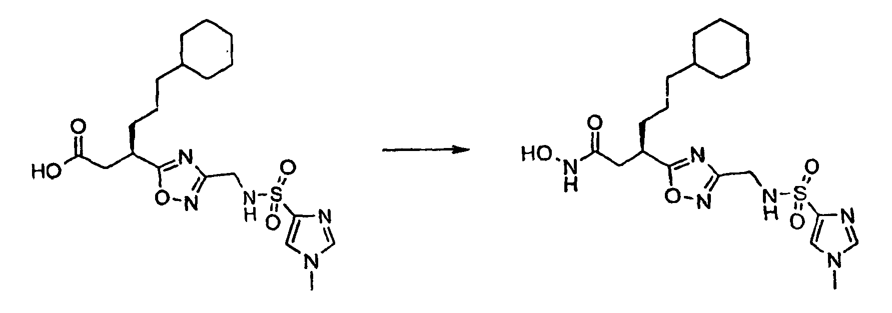

Method same as for example 7 using (3*R*)-6-cyclohexyl-3-[3-({[(1-methyl-1*H*-imidazol-4-yl)sulfonyl]amino}methyl)-1,2,4-oxadiazol-5-yl]hexanoic acid (preparation 20) (205mg, 0.43mmol) as starting material.
Purification: Crude material purified on a silca column eluting with a solvent gradient of DCM : MeOH : NH₃ (98 : 2 : 0) gradually changing to (90 : 10 : 1) to afford the title compound as a white solid (105mg, 54%)
¹H nmr : (d₆DMSO) 0.80 (2H, m), 1.05-1.25 (8H, m), 1.50-1.70 (7H, m), 2.41 (2H, m), 3.40 (1H, m), 3.70 (3H, s), 4.14 (2H, d), 7.66 (1H, s), 7.71 (1H, s), 7.98 (1H, brs), 8.66 (1H, brs), 10.39 (1H, brs).
MS : 477 (MNa⁺)
CHN : Found: C49.86%; H6.67%; N18.28%; C₁₈H₃₀N₆O₅S. 0.1 H₂O requires C50.01%; H6.67%; N18.42%

### Example 9:

### (3R)-6-cyclohexyl-N-hydroxy-3-(3-{[(1H-pyrazol-4-ylsulfonyl)amino]methyl}-1,2,4-oxadiazol-5-yl)hexanamide

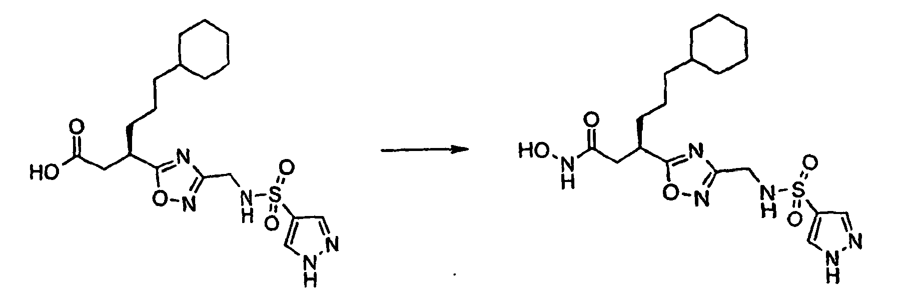

Method same as for example 7 using (3*R*)-6-cyclohexyl-3-(3-{[(1*H*-pyrazol-4-ylsulfonyl)amino]methyl}-1,2,4-oxadiazol-5-yl)hexanoic acid (preparation 23) (122mg, 0.29mmol) as starting material.
Purification: The cude material was purified on 2 silica columns both eluting with a solvent gradient of DCM : MeOH (95 : 5) gradually changing to (90 : 10) to afford the title compound as a white sold (64mg, 50%).
M.pt. 124-126°C dcc (formed a glass at 60-62°C)
¹H nmr : (CD₃OD) 0.85 (2H, m), 1.10-1.30 (8H, m), 1.55-1.75 (7H, m), 2.40-2.60 (2H, m), 3.47 (1 H, m), 4.22 (2H, s), 7.92 (2H, brs).
MS : 463 (MNa⁺)
CHN : Found: C47.87%; H6.58%; N18.53%: C₁₈H₂₈N₆O₅S. 0.6 H₂O requires C47.90%; H6.52%; N18.62%

### Example 10:

### (3R)-6-cyclohexyl-N-hydroxy-3-(3-{[(4H-1,2,4-triazol-3-ylsulfonyl)amino]methyl}-1,2,4-oxadiazol-5-yl)hexanamide

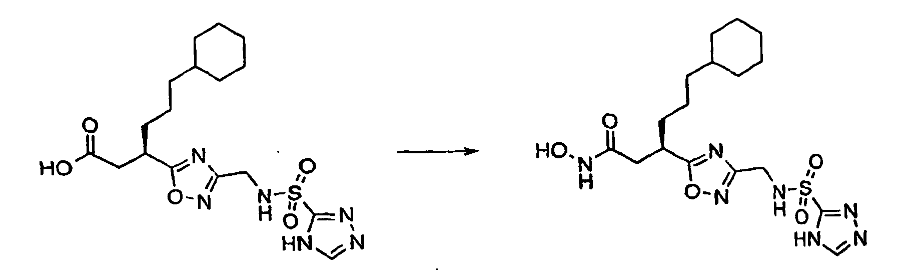

Method as for example 7 using (3*R*)-6-cyclohexyl-3-(3-{[(4*H*-1,2,4-triazol-3-ylsulfonyl)amino]methyl}-1,2,4-oxadiazol-5-yl)hexanoic acid (preparation 26) (200mg, 0.47mmol) as starting material.
Purification: Crude material was purified on a silica column eluting with EtOAc and changing to a solvent gradient of DCM : MeOH (95 : 5) gradually changing to (90 : 10) and finally neat MeOH was used to afford the title compound as a light brown solid (50mg, 24%).
¹H nmr: (CD₃OD) 0.83 (2H, m),1.05-1.30 (8H, m), 1.55-1.75 (7H, m), 2.40-2.65 (2H, m), 3.49 (1H, m), 4.39 (2H, s), 8.28 (1H, s).
MS : 440 (M-H)

### Example 11:

### (3R)-6-cyclohexyl-3-[3-({[(3,5-dimethyl-4-isoxazolyl)sulfonyl]amino}methyl)-1,2,4-oxadiazol-5-yl]-N-hydroxyhexanamide

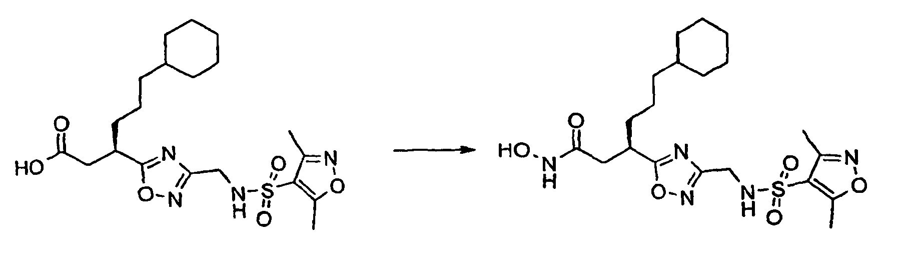

Method as for Example 1 using (3*R*)-6-cyclohexyl-3-[3-({[(3,5-dimethyl-4-isoxazolyl)sulfonyl]amino}methyl)-1,2,4-oxadiazol-5-yl]hexanoic acid (preparation 28) (220mg, 0.48mmol) as starting material.
Purification: The crude material was purified on a silica column eluting with DCM : MeOH (95 : 5) to afford the title compound as a white foam (92mg, 41%).
¹H nmr : (d₆-DMSO) 0.81 (2H, m), 1.05-1.20 (8H, m), 1.50-1.70 (7H, m), 2.27 (3H, s), 2.41 (2H, dd), 2.54 (3H, s), 3.36 (1H, m), 4.21 (2H, d), 8.61 (2H, brs), 10.39 (1H, s).
MS : 492 (MNa⁺)
CHN : Found: C51.08%; H6.77%; N14.16%; C₂₀H₃₁N₅O₆S. 0.15 H₂O. 0.15 EtOAc requires C51.19%; H6.84%; N14.49%

### Example 12:

### (3R)-6-cyclohexyl-N-hydroxy-3-(3-{2-[(methylsulfonyl)amino]ethyl}-1,2,4-oxadiazol-5-yl)hexanamide

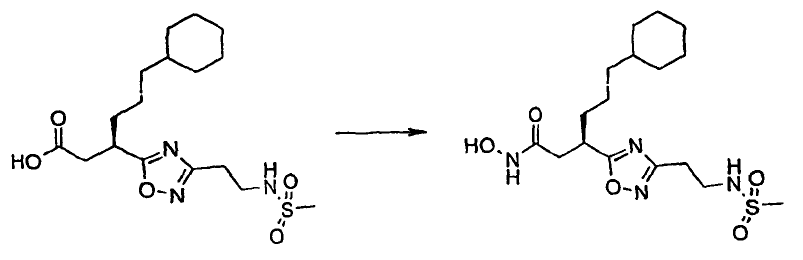

Method as for example 1 using (3*R*)-6-cyclohexyl-3-(3-{2-[(methylsulfonyl)amino]ethyl}-1,2,4-oxadiazol-5-yl)hexanoic acid (preparation 33) (315mg, 0.81 mmol) as starting material.
¹H nmr : (CD₃OD) 0.84 (2H, m), 1.10-1.35 (8H, m), 1.55-1.75 (7H, m), 2.49 (1H, dd), 2.60 (1H, dd), 2.91 (3H, s), 2.96 (2H, t), 3.43 (2H, t), 3.51 (1H, s).
MS : 425 (MNa⁺)
CHN : Found: C47.35%; H7.04%; N12.47%; C₁₇H₃₀N₄O₅S requires C47.24%; H7.02%; N12.59%

### Example 13:

### (3R)-6-cyclohexyl-N-hydroxy-3-{3-[1-(methylsulfonyl)-3-azetidinyl]-1,2,4-oxadiazol-5-yl}hexanamide

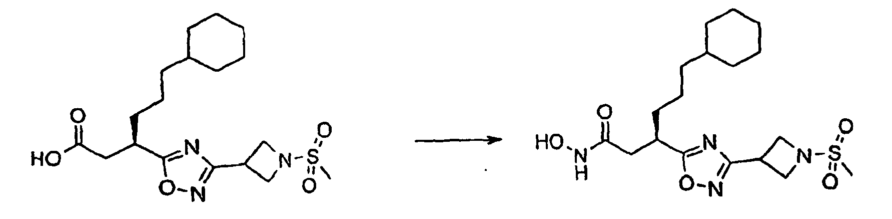

Method as for example 1 using (3*R*)-6-cyclohexyl-3-{3-[1-(methylsulfonyl)-3-azetidinyl]-1,2,4-oxadiazol-5-yl}hexanoic acid (preparation 39) (150mg, 0.38mmol) as starting material. Purification: Crude material was purified on a silica column eluting with a solvent gradient of DCM : MeOH (98 : 2) gradually changing to (90 : 10) to afford the title compound as a sticky, glassy oil (140mg, 90%).
¹H nmr : (CD₃OD) 0.82 (2H, m), 1.05-1.35 (8H, m), 1.55-1.80 (7H, m), 2.53 (1H, dd), 2.61 (1H, dd), 2.98 (3H, s), 3.55 (1H, m), 4.00 (1H, m), 4.15 (2H, m), 4.25 (2H, m).
MS : 437 (MNa⁺)
CHN : Found: C51.04%; H7.26%; N12.93%; C₁₈H₃₀N₄O₅S. 0.25 EtOAC. 0.05DCM requires C51.22%; H7.29%; N13.24%

### Example 14:

### (3R)-6-cyclohexyl-N-hydroxy-3-{3-[1-(methylsulfonyl)-4-piperidinyl]-1,2,4-oxadiazol-5-yl}hexanamide

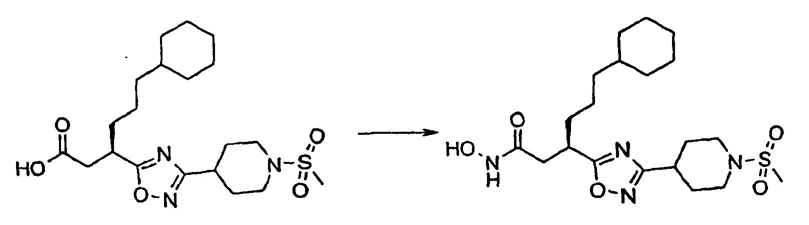

Method as for example 1 using (3*R*)-6-cyclohexyl-3-{3-[1-(methylsulfonyl)-4-piperidinyl]-1,2,4-oxediazol-5-yl}hexanoic acid (preparation 44) (230mg, 0.54mmol) as starting material. Purification: Crude material triturated with DIPE, filtered off and dried under reduced pressure to afford the title compound as fluffy white solid (204mg, 85%).
¹H nmr : (CD₃OD) 0.83 (2H, m), 1.05-1.35 (8H. m). 1.55-1.75 (7H, m), 1.88 (2H, m), 2.09 (2H, m), 2.48 (1H, dd), 2.60 (1H, dd), 2.82 (3H, s), 2.98 (3H, m), 3.52 (1H, m), 3.70 (2H, m).
MS : 465 (MNa⁺)
CHN : Found: C54.08%; H7.75%; N12.48%; C₂₀H₃₄N₄O₅S requires C54.28%; H7.74%; N12.66%

### Example 15:

### (3R)-6-cyclohexyl-3-[3-(1,1-dioxido-2-isothiazolidinyl)-1,2,4-oxadiazol-5-yl]-N-hydroxyhexanamide

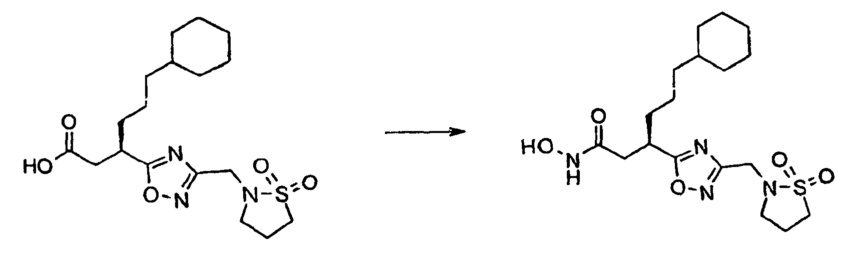

Method as for example 1 using (3*R*)-6-cyclohexyl-3-[3-(1,1-dioxido-2-isothiazolidinyl)-1,2,4-oxadiazol-5-yl]hexanoic acid (preparation 46) (194mg, 0.49mmol) as starting material and NMM as base.
Purification: The crude material was purified on a silica column eluting with Et₂O to removed the impurities and changing to DCM : MeOH (9 : 1) as eluent to afford the title compound as a colourless oil 103mg, 51%).
¹H nmr : (d₆-DMSO) 0.80 (2H, m), 1.00-1.25 (8H, m), 1.50-1.70 (7H, m), 2.22 (2H, m), 2.52 (2H, obs), 2.74 (2H, t), 3.20 (2H, obs), 3.26 (2H, t), 3.41 (1H, m), 4.20 (2H, s), 8.62 (1H, brs), 10.39 (1H, brs).
MS : 437 (MNa⁺)
CHN : Found: C51.86%; H7.37%; N13.03%; C₁₈H₃₀N₄O₅S. 0.2 H₂O requires C51.71 %; H7.33%; N13.40%

### Examples 16 and 17:

### (3R)-3-[3-({[(tert-butylamino)sulfonyl]amino}methyl)-1,2,4-oxadiazol-5-yl]-6-cyclohexyl-N-hydroxyhexanamide(17) and (3R)-3-(3-{[(aminosulfonyl)amino]methyl}-1,2,4-oxadiazol-5-yl)-6-cyclohexyl-N-hydroxyhexanamide(16)

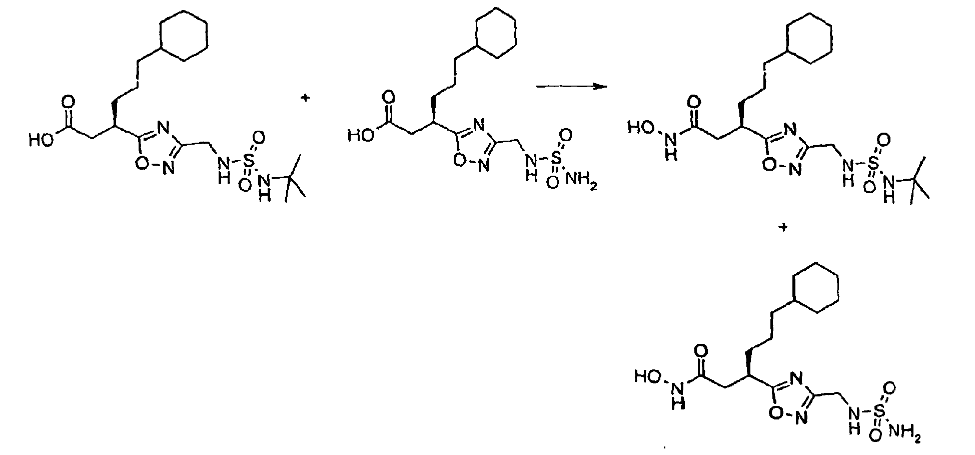

Method as for example 1 using (3*R*)-3-[3-({[(*tert*-butylamino)sulfonyl]amino}methyl)-1,2,4-oxadiazol-5-yl]-6-cyclohexylhexanoic acid and (3*R*)-3-(3-{[(aminosulfonyl)amino]methyl}-1,2,4-oxadiazol-5-yl)-6-cyclohexylhexanoic acid (preparations 48 and 49) as starting material. Purification: The crude mixture of products was purified on a silica column eluting with a solvent gradient of DCM : MeOH (100 : 0) gradually changing to (98 : 2).

Top spot was (3*R*)-3-(3-{[(aminosulfonyl)amino]methyl}-1,2,4-oxadiazol-5-yl)-6-cyclohexyl-*N-*hydroxyhexanamide (example 16) isolated as a white foam (38mg).
¹H nmr : (d₆-DMSO) 0.80 (2H, m), 1.00-1.25 (8H, m), 1.50-1.70 (7H, m), 2.41 (1H, dd), 2.65 (1H, dd), 3.41 (1H, m), 4.16 (2H, d), 6.53 (2H, brs), 7.01 (1H, brs), 8.64 (1H, brs), 10.40 (1H, brs).
MS: 390 (MH⁺)
Accurate mass : Found 390.1794 (MH⁺), Calculated C₁₅H₂₇N₅O₅S, 390.1806

Second spot was (3*R*)-3-[3-({[(*tert*-butylamino)sulfonyl]amino}methyl)-1,2,4-oxadiazol-5-yl]-6-cyclohexyl-*N*-hydroxyhexanamide (example 17) (34mg).
¹H nmr : (d₆-DMSO) 0.81 (2H, m), 1.00-1.20 (8H, m), 2.40 (9H, s), 1.50-1.70 (7H, m), 2.40 (1 H, dd), 2.66 (1H, dd), 3.41 (1H, m), 4.08 (2H, d), 6.52 (1H, brs), 7.17 (1H, brs), 8.63 (1H, brs), 10.38 (1H, brs).
MS : 468 (MNa⁺)
Accurate mass : Found 468.2235 (MNa⁺). Calculated C₁₉H₃₅N₅O₅S, 468.2251

### Example 18:

### (3R)-3-{3-[(acetylamino)methyl]-1,2,4-oxadiazol-5-yl}-6-cyclohexyl-N-hydroxyhexanamide

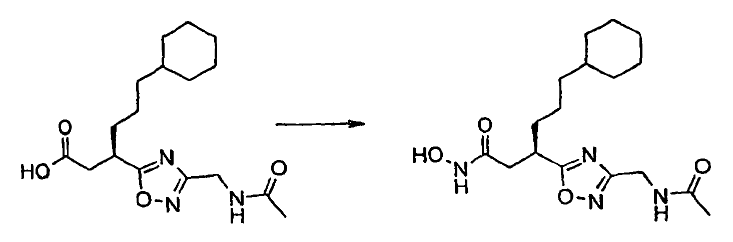

Method as for example 1 using (3*R*)-3-{3-[(acetylamino)methyl]-1,2,4-oxadiazol-5-yl}-6-cyclohexylhexanoic acid (preparation 51) (215mg, 0.64mmol) as strating material and NMM as base.
Purification: Crude material was purified on a silica column eluting with a solvent gradient of Et₂O : MeOH (19 : 1) gradually changing to (9 : 1) to afford the title compound as a colourless gum (39mg, 17%)
¹H nmr : (d₆-DMSO) 0.80 (2H, m), 1.05-1.25 (8H, m), 1.55-1.70 (7H, m), 1.81 (3H, s), 2.40 (2H, m), 3.39 (1H, m), 4.32 (2H, d), 8.31 (1H, brs), 8.62 (1H, s), 10.38 (1H, brs).
MS : 375 (MNa⁺)
CHN : Found: C56.17%; H7.93%; N14.86%; C₁₇H₂₇N₃O₄. 0.4 H₂O. 0.1 DCM requires C55.79%; H7.94%; N15.22%

### Example 19:

### (3R)-3-(3-{[acetyl(methyl)amino]methyl}-1,2,4-oxadiazol-5-yl)-6-cyclohexyl-N-hydroxyhexanamide

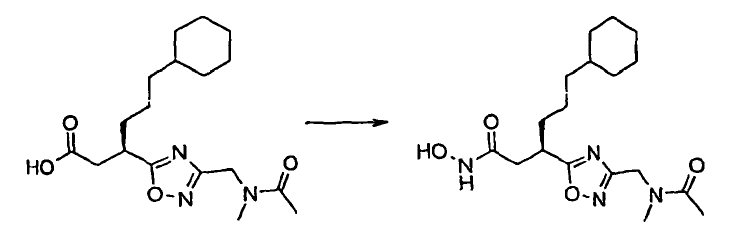

Method as for example 7 using (3*R*)-3-(3-{[acetyl(methyl)amino]methyl}-1,2,4-oxadiazol-5-yl)-6-cyclohexylhexanoic acid (preparation 53) (122mg, 0.37mmol) as starting material.
Purification: Crude material was purified on a silica column eluting with DCM : MeOH (9 : 1) to afford the title compound as a colourless oil (23mg, 18%).
¹H nmr : (CD₃OD): 0.83 (2H,m), 1.10-1.35 (8H, m), 1.60-1.80 (7H, m), 2.12-2.18 (3H, s+s), 2.52 (1H, m), 2.59 (1H, m), 2.95-3.10 (3H, s+s), 3.54 (1H, m), 4.65 (2H, s) and some imidazole.
MS : 389 (MNa⁺)
CHN : Found: C56.04%; H8.20%; N15.26%; C₁₈H₃₀N₄O₄. 0.6 H₂O. 0.1 DCM. 0.1 imidazole requires C56.30%; H8.16%; N14.99%

### Example 20:

### N-[(5-{(1R)-4-cyclohexyl-1-[2-(hydroxyamino)-2-oxoethyl]butyl}-1,2,4-oxadiazol-3-yl)methyl]cyclopropanecarboxamide

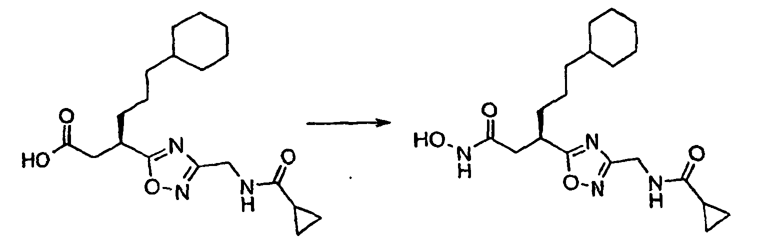

Method as for example 1 using (3*R*)-6-cyclohexyl-3-(3-{[(cyclopropylcarbonyl)amino]methyl}-1,2,4-oxadiazol-5-yl)hexanoic acid (preparation 55) (198mg, 0.54mmol) as starting material and NMM as the base.
Purification: The crude material was purified on a silica column eluting with DCM : MeOH (19 : 1) to afford the title compound as a white foam (141mg, 68%).
¹H nmr : (d₆DMSO) 0.65 (4H, t), 0.80 (2H, m), 1.00-1.25 (8H, m), 1.50-1.70 (8H, m), 2.41 (2H, m), 3.40 (1H, m), 4.37 (2H, d), 8.49 (1H, brs), 8.61 (1H, brs), 10.37 (1H, brs).
MS : 401 (MNa⁺)
CHN : Found: C59.61%; H8.05%; N14.19%; C₁₉H₃₀N₄O₄. 0.3 H₂O requires C59.45%; H8.03%; N14.59%

### Example 21:

### (3R)-6-cyclohexyl-N-hydroxy-3-(3-{[(methoxyacetyl)amino]methyl}-1,2,4-oxadiazol-5-yl)hexanamide

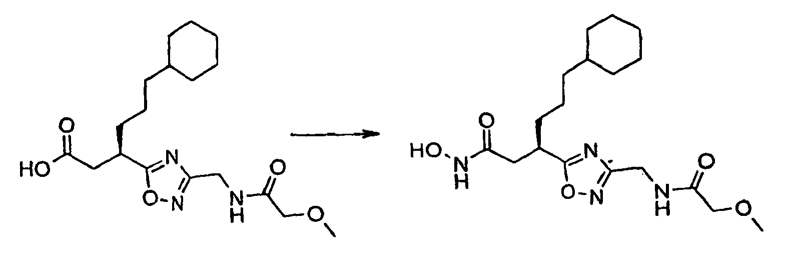

Method as for example 1 using (3*R*)-6-cyclohexyl-3-(3-{[(methoxyacetyl)amino]methyl}-1,2,4-oxadiazol-5-yl)hexanoic acid (preparation 57) (186mg, 0.51mmol) and NMM as the base. Purification: The crude material was purified on a silica column eluting with DCM: MeOH (19: 1) to afford the title compound as a colourless, sticky gum (90mg, 46%).
¹H nmr : (d₆DMSO) 0.80 (2H, m), 1.00-1.25 (8H, m), 1.55-1.70 (7H, m), 2.41 (2H, m), 3.32 (3H, s), 3.40 (1H, m), 3.82 (2H, s), 4.39 (2H, d), 8.21 (1H, brs), 8.63 (1 H, brs), 10.38 (1H, brs).
MS : 405 (MNa⁺)
CHN : Found: C54.09%; H7.79%; N13.81%; C₁₈H₃₀N₄O₅. 0.8 H₂O requires C54.48%; H8.03%; N14.12%

### Example 22:

### (3R)-6-cyclohexyl-3-[3-({[(dimethylamino)acetyl]amino}methyl)-1,2,4-oxadiazol-5-yl]-N-hydroxyhexanamide

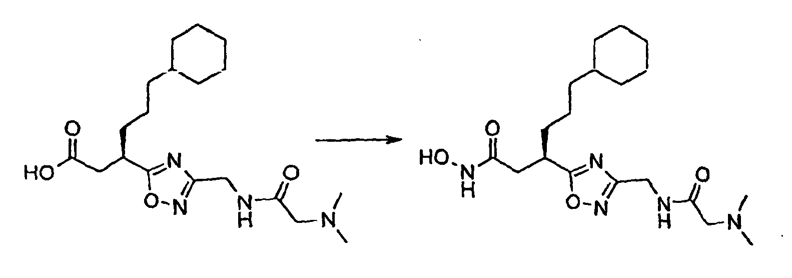

Method as for example 7 using (3*R*)-6-cyclohexyl-3-[3-({[(dimethylamino)aretyl]amino}methyl)-1,2,4-oxadiazol-5-yl]hexanoic acid (preparation 59) (215mg, 0.52mmol) as starting material. Purification: The crude material was purified on a silica column eluting with DCM : MeOH (9 : 1) to afford the title compound as a colourless oil (60mg, 29%).
¹H nmr : (d₆DMSO) 0.80 (2H, m), 1.00-1.20 (8H, m), 1.50-1.65 (8H, m), 2.21 (6H, s), 2.40 (2H, m), 2.91 (2H, s), 3.40 (1H, m), 4.38 (2H, d), 8.18 (1H, brs), 10.37 (1H, brs).
MS : 396 (MH⁺)
CHN : Found: C55.31 %; H7.76%; N22.53%; C₁₉H₃₂N₄O₄. 0.5 H₂O. 1.6 imidazole requires C55.68%; H7.93%; N22.37%

### Example 23:

### N-[(5-{(1R)-4-cyclohexyl-1-[2-(hydroxyamino)-2-oxoethyl]butyl}-1,2,4-oxadiazol-3-yl)methyl]benzamide

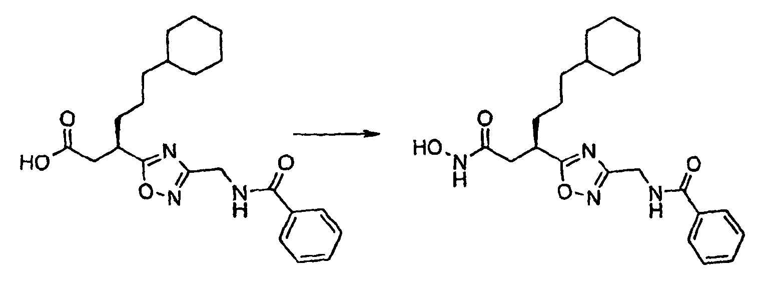

Method as for example 1 using (3*R*)-3-{3-[(benzoylamino)methyl]-1,2,4-oxadiazol-5-yl}-6-cyclohexylhexanoic acid (preparation 61) (202mg, 0.51mmol) and NMM as the base. Purification: The crude material was purified on a silica column eluting with Et₂O : MeOH (19: 1) to afford the title compound as a colourless oil (112mg, 53%).
¹H nmr: (d₆DMSO) 0.79 (2H, m), 1.00-1.25 (8H, m), 1.50-1.70 (7H, m), 2.41 (2H, m), 3.41 (1H, m), 4.54 (2H, d), 7.42 (2H, t), 7.53 (1H, t), 7.83 (2H, d), 8.61 (1H, brs), 8.97 (1H, brs), 10.37 (1 H, brs).
MS : 437 (MNa⁺)
CHN : Found: C61.11%; H7.18%; N11.83%; C₂₂H₃₀N₄O₄. 0.4 H₂O. 0.2 DCM. 0.2 DIPE requires C61.22%; H7.46%; N12.20%

### Example 24:

### N-[(5-{(1R)-4-cyclohexyl-1-[2-(hydroxyamino)-2-oxoethyl]butyl}-1,2,4-oxadiazol-3-yl)methyl]-2-pyridinecarboxamide

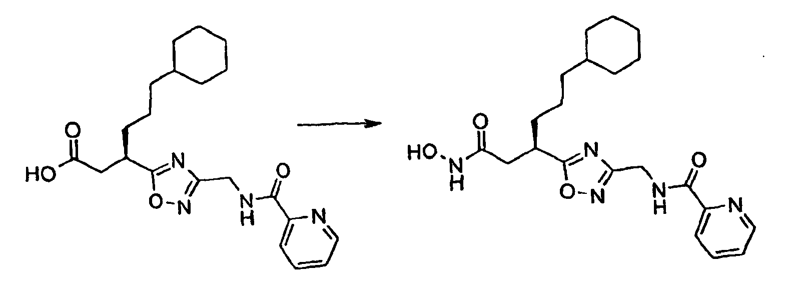

Method as for example 7 using (3*R*)-6-cyclohexyl-3-(3-{[(2-pyridinylcarbonyl)amino]methyl}-1,2,4-oxadiazol-5-yl)hexanoic acid (preparation 63) (188mg, 0.43mmol) as starting material. Purification: The crude material was purified on a silica column eluting with DCM : MeOH (19 : 1) to afford the title compound as a colourless oil (41mg, 23%).
¹H nmr : (d₆DMSO) 0.78 (2H, m), 1.00-1.25 (8H, m), 1.50-1.65 (7H, m), 2.40 (2H, m), 3.40 (1H, m), 4.58 (2H, d), 7.59 (1H, t), 7.98 (2H, m), 8.61 (2H, brs), 9.12 (1H, brs), 10.38 (1H, brs).
MS : 438 (MNa⁺)
CHN : Found: C60.09%; H7.15%; N15.82%; C₂₁H₂₉N₅O₄. 0.4 H₂O. 0.1 DIPE requires C59.93%; H7.26%; N16.18%

### Example 25:

### (3R)-3-(3-{[(aminocarbonyl)amino]methyl}-1,2,4-oxadiazol-5-yl)-6-cyclohexyl-N-hydroxyhexanamide

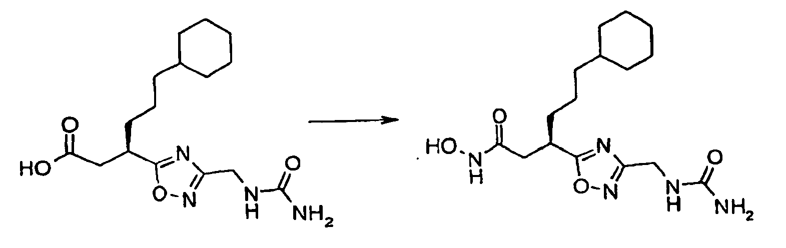

Method as for example 7 using (3*R*)-3-(3-{[(aminocarbonyl)amino]methyl}-1,2,4-oxadiazol-5-yl)-6-cyclohexylhexanoic acid (preparation 65) (78mg, 0.23mmol) as starting material. Purification: Crude material was purified on a silica column eluting with DCM : MeOH : NH₃ (90 : 10 : 1) to afford the title compound as a white solid (13mg)
¹H nmr : (d₆DMSO) 0.81 (2H, m), 1.05-1.30 (8H, m), 1.50-1.70 (7H, m), 2.42 (2H, m), 3.41 (1H, m), 4.31 (2H, d), 6.60 (2H, brs).

### Example 26:

### (3R)-6-cyclohexyl-N-hydroxy-3-[3-({[(methylamino)carbonyl]amino}methyl)-1,2,4-oxadiazol-5-yl]hexanamide

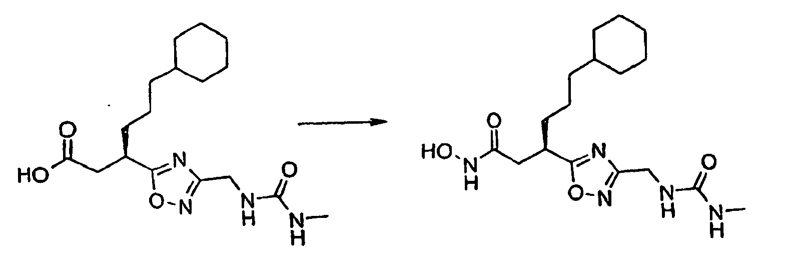

Method as for example 1 using (3*R*)-6-cyclohexyl-3-[3-({[(methylamino)carbonyl]amino}methyl)-1,2,4-oxadiazol-5-yl]hexanoic acid (preparation 67) (119mg, 0.34mmol) as starting material and Et₃N as base.
Purification: Two silica columns were required to purify the crude material the first eluting with DCM : MeOH : NH₃ (90 : 10 : 1) and the second eluting with DCM : MeOH (95 : 5) to afford the title compound as a colourless oil (11mg).
¹H nmr : (d₆DMSO) 0.79 (2H, m), 1.05-1.20 (8H, m), 1.50-1.70 (7H, m), 2.41 (2H, m), 2.55 (3H, d), 3.40 (1H, m), 4.25 (2H, d), 6.42 (1H, brs), 8.80 (1H, brs), 10.50 (1H, brs).
MS : 366 (M-H)

### Example 27:

### (3R)-6-cyclohexyl-3-[3-({[(dimethylamino)carbonyl]amino}methyl)-1,2,4-oxadiazol-5-yl]-N-hydroxyhexanamide

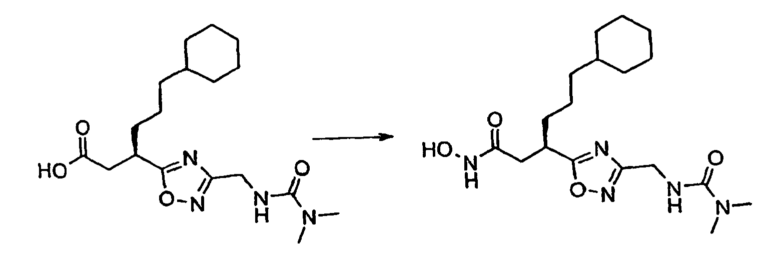

Method as for example 1 using (3*R*)-6-cyclohexyl-3-[3-({[(dimethylamino)carbonyl]amino}methyl)-1,2,4-oxadiazol-5-yl]hexanoic acid (preparation 69) (204mg, 0.56mmol) as starting material and Et₃N as base.
Purification: The crude material was purified on a silica column eluting with a solvent gradient of DCM : MeOH (98 : 2) gradually changing to (90 : 10) to afford the title compound as a crunchy, white foam (147mg).
¹H nmr : (d₆DMSO) 0.80 (2H, m), 1.05-1.30 (8H, m), 1.50-1.70 (7H, m), 2.40 (2H, m), 2.79 (6H, m), 3.40 (1H, m), 4.25 (2H, d), 6.75 (1H, brs), 8.62 (1H, brs), 10.38 (1H, brs).
MS : 380 (M-H)

### Example 28:

### tert-butyl (5-{(1R)-4-cyclohexyl-1-[2-(hydroxyamino)-2-oxoethyl]butyl}-1,2,4-oxadiazol-3-yl)methylcarbamate

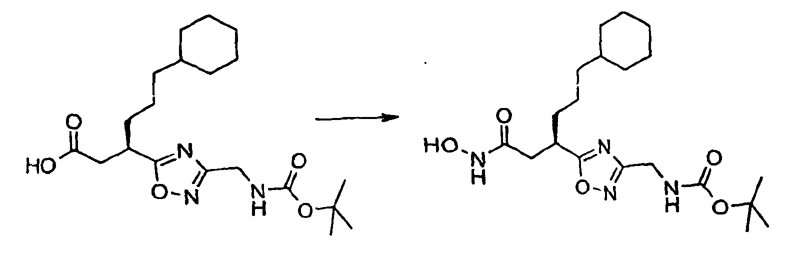

Method as for example 1 using (3*R*)-3-(3-{[(*tert*-butoxycarbonyl)amino]methyl}-1,2,4-oxadiazol-5-yl)-6-cyclohexylhexanoic acid (preparation 70) (113mg, 0.29mmol) as starting material and NMM as the base.
¹H nmr: (CDCl₃) 0.81 (2H, m), 1.10-1.35 (8H, m), 1.43 (9H, s), 1.60-1.80 (7H, m), 2.59 (1H, dd), 2.70 (1H, m), 3.57 (1H, m), 4.40 (2H, d), 5.01 (1H, brs).
MS : 433 (MNa⁺)
CHN : Found: C57.95%; H8.40%; N13.16%; C₂₀H₃₄N₄O₅. 0.25 H₂O requires C57.88%; H8.38%; N13.50%

### Example 29:

### (3R)-3-(3-amino-1,2,4-oxadiazol-5-yl)-6-cyclohexyl-N-hydroxyhexanamide

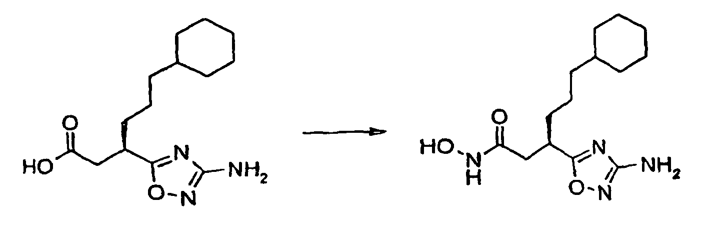

Method as for example 7 using (3*R*)-3-(3-amino-1,2,4-oxadiazol-5-yl)-6-cyclohexylhexanoic acid (preparation 72) (390mg, 1.38mmol) as starting material.
Purification: The crude material was purified on a silica column eluting with a solvent gradient of DCM : MeOH : NH₃ (95 : 5 : 0.5) gradually changing to (70 : 30 : 5) to afford the title compound as a white foam (170mg, 42%).
¹H nmr: (d₆DMSO) 0.80 (2H, m), 1.00-1.20 (8H, m), 1.50-1.70 (7H, m), 2.35 (2H, m), 3.21 (1H, m), 6.10 (2H, brs).
MS : 319 (MNa⁺)

### Example 30:

### tert-butyl (3R)-3-[3-(aminomethyl)-1,2,4-oxadiazol-5-yl]-6-cyclohexylhexanoate

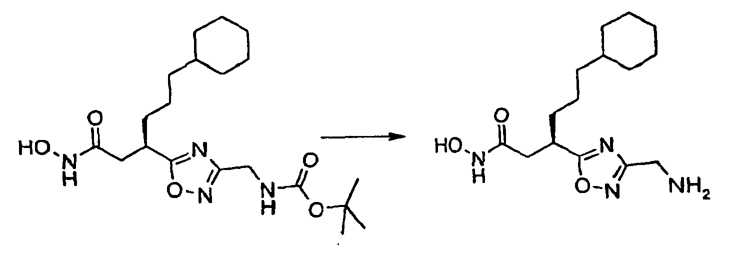

Method as for preparation 11 using *tert*-butyl (5-{(1*R*)-4-cyclohexyl-1-[2-(hydroxyamino)-2-oxoethyl]butyl}-1,2,4-oxadiazol-3-yl)methylcarbamate (example 28) (65mg, 0.16mmol) as starting material. The crude material was azeotroped from DCM (x4) and Et₂O (2x) and the residue dried under reduced pressure to afford the title compound as a pale yellow foam (61 mg, 90%).
¹H nmr : (d₆DMSO) 0.80 (2H, m), 1.05-1.25 (8H, m), 1.50-1.70 (7H, m), 2.40 (2H, m), 3.46 (1H, m), 4.22 (2H, s), 8.45 (2H, brs).
MS : 311 (MH⁺)

### Example 31:

### (3R)-6-cyclohexyl-N-hydroxy-3-{3-[(methylamino)methyl]-1,2,4-oxadiazol-5-yl}hexanamide

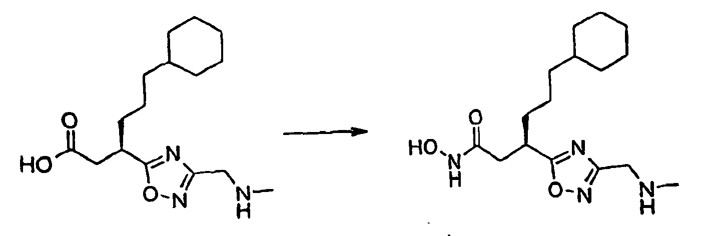

Method as for example 7 using (3*R*)-6-cyclohexyl-3-{3-[(methylamino)methyl]-1,2,4-oxadiazol-5-yl}hexanoic acid (preparation 73) (370mg, 1.07mmol) as starting material.
Purification: The crude material was purified one silica column eluting with DCM : MeOH (9 : 1) to afford the title compound as a colourless oil (93mg, 27%).
¹H nmr : (CD₃OD) 0.85 (2H, m), 1.10-1.35 (8H, m), 1.60-1.80 (7H, m), 2.40 (3H, s), 2.50 (1H, dd), 2.60 (1H, dd), 3.37 (1H, s), 3.54 (1H, m), 3.80 (2H, s).
MS : 325 (MH⁺)
CHN : Found: C57.95%; H8.84%; N16.57%; C₁₆H₂₈N₄O₃. 0.4 H₂O requires C57.95%; H8.75%; N16.89%

### Example 32:

### (3R)-6-cyclohexyl-3-{3-[(ethylamino)methyl]-1,2,4-oxadiazol-5-yl}-N-hydroxyhexanamide

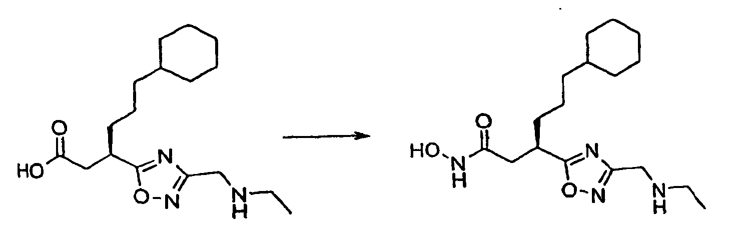

Method as for example 7 using (3*R*)-6-cyclohexyl-3-{3-[(ethylamino)methyl]-1,2,4-oxadiazol-5-yl}hexanoic acid (preparation 75) (360mg, 0.76mmol) as starting material.
Purification: The crude material was purified on a silica column eluting with a solvent gradient of DCM : MeOH : NH₃ (97.5 : 2.5 : 0.5) gradually changing to (90 : 10 :1). The product was recrystallised from Et₂O to afford the title compound as white crystals (79mg, 31 %).
M. Pt. : 69.4-71.4°C
¹H nmr : (d₆DMSO) 0.80 (2H, m), 0.99 (3H, t), 1.05-1.20 (8H, m), 1.50-1.70 (7H, m), 2.40 (2H, m), 2.53 (2H, q), 3.40 (1H, m), 3.71 (2H, s).
MS : 339 (MH⁺)
CHN : Found: C59.58%; H8.85%; N16.47%; C₁₇H₃₀N₄O₃. 0.08 DCM requires C59.42%; H8.81%; N16.23%

### Example 33:

### (3R)-6-cyclohexyl-N-hydroxy-3-{3-[(propylamino)methyl]-1,2,4-oxadiazol-5-yl}hexanamide

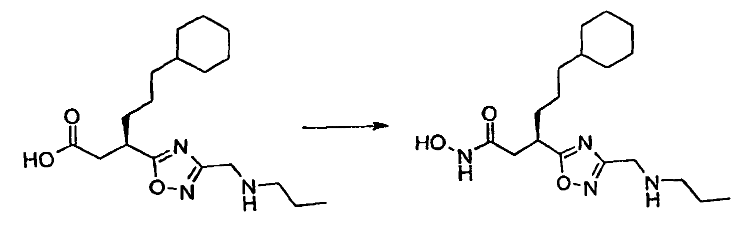

Method as for example 7 using (3*R*)-6-cyclohexyl-3-{3-[(propylamino)methyl]-1,2,4-oxadiazol-5-yl}hexanoic acid (preparation 77) (270mg, 0.60mmol) as starting material.
Purification: The crude material was purified on a silica column eluting with a solvent gradient of DCM : MeOH : NH₃ (97.5 : 2.5 : 0.25) gradually changing to (90 : 10 : 1). The product was recrystallised from Et₂O to afford the title compound as a sticky solid (46mg).
M.Pt. : 90.4-91.8°C
¹H nmr : (d₆DMSO) 0.79 (5H, m), 1.00-1.20 (8H, m), 1.39 (2H, m), 1.50-1.70 (7H, m), 1.99 (1H, brs), 2.30-2.45 (3H, m), 3.41 (1H, m), 3.74 (2H, s), 8.74 (1H, brs), 10.41 (1H, brs).
MS : 353 (MH⁺)
CHN : Found: C58.03%; H8.65%; N15.00%; C₁₈H₃₂N₄O₃. 0.3 DCM requires C58.16; H8.69%; N14.82%

### Example 34:

### (3R)-6-cyclohexyl-N-hydroxy-3-{3-[(isopropylamino)methyl]-1,2,4-oxadiazol-5-yl}hexanamide

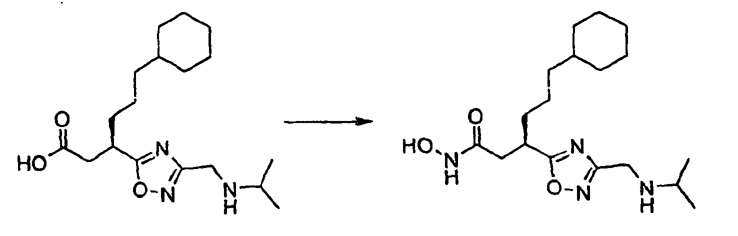

Method as for example 7 using (3*R*)-6-cyclohexyl-3-{3-[(isopropylamino)methyl]-1,2,4-oxadiazol-5-yl}hexanoic acid (preparation 79) (198mg, 0.44mmol) as starting material.
Purification: The crude material was purified on a silica column eluting with a solvent gradient of DCM : MeOH : NH₃ (95 : 5 : 0.5) gradually changing to (90 : 10: 1). The oil was treated with Et₂O to afford the title compound as a white solid (67mg, 44%).
¹H nmr : (CD₃OD) 0.82 (2H, m), 1.09 (6H, d), 1.10-1.30 (8H, m), 1.60-1.80 (7H, m), 2.51 (1 H, dd), 2.61 (1H, dd), 2.81 (1H, m), 3.57 (1H, m), 3.86 (2H, s).
MS : 353 (MH⁺)

### Example 35:

### (3R)-6-cyclohexyl-N-hydroxy-3-{3-[(isobutylamino)methyl]-1,2,4-oxadiazol-5-yl}hexanamide

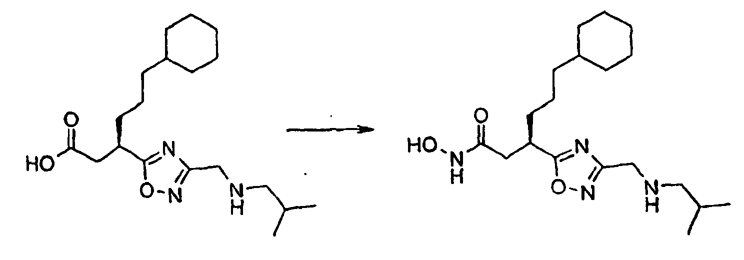

Method as for example 7 using (3*R*)-6-cyclohexyl-3-{3-[(isobutylamino)methyl]-1,2,4-oxadiazol-5-yl}hexanoic acid (preparation 81) (495mg, 1.06mmol) as starting material.
Purification: The crude material was purified on a silica column eluting with a solvent gradient of DCM : MeOH : NH₃ (97.5 : 2.5 : 0.25) gradually changing to (90 : 10 :1). The glass obtained was recrystallised from Et₂O to afford the title compound as colourless crystals (71mg).
M.Pt.: 97.8-99.0°C
¹H nmr : (d₆DMSO) 0.83 (2H, m), 0.92 (6H, d), 1.10-1.35 (8H, m), 1.60-1.80 (8H, m), 2.46 (2H, d), 2.59 (1H, brs), 2.69 (1H, brs), 3.59 (1H, m), 3.88 (2H, s).
MS : 367 (MH⁺)
CHN : Found: C62.21 %; H9.48%; N15.47%; C₁₉H₃₄N₄O₃ requires C62.27; H9.35%; N15.29%

### Example 36:

### (3R)-3-{3-[(tert-butylamino)methyl]-1,2,4-oxadiazol-5-yl}-6-cyclohexyl-N-hydroxyhexanamide

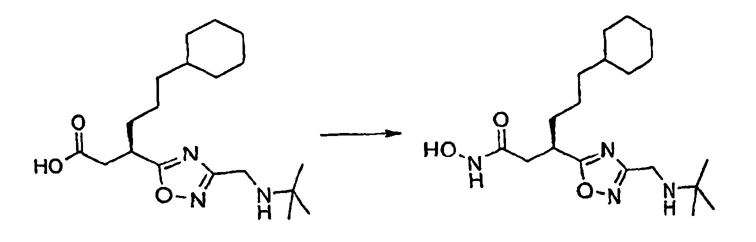

Method as for example 7 using (3*R*)-3-{3-[(*tert*-butylamino)methyl]-1,2,4-oxadiazol-5-yl}-6-cyclohexylhexanoic acid (preparation 83) (274mg, 0.59mmol) as starting material.
Purification: The crude material was purified on a silica column eluting with a solvent gradient of DCM : MeOH : NH₃ (97.5 : 2.5 : 0.25) gradually changing to (90 : 10 :1). Second column was required eluting with DCM : MeOH : NH₃ (95 : 5 : 0.5). The product was partitioned between Et₂O and H₂O. The organic extracts were concentrated under reduced pressure. The residue was dissolved in DCM, dried over anhydrous Na₂SO₄, filtered and the solvent removed under reduced pressure. The residue was dired under reduced pressure to afford the title compound (52mg).
¹H nmr: (CDCl₃) 0.83 (2H, m), 1.10-1.40 (17H, s+m), 1.60-1.80 (7H, m), 2.50-2.75 (2H, brd), 3.59 (1H, m), 3.85 (2H, s).
MS : 367 (MH⁺)
Accurate mass : Found 367.2711 (MH⁺), Calculated C₁₉H₃₅N₄O₃, 367.2709

### Example 37:

### (3R)-6-cyclohexyl-3-(3-{[(1-ethylpropyl)amino]methyl}-1,2,4-oxadiazol-5-yl)-N-hydroxyhexanamide

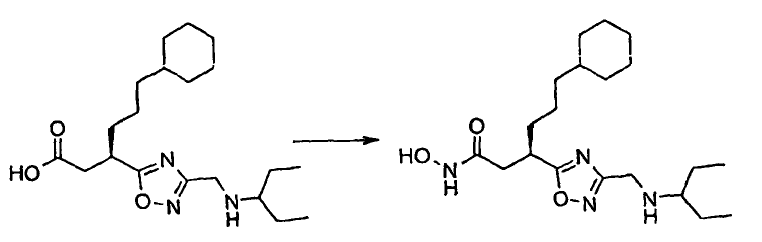

Method as for example 7 using (3*R*)-6-cyclohexyl-3-(3-{[(1-ethylpropyl)amino]methyl}-1,2,4-oxadiazol-5-yl)hexanoic acid (preparation 85) as starting material.
Purification: Crude material purified on a silica column eluting with DCM : MeOH : NH₃ (97.5 : 2.5 : 0.25). The solid was recrystallised from Et₂O to afford the title compound as fluffy crystals (212mg).
M.Pt. : 102.7-104.3°C
¹H nmr : (d₆DMSO) 0.79 (8H, m+t), 1.00-1.20 (8H, m), 1.33 (4H, m),1.50-1.75 (7H, m), 2.29 (1H, t), 2.40 (2H, m), 3.41 (1H, m), 3.74 (2H, s).
MS : 381 (MH⁺)
CHN : Found: C63.12%; H9.64%; N14.85%; C₂₀H₃₆N₄O₃ requires C63.13; H9.54%; N14.72%

### Example 38:

### (3R)-6-cyclohexyl-3-{3-[(cyclopropylamino)methyl]-1,2,4-oxadiazol-5-yl}-N-hydroxyhexanamide

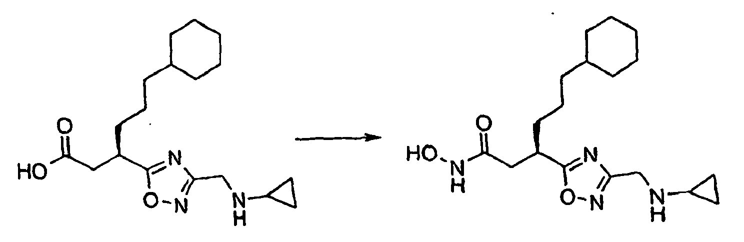

Method as for Example 7 using (3*R*)-6-cyclohexyl-3-{3-[(cyctopropylamino)methyl]-1,2,4-oxadiazol-5-yl}hexanoic acid (preparation 87) (300mg, 0.67mmol) as starting material.

Purification: Crude material purified on a silica column eluting with a solvent gradient of DCM : MeOH : NH₃ (97.5 : 2.5 : 0.25) gradually changing to (90 : 10 : 1). The material was dissolved in Et₂O and washed with H₂O (x5) and the organic extracts were concentrated under reduced pressure. The residue was dissolved in DCM, dried over anhydrous Na₂SO₄ and filtered. The solvent was removed under reduced pressure to afford the title compound (41mg).
¹H nmr : (CDCl₃) 0.40 (2H, s), 0.46 (2H, m), 0.86 (2H, m), 1.10-1.40 (8H, m), 1.60-1.80 (7H. m), 2.19 (1H, m), 2.59 (1H, dd), 2.71 (1H, brs), 3.58 (1H, m), 3.92 (2H, s).
MS : 351 (MH⁺)
CHN : Found: C61.77%; H8.78%; N15.13%; C₁₈H₃₀N₄O₃. 0.25 Et₂O requires C61.85; H8.88%; N15.18%

### Example 39:

### (3R)-3-{3-[(cyclobutylamino)methyl]-1,2,4-oxadiazol-5-yl}-6-cyclohexyl-N-hydroxyhexanamide

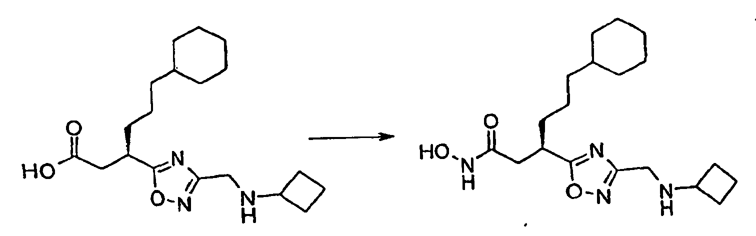

Method as for example 7 using (3*R*)-3-{3-[(cyclobutylamino)methyl]-1,2,4-oxadiazol-5-yl}-6-cyclohexylhexanoic acid (preparation 89) (300mg, 0.67mmol) as starting material.
Purification: Crude material purified on a silica column eluting with a solvent gradient of DCM : MeOH : NH₃ (97.5 : 2.5 : 0.25) gradually changing to (90 : 10 : 1). The material was triturated with DIPE to afford the title compound as a white solid (27mg).
M.Pt. : 82.0-83.9°C
¹H nmr : (d₆DMSO) 0.79 (2H, m), 1.00-1.20 (8H, m), 1.40-1.70 (11H, m), 2.39 (1H, dd), 2.42 (1H, dd), 3.15 (1H, m), 3.40 (1H, m), 3.64 (2H, s), 8.74 (1H, brs), 10.41 (1H, brs).
MS : 365 (MH⁺)
CHN : Found: C62.38%; H8.86%; N15.29%; C₁₉H₃₂N₄O₃ requires C62.61; H8.85%; N15.37%

### Example 40:

### (3R)-6-cyclohexyl-3-{3-[(cyclopentylamino)methyl]-1,2,4-oxadiazol-5-yl}-N-hydroxyhexanamide

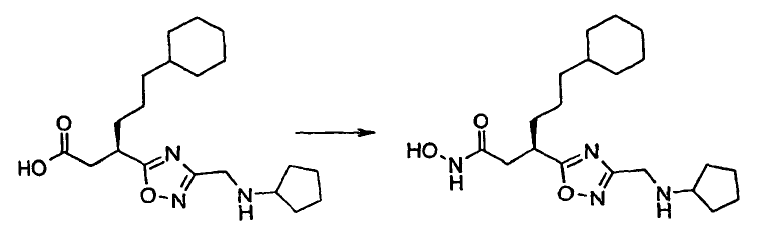

Method as for example 7 using (3*R*)-6-cyclohexyl-3-{3-[(cyclopentylamino)methyl]-1,2,4-oxadiazol-5-yl}hexanoic acid (preparation 91) (240mg, 0.50mmol) as starting material. Purification: The crude material was purified on a silica column eluting with DCM : MeOH : NH₃ (90 : 10 : 1) and the white solid was recrystallised from EtOAc to afford the title compound as a white solid (60mg).
¹H nmr: (d₆DMSO) 0.79 (2H, m), 0.95-1.10 (12H, m), 1.45-1.70 (9H, m), 1.78 (2H, m), 1.95 (1H, brs), 2.25-2.45 (3H, m), 3.41 (1H, m), 3.75 (2H, s), 8.80 (1H, brs), 10.48 (1H, brs).
MS : 379 (MH⁺)
CHN : Found: C63.53%; H9.16%; N14.66%; C₂₀H₃₄N₄O₃ requires C63.46; H9.05%; N14.80%

### Example 41:

### (3R)-6-cyclohexyl-3-{3-[(cyclohexylamino)methyl]-1,2,4-oxadiazol-5-yl}-N-hydroxyhexanamide

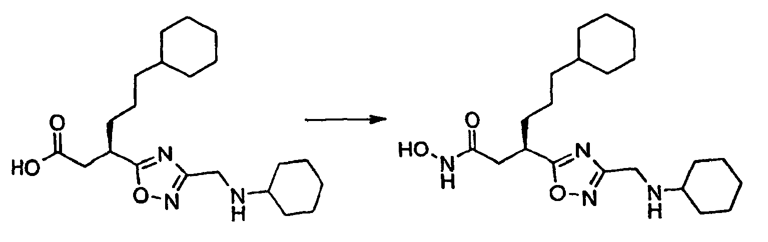

Method as for example 7 using (3*R*)-6-cyclohexyl-3-{3-[(cyclohexylamino)methyl]-1,2,4-oxadiazol-5-yl}hexanoic acid (preparation 93) (262mg, 0.53mmol) as starting material. Purification: The crude material was purified on a silica column eluting with DCM : MeOH : NH₃ (90 : 10 : 1) to afford the title compound as a white solid (130mg).
M. Pt. : 127°C
¹H nmr : (d₆DMSO) 0.79 (2H, m), 0.95-1.20 (10H, m), 1.27 (2H, m), 1.41 (2H, m), 1.50-1.70 (11H, m), 2.40 (2H, m), 2.97 (1H, m), 3.40 (1H, m), 3.71 (2H, s), 8.79 (1H, brs), 10.45 (1H, brs).
MS : 393 (MH⁺)
CHN : Found: C63.79%; H9.24%; N14.32%; C₂₁H₃₆N₄O₃ requires C64.20; H9.24%; N14.27%

### Example 42:

### (3R)-6-cyclohexyl-N-hydroxy-3-(3-{[(2-hydroxyethyl)amino]methyl}-1,2,4-oxadiazol-5-yl)hexanamide

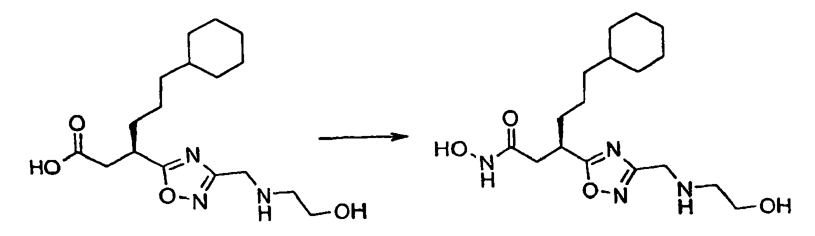

A solution of (3*R*)-6-cyclohexyl-3-(3-{[(2-hydroxyethyl)amino]methyl}-1,2,4-oxadiazol-5-yl)hexanoic acid (preparation 95) (398mg, 0.82mmol) in DCM (8ml) was treated with imidazole (56mg, 0.82mmol) and stirred at room temperature until all imidazole had dissolved. TMSCI (100µl, 0.82mmol) was added and the reaction mixture was stirred at room temperature for 1 hour. Method as for example 7 was then followed.
Purification: The crude material was purified on 2 silica columns. The first eluting with DCM : MeOH : NH₃ (90 : 10 : 1) and the second eluting with DCM : MeOH : NH₃ (80 : 20 : 2) to afford the title compound as a oil (90mg).
¹H nmr : (CD₃OD) 0.82 (2H, m), 1.10-1.35 (8H, m), 1.60-1.80 (7H, m). 2.50-2.70 (2H, m), 2.86 (2H, t), 3.55 (1H, m), 3.70 (2H, t), 4.04 (2H, s) + some impurities.
MS : 355 (MH⁺)

### Example 43:

### (3R)-6-cyclohexyl-N-hydroxy-3-(3-{[(2-hydroxy-1,1-dimethylethyl)amino]methyl}-1,2,4-oxadiazol-5-yl)hexanamide

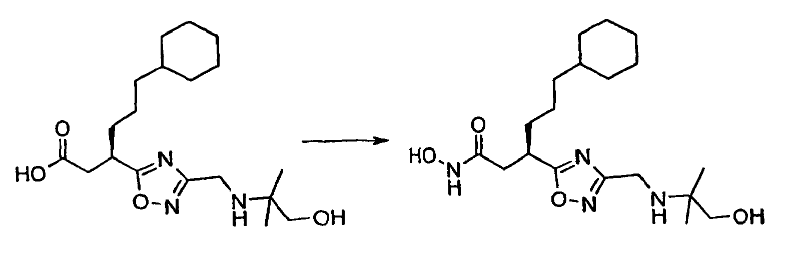

Method as for example 42 using (3*R*)-6-cyclohexyl-3-(3-{[(2-hydroxy-1,1-dimethylethyl)amino]methyl}-1,2,4-oxediazol-5-yl)hexanoic acid (preparation 97) (2.59g, 4.95mmol) as starting material.
Purification: The crude material was purififed on a silica column eluting with DCM : MeOH : NH₃ (98 : 2 : 0.2) gradually changing to (90 : 10: 1). The material was azeotroped from DCM followed by Et₂O to afford the title compound as a white foam (980mg, 52%).
M.Pt. : 49-51 °C
¹H nmr : (d₆DMSO) 0.80 (2H, m), 0.98 (6H, s), 1.05-1.25 (8H, m), 1.50-1.70 (7H, m), 2.40 (2H, m), 3.20 (2H, m), 3.40 (1H, m), 3.73 (2H, s), 4.43 (1H, brs), 8.62 (1H, brs), 10.38 (1H, brs).
MS : 383 (MH⁺)
CHN : Found: C59.27%; H9.06%; N14.22%; C₁₉H₃₄N₄O₄. 0.05 H₂O requires C59.52; H8.96%; N14.61%

### Example 44:

### (3R)-6-cyclohexyl-N-hydroxy-3-(3-{[(2-methoxyethyl)amino]methyl}-1,2,4-oxadiazol-5-yl)hexanamide

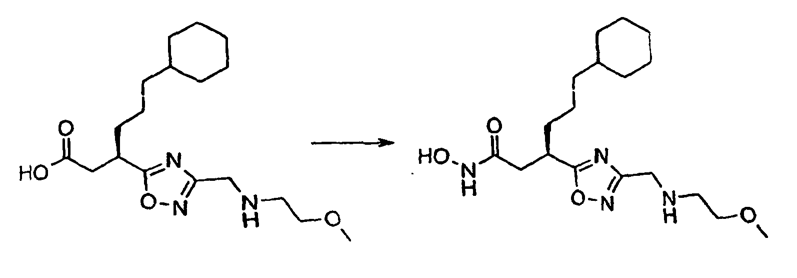

Method as for example 7 using (3*R*)-6-cyclohexyl-3-(3-{[(2-methoxyethyl)amino]methyl}-1,2,4-oxadiazol-5-yl)hexanoic acid (preparation 99) (429mg, 0.93mrnol) as starting material. Purification: The oil solidified on standing to afford the title compound (44mg).
¹H nmr: (d₆DMSO) 0.79 (2H, m), 1.05-1.20 (8H, m), 1.55-1.65 (7H, m), 2.42 (2H, m), 2.69 (2H, t), 3.20 (3H, s), 3.39 (2H, t), 3.41 (1H, m), 3.80 (2H, s), 8.78 (1H, brs), 10.46 (1H, brs).

### Example 45:

### (3R)-6-cyclohexyl-N-hydroxy-3-(3-{[(3-methoxypropyl)amino]methyl}-1,2,4-oxadiazol-5-yl)hexanamide

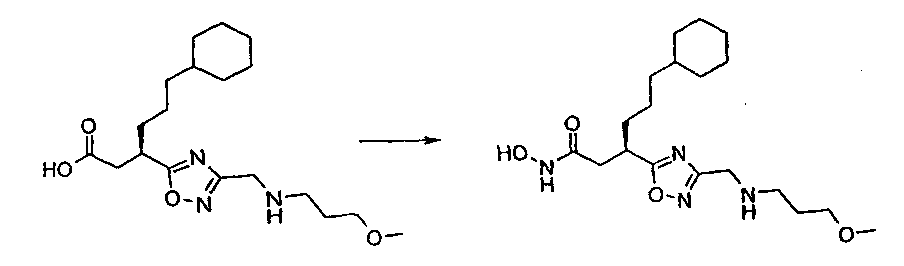

Method as for example 7 using (3*R*)-6-cyclohexyl-3-(3-{[(3-methoxypropyl)amino]methyl}-1,2,4-oxadiazol-5-yl)hexanoic acid (preparation 101) (226mg, 0.62mmol) as starting material. Purification: The crude material was purified on a silica column eluting with DCM : MeOH : NH₃ (92.5 : 7.5: 0.5) to afford the title compound as a white solid (52mg, 25%).
M.Pt. : 60°C
¹H nmr : (CD₃OD) 0.83 (2H, m), 1.10-1.30 (8H, m), 1.60-1.80 (9H, m). 2.52 (1H, dd), 2.61 (1H, dd), 2.68 (2H, t), 3.33 (3H, obs), 3.42 (2H, t), 3.58 (1H, m), 3.83 (2H, s).
MS : 383 (MH⁺)

### Example 46:

### (3R)-6-cyclohexyl-N-hydroxy-3-(3-{[(4-hydroxycyclohexyl)amino]methyl}-1,2,4-oxadiazol-5-yl)hexanamide

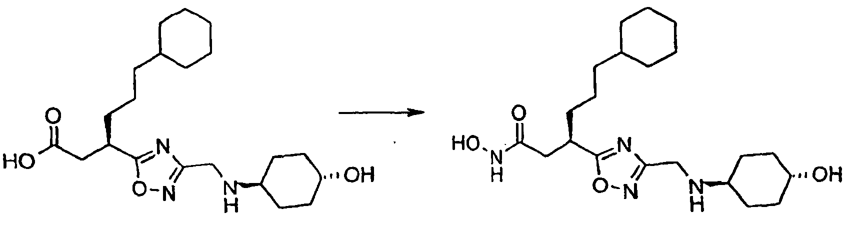

Method as for example 7 using (3*R*)-6-cyclohexyl-3-(3-{[(4-hydroxycyclohexyl)amino]methyl}-1,2,4-oxadiazol-5-yl)hexanoic acid (preparation 103) (179mg, 0.35mmol) as starting material.. Purification: The crude material was purified on a silica column eluting with a solvent gradient of DCM : MeOH : NH₃ (90 : 10 : 1) gradually changing to (80 : 20 : 1) to afford the title compound as a colourless oil (70mg).
¹H nmr: (CD₃OD) 0.82 (2H, m), 1.10-1.35 (12H, m), 1.60-1.80 (7H, m), 1.99 (4H, brd), 2.52 (1H, dd), 2.60 (1H, dd), 2.63 (1H, m), 3.54 (2H, m), 4.01 (2H, s).
MS : 409 (MH⁺)

### Example 47:

### (3R)-6-cyclohexyl-N-hydroxy-3-(3-{[(4-methoxycyclohexyl)amino]methyl}-1,2,4-oxadiazol-5-yl)hexanamide

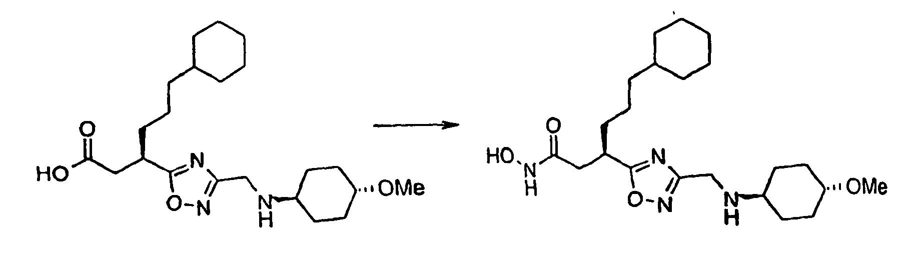

Method as for example 7 using (3*R*)-6-cyclohexyl-3-(3-{[(4-methoxycyclohexyl)amino]methyl}-1,2,4-oxadiazol-5-yl)hexanoic acid (preparation 105) (175mg, 0.34mmol) as starting material.
Purification: The crude material was purified on a silica column eluting with a solvent gradient of DCM: MeOH : NH₃ (97.5 : 2.5 : 0.25) gradually changing to (90 : 10 : 1) to afford the title compound as a gum (68mg).
¹H nmr: (CDCl₃) 0.82 (2H, m), 1.10-1.35 (10H, m),1.40-1.90 (13H, m), 2.60 (3H, m), 3.26 (4H, s+m), 3.59 (1 H, m), 3.91 (2H, s). Evidence of the presence of the cis-isomer of the cyclohexanamine.
MS : 424 (MH⁺)

### Example 48:

### methyl {[(5-{(1R)-4-cyclohexyl-1-[2-(hydroxyamino)-2-oxoethyl]butyl}-1,2,4-oxadiazol-3-yl)methyl]amino}acetate

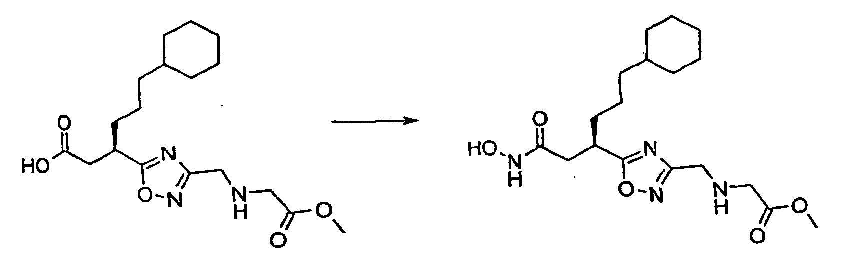

Method as for example 7 using (3*R*)-6-cyclohexyl-3-(3-{[(2-methoxy-2-oxoethyl)amino]methyl}-1,2,4-oxadiazol-5-yl)hexanoic acid (preparation 107) (135mg, 0.37mmol) as starting material. Purification: The crude material was purified on a silica column eluting with a solvent gradient of DCM : MeOH (100 : 0) gradually changing to (90 : 10) to afford the title compound as a colourless oil (30mg, 21%).
¹H nmr : (CDCl₃) 0.82 (2H, m), 1.10-1.40 (8H, m), 1.60-1.85 (7H, m), 2.55-2.79 (2H, m), 3.49 (2H, s), 3.59 (1H, m), 3.77 (3H, s), 3.93 (2H, s).
MS : 383 (MH⁺)
Accurate mass : Found 383.2300 (MH⁺), Calculated C₁₈H₃₀N₄O₅, 383.2289

### Example 49:

### (3R)-3-(3-{[(2-amino-2-oxoethyl)amino]methyl}-1,2,4-oxadiazol-5-yl)-6-cyclohexyl-N-hydroxyhexanamide

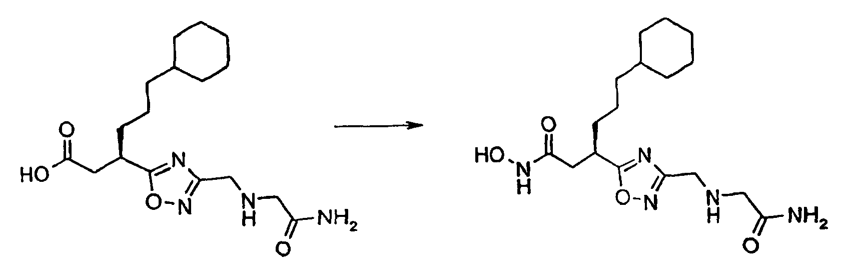

Method as for example 7 using (3*R*)-3-(3-{[(2-amino-2-oxoethyl)amino]methyl}-1,2,4-oxadiazol-5-yl)-6-cyclohexylhexanoic acid (preparation 109) (137mg, 0.35mmol) as starting material. Purification: The crude material was purified on a silica column eluting with a solvent gradient of DCM : MeOH (9 : 1) gradually changing to (1: 1) To afford the title compound as a white solid (15mg).
M.Pt. : 60-70°C
¹H nmr : (d₆DMSO) 0.80 (2H, m), 1.00-1.25 (8H, m), 1.50-1.70 (7H, m), 2.41 (2H, m), 3.08 (2H, s), 3.40 (1H, m), 3.79 (2H, s), 6.82 (1H, brs), 7.18 (1H, brs), 8.62 (1 H, brs), 10.38 (1H, brs).
MS : 390 (MNa⁺)

### Example 50:

### (3R)-6-cyclohexyl-N-hydroxy-3-{3-[(tetrahydro-2H-pyran-4-ylamino)methyl]-1,2,4-oxadiazol-5-yl}hexanamide

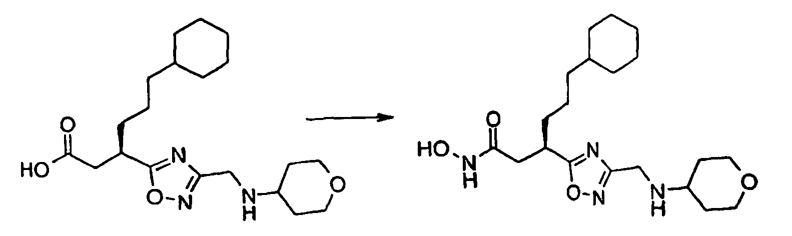

Method as for example 7 using (3*R*)-6-cyclohexyl-3-{3-[(tetrahydro-2*H*-pyran-4-ylamino)methyl]-1,2,4-oxadiazol-5-yl}hexanoic acid (preparation 111) (223mg, 0.45mmol) as starting material. Purification: The crude material was purified on a silica column eluting with a solvent gradient of DCM : MeOH : NH₃ (97.5 : 2.5 : 0.25) gradually changing to (90 : 10: 1). The material was azeotroped from Et₂O and then triturated with Et₂O to afford the title compound as a white solid, which was dried under reduced pressure (35.3mg).
M. Pt. : 108.9-110.3°C
¹H nmr : (CDCl₃) 0.81 (2H, m), 1.10-1.35 (8H, m),1.43 (2H, m), 1.60-1.80 (7H, m),1.82 (2H, brd), 2.55 (1H, m), 2.62 (1H, m), 2.78 (1H, m), 3.39 (2H, t), 3.59 (1H, m), 3.93 (2H, s), 3.98 (2H, d).
MS : 395 (MH⁺)
CHN : Found: C60.53%; H8.73%; N14.04%; C₂₀H₃₄N₄O₄ requires C60.89; H8.69%; N14.20%

### Example 51:

### (3R)-6-cyclohexyl-N-hydroxy-3-{3-[(1H-pyrazol-3-ylamino)methyl]-1,2,4-oxadiazol-5-yl}hexanamide

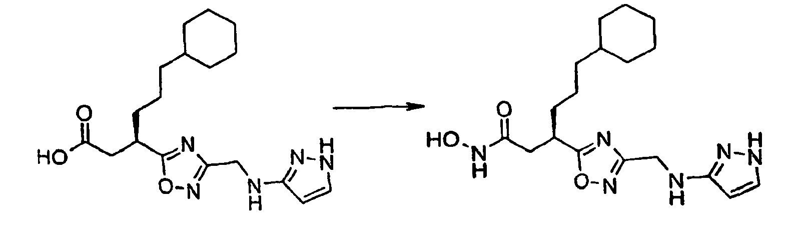

Method as for example 7 using (3*R*)-6-cyclohexyl-3-{3-[(1*H*-pyrazol-3-ylamino)methyl]-1,2,4-oxadiazol-5-yl}hexanoic acid (preparation 113) (132mg, 0.27mmol) as starting material. Purification: The crude material was purified on a silica column eluting with DCM : MeOH : NH₃ (90 : 10 : 1) to afford the title compound as a colourless oil (4.1 mg)
¹H nmr : (d₆DMSO) 0.79 (2H, m), 1.00-1.20 (8H, m), 1.50-1.65 (7H, m), 2.40 (2H, m), 3.40 (1H, m), 5.19 (2H, s), 5.24 (3H, s), 7.02 (1H, s).

### Example 52:

### (3R)-6-cyclohexyl-3-(3-{[(1-ethyl-1H-pyrazol-5-yl)amino]methyl}-1,2,4-oxadiazol-5-yl)-N-hydroxyhexanamide

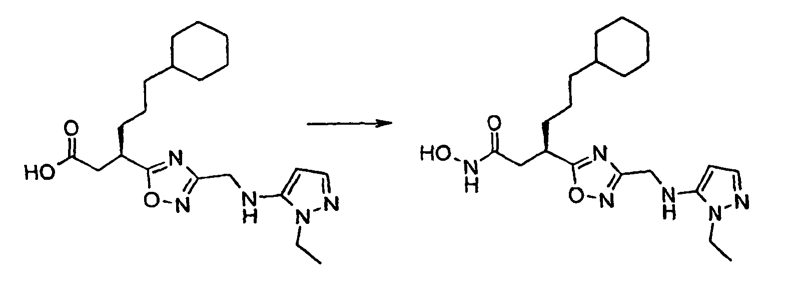

Method as for example 1 using (3*R*)-6-cyclohexyl-3-(3-{[(1-ethyl-1*H*-pyrazol-5-yl)amino]methyl}-1,2,4-oxadiazol-5-yl)hexanoic acid (preparation 115) (40mg, 0.10mmol) as starting material and NMM as the base.
Purification: The crude material was purified on a silica column eluting with DCM : MeOH : NH₃ (97 : 3 : 0.3) gradually changing to (90 : 10 : 1) to afford the title compound as a white foam (31mg).
¹H nmr : (CDCl₃) 0.79 (2H, m), 1.05-1.40 (11 H, m), 1.55-1.80 (7H, m), 2.52 (2H, brs), 3.59 (1H, m), 3.92 (2H, brs), 4.33 (2H, brs), 5.51 (1H, brs), 7.26 (1H, obs).
MS : 405 (MH⁺)
CHN : Found: C57.94%; H7.92%; N20.08%; C₂₀H₃₂N₆O₃. 0.5 H₂O requires C58.09; H8.04%; N20.32%

### Example 53:

### tert-butyl (2S)-2-(5-{(1R)-4-cyclohexyl-1-[2-(hydroxyamino)-2-oxoethyl]butyl}-1,2,4-oxadiazol-3-yl)-1-pyrrolidinecarboxylate

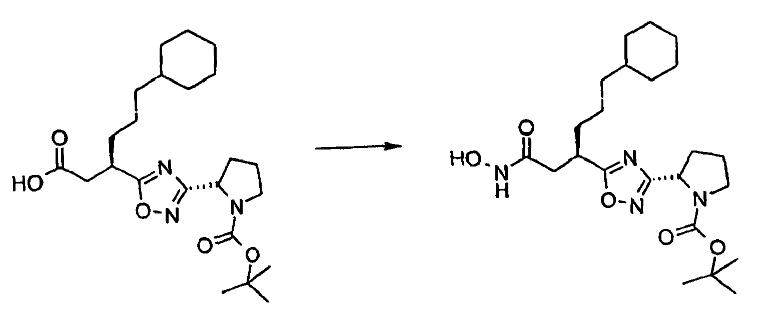

Method as for example 7 using (3*R*)-3-{3-[(2*S*)-1-(*tert*-butoxycarbonyl)pyrrolidinyl]-1,2,4-oxadiazol-5-yl}-6-cyclohexylhexanoic acid (preparation 120) (220mg, 0.50mmol) as starting material.
Purification: The crude material was purified on a silica column eluting with DCM : MeOH (95 : 5) to afford the title compound as a colourless oil (95mg).
¹H nmr : (d₆DMSO) 0.79 (2H, m), 1.10-1.40 (17H, m+s+m), 1.50-1.65 (7H, m), 1.70-1.95 (3H, m), 2.22 (1H, m), 2.40 (2H, m), 3.39 (3H, m), 4.81 (1H, brs), 8.78 (1H, brs), 10.42 (1H, brs).
MS : 473 (MNa⁺)

### Example 54:

### (3R)-6-cyclohexyl-N-hydroxy-3-{3-[(2S)-pyrrolidinyl]-1,2,4-oxadiazol-5-yl}hexanamide

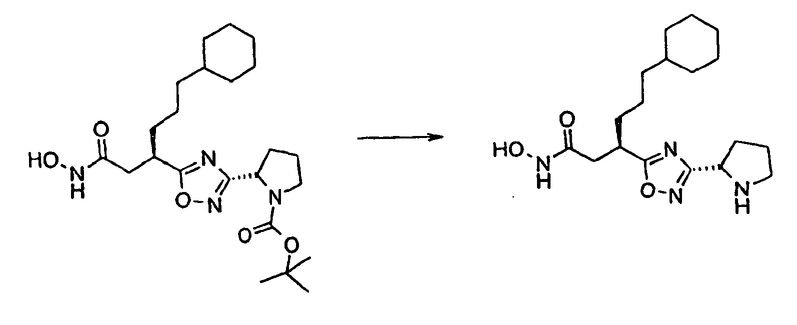

Method as for preparation 11 using *tert*-butyl (2*S*)-2-(5-{(1*R*)-4-cyclohexyl-1-[2-(hydroxyamino)-2-oxoethyl]butyl}-1,2,4-oxadiazol-3-yl)-1-pyrrolidinecarboxylate (example 53) (90mg, 0.2mmol) as starting material to afford the title compound as a colourless gum.
¹H nmr : (d₆DMSO) 0.80 (2H, m), 1.00-1.25 (8H, m), 1.50-1.70 (7H, m), 2.02 (4H, m), 2.40 (2H, m), 3.31 (2H, m), 4.48 (1H, m), 4.83 (1H, brs), 9.28 (1H, brs), 9.80 (1H, brs).
MS : 351 (MH⁺)

### Example 55:

### (3R)-6-cyclohexyl-N-hydroxy-3-{3-[(2S)-piperidinyl]-1,2,4-oxadiazol-5-yl}hexanamide

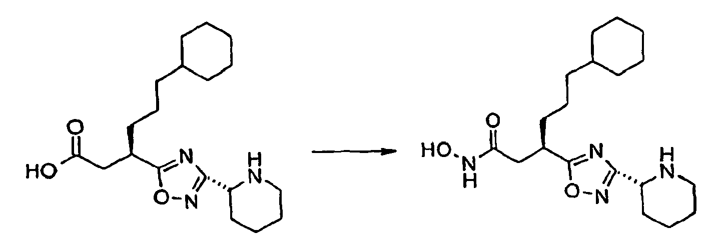

Method as for example 7 using (3*R*)-6-cyclohexyl-3-{3-[(2*S*)-piperidinyl]-1,2,4-oxadiazol-5-yl}hexanoic acid (preparation 123) (319mg, 0.83mmol) as starting material.
Purification: The crude material was purified on a silica column eluting with a solvent gradient of DCM : MeOH (100 : 0) gradually changing to (95: 5) to afford the title compound contaminated with imidazole. The material was dissolved in EtOAc and washed with H₂O (x2) followed by brine. The organic extract was dried over anhydrous MgSO₄ and filtered. The solvent was removed under reduced pressure to afford the title compound as a white foam.
¹H nmr : (d₆DMSO) 0.80 (2H, m), 1.00-1.30 (8H, m), 1.30-1.65 (11H, m), 1.74 (1H, m), 1.82 (1H, d), 2.45 (2H, obs), 2.60 (1H, t), 2.97 (1H, d), 3.40 (1H, m), 3.79 (1H, d), 8.61 (1H, brs), 10.38 (1H, brs).
MS : 365 (MH⁺)
CHN : Found: C60.88%; H8.82%; N15.06%; C₁₉H₃₂N₄O₃. 0.5H₂O requires C61.10; H8.91%; N15.00%

### Example 56:

### (3R)-6-cyclohexyl-3-{3-[(dimethylamino)methyl]-1,2,4-oxadiazol-5-yl}-N-hydroxyhexanamide

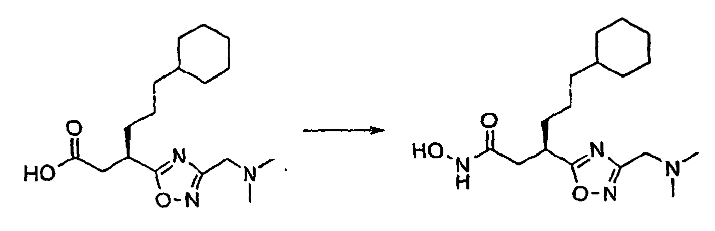

Method as for example 7 using (3*R*)-6-cyclohexyl-3-{3-[(dimethylamino)methyl]-1,2,4-oxadiazol-5-yl}hexanoic acid (Preparation 125) (495mg, 1.14mmol) as starting material.
Purification: The residue was purified on a silica column eluting with DCM : MeOH : NH₃ (90 : 10 : 1) to afford the title compound (90mg).
¹Hnmr (CD₃OD) : 0.85 (2H, m), 1.10-1.30 (8H, m), 1.60-1.80 (7H, m), 2.38 (6H, s), 2.51 (1H, dd), 2.61 (1H, dd), 3.54 (1H, m), 3.65 (2H, s).
MS : 339 (MH⁺)

### Example 57:

### (3R)-6-cyclohexyl-N-hydroxy-3-(3-{[(2-methoxyethyl)(methyl)amino]methyl}-1,2,4-oxadiazol-5-yl)hexanamide

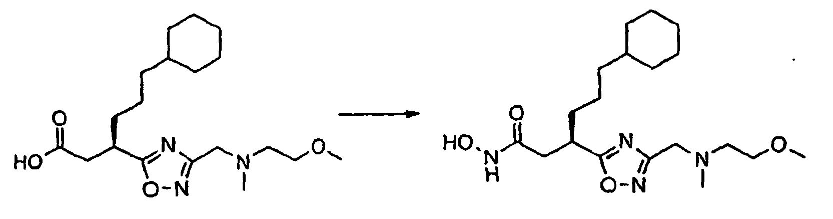

Method as for example 7 using (3*R*)-6-cyclohexyl-3-(3-{[(2-methoxyethyl)(methyl)amino]methyl}-1,2,4-oxadiazol-5-yl)hexanoic acid (preparation 127) (209mg, 0.57mmol) as starting material. Purification: The crude material was purified on a silica column eltuing with DCM : MeOH : NH₃ (90 : 10 : 1) to afford the title compound as a colourless gum (145mg, 66%).
¹Hnmr (CD₃OD) : 0.83 (2H, m), 1.10-1.30 (8H, m), 1.60-1.80 (7H, m), 2.37 (3H, s), 2.53 (1H, dd), 2.61 (1H, dd), 2.71 (2H, t), 3.36 (3H, s), 3.55 (3H, t+m), 3.78 (2H, s).
MS : 383 (MH⁺)

### Example 58:

### (3R)-6-cyclohexyl-3-(3-{[[2-(dimethylamino)ethyl](methyl)amino]methyl}-1,2,4-oxadiazol-5-yl)-N-hydroxyhexanamide

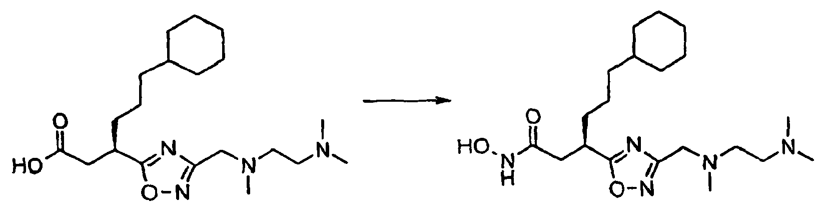

Method as for example 7 using (3*R*)-6-cyclohexyl-3-(3-{[[2-(dimethylamino)ethyl](methyl)amino]methyl}-1,2,4-oxadiazol-5-yl)hexanoic acid (preparation 129) (354mg, 0.58mmol) as starting material.
Purification: The crude material was purified on a silica column eluting with DCM : MeOH : NH₃ (90 : 10 : 1) to afford the title compound as a colourless oil (40mg).
¹Hnmr (d₆DMSO) : 0.78 (2H, m), 1.00-1.20 (8H, m), 1.50-1.70 (7H, m), 2.08 (6H, s), 2.19 (3H, s), 2.32 (2H, t), 2.42 (4H, m), 3.41 (1H, m), 3.65 (2H, s).
MS : 397 (M-H)

### Example 59:

### (3R)-3-(3-{[(2-amino-2-oxoethyl)(methyl)amino]methyl}-1,2,4-oxadiazol-5-yl)-6-cyclohexyl-N-hydroxyhexanamide

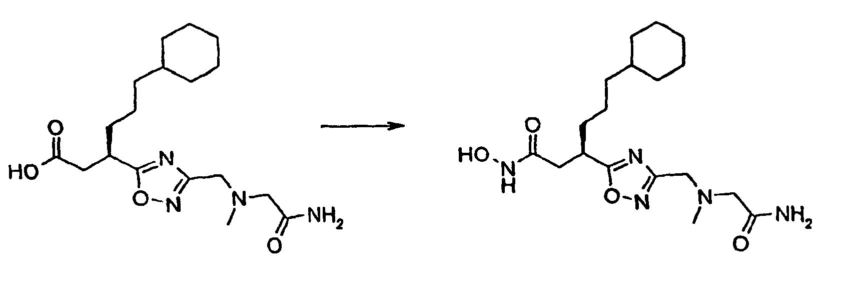

Method as for example 7 using (3*R*)-3-(3-{[(2-amino-2-oxoethyl)(methyl)amino]methyl}-1,2,4-oxadiazol-5-yl)-6-cyclohexylhexanoic acid (preparation 131) (223mg, 0.57mmol) as starting material.
Purification: The crude material was purified on a silica column eluting with DCM : MeOH (9 : 1) to afford the title compound as a colourless oil (74mg).
¹H nmr : (CD₃OD) 0.83 (2H, m), 1.10-1.30 (8H, m), 1.60-1.80 (7H, m), 2.39 (3H, s), 2.52 (1H, dd), 2.61 (1H, dd), 3.18 (2H, s), 3.56 (1H, m), 3.80 (2H, s) + imidazole
MS : 404 (MNa⁺)
CHN : Found: C55.09%; H8.03%; N18.63%; C₁₈H₃₁N₅O₄. 0.5 H₂O. 0.1 imidazole requires C55.33; H8.22%; N18.33%

### Example 60:

### (3R)-6-cyclohexyl-N-hydroxy-3-[3-({methyl[2-(methylamino)-2-oxoethyl]amino}methyl)-1,2,4-oxadiazol-5-yl]hexanamide

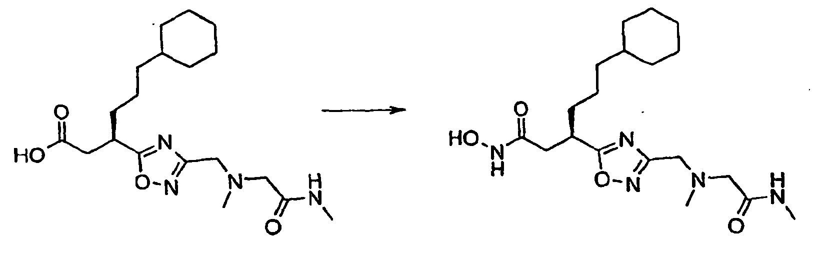

Method as for example 7 using (3*R*)-6-cyclohexyl-3-[3-({methyl[2-(methylamino)-2-oxoethyl]amino}methyl)-1,2,4-oxediazol-5-yl]hexanoic acid (preparation 135) (398mg, 0.95mmol) as starting material.
Purification: The crude material was purified on a silica column eluting with DCM : MeOH (19 : 1) to afford the title compound as a colourless gum (198mg, 52%).
¹H nmr: (d₆DMSO) 0.80 (2H, m), 1.00-1.25 (8H, m), 1.50-1.70 (7H, m), 2.23 (3H, s), 2.41 (2H, m), 2.60 (3H, d), 3.02 (2H, s), 3.41 (1H, m), 3.78 (2H, s), 7.58 (1H, brs), 8.63 (1H, brs), 10.39 (1H, brs).
MS : 418 (MNa⁺)
CHN : Found: C56.22%; H8.22%; N17.23%; C₁₉H₃₃N₅O₄. 0.5 H₂O requires C56.42; H8.47%; N17.31%

### Example 61:

### (3R)-6-cyclohexyl-3-(3-{[[2-(dimethylamino)-2-oxoethyl](methyl)amino]methyl}-1,2,4-oxadiazol-5-yl)-N-hydroxyhexanamide

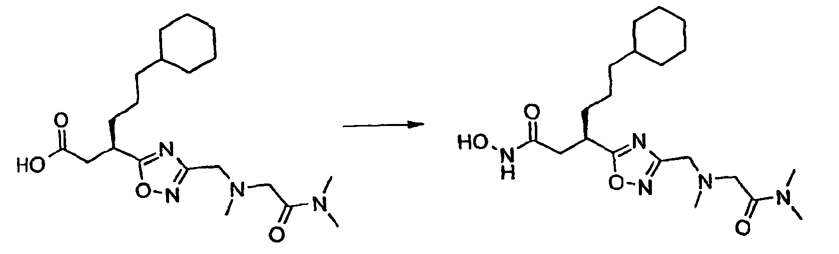

Method as for example 7 using (3*R*)-6-cyclohexyl-3-(3-{[[2-(dimethylamino)-2-oxoethyl](methyl)amino]methyl}-1,2,4-oxadiazol-5-yl)hexanoic acid (preparation 137) (403mg, 0.94mmol) as starting material.
Purification: The crude material was purified on a 2 silica columns eluting with DCM : MeOH (19 : 1) to afford the title compound as a colourless sticky gum (76mg).
¹H nmr : (d₆DMSO) 0.80 (2H, m), 1.00-1.25 (8H, m), 1.50-1.70 (7H, m), 2.27 (3H, s), 2.41 (2H, m), 2.80 (3H, s), 2.93 (3H, s), 3.27 (2H, s), 3.41 (1H, m), 3.78 (2H, s), 8.64 (1H, brs), 10.39 (1H, brs).
MS : 432 (MNa⁺)
CHN : Found: C56.58%; H8.56%; N16.46%; C₂₀H₃₅N₅O₄. 0.7 H₂O requires C56.91; H8.69%; N16.59%

### Example 62:

### (3R)-3-(3-{[bis(2-methoxyethyl)amino]methyl}-1,2,4-oxadiazol-5-yl)-6-cyclohexyl-N-hydroxyhexanamide

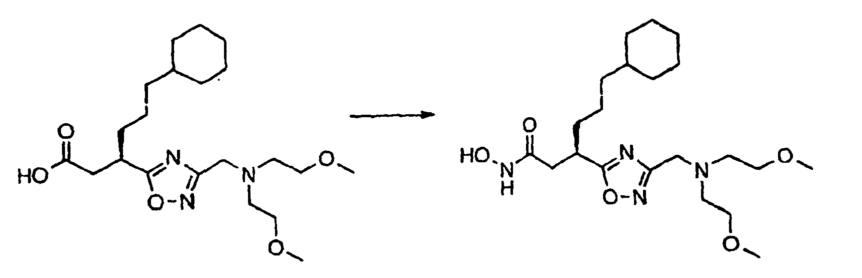

Method as for example 7 using (3*R*)-3-(3-{[bis(2-methoxyethyl)amino]methyl}-1,2,4-oxadiazol-5-yl)-6-cyclohexylhexanoic acid (preparation 139) (176mg, 0.42mmol) as starting material. Purification: The crude material was purififed on a silica column eluting with DCM : MeOH : NH₃ (90 : 10 : 1) to afford the title compound as a colourless oil (76mg).
¹Hnmr (d₆DMSO) : 0.79 (2H, m), 1.00-1.20 (8H, m), 1.50-1.70 (7H, m), 2.42 (2H, m), 2.69 (4H, t), 3.20 (6H, s), 3.19 (4H, t), 3.20 (1H, m), 3.80 (2H, s).

### Example 63:

### (3R)-6-cyclohexyl-N-hydroxy-3-[3-(1-pyrrolidinylmethyl)-1,2,4-oxadiazol-5-yl]hexonamide

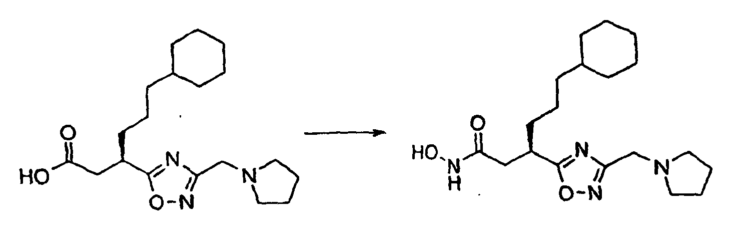

Method as for example 7 using (3*R*)-6-cyclohexyl-3-[3-(1-pyrrolidinylmethyl)-1,2,4-oxadiazol-5-yl]hexanoic acid (Preparation 141) (97mg, 0.24mmol).
Purification: The residue was purified a silica column eluting with a solvent gradient system of DCM : MeOH : NH₃ (95 : 5 : 0.5) gradually changing to (90 : 10 : 1) to afford the title compound (14mg).
¹Hnmr (CD₃OD) : 0.86 (2H, m), 1.05-1.35 (8H, m), 1.60-1.90 (11H, m), 2.52 (1H, dd), 2.60 (1H, dd), 2.72 (4H, m), 3.54 (1H, m), 3.83 (2H, s),
MS : 365 (MH⁺), 387 (MNa⁺)

### Example 64:

### (3R)-6-cyclohexyl-N-hydroxy-3-{3-[(4-hydroxy-1-piperidinyl)methyl]-1,2,4-oxadiazol-5-yl}hexanamide

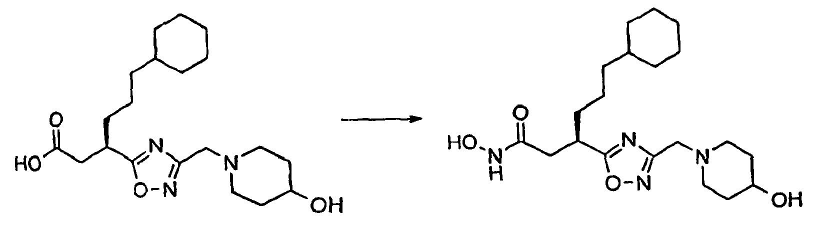

Method as for example 7 using (3*R*)-6-cyclohexyl-3-{3-[(4-hydroxy-1-piperidinyl)methyl]-1,2,4-oxadiazol-5-yl}hexanoic acid trifluoroacetate (Preparation 143) (414mg, 0.84mmol) as starting material.
Purifcation:The crude material was purified on a silica column eluting with DCM : MeOH : NH₃ (90 : 10 : 1). The residue was azeotroped from Et₂O to afford the title compound as a white foam (240mg).
M.Pt. : 85°C
¹Hnmr (CD₃OD) : 0.85 (2H, m), 1.05-1.35 (8H, m), 1.55-1.80 (9H, m), 1.88 (2H, m), 2.42 (2H, m), 2.54 (1H, dd), 2.61 (1H, dd), 2.93 (2H, m), 3.55 (1H, m), 3.63 (1H, m), 3.76 (2H, s)
MS : 395 (MH⁺), 417 (MNa⁺)

### Example 65:

### (3R)-6-cyclohexyl-N-hydroxy-3-[3-(4-morpholinylmethyl)-1,2,4-oxadiazol-5-yl]hexanamide

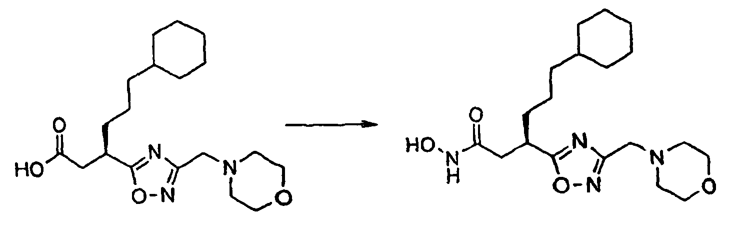

Method as for example 7 using (3*R*)-8-cyclohexyl-3-[3-(4-morpholinylmethyl)-1,2,4-oxadiazol-5-yl]hexanoic acid (Preparation 145) (120mg, 0.33mmol) as starting material.
Purification: The crude material was purified on a silica column eluting with DCM : MeOH : NH₃ (90 : 10 : 1). The residue was triturated with Et₂O to afford the title compound as a white solid (29mg).
M.Pt. : 138-139°C
¹Hnmr (d₆DMSO) : 0.77 (2H, m), 1.00-1.25 (8H, m), 1.50-1.70 (7H, m), 2.35-2.55 (6H, m), 3.42 (1H, m), 3.53 (4H, m), 3.60 (2H, s), 8.74 (1H, s), 10.45 (1H, s)
MS : 381 (MH⁺)

### Example 66:

### (3R)-6-cyclohexyl-N-hydroxy-3-{3-[(4-methyl-1-piperazinyl)methyl]-1,2,4-oxadiazol-5-yl}hexanamide

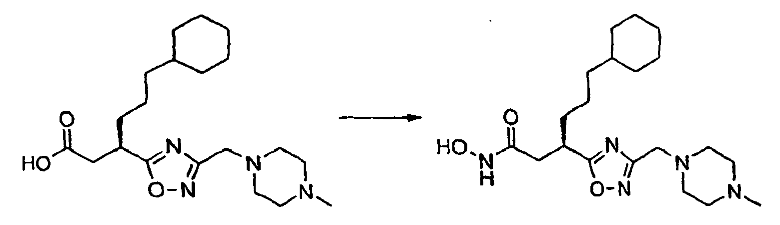

Method as for example 7 using (3*R*)-6-cyclohexyl-3-{3-[(4-methyl-1-piperazinyl)methyl]-1,2,4-oxadiazol-5-yl}hexanoic acid trifluoroacetate (Preparation 147) (343mg, 0.91mmol) as starting material.
Purification: The crude material was purified on a silica column eluting with a gradient system of DCM : MeOH : NH₃ (95 : 5 : 0.5) gradually changing to (80 : 20 : 1) to afford the title compound as a white gum (140mg).
¹Hnmr (CD₃OD) : 0.87 (2H, m), 1.10-1.40 (8H, m), 1.60-1.80 (7H, m), 2.50-3.45 (13H, m), 3.52 (1H, m), 3.84 (2H, s)
MS : 394 (MH⁺)

### Example 67:

### (3R)-6-cyclohexyl-N-hydroxy-3-[3-(1H-1,2,4-tnazol-1-ylmethyl)-1,2,4-oxadiazol-5-yl]hexanamide

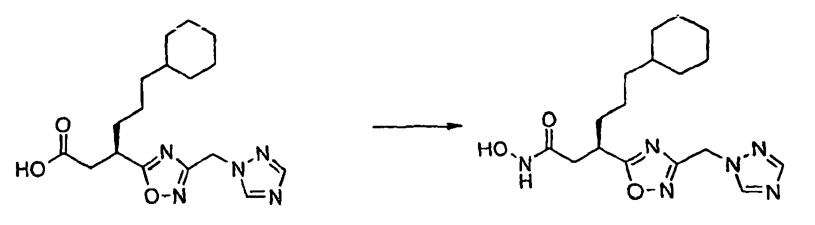

Method as for example 7 using (3*R*)-6-cyclohexyl-3-[3-(1*H*-1,2,4-triazol-1-ylmethyl)-1,2,4-oxadiazol-5-yl]hexanoic acid (Preparation 150) (174mg, 0.50mmol) as starting material to afford the title compound as a white solid (68mg).
M.Pt. : 118-120°C
¹Hnmr (d₆DMSO) : 0.75 (2H, m), 1.0-1.20 (8H, m), 1.45-1.65 (7H, m), 2.35-2.55 (2H, m), 3.40 (1H, m), 5.61 (2H, s), 7.98 (1H, s), 8.63 (1H, s), 8.76 (1H, s), 10.45 (1H, s)

### Example 68:

### (3R)-6-cyclohexyl-N-hydroxy-3-{4-[(isopropylamino)methyl]-1,3-oxazol-2-yl}hexanamide

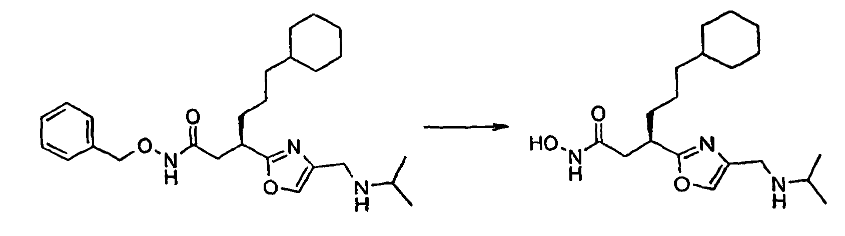

A solution of (3*R*)-*N*-(benzyloxy)-6-cyclohexyl-3-{4-[(isopropylamino)methyl]-1,3-oxazol-2-yl}hexanamide (preparation 157) (85mg, 0.20mmol) in EtOH (5ml) was treated with HCO₂NH₄ (63mg, 1.00mmol) and Pd(OH)₂ (20mg) and the reaction mixture heated at 43°C for 18 hours. 2 further portions of HCO₂NH₄ (30mg + 30mg) and Pd(OH)₂ (10mg + 30mg) were added over a period of 4 hours. The catalyst was filtered off and washed with EtOH. The solvent was removed under reduced pressure. The crude material was purified on a silica column eluting with DCM : MeOH : NH₃ (90 : 10 : 1) to afford the title compound.
¹H nmr: (d₆DMSO) 0.79 (2H, m), 0.96 (6H, d), 1.00-1.20 (8H, m), 1.50-1.65 (7H, m), 2.24 (1H, m), 2.40 (1H, m), 3.70 (1H, m), 3.20 (1H, obs), 3.52 (2H, s), 7.68 (1H, s).
MS : 352 (MH⁺)
Acc. Mass : Found 374.2415 (MNH₄⁺); Calculated C₁₉H₃₃N₃O₃ 374.2420 (MNH₄⁺)

### Example 69:

### (3R)-6-cyclohexyl-3-{4-[(cyclopentylamino)methyl]-1,3-oxazol-2-yl}-N-hydroxyhexanamide

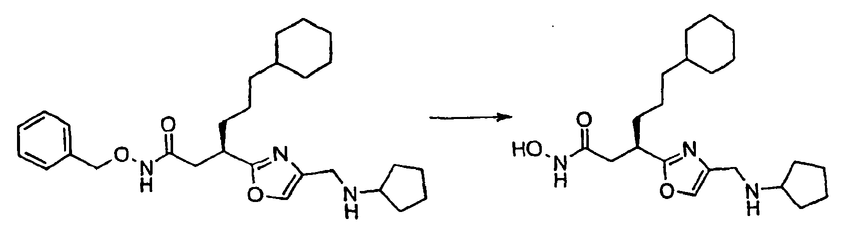

Method and purification as for example 67 using (3*R*)-*N-*(benzyloxy)-6-cyclohexyl-3-{4-[(cyclopentylamino)methyl]-1,3-oxazol-2-yl}hexanamide (preparation 158) (112mg, 023mmol) as starting material to afford the title compound as a white solid (30mg, 35%).
M.Pt. : 124-127°C
¹H nmr : (d₆DMSO) 0.80 (2H, m), 1.00-1.20 (8H, m+Et₂O), 1.28 (2H, m), 1.42 (2H, m), 1.50-1.75 (11H, m), 2.25 (1H, m), 2.39 (1H, m), 2.99 (1H, m), 3.20 (1H, m), 3.49 (2H, s), 7.64 (1H, s).
MS : 378 (MH⁺)
CHN : Found: C66.40%; H9.48%; N11.08%; C₂₁H₃₅N₃O₃. 0.1 H₂O requires C66.49; H9.35%; N11.08%

### Example 70:

### (3R)-6-cyclohexyl-N-hydroxy-3-[4-(4-morpholinylmethyl)-1,3-oxazol-2-yl]hexanamide

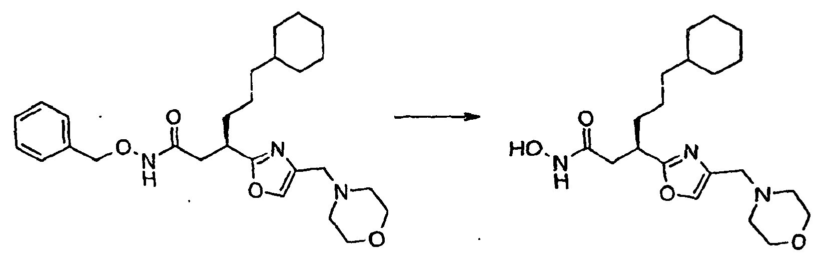

Method as for example 67 using (3*R*)-*N*-(benzyloxy)-6-cyclohexyl-3-[4-(4-morpholinylmethyl)-1,3-oxazol-2-yl]hexanamide (preparation 159) (130mg, 0.28mmol) as starting material. Purification: The crude material was purified on a silica column eluting with a solvent gradient of DCM : MeOH : NH₃ (98 : 2 : 0.2) gradually changing to (90 : 10 : 1) to afford the title compound as a white solid (32mg, 30%).
M.Pt. : 117-119°C
¹H nmr : (d₆DMSO) 0.79 (2H, m), 1.00-1.20 (8H, m), 1.50-1.65 (7H, m), 2.25 (1H, m), 2.26 (1H, m), 2.38 (4H, t), 2.41 (1H, m), 3.20 (1H, m), 3.53 (4H, t), 3.90 (2H, d), 7.77 (1H, s), 8.58 (1H, brs), 10.28 (1H, brs).
MS : 402 (MNa⁺)
CHN : Found: C63.09%; H8.80%; N11.01%; C₂₀H₃₃N₃O₄ requires C63.30; H8.76%; N11.07%

### Example 71:

### (3R)-6-cyclohexyl-N-hydroxy-3-{5-methyl-4-[(tetrahydro-2H-pyran-4-ylamino)methyl]-1,3-oxazol-2-yl}hexanamide

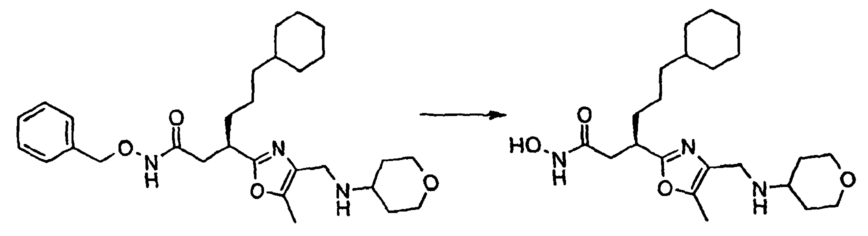

Method as for example 67 using (3*R*)-*N*-(benzyloxy)-6-cyclohexyl-3-{5-methyl-4-[(tetrahydro-2*H-*pyran-4-ylamino)methyl]-1,3-oxazol-2-yl}hexanamide (preparation 161) (200mg, 0.40mmol) as starting material.
Purification: The crude material was purified on a silica column eluting with DCM : MeOH : NH₃ (95 : 5 : 0.5) to afford the title compound as a white solid (28mg, 17%).
M.Pt. : 108-110°C
¹H nmr : (d₆DMSO) 0.79 (2H, m), 1.05-1.30 (10H, m), 1.50-1.65 (7H, m), 1.70 (2H, brd), 2.20 (3H, s), 2.37 (1H, m), 2.50 (2H, obs), 3.15 (1H, obs), 3.21 (2H, t), 3.46 (2H, s), 3.79 (2H, m), 8.58 (1H, brs), 10.27 (1H, brs).
MS : 408 (MH⁺)
CHN : Found: C64.37%; H9.25%; N10.27%; C₂₂H₃₇N₃O₄. 0.1 H₂O requires C64.55; H9.16%; N10.26%

### Example 72:

### (3R)-6-cyclohexyl-N-hydroxy-3-[5-methyl-4-(4-morpholinylmethyl)-1,3-oxazol-2-yl]hexanamide

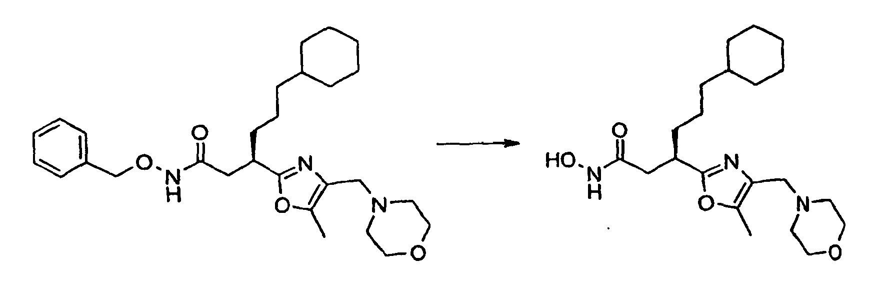

Method as for example 67 using (3*R*)-*N*-(benzyloxy)-6-cyclohexyl-3-[5-methyl-4-(4-morpholinylmethyl)-1,3-oxazol-2-yl]hexanamide (preparation 162) (154mg, 0.32mmol) as starting material.
Purification: 3 silica columns were required to purify the crude material. First column eluting with a solvent gradient of DCM : MeOH : NH₃ (100 : 0 : 0) gradually changing to (95 : 5 : 0.5). The second column eluting with toluene : EtOAc (9 : 1) and the final column eluting with DCM : MeOH : NH₃ (95 : 5 : 0.5) to afford the title compound as a white foam (50mg, 40%).
¹H nmr: (d₆DMSO) 0.79 (2H, m), 1.00-1.20 (8H, m), 1.50-1.65 (7H, m), 2.21 (3H, s), 2.35 (5H, m), 2.50 (1H, obs), 3.14 (1H, obs)3.22 (2H, s), 3.51 (4H, m), 8.58 (1H, brs), 10.28 (1H, brs).
MS : 394 (MH⁺)
CHN : Found: C63.15%; H9.20%; N10.57%; C₂₁H₃₅N₃O₄. 0.2 H₂O requires C63.51; H8.98%; N10.58%

### Preparation 1: (1Z)-N'-hydroxy-2-[(methylsulfonyl)amino]ethanimidamide

### (a) N-(Cyanomethyl)methanesulfonamide (known compound, WO 92/02521)

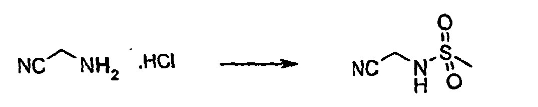

N-methyl morpholine (1.12Kg, 11.1mol) was added dropwise over 30 min to a stirred suspension of aminoacetonitrile.hydrochloride (500g, 5.4mol) in dichloromethane (2.6litres), between 25 and 28°C. The mixture was stirred at ambient temperature for 30 min. Methanesulfonyl chloride (619g, 5.4mol) was added dropwise over 60 min, maintaining the temperature between 15 and 22°C. The mixture was stirred at ambient temperature for 45 min and then cooled to 12°C. The mixture was filtered and the filtrate concentrated *in vacuo* to a brown suspension. Acetone (2 litres) was added and the mixture was cooled to 10°C and then filtered. Silica (1Kg) was added to the filtrate, which was then filtered through a plug of silica (1Kg). The residue was washed with acetone (2x2.5litres) and the combined filtrate was concentrated *in vacuo* to afford the title compound as a brown oil (725g, quantitative).

### (b) (1Z)-N'-hydroxy-2-[(methylsulfonyl)amino]ethanimidamide

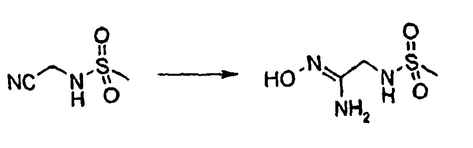

METHOD A. A solution of 50% aqueous hydroxylamine (367g, 5.4mol) was added over 60 min to a solution of *N*-(cyanomethyl)methanesulfonamide (725g, 5.4mol) in EtOH (3.6litres), between 25 and 35°C. The mixture was stirred at ambient temperature for 60 min and then cooled to 5°C. The mixture was filtered and the residue washed with EtOH (3x250ml) and then dried by suction overnight to afford the title compound as white crystals (781g, 87%).

Method B. A solution of *N*-(cyanomethyl)methanesulfonamide (WO 92102521) (10.85g, 81.0mmol) in EtOH (370ml) was treated with hydroxylamine.HCl (5.63g, 81.0mmol) followed by a solution of aqueous NaOH (3.24g, 81.0mmol in125ml) and stirred at room temperature, under a nitrogen atmosphere for 20 hours. The solvent was removed under reduced pressure. The solid was dissolved in hot EtOH and the solid NaCl was filtered off. A solid began to crystallise out after 1 hour. The solid was filtered off and washed with Et₂O to afford the title compound as white crystals (9.77g, 72%).
¹H nmr : (d₆DMSO) 2.87 (3H, s), 3.49 (2H, s), 5.22 (2H, brs), 7.09 (1H, brs), 9.00 (1H, s)
MS : 190 (MNa⁺)
CHN : Found: C21.46%; H5.41%; N24.58%; C₃H₉N₃O₃S. 0.1 H₂O requires C21.32%; H5.49%; N24.87%

### Preparation 2: tert-butyl (5Z,9R)-5-amino-9-(3-cyclohexylpropyl)-8-oxo-7-oxa-2-thia-3,6-diazaundec-5-en-11-oate 2,2-dioxide

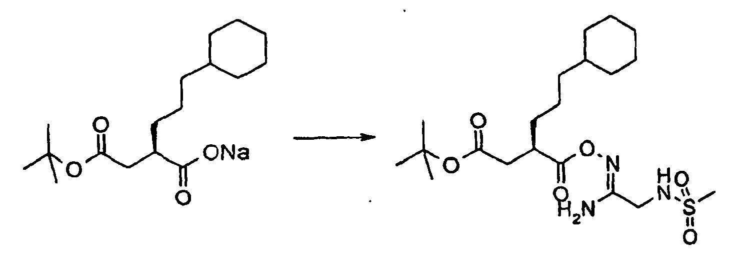

Method A. Sodium (2*R*)-2-(2-*tert*-butoxy-2-oxoethyl)-5-cyclohexylpentanoate (Preparation 167) (18.68g, 58.4mmol) was partitioned between 10% citric acid solution (190ml) and EtOAc (190ml). The organic layer was washed with brine, dried over anhydrous MgSO₄, filtered and the solvent removed under reduced pressure to afford the free acid as a colourless oil. A solution of the free acid in DCM (185ml) was treated CDI (9.46g, 58.4mmol) and the reaction mixture was stirred at room temperature for 1.5 hours. (12)-*N*'-hydroxy-2-[(methylsulfonyl)amino]ethanimidamide (preparation 1) (9.75g, 58.4mmol) was added portionwise but required the addition of DMF (50ml) to dissolve the solid. The reaction mixture was stirred at room temperature for 18 hours. The solvents were removed under reduced pressure. The residue was dissolved on EtOAc (500ml) and washed with water (2x500ml) and brine, dried over anhydrous MgSO₄, filtered and the solvent removed under reduced pressure to afford the title compound as a white solid (26.3g, ~100%).

METHOD B. Sodium (2*R*)-2-(2-*tert*-butoxy-2-oxoethyl)-5-cyclohexylpentanoate (Preparation 167) (50.0g, 0.16mol) was partitioned between 10% citric acid solution (500ml) and dichloromethane (500ml). The organic layer was separated, washed with demineralised water (500ml), and then dried by azeotropic distillation at constant volume of dichloromethane. The mixture was allowed to cool to ambient temperature and then CDI (25.3g, 0.16mol) was added portionwise over 2 min, under nitrogen. The mixture was stirred at ambient temperature for 30 min and then (1*Z*)-*N*'-hydroxy-2-[(methylsulfonyl)amino]ethanimidamide (preparation 1) (26.1g, 0.16mol) was added in one portion. The mixture was stirred at ambient temperature for 18 hours and then filtered. The filtrate was washed with water (2x500ml), 10% aqueous citric acid solution (500ml) and water (500ml) and then concentrated *in vacuo* to afford the title compound as a white solid (58.3g, 83%).
¹H nmr : (d₆DMSO) 0.81 (2H, m), 1.00-1.30 (9H, m), 1.37 (9H, s), 1.40-1.65 (6H, m), 2.39 (1H, dd), 2.55 (1H, dd), 2.79 (1H, m), 2.94 (3H, s), 3.62 (2H, s), 6.19 (2H, brs), 7.30 (1H, brs)
MS : 470 (MNa⁺)

### Preparation 3:

### tert-butyl (3R)-6-cyclohexyl-3-(3-{[(methylsulfonyl)amino]methyl}-1,2,4-oxadiazol-5-yl)hexanoate

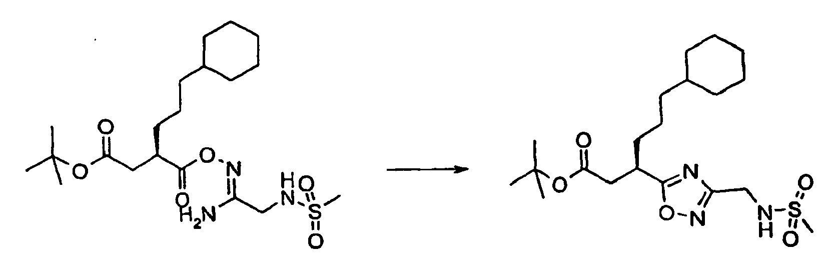

*METHOD A*. *tert*-butyl (5*Z*,9*R*)-5-amino-9-(3-cyclohexylpropyl)-8-oxo-7-oxa-2-thia-3,6-diazaundec-5-en-11-oate 2,2-dioxide (preparation 2) (26.0g, 58.2mmol) was heated at 130°C in xylene (500ml) under a nitrogen atmosphere for 20 hours. The crude reaction mixture was purified on a silica column diluted at first by pentane and eluting the column with a solvent gradient of DCM : MeOH (100 : 0) gradually changing to (90 : 10). Mixed fractions were combined and the solvent removed under reduced pressure to give a brown residue. This was triturated with pentane and the solid filtered off to afford the title compound as a white solid (9.43g, 38%).

METHOD B. A stirred suspension of *tert*-butyl (5*Z*,9*R*)-5-amino-9-(3-cyclohexylpropyl)-8-oxo-7-oxa-2-thia-3,6-diazaundec-5-en-11-oate 2,2-dioxide (preparation 2) (50.0g, 0.11mol) and basic aluminium oxide (150g) in toluene was heated to reflux for 5 hours, under nitrogen. Basic aluminium oxide (50g) was added and the mixture was held at reflux for a further 1 hour. The mixture was allowed to cool to ambient temperature and then filtered. The residue was washed with toluene (100ml) and the combined filtrate was concentrated *in vacuo* to afford the title compound as a white solid (24.0g, 50%).
¹H nmr : (CD₃OD) 0.83 (2H, m), 1.10-1.30 (9H, m), 1.38 (9H, s), 1.60-1.75 (7H, m), 2.67 (1 H, dd), 2.79 (1H, dd), 2.98 (3H, s), 3.42 (1H, m), 4.39 (2H, s)
MS: 452 (MNa⁺)

### Alternative synthesis:

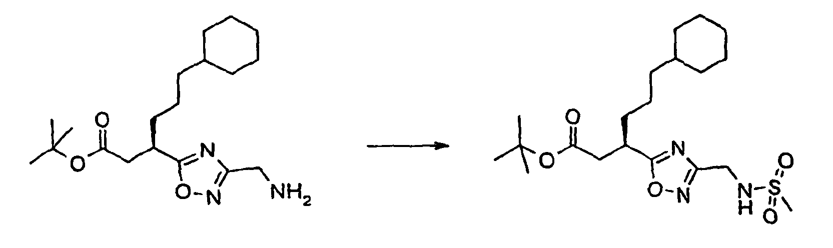

A solution of tert-butyl (3R)-3-[3-(aminomethyl)-1,2,4-oxadiazol-5-yl]-6-cyclohexylhexanoate (preparation 18) (1.0g, 2.85mmol) in pyridine (10ml) at 0°C was treated with methanesulphonylchloride (221 µl, 2.85mmol) and stirred under a nitrogen atmosphere for 5 hours. The solvent was removed under reduced pressure. The residue was dissolved in ethyl acetate and washed with 1M HCl followed by brine. The organic extract was dried over anhydrous MgSO₄, filtered and the solvent removed under reduced pressure. The residue was purified on a silica column eluting with a gradient solvent system EtOAc : pentane (0 : 100) gradually changing to (35: 55) to afford the title compound as a pale yellow oil (1.22g, 98%).
¹H nmr : (CDCl₃) 0.84 (2H, m), 1.10-1.35 (8H, m), 1.40 (9H, s), 1.60-1.80 (7H, m). 2.62 (1H, dd), 2.79 (1H, dd), 2.99 (3H, s), 3.42 (1H, m), 4.45 (2H, d), 4.82 (1H, s).
MS : 452 (MNa⁺)

### Preparation 4:

### (3R)-6-cyclohexyl-3-(3-{[(methylsulfonyl)amino]methyl}-1,2,4-oxadiazol-5-yl)hexanoic acid

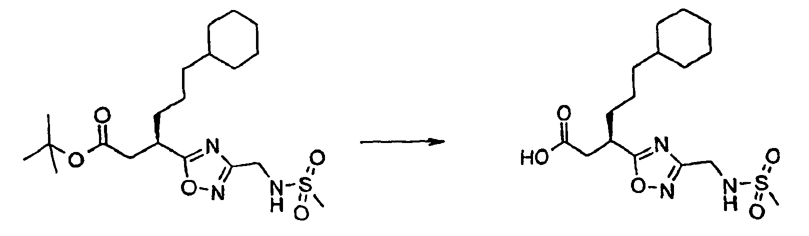

METHOD A. A solution of *tert*-butyl (3*R*)-6-cyclohexyl-3-(3-{[(methylsulfonyl)amino]methyl}-1,2,4-oxadiazol-5-yl)hexanoate (preparation 3) (9.36g, 21.8mmol) in toluene (100ml) was treated with TFA (50ml) and stirred under a nitrogen atmosphere, at room temperature for 20 hours. The solvent was removed under reduced pressure and azeotroped with EtOAc. The residue was dissolved in EtOAc (250ml) and washed with H₂O followed by brine. The organic layer was dried over anhydrous MgSO₄, filtered and the solvent was removed under reduced pressure. The solid was triturated with Et₂O, filtered off and dried under reduced pressure to afford the title compound as an off-white solid (7.06g, 87%).

METHOD B. Trifluoroacetic acid (48ml) was added in one portion to a stirred solution of *tert-*butyl (3*R*)-6-cyclohexyl-3-(3-{[(methylsulfonyl)amino]methyl}-1,2,4-oxadiazol-5-yl)hexanoate (preparation 3) (24.0g, 56mmol) in toluene (120ml), under nitrogen. The mixture was stirred at ambient temperature for 20 hours and then concentrated in vacuo to a yellow oil. Toluene (100ml) was added and the mixture was filtered. The residue was washed with toluene (2x20ml) and then dried *in vacuo* at 45°C to afford the title compound as an off-white solid (16.4g, 78%).
¹H nmr : (CD₃OD) 0.83 (2H, m), 1.10-1.35 (8H, m), 1.60-1.80 (7H, m), 2.75 (1H, dd), 2.85 (1H, dd), 2.97 (3H, s), 3.48 (1H, m), 4.39 (2H, s)
MS : 396 (MNa⁺)
CHN : Found: C51.45%; H7.29%; N11.20%; C₁₆H₂₇N₃O₅S requires C51.46%; H7.29%; N11.25%

### Preparation 5:

### tert-butyl (3R)-6-cyclohexyl-3-{3-[(methylamino)methyl]-1,2,4-oxadiazol-5-yl}hexanoate

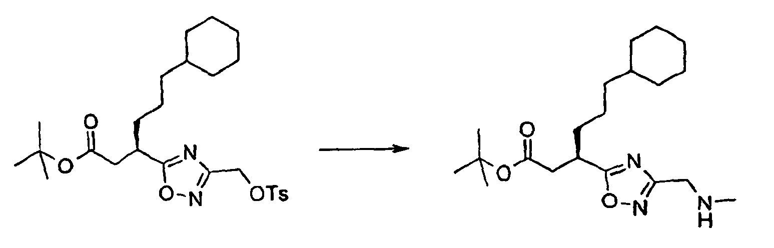

A solution of *tert*-butyl (3*R*)-6-cyclohexyl-3-[3-({[(4-methylphenyl)suffonyl]oxy}methyl)-1,2,4-oxadiazol-5-yl]hexanoate (preparation 177) (492mg, 0.97mmol) in THF (1ml) was treated with a solution of 2M methylamine in THF (7ml, 14.4mmol) and stirred at 40°C in a sealed vessel for 75 minutes. The reaction mixture was allowed to cool to room temprature and dissolved in EtOAc (100ml) which was washed with sat. NaHCO₃ (100ml) solution followed by brine. The organic extract was dried over MgSO₄, filtered and the solvent was removed under reduced pressure to afford the title compound as a colourless oil (337mg, 95%).
¹H nmr : (CDCl₃) 0.82 (2H, m), 1.10-1.35 (8H, m), 1.39 (9H, s), 1.55-1.80 (7H, m), 2.43 (3H, s), 2.60 (1H, dd), 2.79 (1H, dd), 3.43 (1H, m), 3.83 (2H, s).
MS : 366 (MH⁺)
CHN : Found: C65.32%; H9.67%; N11.22%: C₂₀H₃₅N₃O₃ requires C65.72%; H9.65%; N11.50%

### Preparation 6:

### tert-butyl (3R)-6-cyclohexyl-3-(3-{[methyl(methylsulfonyl)amino]methyl}-1,2,4-oxadiazol-5-yl)hexanoate

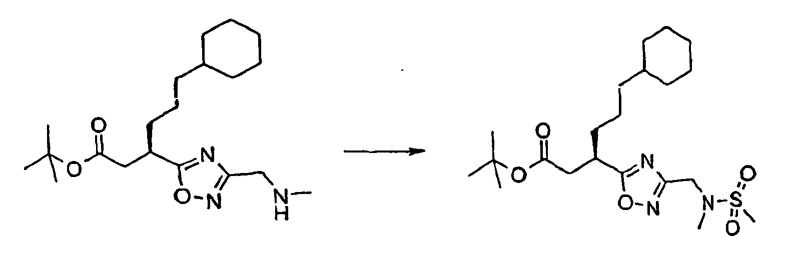

A solution of *tert*-butyl (3*R*)-6-cyclchexyl-3-{3-[(methylemino)methyl]-1,2,4-oxediazol-5-yl}hexanoate (preparation 5) (300mg, 0.82mmol) in pyridine (2ml) in a water bath was treated with methanesulphonylchloride (64µl, 0.82mmol) and stirred under a nitrogen atmosphere for 20 minutes. The solvent was removed under reduced pressure. The residue was dissolved in EtOAc and washed with 2M HCl followed by brine. The organic extract was dried over MgSO₄, filtered and the solvent removed under reduced pressure. The residue was purified on a silica column eluting with a solvent gradient of EtOAc : pentane (5 : 95) gradually changing to (30 : 70) to afford the title compound as a colourless oil (273mg, 75%).
¹H nmr : (CDCl₃) 0.84 (2H, m), 1.10-1.35 (8H, m), 1.39 (9H, s), 1.60-1.80 (7H, m), 2.61 (1H, dd), 2.79 (1H, dd), 2.93 (3H, s), 2.97 (3H, s), 3.43 (1H, m), 3.59 (2H, s).
MS : 466 (MNa⁺)
CHN : Found: C57.03%; H8.43%; N9.39%; C₂₁H₃₇N₃O₅S requires C56.86%; H8.41%; N9.47%

### Preparation 7:

### (3R)-6-cyclohexyl-3-(3-{[methyl(methylsulfonyl)amino]methyl}-1,2,4-oxadiazol-5-yl)hexanoic acid

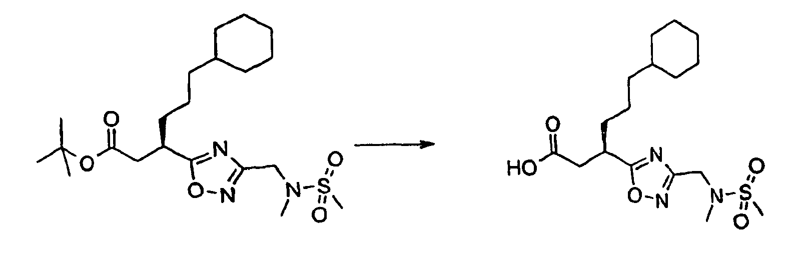

A solution of *tert*-butyl (3*R*)-6-cyclohexyl-3-(3-{[methyl(methylsulfonyl)amino]methyl}-1,2,4-oxadiazol-5-yl)hexanoate (preparation 6) (264mg, 0.60mmol) in 4M HCl in dioxan (2ml) was stirred at room temperature for 20 hours. Further 4M HCl in dioxan (2ml) was added and stirred for a further 3 hours. A few drops of concentrated HCl was added and the reaction mixture was stirred at room temperature for 18 hours. The solvent was removed under reduced pressure to afford the title compound (245mg - contained some dioxan as impurity).
¹H nmr : (CDCl₃) 0.84 (2H, m), 1.10-1.35 (8H, m), 1.60-1.85 (7H, m), 2.61 (1H, dd), 2.79 (1H, dd), 2.88-2.99 (7H, m), 3.47 (1H, m), 4.58 (2H, s).
MS : 386 (M-H)
CHN : Found: C50.82%; H7.45%; N9.93%; C₁₇H₂₅N₃O₅S. 0.75 H₂O. 0.15 dioxan requires C51.03%; H7.71%; N10.14%

### Preparation 8:

### tert-butyl (3R)-6-cyclohexyl-3-(3-{[(ethylsulfonyl)amino]methyl}-1,2,4-oxadiazol-5-yl)hexanoate

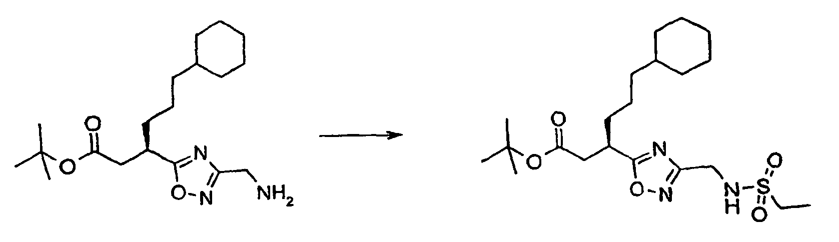

Method the same as for Preparation 6 using tert-butyl (3R)-3-[3-(aminomethyl)-1,2,4-oxadiazol-5-yl]-6-cyclohexylhexanoate (preparation 18) (200mg, 0.57mmol) and ethanesulphonylchloride (54µl, 0.57mmol) as starting materials. The reaction was carried out at 0°C.
Purification: Crude material was purified on a silica column eluting with a solvent gradient of DCM : MeOH (100 : 0) gradually changing to (90 :10) to yield the title compound as a colourless oil (190mg, 90%).
¹H nmr : (CDCl₃) 0.84 (2H, m), 1.05-1.35 (11H, m), 1.40 (9H, s), 1.60-1.80 (7H, m), 2.61 (1H, dd), 2.79 (1H, dd), 3.09 (2H, q), 3.42 (1H, m), 4.43 (2H, d), 4.73 (1H, brs).
MS : 466 (MNa⁺)
CHN : Found: C57.14%; H8.48%; N9.47%; C₂₁H₃₇N₃O₅S requires C56.86%; H8.41%; N9.47%

### Preparation 9:

### (3R)-6-cyclohexyl-3-(3-{[(ethylsulfonyl)amino]methyl}-1,2,4-oxadiazol-5-yl)hexanoic acid

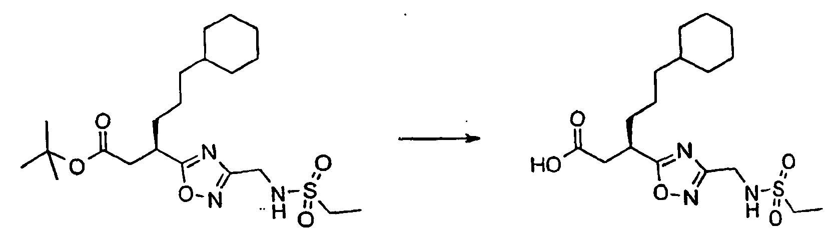

Method same as for preparation 7 using *tert*-butyl (3*R*)-6-cyclohexyl-3-(3-{[(ethylsulfonyl)amino]methyl}-1,2,4-oxadiazol-5-yl)hexanoate (preparation 8) (174mg, 0.40mmol) as starting material. After 24 hours the reaction had not gone to completion. The solvent was removed under reduced pressure. DCM (4ml) and TFA (2ml) were added and the reaction mixture was stirred at room temperature for 2 hours. The solvent was removed under reduced pressure and azeotroped with DCM (x3) and Et₂O. The residue was dissolved in EtOAc and washed with H₂O and brine. The organic extracts were dried over MgSO₄, filtered and the solvent removed under reduced pressure to afford the title compound as a colourless oil (148mg, 96%).
¹H nmr : (CDCl₃) 0.81 (2H, m), 1.05-1.35 (11H, m), 1.60-1.80 (7H, m), 2.78 (1H, dd), 2.97 (1H, dd), 3.09 (2H, q), 3.48 (1H, m), 4.43 (2H, d), 5.14 (1H, brs).
MS : 386 (M-H)
CHN : Found: C52.07%; H7.38%; N10.30%; C₁₇H₂₉N₃O₅S. 0.1TFA requires C51.79%; H7.35%; N10.53%

### Preparation 10:

### tert-butyl (3R)-6-cyclohexyl-3-(3-{[(isopropylsulfonyl)amino]methyl}-1,2,4-oxadiazol-5-yl)hexanoate

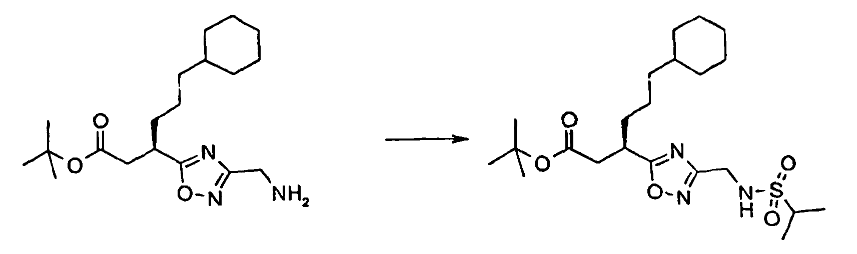

A solution of tert-butyl (3R)-3-[3-(aminomethyl)-1,2,4-oxadiazol-5-yl]-6-cyclohexylhexanoate (preparation 18) (216mg, 0.61mmol) and 2,6-lutidine (180µl, 1.50mmol) in DCM (3ml) was treated with isopropanesulphonylchloride (100µl, 0.90mmol) and stirred at room temperature for 18 hours. Reaction was complete but it was left for 12 days before purification commenced. The reaction mixture was diluted with DCM and washed with 1M HCl (2x20ml), sat. NaHCO₃ solution and brine. The organic extract was dried over anhydrous MgSO₄, filtered and the solvent removed under reduced pressure. The residue was purified on a silica column eluting with a solvent gradient of pentane : EtOAc (90 : 10) gradually changing to (50 : 50) to afford the title compound as a colourless oil (157mg, 56%).
¹H nmr : (CDCl₃) 0.80-0.90 (2H, m), 1.00-1.30 (8H, m), 1.30-1.40 (15H, d+s),1.60-1.80 (7H, m), 2.60 (1H, dd), 2.80 (1H, dd), 3.20 (1H, m), 3.45 (1H, m), 4.45 (2H, d), 4.60 (1H, m).
MS : 458 (MH⁺)

### Preparation 11:

### (3R)-6-cyclohexyl-3-(3-{[(isopropylsulfonyl)amino]methyl}-1,2,4-oxadiazol-5-yl)hexanoic acid

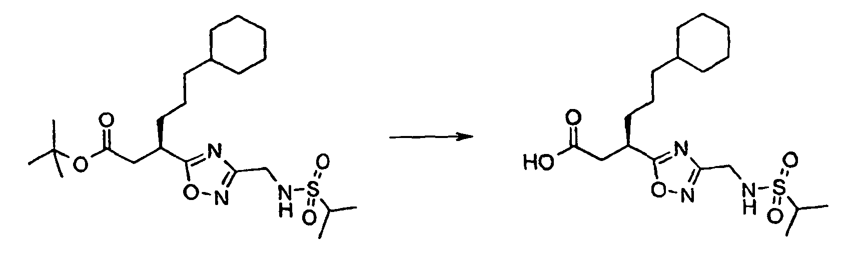

A solution of tert-butyl (3R)-3-[3-(aminomethyl)-1,2,4-oxadiazol-5-yl]-6-cyclohexylhexanoate (preparation 10) (145mg, 0.32mmol) in DCM (4ml) was treated with TFA (2ml) and stirred at room temperature for 6 hours. The solvent was removed under reduced pressure and azeotroped with toluene (x3) and DCM (x3) to afford the title compound as a yellow oil (117mg, 92%).
¹H nmr : (CDCl₃) 0.80-0.90 (2H, m), 1.10-1.30 (8H, m), 1.35 (6H, d), 1.60-1.80 (7H, m), 2.75 (1H, dd), 2.90 (1H, dd), 3.20 (1H, m), 3.50 (1H, m), 4.40 (2H, d), 4.90 (1H, m).
MS : 400 (M-H)

### Preparation 12:

### tert-butyl (3R)-6-cyclohexyl-3-(3-{[(phenylsulfonyl)amino]methyl}-1,2,4-oxadiazol-5-yl)hexanoate

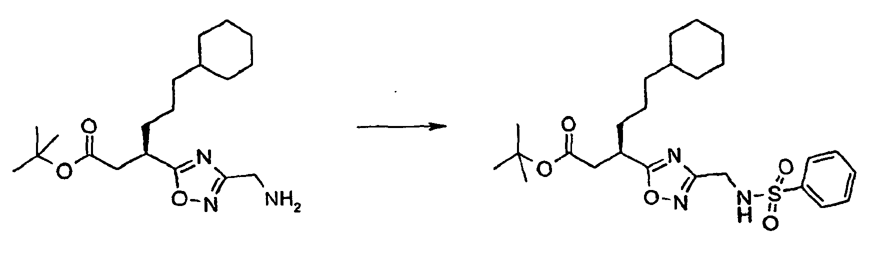

Method as for preparation 6 using tert-butyl (3R)-3-[3-(aminomethyl)-1,2,4-oxadiazol-5-yl]-6-cyclohexylhexanoate (preparation 18) (205mg, 0.58mmol) and benzenesulphonylchloride (75µl, 0.58mmol) as starting materials.
Purification: The crude materal was purified on a silica column eluting with a solvent gradient of pentane : EtOAc (100 : 0) gradually changing to (65 : 35) to afford the title compound as a colourless oil (241mg, 85%).
¹H nmr : (CDCl₃) 0.83 (2H, m), 1.10-1.30 (8H, m), 1.39 (9H, s), 1.60-1.75 (7H, m), 2.55 (1H, dd), 2.68 (1H, dd), 3.37 (1H, m), 4.31 (2H, d), 5.00 (1H, brs), 7.47 (2H, t), 7.56 (1H, d), 7.86 (2H, d)
MS : 514 (MNa⁺)
CHN : Found: C60.34%; H7.60%; N8.29%; C₂₅H₃₇N₃O₅S. 0.25 H₂O requires C60.52%; H7.62%; N8.47%

### Preparation 13:

### (3R)-6-cyclohexyl-3-(3-{[(phenylsulfonyl)amino]methyl}-1,2,4-oxadiazol-5-yl)hexanoic acid

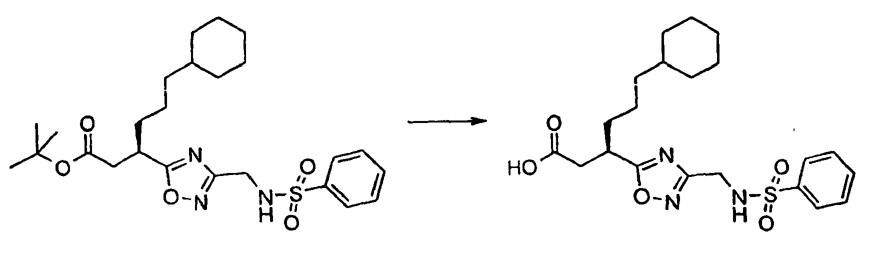

Method same as preparation 11 using *tert*-butyl (3*R*)-6-cyclohexyl-3-(3-{[(phenylsulfonyl)amino]methyl}-1,2,4-oxadiazol-5-yl)hexanoate (preparation 12) (231mg, 0.47mmol) as starting material.
Purification: The crude product was dissolved in EtOAc and washed with H₂O and brine. The organic extract was dried over anhydrous MgSO₄, filtered and the solvent removed under reduced pressure to afford the title compound as a white solid (168mg, 82%).
¹H nmr : (CD₃OD) 0.85 (2H, m), 1.10-1.30 (8H, m), 1.60-1.75 (7H, m), 2.62 (1H, dd), 2.76 (1H, dd), 3.38 (1H, m), 4.21 (2H, d), 7.50 (2H, m), 7.58 (1H, m), 7.81 (2H, d)
MS : 458 (MNa⁺)
CHN : Found: C57.73%; H6.68%; N9.51%; C₂₁H₂₉N₃O₅S requires C57.91%; H6.71%; N9.65%

### Preparation 14:

### tert-butyl (3R)-6-cyclohexyl-3-(3-{[(2-pyridinylsulfonyl)amino]methyl}-1,2,4-oxadiazol-5-yl)hexanoate

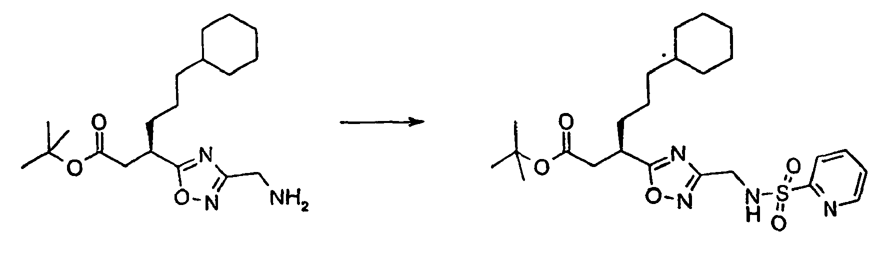

Method same as for preparation 10 using tert-butyl (3R)-3-[3-(aminomethyl)-1,2,4-oxadiazol-5-yl]-6-cyclohexylhexanoate (preparation 18) (210mg, 057mmol) and 2-pyridinesulphonylchloride (J. Med. Chem; 1997, 40, 1149) (170mg, 0.96mmol) as starting materials.
Purification: Attempted purification on a silica column eluting with DCM failed as the title compound could only be isolated with 0.5eq 2,6-lutidine as an impurity.

### Preparation 15:

### (3R)-6-cyclohexyl-3-(3-{[(2-pyridinylsulfonyl)amino]methyl}-1,2,4-oxadiazol-5-yl)hexanoic acid

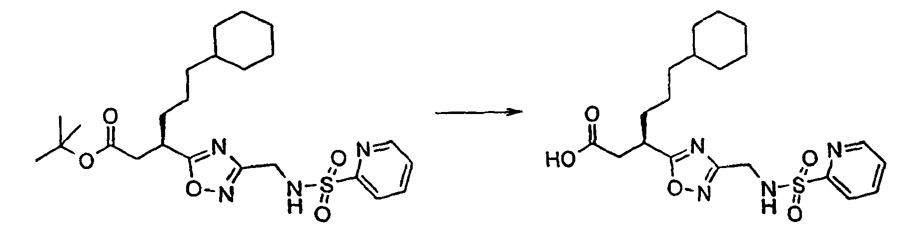

Method same as for preparation 7 using *tert*-butyl (3*R*)-6-cyclohexyl-3-(3-{[(2-pyridinylsulfonyl)amino]methyl}-1,2,4-oxadiazol-5-yl)hexanoate (preparation 14) (300mg, 0.61mmol) as starting material to afford the title compound as a pale yellow oil (250mg).
¹H nmr : (d₆DMSO) 0.81 (2H, m), 1.05-1.25 (8H. m), 1.50-1.70 (7H, m), 2.68 (2H, d), 3.31 (1H, m), 4.32 (2H, d), 7.60 (1H, m), 7.88 (1H, d), 8.03 (1H, t), 8.48 (1H, brs), 8.66 (1H, d).
MS : 459 (MNa⁺)
CHN : Found: C54.80%; H6.79%; N11.21%; C₂₀H₂₆N₄O₅S. 0.2 H₂O. 0.5 dioxan requires C54.57%; H6.74%; N11.57%

### Preparation 16:

### tert-butyl (3R)-6-cyclohexyl-3-(3-{[(3-pyridinylsulfonyl)amino]methyl}-1,2,4-oxadiazol-5-yl)hexanoate

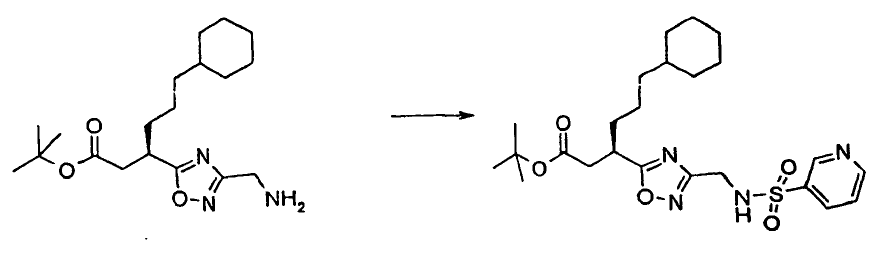

Method same as Preparation 14 using tert-butyl (3R)-3-[3-(aminomethyl)-1,2,4-oxadiazol-5-yl]-6-cyclohexylhexanoate (preparation 18) (200mg, 057mmol) and 3-pyridinesulphonylchloride (EP911333) (134mg, 0.63mmol) as starting materials.
Purification: The crude material was purified on a silica column eluting with a solvent gradient of pentane : EtOAc (100 : 0) gradually changing to (50 : 50) to afford the title compound as a colourless oil (192mg, 68%).
¹H nmr : (CDCl₃) 0.84 (2H, m), 1.10-1.30 (8H, m), 1.40 (9H, s), 1.60-1.75 (7H, m), 2.58 (1H, dd), 2.67 (1H, dd), 3.38 (1H, m), 4.40 (2H, d), 5.19 (1H, brs), 7.40 (1H, m), 8.14 (1H, d), 8.78 (1H, d), 9.08 (1H, s).
MS : 515 (MNa⁺)
CHN : Found: C57.96%; H7.48%; N10.99%; C₂₄H₃₆N₄O₅S. 0.1 H₂O requires C58.30%; H7.38%; N11.33%

### Preparation 17:

### (3R)-6-cyclohexyl-3-(3-{[(3-pyridinylsulfonyl)amino]methyl}-1,2,4-oxadiazol-5-yl)hexanoic acid

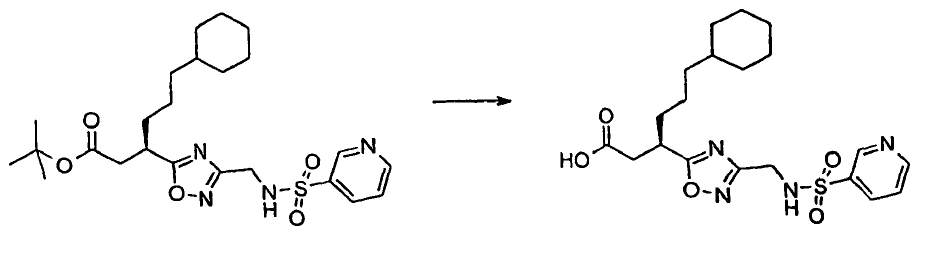

Method same as for preparation 11 using *tert*-butyl (3*R*)-6-cyclohexyl-3-(3-{[(3-pyridinylsulfonyl)amino]methyl}-1,2,4-oxadiazol-5-yl)hexanoate (preparation 16) (181mg, 0.37mmol) as starting material to afford the title compound as a white foam (177mg, 94%)
¹H nmr: (CDCl₃) 0.82 (2H, m), 1.05-1.30 (8H, m + EtOAc), 1.55-1.75 (7H, m), 2.63-2.84 (2H, m), 3.37 (1H, m), 4.43 (2H, d), 7.58 (1H, m), 8.28 (1H, d), 8.78 (1H, brs), 9.00 (1H, brs).
MS : 435 (M-H)
CHN : Found: C50.67%; H5.92%; N10.76%; C₂₀H₂₈N₄O₅S. 0.1 H₂O. 0.5 TFA. 0.15 EtOAc requires C51.01%; H5.93%; N11.02%

### Preparation 18:

### tert-butyl (3R)-3-[3-(aminomethyl)-1,2,4-oxadiazol-5-yl]-6-cyclohexylhexanoate

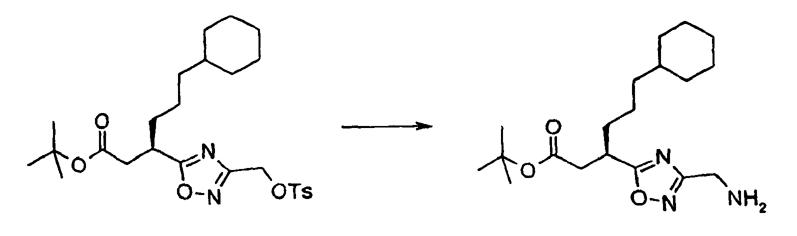

A soluton of *tert*-butyl (3*R*)-6-cyclohexyl-3-[3-({[(4-methylphenyl)sulfonyl]oxy}methyl)-1,2,4-oxadiazol-5-yl]hexanoate (preparation 177) (1.2g, 2.37mmol) in THF (40ml) was treated with concentrated ammonia solution and heated in a bomb at 40°C for 18 hours. The reaction mixture was diluted with EtOAc and washed with sat. NaHCO₃ solution followed by brine. The organic extract was dried over anhydrous MgSO₄, filtered and the solvent removed under reduced pressure. The colourless oil was purified on a silica column eluting with a solvent gradient of pentane : EtOAc (4 : 1) gradually changing to (1 : 1) and then changing to DCM : MeOH : NH₃ (95 : 5 : 0.5) to afford the title compound as a colourless oil (782mg, 94%).
¹H nmr : (CDCl₃) 0.84 (2H, m), 1.10-1.35 (8H, m), 1.40 (9H, s), 1.60-1.80 (7H, m), 2.61 (1H, dd), 2.79 (1H, dd), 3.42 (1H, m), 3.95 (2H, s).
MS : 374 (MNa⁺)

### Preparation 19:

### tert-butyl (3R)-6-cyclohexyl-3-[3-({[(1-methyl-1H-imidazol-4-yl)sulfonyl]amino}methyl)-1,2,4-oxadiazol-5-yl]hexanoate

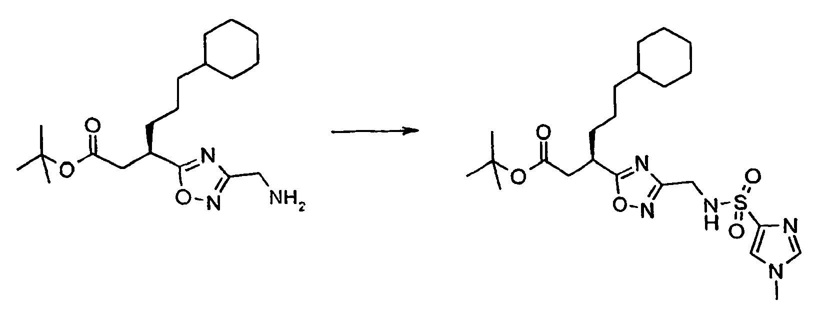

Method same as for preparation 10 using tert-butyl (3R)-3-[3-(aminomethyl)-1,2,4-oxadiazol-5-yl]-6-cyclohexylhexanoate (preparation 18) (145mg, 0.41mmol) and 1-methyl-1H-imidazole-4-sulphonyl chloride (75mg, 0.41mmol) as starting materials.
Purification: The crude matrial was purified on a silica column eluting with a solvent gradient of DCM : MeOH (100 : 0) gradually changing to (90 : 10) to afford the title compound as a colourless oil.
¹H nmr: (CDCl₃) 0.83 (2H, m), 1.10-1.30 (8H, m), 1.40 (9H, s), 1.60-1.75 (7H, m), 2.59 (1H, dd), 2.75 (1H, dd), 3.40 (1H, m), 3.73 (3H, s), 4.36 (2H, d), 5.38 (1H, brs), 41 (1H, s), 7.46 (1H, s).
MS : 435 (MNa⁺)
CHN : Found: C52.40%; H7.07%; N13.12%; C₂₃H₃₇N₅O₅S. 0.5 DCM requires C52.45%; H7.12%; N13.01%

### Preparation 20:

### (3R)-6-cyclohexyl-3-[3-({[(1-methyl-1H-imidazol-4-yl)sulfonyl]amino}methyl)-1,2,4-oxadiazol-5-yl]hexanoic acid

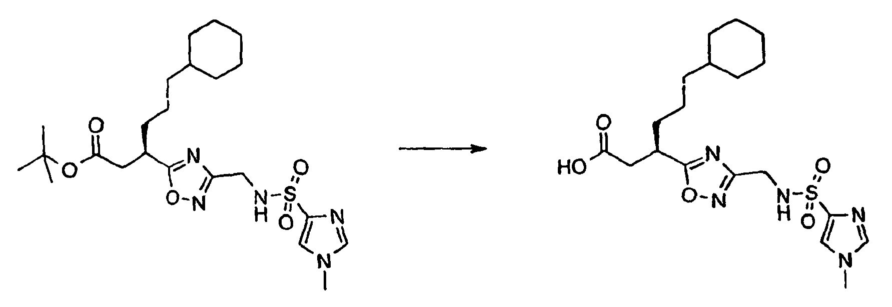

Method as for preparation 7 using *tert*-butyl (3*R*)-6-cyclohexyl-3-[3-({[(1-methyl-1*H*-imidazol-4-yl)sulfonyl]amino}methyl)-1,2,4-oxadiazol-5-yl]hexanoate (preparation 19) (490mg, 1.00mmol) as starting material.
Purification: Triturated with Et₂O to afford the title compound as a white solid 320mg (320mg, 74%)
¹H nmr : (CD₃OD) 0.83 (2H, m), 1.10-1.30 (8H, m), 1.60-1.75 (7H, m), 2.78 (2H, m), 3.40 (1H, m), 3.91 (3H, s), 4.40 (2H, d), 7.98 (1H, s), 8.78 (1H, s).
MS : 438 (M-H)
CHN : Found: C47.18%; H6.25%; N14.33%; C₁₉H₂₉N₅O₅S. 1 HCl. 0.1 DCM requires C47.35%; H6.28%; N14.46%

### Preparation 21:

### 1H-pyrazole-4-sulfonyl chloride

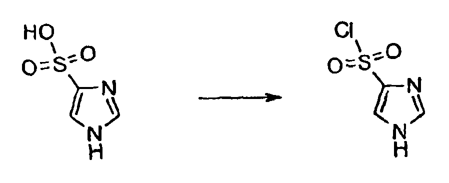

A mixture of 1*H*-pyrazole-4-sulfonic acid (J. Am. Chem. Soc.; 1955, 77, 6532) (1.0g, 4.0mmol) and phosphorous pentachloride (1.6g, 7.7mmol) were heated up to 180°C over the period of an hour. The mixture began to reflux but not fully molten. The reaction mixture was cooled to 130°C and toluene (5ml) was added and allowed to cool to room temperature with stirring. The white solid was filtered off. The filtrate solvent was removed under reduced pressure and azeotroped with toluene and DCM to afford the title compound as a colourless oil and impure.

### Preparation 22:

### tert-butyl (3R)-6-cyclohexyl-3-(3-{[(1H-pyrazol-4-ylsulfonyl)amino]methyl}-1,2,4-oxadiazol-5-yl)hexanoate

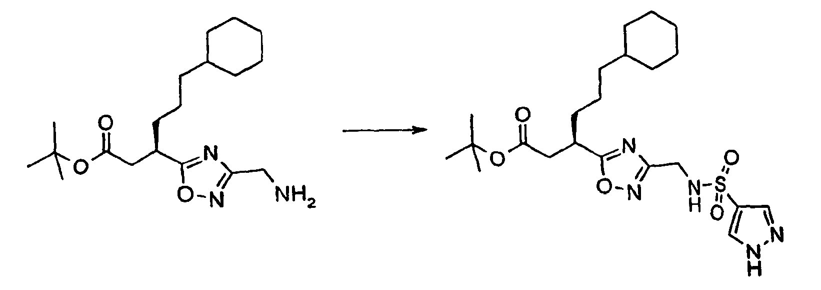

Method as for Preparation 10 using tert-butyl (3R)-3-[3-(aminomethyl)-1,2,4-oxadiazol-5-yl]-6-cyclohexylhexanoate (preparation 18) (310mg, 0.88mmol) and 1*H*-pyrazole-4-sulfonyl chloride (preparation 21) (220mg, 1.32mmol) as starting materials.
Purification: The crude material was purified on a silica column eluting with a solvent gradient of DCM : MeOH (98 : 2) gradually changing to (90 : 10) to afford the title compound.
¹H nmr : (CDCl₃) does not integrate for the pyrrole protons
MS : 503 (MNa⁺)

### Preparation 23:

### (3R)-6-cyclohexyl-3-(3-{[(1H-pyrazol-4-ylsulfonyl)amino]methyl}-1,2,4-oxadiazol-5-yl)hexanoic acid

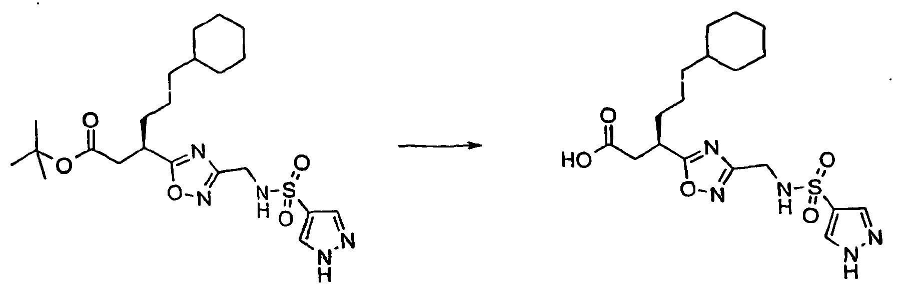

Method as for preparation 7 using *tert*-butyl (3*R*)-6-cyclohexyl-3-(3-{[(1*H*-pyrazol-4-ylsulfonyl)amino]methyl}-1,2,4-oxadiazol-5-yl)hexanoate (preparation 22) (420mg, 0.87mmol) as starting material.
Purification: Crude material taken up in Et₂O and DIPE and left in the fridge for 2 days. Solid was filtered off and dried under reduced pressure to afford the title compound as a white solid (140mg, 38%).
¹H nmr : (CD₃OD) 0.83 (2H, m), 1.05-1.30 (8H, m), 1.55-1.75 (7H, m), 2.69 (1H, dd), 2.81 (1H, dd), 3.41 (1H, m), 4.22 (2H, s), 7.91 (2H, brs).
MS : 424 (M-H)
CHN : Found: C50.23%; H6.33%; N16.46%; C₁₈H₂₇N₅O₅S. 0.2 H₂O requires C50.38%; H6.44%; N16.32%

### Preparation 24:

### 4H-1,2,4-triazole-3-sulfonyl chloride

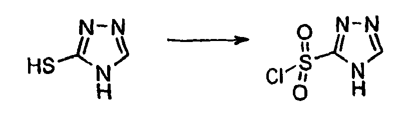

Chlorine gas was bubbled through a suspension of 4*H*-1,2,4-triazole-3-thiol (2.7g, 26.7mmol) in 2M HCl (5ml) for 1 hour, at 0°C. The reaction mixture became homogenous. Aqueous Na₂S₂O₅ was added to the reaction mixture and nitrogen gas was bubbled through for 10 minutes. The white solid was filtered and washed with cold H₂O to afford the title compound (460mg, 10%).
¹H nmr : (CD₃OD) 8.69 (1H, s).
MS : 166 (M-H)

### Preparation 25:

### tert-butyl (3R)-6-cyclohexyl-3-(3-{[(4H-1,2,4-triazol-3-ylsulfonyl)amino]methyl}-1,2,4-oxadiazol-5-yl)hexanoate

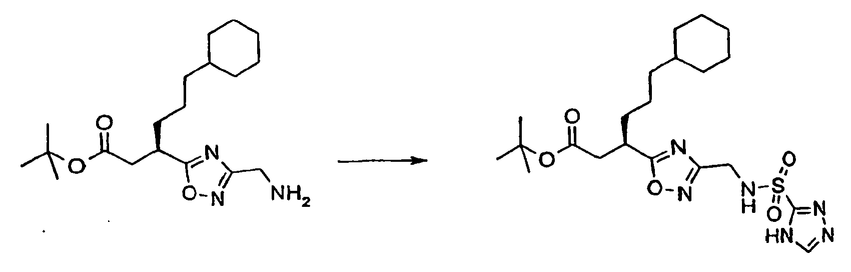

Method as for preparation 10 using tert-butyl (3R)-3-[3-(aminomethyl)-1,2,4-oxadiazol-5-yl]-6-cyclohexylhexanoate (preparation 18) (292mg, 0.83mmol) and 4*H*-1,2,4-triazole-3-sulfonyl chloride (preparation 24) (208mg, 1.24mmol) as starting materials.
Purification: The crude material was purified on a silica column eluting with a solvent gradient of DCM : MeOH (98 : 2) gradually changing to (90 : 10) to afford the title compound as a colourless oil (412mg, 102%, contains DCM).
¹H nmr : (CD₃OD) 0.83 (2H, m), 1.05-1.30 (8H, m), 1.38 (9H, s), 1.55-1.75 (7H, m), 2.60-2.80 (2H, m), 3.40 (1H, m), 4.41 (2H, s), 8.50 (1H, s).
MS : 505 (MNa⁺)

### Preparation 26:

### (3R)-6-cyclohexyl-3-(3-{[(4H-1,2,4-triazol-3-ylsulfonyl)amino]methyl}-1,2,4-oxadiazol-5-yl)hexanoic acid

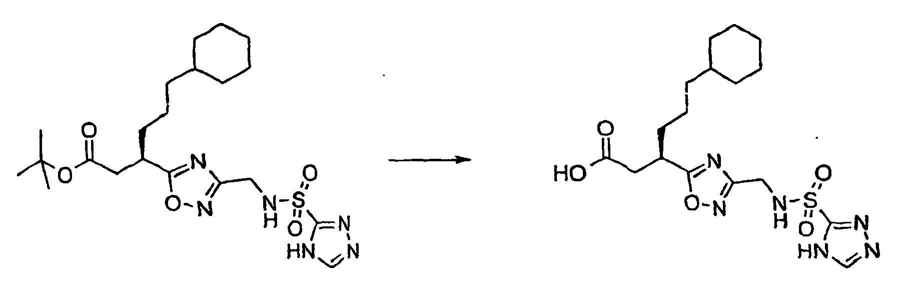

Method as for preparation 7 using *tert*-butyl (3*R*)-6-cyclohexyl-3-(3-{[(4*H*-1,2,4-triazol-3-ylsulfonyl)amino]methyl}-1,2,4-oxadiazol-5-yl)hexanoate (preparation 25) (410mg, 0.85mmol) as starting material.
Purification: Material was azeotroped with toluene followed by DCM to afford the title compound as a white solid (320mg, 88%).
M.pt. 151-153°C
¹H nmr : (CD₃OD) 0.83 (2H, m), 1.10-1.30 (8H, m), 1.55-1.75 (7H, m), 2.69 (1H, dd), 2.81 (1H, dd), 3.42 (1H, m), 4.40 (2H, s), 8.48 (1H, s).
MS : 425 (M-H)

### Preparation 27:

### tert-butyl (3R)-6-cyclohexyl-3-[3-({[(3,5-dimethyl-4-isoxazolyl)sulfonyl]amino}methyl)-1,2,4-oxadiazol-5-yl]hexanoate

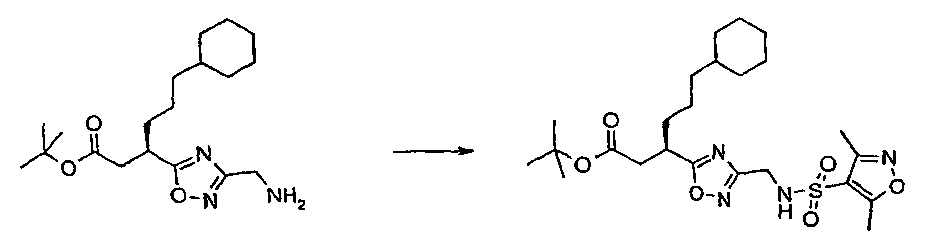

Method as for preparation 6 using tert-butyl (3R)-3-[3-(aminomethyl)-1,2,4-oxadiazol-5-yl]-6-cyclohexylhexanoate (preparation 18) (200mg, 0.57mmol) and 3,5-dimethylisoxazolesulphonyl chloride (112mg, 0.57mmol) as starting materials.
Purification: The crude material was purified on a silica column eluting with a solvent gradient of pentane : EtOAc (100 : 0) gradually changing to (70 : 30) to afford the title compound as a colourless oil (252mg, 87%).
¹H nmr : (CDCl₃) 0.82 (2H, m), 1.10-1.30 (8H, m), 1.39 (9H, s), 1.60-1.75 (7H, m), 2.39 (3H, s), 2.58 (2H, dd), 2.61 (3H, s), 2.71 (1H, dd), 3.38 (1H, m), 4.37 (2H, d), 5.26 (1H, brs).
MS : 533 (MNa⁺)
CHN : Found: C56.17%; H7.48%; N10.65%; C₂₄H₃₈N₄O₆S requires C56.45%; H7.50%; N10.97%

### Preparation 28:

### (3R)-6-cyclohexyl-3-[3-({[(3,5-dimethyl-4-isoxazolyl)sulfonyl]amino}methyl)-1,2,4-oxadiazol-5-yl]hexanoic acid

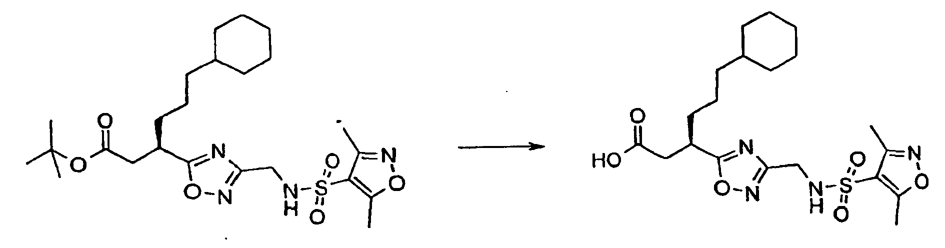

Method same as for Preparation 11 using *tert*-butyl (3*R*)-6-cyclohexyl-3-[3-({[(3,5-dimethyl-4-isoxazolyl)sulfonyl]amino}methyl)-1,2,4-oxadiazol-5-yl]hexanoate (preparation 27) (246mg, 0.48mmol) as starting material to afford the title compound as a yellow gum (238mg, 109%, contains EtOAc).
¹H nmr : (CDCl₃) 0.82 (2H, m), 1.10-1.25 (8H, m), 1.55-1.75 (7H, m), 2.35 (3H, s), 2.59 (3H, s), 2.74 (1H, dd), 2.83 (1H, dd), 3.41 (1H, m), 4.38 (2H, d), 5.69 (1H, brs).
MS : 453 (M-H)

### Preparation 29:

### N-(2-cyanoethyl)methanesulfonamide

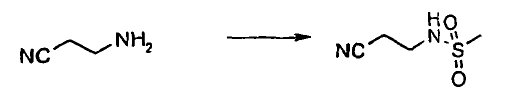

A solution of aminopropionitrile fumarate salt (6.0g, 47mmol) in 2M NaOH solution (40ml) was stirred at room temperaute for 15 minutes. The reaction mixture extracted with DCM (50ml). The organic extract was dried over anhydrous Na₂SO₄ and filtered. The filtrate was treated pyridine (11ml, 136mmol) and methanesulphonyl chloride (3.9ml, 50mmol) and stirred under at nitrogen atmosphere for 18 hours. The solvent was removed under reduced pressure. The oil was dissolved in DCM and washed with 2M HCl (2x100ml) and saturated NaHCO₃ solution. All the product had been extracted into the aqueous washings. NaHCO₃ solution was acidified with conc. HCl and combined with the other acidic washes. The aqueous washes were reduced to ~50ml in volume and extracted with EtOAc. The organic extract was evaporated under reduced pressure to afford the title compound as a pink oil (2.1g, 30%).
¹H nmr : (CDCl₃) 2.64 (2H, t), 3.00 (3H, s), 3.42 (2H, m), 4.83 (1H, brs).
MS : 171 (MNa⁺)

### Preparation 30:

### (1 Z)-N'-hydroxy-3-[(methylsulfonyl)amino]propanimidamide

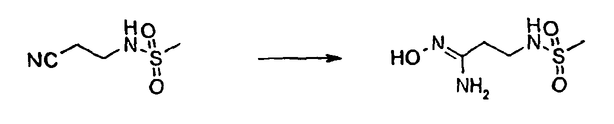

Method as for preparation 1 using *N*-(2-cyanoethyl)methanesulfonamide (preparation 29) (1.0g, 6.75mmol) as starting material.
Purification: Crude material was dissolved in hot EtOH and filtered. The solvent in the filtrate was removed under reduced pressure to afford the title compound as a colourless oil (1.13g).
¹H nmr : (CD₃OD) contains starting material in ratio product : SM (3 : 2)
MS : 204 (MNa⁺)

### Preparation 31:

### tert-butyl (6Z,10R)-6-amino-10-(3-cyclohexylpropyl)-9-oxo-8-oxa-2-thia-3,7-diazadodec-6-en-12-oate 2,2-dioxide

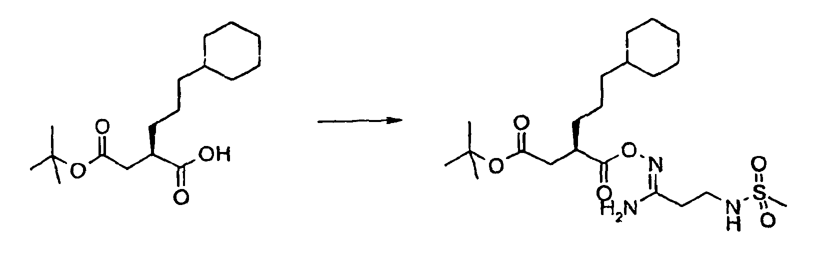

Method as for preparation 2 using (2*R*)-2-(2-*tert*-butoxy-2-oxoethyl)-5-cyclohexylpentanoic acid (preparation 168) (670mg, 2.25mmol) and (1*Z*)-*N*'-hydroxy-3-[(methylsulfonyl)amino]propanimidamide (preparation 30) (700mg, 2.50mmol) as starting materials to afford the title compound as a yellow oil.

Compound only 90% pure.

### Preparation 32:

### tert-butyl (3R)-6-cyclohexyl-3-(3-{2-[(methylsulfonyl)amino]ethyl}-1,2,4-oxadiazol-5-yl)hexanoate

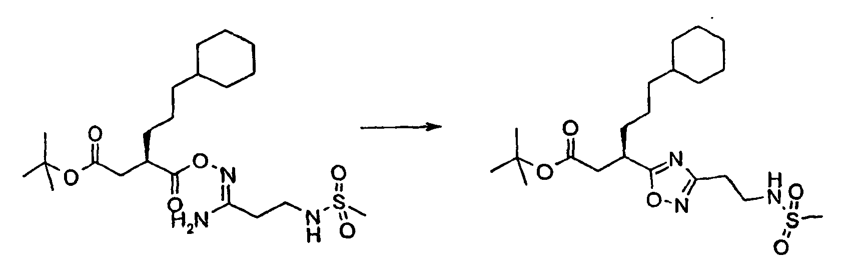

Method as for preparation 3 using *tert*-butyl (6*Z*,10*R*)-6-amino-10-(3-cyclohexylpropyl)-9-oxo-8-oxa-2-thia-3,7-diazadodec-6-en-12-oate 2,2-dioxide (preparation 31) (1.14g, 2.48mmol) as starting material.
Purification: The crude material was purified on a silica column eluting with a solvent gradient of pentane : EtOAc (90 : 10) gradually changing to (50 : 50) to afford the title compound as a colourless oil (910mg, 83%).
¹H nmr : (CD₃OD) 0.85 (2H, m), 1.10-1.35 (8H, m), 1.39 (9H, s), 1.60-1.75 (7H, m), 2.66 (1H, dd), 2.77 (1H, dd), 2.91 (3H, s), 2.98 (2H, t), 3.42 (3H, m).
MS : 466 (MNa⁺)
CHN : Found: C56.77%; H8.39%; N9.36%; C₂₁H₃₇N₃O₅S requires C56.86%; H8.41%; N9.47%

### Preparation 33:

### (3R)-6-cyclohexyl-3-(3-{2-[(methylsulfonyl)amino]ethyl}-1,2,4-oxadiazol-5-yl)hexanaic acid

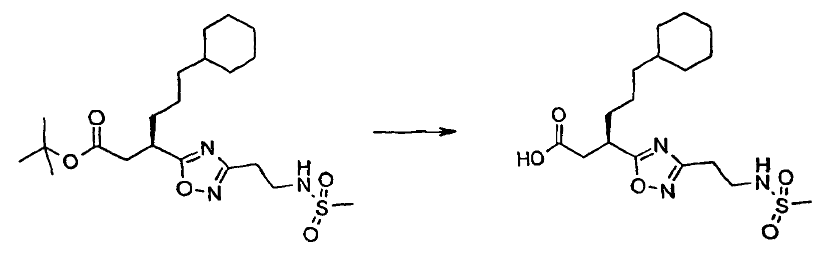

Method as for preparation 11 using *tert*-butyl (3*R*)-6-cyclohexyl-3-(3-{2-[(methylsulfonyl)amino]ethyl}-1,2,4-oxadiazol-5-yl)hexanoate (preparation 32) (400mg, 0.9mmol) as starting material.
Purification: Crude material was purified on a silica column eluting with a solvent gradient of DCM : MeOH : AcOH (100 : 0 : 0) gradually changing to (90 : 10: 1) to afford the title compound as a colourless oil (370mg)
¹H nmr : (CD₃OD) 0.85 (2H, m), 1.05-1.35 (8H, m), 1.55-1.75 (7H, m), 2.73 (1H, dd), 2.84 (1H, dd), 2.90 (3H, s), 2.95 (2H, t), 3.43 (3H, t).
MS : 386 (M-H)
CHN : Found: C52.54%; H7.62%; N10.58%; C₁₇H₂₉N₃O₅S requires C52.69%; H7.54%; N10.84%

### Preparation 34:

### 1-benzhydryl-N'-hydroxy-3-azetidinecarboximidamide

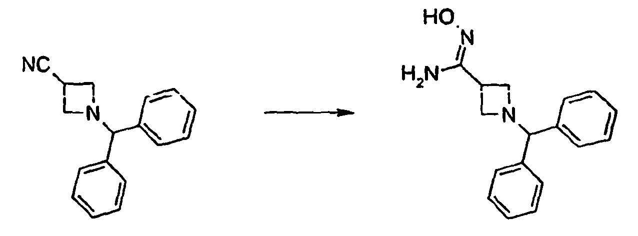

Method as for preparation 1 using 1-benzhydryl-3-azetidinecarbonitrile (2.0g, 8.0mmol) was used as starting material.

The title material isolated 90% pure and used as such in the following step.

### Preparation 35:

### tert-butyl (3R)-3-[({[(Z)-amino(1-benzhydryl-3-azetidinyl)methylidene]amino}oxy)carbonyl]-6-cyclohexylhexanoate

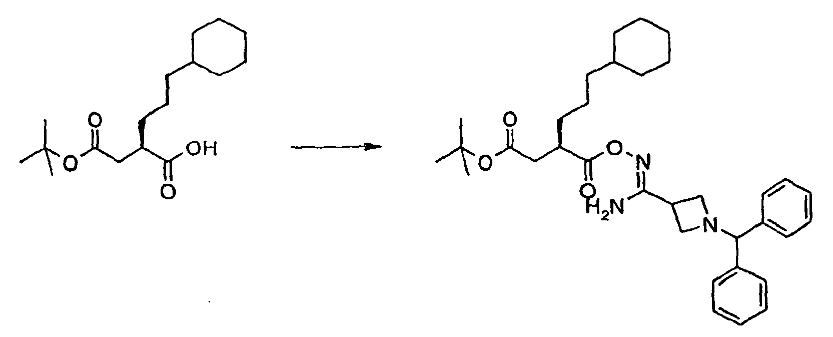

Method as for preparation 2 using (2*R*)-2-(2-*tert*-butoxy-2-oxoethyl)-5-cyclohexylpentanoic acid (1.65g, 5.51mmol) and 1-benzhydryl-*N*'-hydroxy-3-azetidinecarboximidamide (preparation 34) (1.74g, 6.17mmol) as starting materials.
Purification: The crude material was dissolved in EtOAc and washed with H₂O (3x50ml) and brine. The organic extract was dried over Na₂SO₄, filterd and the solvent removed under reduced pressure to afford the title compound as a brown oil (3.25g).
¹H nmr : (CDCl₃) 0.83 (2H, m), 1.05-1.30 (8H, m), 1.41 (9H, s), 1.60-1.75 (7H, m), 2.41 (1H, brd), 2.65 (1H, m), 2.87 (1H, brs), 3.09 (1H, brs), 3.30 (4H, brs), 4.36 (1H, s), 5.87 (2H, brs), 7.15-7.40 (10H, m + CHCl₃)
MS : 584 (MNa⁺)

### Preparation 36:

### tert-butyl (3R)-3-[3-(1-benzhydryl-3-azetidinyl)-1,2,4-oxadiazol-5-yl]-6-cyclohexylhexanoate

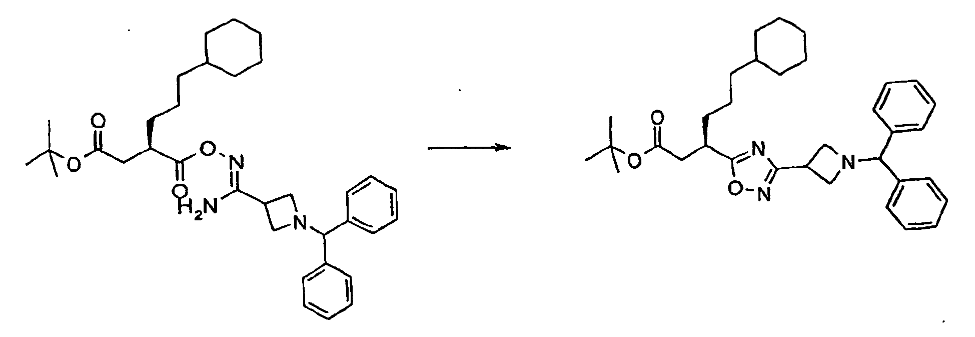

A solution of *tert*-butyl (3*R*)-3-[({[(*Z*)-amino(1-benzhydryl-3-azetidinyl)methylidene]amino}oxy)carbonyl]-6-cyclohexylhexanoate (preparation 35) (2.85g, 5.07mmol) in toluene (90ml) was heated at 140°C under Dean and Stark conditions for 18 hours. The reaction mixture was allowed to cool to room temperature. The solvent was removed under reduced pressure. The residue was purified on a silica column eluting with a solvent gradient of cyclohexane : Et₂O (90 : 10) gradually changing to (60 : 40) to afford the title compound as a colourless oil (2.0g, 73%).
¹H nmr: (CDCl₃) 0.83 (2H, m), 1.05-1.35 (8H, m), 1.39 (9H, s), 1.60-1.80 (7H, m), 2.60 (1H, dd), 2.79 (1H, dd), 3.34 (2H, t), 3.43 (1H, m), 3.60 (2H, t), 3.80 (1H, m), 4.43 (1H, s), 7.18 (2H, m), 7.26 (4H, t+CHCl₃), 7.40 (4H, m).
MS : 544 (MH⁺)
CHN : Found: C73.58%; H8.15%; N7.66%; C₃₄H₄₅N₃O₃. 0.2 DCM requires C73.26%; H8.16%; N7.49%

### Preparation 37:

### methyl (3R)-3-[3-(3-azetidinyl)-1,2,4-oxadiazol-5-yl]-6-cyclohexylhexanoate

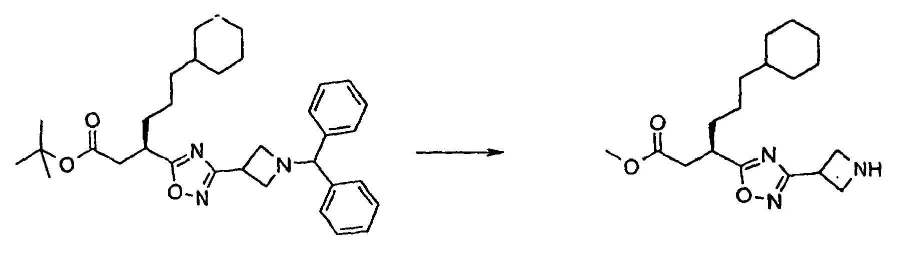

A solution of *tert*-butyl (3*R*)-3-[3-(1-benzhydryl-3-azetidinyl)-1,2,4-oxadiazol-5-yl]-6-cyclohexylhexanoate (preparation 36) (1.99g, 3.66mmol) in DCM (15ml) was treated with ACE-Cl (530µl, 4.90mmol) and heated at reflux for 4 hours. The solvent was removed under reduced pressure. The residue was dissolved in MeOH (15ml) and heated at reflux for 1.5 hours. The solvent was removed under reduced pressure. The oil was dissolved in EtOAc (150ml) and washed with sat. NaHCO₃ solution (50ml) and brine (40ml). The aqueous extracts were combined and extracted with EtOAc. All of the organic extracts were combined, dried over anhydrous Na₂SO₄ and filtered. The solvent was removed under reduced pressure to afford the title compound as a colourless oil (900mg).
¹H nmr: (CD₃OD) 0.85 (2H, m), 1.10-1.35 (8H, m), 1.60-1.80 (7H, m), 2.78 (1H, dd), 2.86 (1H, dd), 3.49 (1H, m), 3.61 (3H, s), 3.95 (4H, m), 4.06 (1H, m).
MS : 336 (MH⁺)

### Preparation 38:

### methyl (3R)-6-cyclohexyl-3-{3-[1-(methylsulfonyl)-3-azetidinyl]-1,2,4-oxadiazol-5-yl}hexanoate

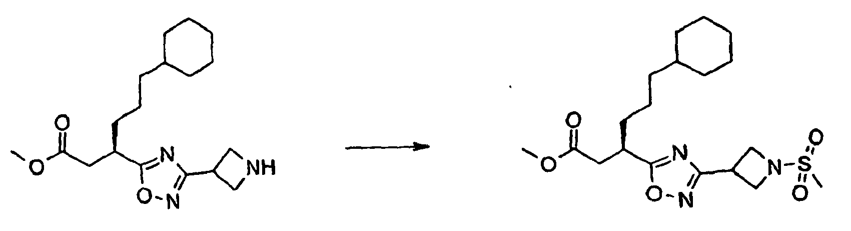

Method as for preparation 10 using methyl (3*R*)-3-[3-(3-azetidinyl)-1,2,4-oxadiazol-5-yl]-6-cyclohexylhexanoate (preparation 37) (880mg, 2.64mmol) and methanesulphonyl chloride (1.53ml, 19.77mmol) as starting materials.
Purification: After aqueous work-up MeOH was added to quench the excess methanesulphonyl chloride. The solvent was removed under reduced pressure. The crude material was purified on a silica column eluting with a solvent gradient of cyclohexane : EtOAc (90 : 10) gradually changing to (50 : 50) to afford the title compound as a yellow oil (900mg, contains 3 equivalents of MeSO₃Me).
¹H nmr: (CDCl₃) 0.82 (2H, m), 1.10-1.35 (8H, m), 1.60-1.80 (7H, m), 2.72 (1H, dd), 2.82-2.94 (4H, dd+s), 3.50 (1H, m), 3.64 (3H, s), 3.96 (1H, m), 4.19 (2H, m), 4.28 (2H, m).
MS : 436 (MNa⁺)

### Preparation 39:

### (3R)-6-cyclohexyl-3-{3-[1-(methylsulfonyl)-3-azetidinyl]-1,2,4-oxadiazol-5-yl}hexanoic acid

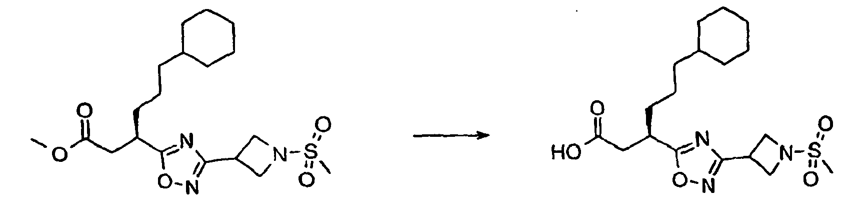

A solution of methyl (3*R*)-6-cyclohexyl-3-{3-[1-(methylsulfonyl)-3-azetidinyl]-1,2,4-oxadiazol-5-yl}hexanoate (preparation 38) (780mg, 1.05mmol) in dioxan (20ml) and H₂O (10ml) was treated with LiOH.H₂O (218mg, 5.2mmol) and stirred at room temperature for 2.25 hours. The reaction mixture was acidified with 2M HCl (20ml) and extracted with EtOAc. The organic extract was washed with H₂O and the solvent was removed under reduced pressure. The oil was purified on a silica column eluting with a solvent gradient of DCM : MeOH : AcOH (100 : 0 : 0) gradually changing to (90 : 10 : 1) to afford a colourless oil which solidified on standing. The solid was triturated with pentane, filtered off and dried under reduced pressure to afford the title compound as a white solid (372mg, 78%).
¹H nmr : (CD₃OD) 0.83 (2H, m), 1.05-1.35 (8H, m), 1.55-1.80 (7H, m), 2.75 (1H, dd), 2.84 (1H, dd), 2.98 (3H, s), 3.47 (1H, m), 4.01 (1H, m), 4.12 (2H, m), 4.26 (2H, m).
MS : 398 (M-H)
CHN : Found: C54.08%; H7.34%; N10.37%; C₁₈H₂₉N₃O₅S requires C54.12%; H7.32%; N10.52%

### Preparation 40:

### 1-(methylsulfonyl)-4-piperidinecarbonitrile

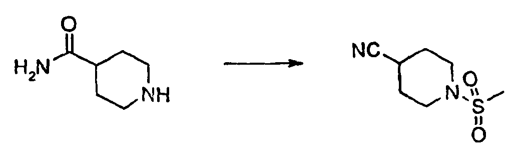

Method as for preparation 6 using 4-piperidinecarboxamide (4.0g, 31.2mmol) as starting material and DMF (2ml) was added.

Purification: The crude material was triturated with Et₂O, filtered off and dried under reduced pressure to afford the title compound as a white solid (2.48g, 42%).
¹H nmr : (CD₃OD) 1.85 (2H, m), 2.01 (2H, m), 2.81 (3H, s), 2.95 (1H, m), 3.18 (2H, m), 3.41 (2H, m).
MS : 211 (MNa⁺)
CHN : Found: C44.52%; H6.46%; N14.77%; C₇H₁₂N₂O₂S requires C44.66%; H6.43%; N14.88%

### Preparation 41:

### N'-hydroxy-1-(methylsulfonyl)-4-piperidinecarboximidamide

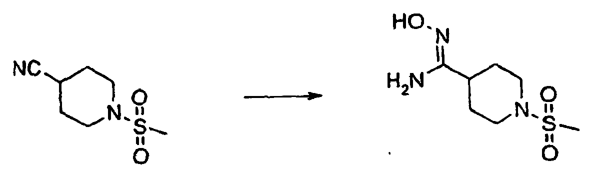

Method as for preparation 1 using 1-(methylsulfonyl)-4-piperidinecarbonitrile (preparation 40) (1.47g, 7.81mmol) as starting material.
Purification: The crude material was dissolved in EtOH, filtered and the solvent removed under reduced pressure to afford the title compound as a white solid.
¹H nmr : shows 2 : 1 mixture starting material : product. Taken on crude to next step.

### Preparation 42:

### tert-butyl (3R)-3-{[({(Z)-amino[1-(methylsulfonyl)-4-piperidinyl]methylidene}amino)oxy]carbonyl}-6-cyclohexylhexanoate

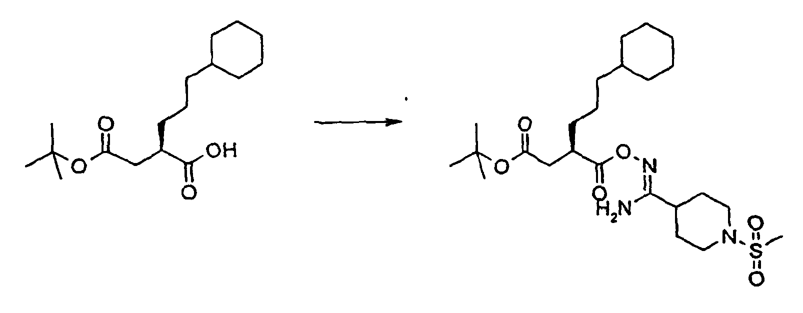

Method as for preparation 2 using (2*R*)-2-(2-*tert*-butoxy-2-oxoethyl)-5-cyclohexylpentanoic acid (54mg, 1.83mmol) and *N*'-hydroxy-1-(methylsulfonyl)-4-piperidinecarboximidamide (preparation 41) (1.20g, 2.0mmol) as starting materials.
Title compound isolated with 1-(methylsulfonyl)-4-piperidinecarbonitrile (preparation 40) as a major impurity in the ratio product : nitrile 1 : 1.5. Taken on crude to following stage.

### Preparation 43:

### tert-butyl (3R)-6-cyclohexyl-3-{3-[1-(methylsulfonyl)-4-piperidinyl]-1,2,4-oxadiazol-5-yl}hexanoate

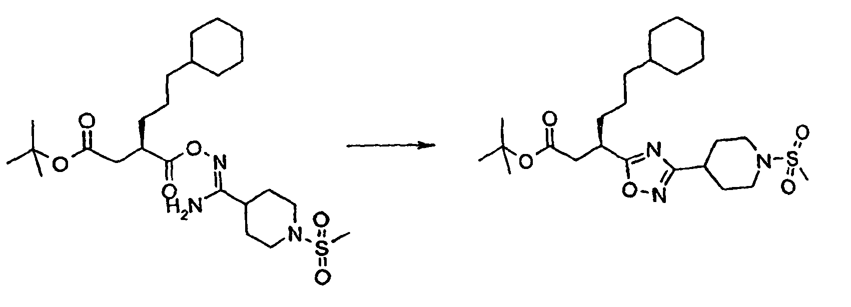

A solution of *tert*-butyl (3*R*)-3-{[({(Z)-amino[1-(methylsulfonyl)-4-piperidinyl]melhylidene}amino)oxy]carbonyl}-6-cyclohexylhexanoate (preparation 42) (1.50g, 1.83mmol) in toluene (100ml) was treated with pyridine (90µl, 1.83mmol) and anhydrous ZnCl₂ (150mg, 1.83mmol) and heated at reflux for 20 hours. The reaction mixture was diluted with EtOAc and washed with H₂O, brine and H₂O, dried over anhydrous Na₂SO₄ and filtered. The solvent was removed under reduced pressure. The residue was purified on a silica column eluting with a solvent gradient of cyclohexane : EtOAc (90 : 10) gradually changing to (50 : 50). The oil was recrystallised from pentane to afford the title compound as a white solid (320mg, 36%).
¹H nmr: (CDCl₃) 0.83 (2H, m), 1.10-1.35 (8H, m), 1.40 (9H, s), 1.60-1.80 (7H, m), 1.99 (2H, m), 2.14 (2H, brd), 2.60 (1H, dd), 2.71-2.80 (4H, dd+s), 2.95 (3H, m), 3.41 (1H, m), 3.74 (2H, m).
MS : 506 (MNa⁺)
CHN : Found: C59.55%; H8.58%; N8.68%; C₂₄H₄₁N₃O₅S requires C59.60%; H8.54%; N8.69%

### Preparation 44:

### (3R)-6-cyclohexyl-3-{3-[1-(methylsulfonyl)-4-piperidinyl]-1,2,4-oxadiazol-5-yl}hexanoic acid

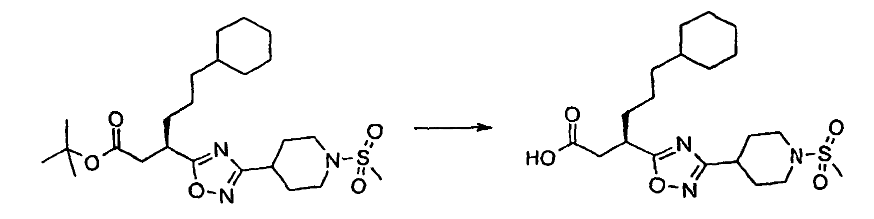

Method as for preparation 11 using *tert*-butyl (3*R*)-6-cyclohexyl-3-{3-[1-(methylsulfonyl)-4-piperidinyl]-1,2,4-oxadiazol-5-yl}hexanoate (preparation 43) (310mg, 0.64mmol) as starting material.
Purification: The crude material was triturated with pentane and filtered off to afford the title compound as a white solid (245mg, 89%).
¹H nmr : (CDCl₃) 0.81 (2H, m), 1.05-1.35 (8H, m), 1.55-1.85 (7H, m), 1.97 (2H, m), 2.10 (2H, m), 2.70-2.80 (4H, dd+s), 2.93 (4H, m), 3.45 (1H, m), 3.72 (2H, m).
MS : 426 (M-H)
CHN : Found: C56.15%; H7.77%; N9.64%; C₂₀H₃₃N₃O₅S requires C56.18%; H7.78%; N9.83%

### Preparation 45:

### tert-butyl (3R)-3-(3-{[(3-chloropropyl)sulfonyl]amino}-1,2,4-oxadiazol-5-yl)-6-cyclohexylhexanoate

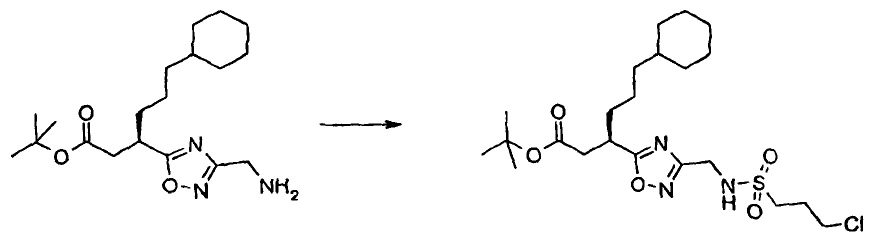

Method as for preparation 10 using tert-butyl (3R)-3-[3-(aminomethyl)-1,2,4-oxadiazol-5-yl]-6-cyclohexylhexanoate (preparation 18) (200mg, 0.57mmol) and 3-chloropropanesulphonyl chloride (83µl, 0.68mmol) as starting materials and pyridine as the base.
Pruification: The crude material was purified on a silica column eluting with pentane : Et2O (1 : 1) to afford the title compound as a colourless oil (225mg, 93%).
¹H nmr : (CDCl₃) 0.83 (2H, m), 1.10-1.35 (8H, m), 1.40 (9H, s), 1.60-1.80 (7H, m), 2.28 (2H, m), 2.61 (1H, dd), 2.78 (1H, dd), 3.23 (2H, m), 3.42 (1H, m), 3.62 (2H, t), 4.45 (2H, d), 5.79 (1H, brs).
MS : 514 (MNa⁺)
CHN : Found: C53.01%; H7.70%; N8.17%; C₂₂H₃₆ClN₃O₅S, 0.2 H₂O requires C53.31%; H7.81%; N8.48%

### Preparation 46:

### (3R)-6-cyclohexyl-3-[3-(1,1-dioxido-2-isothtazolidinyl)-1,2,4-oxadiazol-5-yl]hexanoic acid

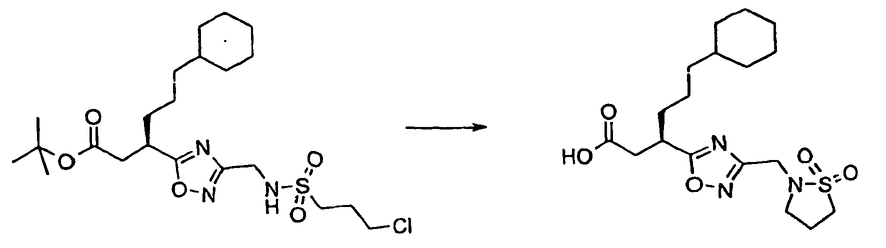

Sodium metal (114mg, 5.00mmol) was added in portions to MeOH (10ml) at 0°C and stirred until all of the sodium had dissloved (-10 minutes). A solution of *tert*-butyl (3*R*)-3-(3-{[(3-chloropropyl)sulfonyl]amino}-1,2,4-oxadiazol-5-yl)-6-cyclohexylhexanoate (preparation 45) (245mg, 0.50mmol) in MeOH (3ml) was added to the reaction mixture and was stirred under a nitrogen atmosphere for 3 days warming to room temperature over this time. The reaction was quenched with H₂O and the solvent removed under reduced pressure. The residue was diluted with water and acidified with 2M HCl (pH-3) and extracted with Et₂O followed by EtOAc. The organic extracts were combined, dried over anhydrous MgSO₄ and filtered. The solvent was removed under reduced pressure to afford the title compound as a colouriess oil (203mg, 98%).
¹H nmr : (d₆-DMSO) 0.81 (2H, m), 1.00-1.25 (8H, m),1.50-1.70 (7H, m), 2.21 (2H, m), 2.74 (2H, t), 3.26 (2H, t), 3.40 (1H, m), 4.21 (2H, s).
MS : 422 (MNa⁺)
CHN : Found: C54.33%; H7.49%; N10.10%; C₁₈H₂₉N₃O₅S. 0.05 H₂O requires C53.99%; H7.33%; N10.49%

### Preparation 47:

### terl-butyl (3R)-3-[3-({[(tert-butylamino)sulfonyl]amino}methyl)-1,2,4-oxadiazol-5-yl]-6-cyclohexylhexanoate

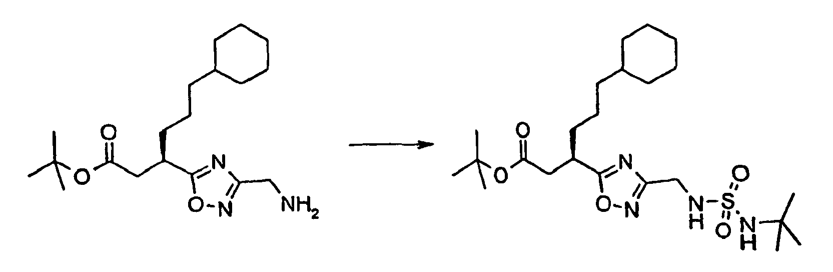

Method as for preparation 10 using tert-butyl (3R)-3-[3-(aminomethyl)-1,2,4-oxadiazol-5-yl]-6-cyclohexylhexanoate (preparation 18) (200mg, 0.57mmol) and *N*-*tert*-butylsulfamoyl chloride (J. Org. Chem.; 1976, *41*, 4028) (108mg, 0.63mmol) as starting materials and pyridine as base. Purification: The crude product was purified on a silica column eluting with a solvent gradient of DCM : MeOH (100 : 0) gradually changing to (93 : 7) to afford the title compound as a colourless oil (186mg, 67%).
¹H nmr : (CDCl₃) 0.83 (2H, m), 1.10-1.30 (8H, m), 1.38 (9H, s), 1.40 (9H, s), 1.60-1.80 (7H, m), 2.61 (1H, dd), 2.79 (1H, dd), 3.43 (1H, m), 4.22 (1H, brs), 4.38 (2H, d), 4.70 (1H, brs).
MS : 509 (MNa⁺)
CHN : Found: C56.20%; H8.67%; N11.32%; C₂₃H₄₂N₄O₅S. 0.15 H₂O requires C56.45%; H8.71%; N11.45%

### Preparations 48 and 49:

### (3R)-3-[3-({[(tert-butylamino)sulfonyl]amino}methyl)-1,2,4-oxadiazol-5-yl]-6-cyclohexylhexanoic acid and (3R)-3-(3-{[(aminosulfonyl)amino]methyl}-1,2,4-oxadiazol-5-yl)-6-cyclohexylhexanoic acid

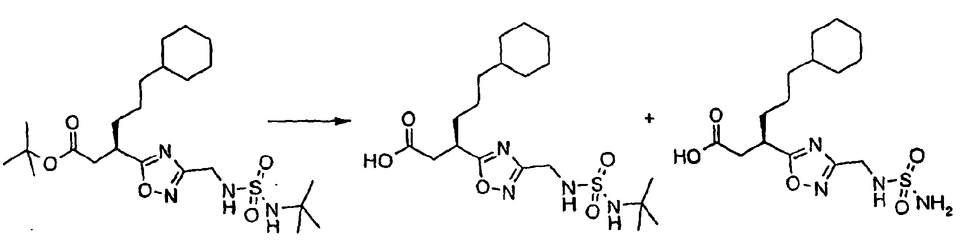

Method as for preparation 11 using *tert*-butyl (3*R*)-3-[3-({[(*tert-*butylamino)sulfonyl]amino}methyl)-1,2,4-oxadiazol-5-yl]-6-cyclohexylhexanoate (preparation 47) (171mg, 0.35mmol) as starting material.
Purification: The crude product was dissolved in EtOAc and washed with H₂O and brine. The orgainc extract was dried over anhydrous MgSO₄ and filtered. The solvent was removed under reduced pressure to afford a mixture of (3*R*)-3-[3-({[(*tert*-butylamino)sulfonyl]amino}methyl)-1,2,4-oxadiazol-5-yl]-6-cyclohexylhexanoic acid and (3*R*)-3-(3-{[(aminosulfonyl)amino]methyl}-1,2,4-oxadiazol-5-yl)-6-cyclohexylhexanoic acid (130mg).

Taken on as a mixture to the following step (see Examples 16,17).

### Preparation 50:

### tert-butyl (3R)-3-{3-[(acetylamino)methyl]-1,2,4-oxadiazol-5-yl}-6-cyclohexylhexanoate

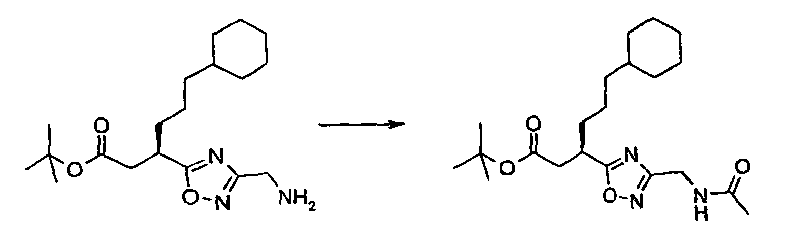

A solution of tert-butyl (3R)-3-[3-(aminomethyl)-1,2,4-oxadiazol-5-yl]-6-cyclohexylhexanoate (preparation 18) (219mg, 0.62mmol) in THF (1 0ml) was treated with AcCl (53µl, 0.75mmol) and stirred at room temperature for 1 hour. More AcCl (200µl) was added and stirred for a further hour. Sat. NaHCO₃ solution was added and the layers separated. The organic extract was washed with brine, dried over anhydrous Na₂SO₄ and filtered. The solvent was removed under reduced pressure to afford the title compound as a colourless oil (247mg, 100%).
¹H nmr : (CDCl₃) 0.83 (2H, m), 1.10-1.35 (8H, m), 1.40 (9H, s), 1.60-1.80 (7H, m), 2.03 (3H, s), 2.60 (1H, dd), 2.79 (1H, dd), 3.42 (1H, m), 4.58 (2H, d), 5.94 (1H, brs).
MS : 416 (MNa⁺)

### Preparation 51:

### (3R)-3-{3-[(acetylamino)methyl]-1,2,4-oxadiazol-5-yl}-6-cyclohexylhexanoic acid

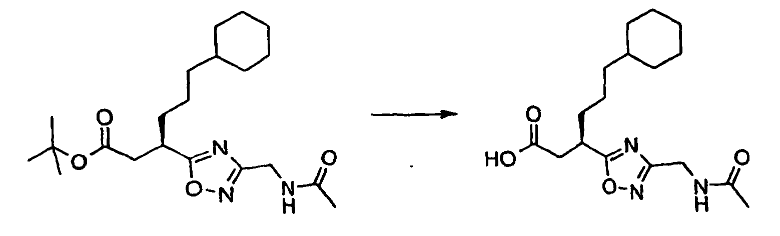

Method as for preparation 7 using *tert*-butyl (3*R*)-3-{3-[(acetylamino)methyl]-1,2,4-oxadiazol-5-yl}-6-cyclohexylhexanoate (preparation 50) (229mg, 0.58mmol) as starting material to afford the title compound as a pale yellow oil (235mg)
¹H nmr : (d₆-DMSO) 0.81 (2H, m), 1.05-1.25 (8H, m), 1.50-1.70 (7H, m), 1.83 (3H, s), 2.70 (2H, m), 3.39 (1H, m), 4.32 (2H, d), 8.34 (1H, brs).
MS : 360 (MNa⁺)
CHN : Found: C57.98%; H8.24%; N10.18%; C₁₇H₂₇N₃O₄. 0.8 H₂O. 0.6 dioxan requires C57.58%; H8.32%; N10.38%

### Preparation 52:

### tert-butyl (3R)-3-(3-{[acetyl(methyl)amino]methyl}-1,2,4-oxadiazol-5-yl)-6-cyclohexylhexanoate

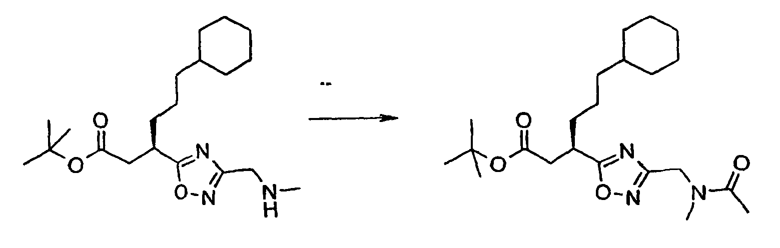

Method as for preparation 50 using *tert*-butyl (3*R*)-6-cyclohexyl-3-{3-[(methylamino)methyl]-1,2,4-oxadiazol-5-yl}hexanoate (preparation 5) as starting material.
Purification: Crude material purified on a silica column eluting with DCM : MeOH (19 : 1) to afford the title compound as a colourless oil (255mg, 94%).
¹H nmr : (CDCl₃) contains rotamers: 0.86 (2H, m), 1.10-1.35 (8H, m), 1.40 (9H, s), 1.60-1.80 (7H, m), 2.10-2.20 (3H, s+s), 2.60 (1H, m), 2.78 (1H, m), 2.98-3.15 (3H, s+s), 3.42 (1H, m), 4.50-4.70 (2H, s+s).
MS : 430 (MNa⁺)

### Preparation 53:

### (3R)-3-(3-{[acetyl(methyl)amino]methyl}-1,2,4-oxadiazol-5-yl)-6-cyclohexylhexanoic acid

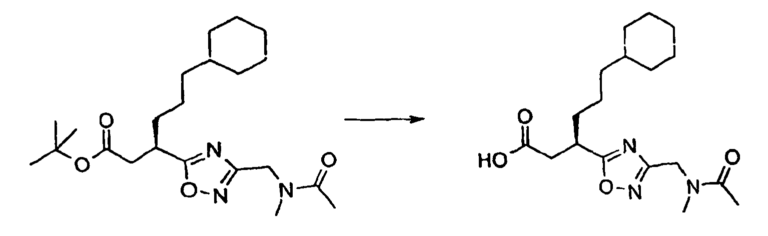

Method as for preparation 7 using *tert*-butyl (3*R*)-3-(3-{[acetyl(methyl)amino]methyl}-1,2,4-oxadiazol-5-yl)-6-cyclohexylhexanoate (preparation 52) (241mg, 0.59mmol) as starting material to afford the title compound as a colourless oil (146mg, 46%).
¹H nmr : (CDCl₃) contains rotamers: 0.83 (2H,br), 1.05-1.50 (8H, br), 1.60-1.90 (7H, br), 2.10-2.20 (3H, s+s), 2.62-3.10 (5H, m), 3.47 (1H, m), 4.50-4.70 (2H, s+s).
MS : 374 (MNa⁺)
CHN : Found: C59.73%; H8.21%; N11.07%; C₁₈H₂₉N₃O₄. 0.45 H₂O. 0.1 dioxan requires C60.00%; H8.40%; N11.41%

### Preparation 54:

### tert-butyl (3R)-6-cyclohexyl-3-(3-{[(cyclopropylcarbonyl)amino]methyl}-1,2,4-oxadiazol-5-yl)hexanoate

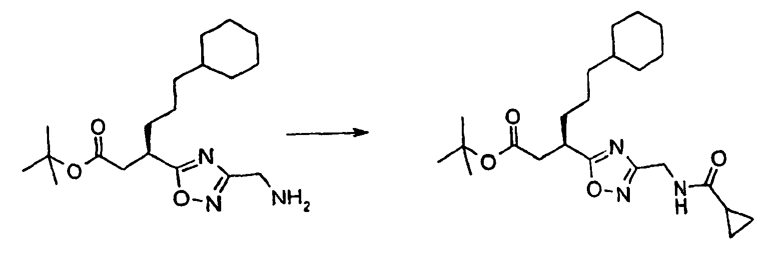

Method as for preparation 50, using tert-butyl (3R)-3-[3-(aminomethyl)-1,2,4-oxadiazol-5-yl]-6-cyclohexylhexanoate (preparation 18) (204mg, 0.57mmol) and cyclopropane carboxylic acid chloride (62µl, 0.68mmol) as starting materials to afford the title compound as a colourless oil (257mg).
¹H nmr: (CDCl₃) 0.75-1.80 (31 H, m), 2.61 (1H, dd), 2.78 (1 H, dd), 3.42 (1 H, m), 4.59 (2H, d), 6.20 (1H, brs).
MS : 442 (MNa⁺)
CHN : Found: C64.74%; H8.80%; N9.12%; C₂₃H₃₇N₃O₄. 0.5 H₂O requires C64.53%; H9.01%; N9.49%

### Preparation 55:

### (3R)-6-cyclohexyl-3-(3-{[(cyclopropylcarbonyl)amino]methyl}-1,2,4-oxadiazol-5-yl)hexanoic acid

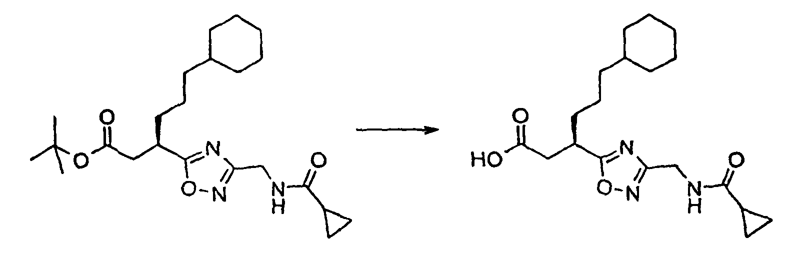

Method as for preparation 7 using *tert*-butyl (3*R*)-6-cyclohexyl-3-(3-{[(cyclopropylcarbonyl)amino]methyl}-1,2,4-oxadiazol-5-yl)hexanoate (preparation 54) (236mg, 0.56mmol) as starting material to afford the title compound as a yellow oil (224mg).
¹H nmr : (d₆DMSO) 0.68 (4H, m), 0.80 (2H, m), 1.05-1.25 (8H, m), 1.55-1.70 (8H, m), 2.69 (2H, m), 3.38 (1H, m), 4.36 (2H, d), 8.51 (1H, brs).
MS : 386 (MNa⁺)
CHN : Found: C60.81 %; H8.09%; N9.47%; C₁₉H₂₉N₃O₄. 0.2 H₂O. 0.7 dioxan requires C61.07%; H8.23%; N9.80%

### Preparation 56:

### tert-butyl (3R)-6-cyclohexyl-3-(3-{[(methoxyacetyl)amino]methyl}-1,2,4-oxadiazol-5-yl)hexanoate

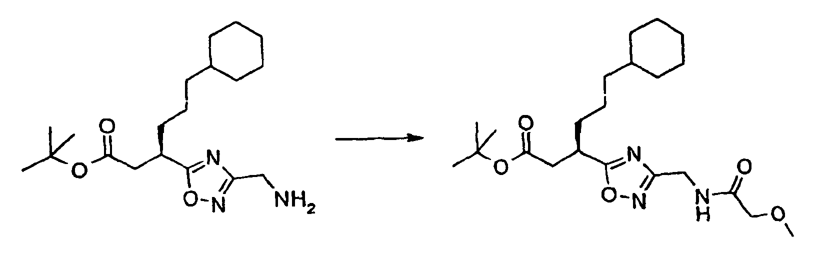

A solution of tert-butyl (3R)-3-[3-(aminomethyl)-1,2,4-oxadiazol-5-yl]-6-cyclohexylhexanoate (preparation 18) (206mg, 0.57mmol) and methoxyacetic acid (48µl, 0.63mmol) in DCM (5ml) was treated with a solution of WSCDI (120mg, 0.63mmol), HOBt (85mg, 0.63mmol) and NMM (69µl, 0.63mmol) in DCM (10ml) and stirred at room temperature for 18 hours. The reaction mixture was washed with sat. NaHCO₃ solution followed by brine, dried over MgSO₄ and filtered. The solvent was removed under reduced pressure. The residue was purified on a silica column eluting with Et₂O to afford the title compound as a colourless oil (230mg, 93%).
¹H nmr : (CDCl₃) 0.82 (2H, m), 1.10-1.30 (8H, m), 1.40 (9H, s), 1.60-1.80 (7H, m), 2.60 (1H, dd), 2.79 (1H, dd), 3.40-3.50 (4H, s+m), 3.97 (2H, s), 4.60 (2H, d), 7.00 (1H, brs).
MS : 446 (MNa⁺)
CHN : Found: C62.28%; H8.84%; N9.62%; C₂₂H₃₇N₃O₅ requires C62.39%; H8.81%; N9.92%

### Preparation 57:

### (3R)-6-cyclohexyl-3-(3-{[(methoxyacetyl)amino]methyl}-1,2,4-oxadiazol-5-yl)hexanoic acid

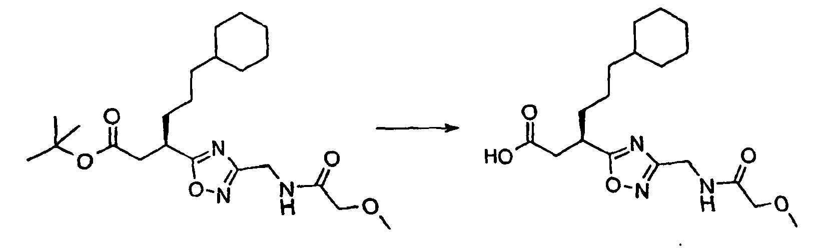

Method as for preparation 7 using *tert*-butyl (3*R*)-6-cyclohexyl-3-(3-{[(methoxyacetyl)amino]methyl}-1,2,4-oxadiazol-5-yl)hexanoate (preparation 56) (216mg, 0.51 mmol) as starting material to afford the title compound as a yellow oil (196mg).
¹H nmr : (d₆DMSO) 0.80 (2H, m), 1.05-1.25 (8H, m), 1.50-1.70 (7H, m), 2.68 (2H, m), 3.32 (3H, s), 3.39 (1H, m), 3.81 (2H, s), 4.39 (2H, d), 8.21 (1H, brs).
MS : 368 (MH⁺)
CHN : Found: C58.39%; H8.18%; N10.05%; C₁₈H₂₉N₃O₅. 0.4 dioxan requires C58.46%; H8.06%; N10.43%

### Preparation 58:

### tert-butyl (3R)-6-cyclohexyl-3-[3-({[(dimethylamino)acetyl]amino}methyl)-1,2,4-oxadiazol-5-yl]hexanoate

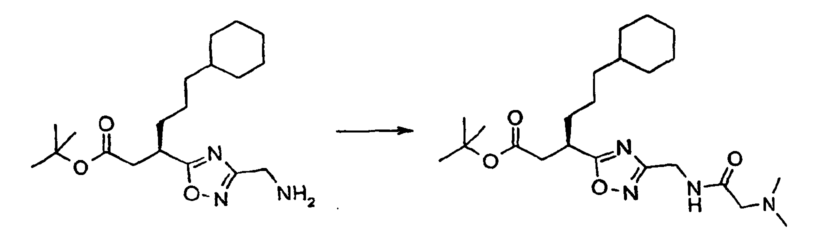

Method as for preparation 56 using tert-butyl (3R)-3-[3-(aminomethyl)-1,2,4-oxadiazol-5-yl]-6-cyclohexylhexanoate (preparation 18) (206mg, 0.57mmol) and *N,N*-dimethylglycine (65mg, 0.63mmol) as starting materials.
Purification: The crude material was purified on a silica column eluting with Et₂O : MeOH (9 : 1) to afford the title compound as a colourless oil (227mg, 89%).
¹H nmr : (CDCl₃) 0.83 (2H, m), 1.10-1.35 (8H, m), 1.40 (9H, s), 1.60-1.75 (7H, m), 2.32 (6H, s), 2.61 (1H, dd), 2.79 (1H, dd), 3.00 (2H, s), 2.44 (1H, m), 4.60 (2H, d), 7.60 (1H, brs).
MS : 459 (MNa⁺)
CHN : Found: C63.04%; H9.30%; N12.67%; C₂₃H₄₀N₄O₄ requires C63.27%; H9.23%; N12.83%

### Preparation 59:

### (3R)-6-cyclohexyl-3-[3-({[(dimethylamino)acetyl]amino}methyl)-1,2,4-oxadiazol-5-yl]hexanoic acid

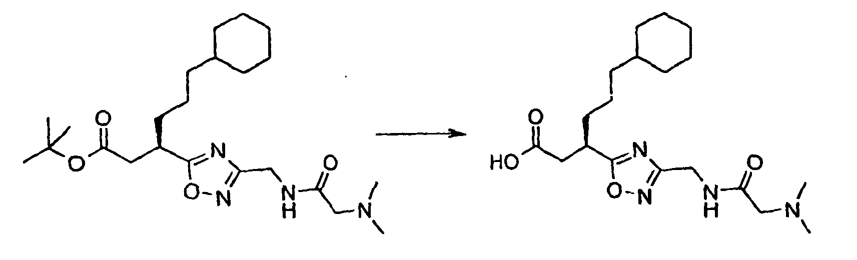

Method as for preparation 7 using *tert*-butyl (3*R*)-6-cyclohexyl-3-[3-({[(dimethylamino)acetyl]amino}methyl)-1,2,4-oxadiazol-5-yl]hexanoate (preparation 58) (216mg, 0.49mmol) as starting material to afford the title compound as a white solid (225mg).
¹H nmr : (d₆DMSO) 0.81 (2H, m), 1.05-1.25 (8H, m), 1.50-1.70 (8H, m), 2.70 (2H, m), 2.79 (6H, s), 3.39 (1H, m), 3.93 (2H, s), 4.44 (2H, d), 9.15 (1H, brs).
MS : 381 (MH⁺)
CHN : Found: C54. 46%; H7.96%; N13.17%; C₁₉H₃₂N₄O₄. HCl requires C54.73%; H7.98%; N13.44%

### Preparation 60:

### tert-butyl (3R)-3-{3-[(benzoylamino)methyl]-1,2,4-oxadiazol-5-yl}-6-cyclohexylhexanoate

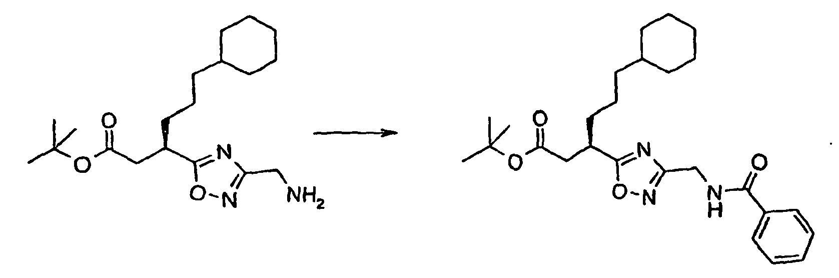

Method as for preparation 50 using tert-butyl (3R)-3-[3-(aminomethyl)-1,2,4-oxadiazol-5-yl]-6-cyclohexylhexanoate (preparation 18) (200mg, 0.57mmol) and benzoylchloride (79µl, 0.68mmol) as starting materials.
Purification: The crude material was purified on a silica column eluting with Et₂O : pentane(1 : 1) to afford the title compound as a colourless oil (243mg, 94%).
¹H nmr : (CDCl₃) 0.82 (2H, m), 1.10-1.80 (24H, m), 2.61 (1H, dd), 2.79 (1H, dd), 3.43 (1H, m), 4.79 (2H, d), 6.60 (1H, brs), 7.42 (2H, t), 7.51 (2H, d), 7.80 (2H, d).
MS : 478 (MNa⁺)
CHN : Found: C68.40%; H8.26%; N9.04%; C₂₆H₃₇N₃O₄. 0.1 H₂O. 0.1 Et₂O requires C68.22%; H8.28%; N9.04%

### Preparation 61:

### (3R)-3-{3-[(benzoylamino)methyl]-1,2,4-oxadiazol-5-yl}-6-cyclohexylhexanoic acid

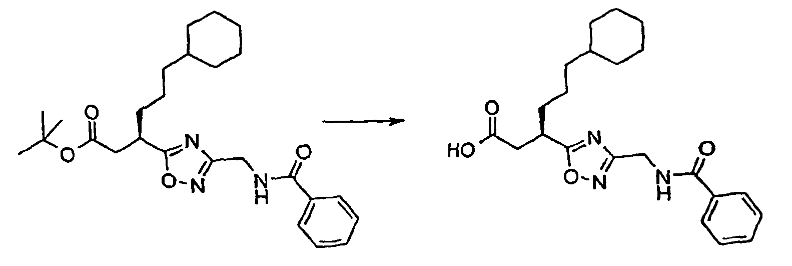

Method as for preparation 7 using *tert*-butyl (3*R*)-3-{3-[(benzoylamino)methyl]-1,2,4-oxadiazol-5-yl}-6-cyclohexylhexanoate (preparation 60) (227mg, 0.50mmol) as starting material to afford the title compound as a yellow oil (227mg).
¹H nmr : (d₆DMSO) 0.79 (2H, m), 1.00-125 (8H, m), 1.50-1.70 (7H, m), 2.68 (2H, m), 3.39 (1H, m), 4.54 (2H, d), 7.43 (2H, t), 7.50 (1H, t), 7.82 (2H, d), 8.98 (1H, brs).
MS : 422 (MNa⁺)
CHN : Found: C64.32%; H7.64%; N8.73%; C₂₂H₂₉N₃O₄. 0.7 dioxan requires C64.59%; H7.56%; N9.11%

### Preparation 62:

### tert-butyl (3R)-6-cyclohexyl-3-(3-{[(2-pyridinylcarbonyl)amino]methyl}-1,2,4-oxadiazol-5-yl)hexanoate

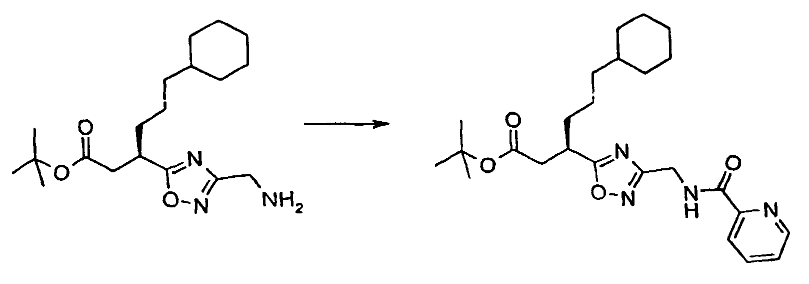

Method as for preparation 56 using tert-butyl (3R)-3-[3-(aminomethyl)-1,2,4-oxadiazol-5-yl]-6-cyclohexylhexanoate (preparation 18) (202mg, 0.57mmol) and picolininc acid (77mg, 0.63mmol) as starting materials.
Purification: The crude material was purified on a silica column eluting with pentane : Et₂O (1 : 1) to afford the title compound as a colourless oil.
¹H nmr : (CDCl₃) 0.81 (2H, m), 1.05-1.35 (8H, m), 1.39 (9H, s), 1.55-1.80 (7H, m), 2.60 (1H, dd), 2.80 (1H, dd), 3.43 (1H, m), 4.79 (2H, d), 7.41 (1H, m), 7.82 (1H, t), 8.20 (1H, d), 8.50 (1H, brs), 8.59 (1H, d).
MS : 479 (MNa⁺)
CHN : Found: C65.39%; H7.98%; N12.00%; C₂₅H₃₆N₄O₄ requires C65.77%; H7.95%; N12.27%

### Preparation 63:

### (3R)-6-cyclohexyl-3-(3-{[(2-pyridinylcarbonyl)amino]methyl}-1,2,4-oxadiazol-5-yl)hexanoic acid

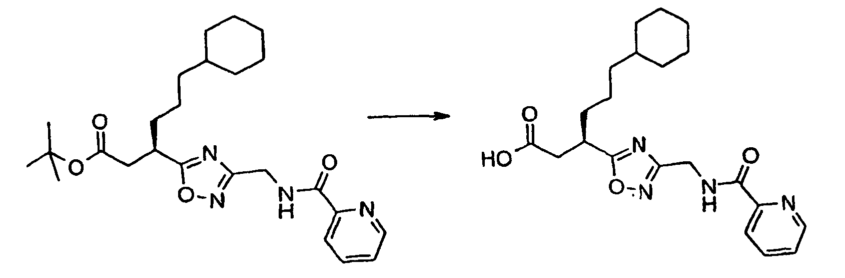

Method as for preparation 7 using *tert*-butyl (3*R*)-6-cyclohexyl-3-(3-{[(2-pyridinylcarbonyl)amino]methyl}-1,2,4-oxadiazol-5-yl)hexanoate (preparation 62) (216mg, 0.47mmol) as starting material to afford the title compound as a yellow oil (213mg).
¹H nmr: (d₆DMSO) 0.77 (2H, m), 1.00-9.20 (8H, m), 1.50-1.65 (7H, m), 2.79 (2H, m), 3.40 (1H, m), 4.58 (2H, d), 7.60 (1H, t), 8.00 (2H, m), 8.62 (1H, d), 9.30 (1H, t).
MS : 399 (M-H)
CHN : Found: C61.20%; H7.16%; N12.65%; C₂₁H₂₈N₄O₄. 0.4 H₂O. 0.3 dioxan requires C61.42%; H7.24%; N12.91%

### Preparation 64:

### tert-butyl (3R)-3-(3-{[(aminocarbonyl)amino]methyl}-1,2,4-oxadiazol-5-yl)-6-cyclohexylhexanoate

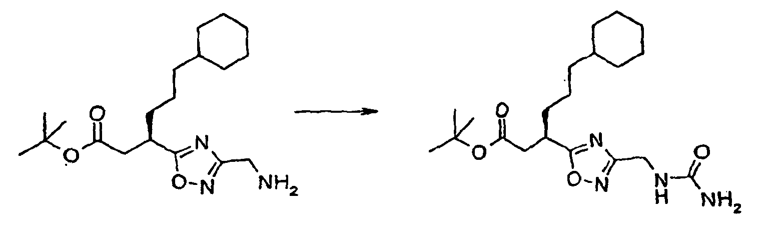

A solution of tert-butyl (3R)-3-[3-(aminomethyl)-1,2,4-oxadiazol-5-yl]-6-cyclohexythexanoate (preparation 18) (310mg, 0.88mmol) in MeCN (10ml) was treated with N,N-disuccinimidyl carbonate (271 mg, 1.06mmol) and stirred at room temperature, under a nitrogen atmosphere for 4.5 hours. NH₃ (0.5M in dioxan) was added forming a white precipitate. The reaction mixture was stirred at room temperature for 18hours. The reaction mixture was filtered and the solvent removed under reduced pressure. The residue was purified on a silica column eluting with a solvent gradient of DCM : MeOH (100 : 0) gradually changing to (95: 5) to afford the title compound as a colourless oil (253mg, 73%).
¹H nmr : (CDCl₃) 0.83 (2H, m), 1.10-1.35 (8H, m), 1.40 (9H, s), 1.55-1.80 (7H, m), 2.60 (1H, dd), 2.78 (1H, dd). 3.42 (1H, m), 4.54 (2H, d), 5.08 (1H, brs).

### Preparation 65:

### (3R)-3-(3-{[(aminocarbonyl)amino]methyl}-1,2,4-oxadiazol-5-yl)-6-cyclohexylhexanoic acid

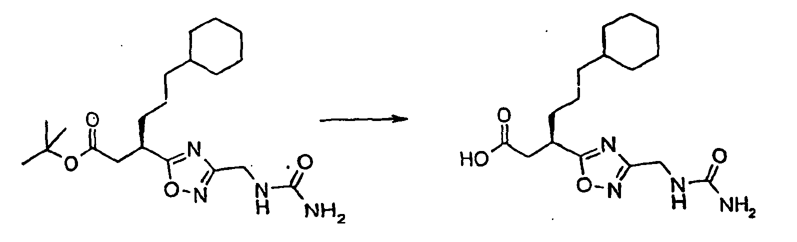

Method as for preparation 11 using *tert*-butyl (3*R*)-3-(3-{[(aminocarbonyl)amino]methyl}-1,2,4-oxadiazol-5-yl)-6-cyclohexylhexanoate (preparation 64) (261mg, 0.66mmol) as starting material to afford the title compound as a yellow oil (191 mg, 86%).
¹H nmr : (CDCl₃) 0.85 (2H, m), 1.10-1.40 (8H, m), 1.60-1.85 (7H, m), 2.80 (2H, m), 3.44 (1H, m), 4.45 (2H, d).

### Preparation 66:

### tert-butyl (3R)-6-cyclohexyl-3-[3-({[(methylamino)carbonyl]amino}methyl)-1,2,4-oxadiazol-5-yl]hexanoate

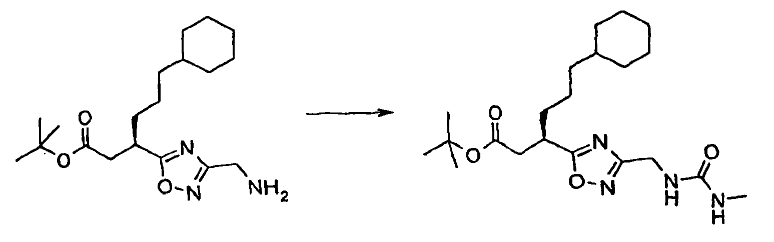

A solution of tert-butyl (3R)-3-[3-(aminomethyl)-1,2,4-oxadiazol-5-yl]-6-cyclohexylhexanoate (preparation 18) (148mg, 0.42mmol) in MeCN (10ml) was treated with N,N-disuccinimidyl carbonate (130mg, 0.51mmol) and stirred at room temperature, under a nitrogen atmosphere for 4 hours. The solvent was removed under reduced pressure. The oil was dissolved in THF (5ml) and treated with MeNH₂ (2.0M In THF) (1.0ml) forming a white precipitate. The reaction mixture was stirred at room temperature for 2 days and filtered. The solvent was removed under reduced pressure. The oil was purified on a silica column eluting with a solvent gradient of DCM : MeOH (95 : 5) gradually changing to (90 : 10) to afford the title compound as a colourless oil (134mg, 78%).
¹H nmr: (CDCl₃) 0.82 (2H, m), 1.10-1.30 (8H, m),1.39 (9H, s), 1.60-1.80 (7H, m), 2.60 (1H, dd), 2.78 (1H, dd), 2.80 (3H, d), 3.42 (1H, m), 4.50 (2H, d), 4.83 (1H, brs).
MS : 431 (MNa⁺)

### Preparation 67:

### (3R)-6-cyclohexyl-3-[3-({[(methylamino)carbonyl]amino}methyl)-1,2,4-oxadiazol-5-yl]hexanoic acid

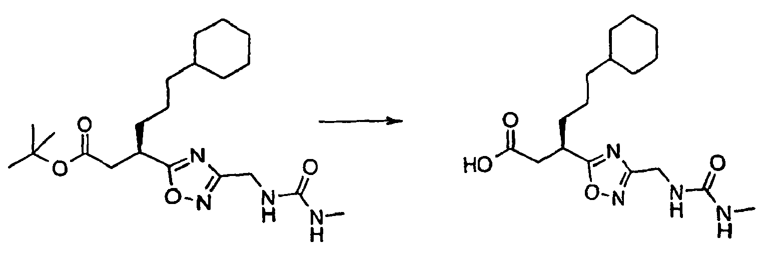

Method as for preparation 11 using *tert*-butyl (3R)-6-cyclohexyl-3-[3-({[(methylamino)carbonyl]amino}methyl)-1,2,4-oxadiazol-5-yl]hexanoate (preparation 66) (140mg, 0.34mmol) as starting material to afford the title compound as a colourless oil (141mg).
¹H nmr : (d₆DMSO) 0.81 (2H, m), 1.05-1.30 (8H, m), 1.50-1.70 (7H, m), 2.56 (3H, d), 2.67 (2H, m), 3.38 (1 H, m), 4.25 (2H, s).
MS : 375 (MNa⁺)

### Preparation 68:

### tert-butyl (3R)-6-cyclohexyl-3-[3-({[(dimethylamino)carbonyl]amino}methyl)-1,2,4-oxadiazol-5-yl]hexanoate

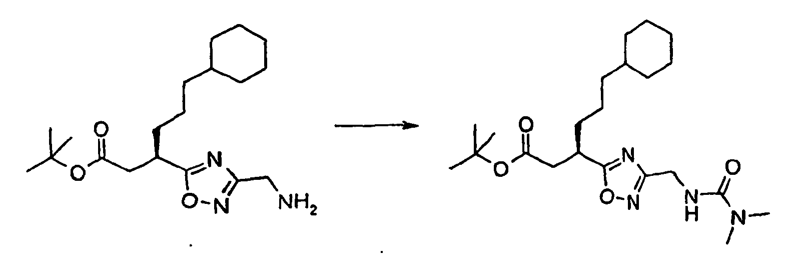

Method as for preparation 66 using tert-butyl (3R)-3-[3-(aminomethyl)-1,2,4-oxadiazol-5-yl]-6-cyclohexylhexanoate (preparation 18) (310mg, 0.88mmol) and Me₂NH (2.0M in THF) (1.80ml, 3.52mmol) as starting materials.
Purification: The crude material was purified on a silica column eluting with a solvent gradient of DCM : MeOH (98: 2) gradually changing to (95 : 5) to afford the title compound (309mg, 83%).
¹H nmr : (CDCl₃)0.81 (2H, m), 1.10-1.30 (8H, m), 1.39 (9H, s), 1.60-1.80 (7H, m), 2.61 (1H, dd), 2.78 (1H, m), 2.94 (6H, s), 3.42 (1H, m), 4.57 (2H, m), 5.01 (1H, brs).
MS : 423 (MH⁺)

### Preparation 69:

### (3R)-6-cyclohexyl-3-[3-({[(dimethylamino)carbonyl]amino}methyl)-1,2,4-oxadiazol-5-yl]hexanoic acid

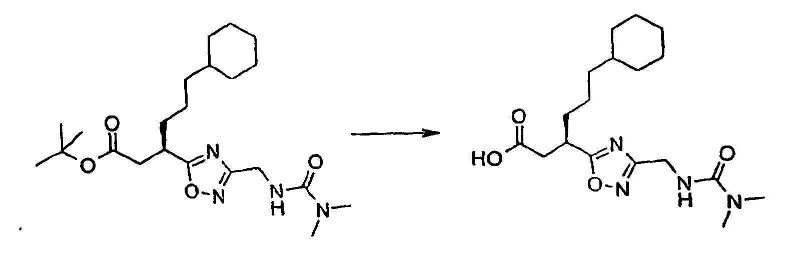

Method as for preparation 11 using *tert*-butyl (3*R*)-6-cyclohexyl-3-[3-({[(dimethylamino)carbonyl]amino}methyl)-1,2,4-oxadiazol-5-yl]hexanoate (preparation 68) 9298mg, 0.71mmol) as starting material.
Purification: The crude material was purified on a silica column eluting with a solvent gardient of DCM : MeOH (98 : 2) gradually changing to (90 : 10) to afford the title compound (214mg, 83%).
¹H nmr : (CDCl₃) 0.80 (2H, m), 1.05-1.30 (8H, m), 1.60-1.80 (7H, m), 2.73 (1H, dd), 2.92 (7H, m), 3.49 (1H, m), 4.51 (2H, m), 5.37 (1H, brs).
MS : 365 (M-H)

### Preparation 70:

### (3R)-3-(3-{[(tert-butoxycarbonyl)amino]methyl}-1,2,4-oxadiazol-5-yl)-6-cyclohexylhexanoic acid

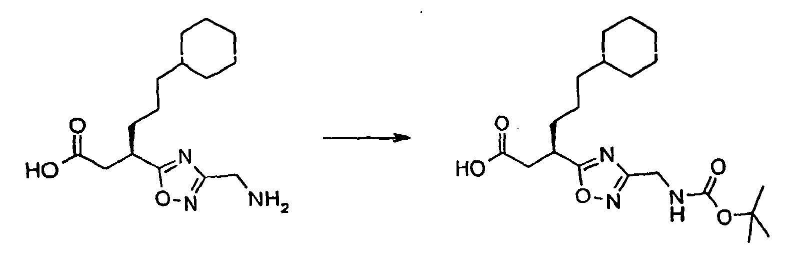

A solution of (3*R*)-3-[3-(aminomethyl)-1,2,4-oxadiazol-5-yl]-6-cyclohexylhexanoic acid (preparation 163) (177mg, 0.53mmol) and Et₃N (186µl, 1.33mmol) In dioxan (500µl) and H₂O (500µl) was treated with Boc-ON (145mg, 0.58mmol) and stirred at room temperature for 18hours. The reaction mixture was diluted with H₂O and extracted with EtOAc (x2). The organic extracts were combined and the solvent removed under reduced pressure. The crude material was purified on a silica column eluting with DCM : MeOH (100 : 0) gradually changing to (90 : 10) to afford the title compound as a colourless gum (115mg, 55%).
¹H nmr : (CDCl₃) 0.84 (2H, m), 1.10-1.35 (8H, m), 1.42 (9H, s), 1.60-1.80 (7H, m), 2.74 (1H, dd), 2.94 (1H, dd), 3.48 (1H, m), 4.40 (2H, d).
MS : 394 (M-H)
Accurate mass : Found 396.2500 (MH⁺), Calculated C₂₀H₃₄N₃O₅, 396.2493

### Preparation 71:

### tert-butyl (3R)-6-cyclohexyl-3-[3-(hydrazinocarbonyl)-1,2,4-oxadiazol-5-yl]hexanoate

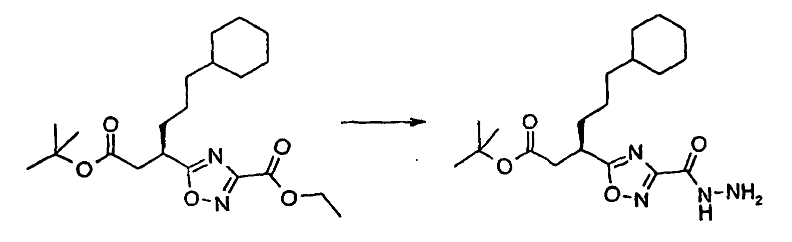

A solution of ethyl 5-[(1*R*)-1-(2-*tert*-butoxy-2-oxoethyl)-4-cyclohexylbutyl]-1,2,4-oxadiazole-3-carboxylate (preparation 170) (1.14g, 2.89mmol) in EtOH (20ml) was treated with NH₂NH₂.H₂O (280µl, 5.78mmol) and stirred at room temperature for 45 minutes. The solvent was removed under reduced pressure. The residue was partitioned between EtOAc and H₂O/brine. The organic solvent was removed under reduced pressure to afford the title compound (1.06g, 90%).
¹H nmr: (d₆DMSO) 0.80 (2H, m), 1.00-1.25 (8H, m), 1.38 (9H, s). 1.50-1.70 (7H, m), 2.74 (2H, d), 3.42 (1H, m), 4.66 (2H, brs), 10.20 (1H, brs).
MS : 403 (MNa⁺)

### Preparation 72:

### (3R)-3-(3-amino-1,2,4-oxadiazol-5-yl)-6-cyclohexylhexanoic acid

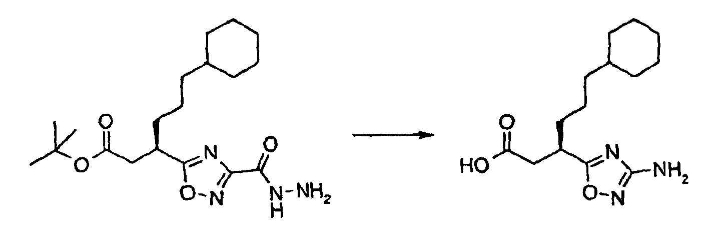

A solution of *tert*-butyl (3*R*)-6-cyclohexyl-3-[3-(hydrazinocarbonyl)-1,2,4-oxadiazol-5-yl]hexanoate (preparation 71) (1.06g, 2.76mmol) in glacial AcOH (10ml) was treated with 2M HCl (10ml) and stirred at room temperature for 30 minutes. The reaction mixture was cooled to 0°C before being treated with a solution of NaNO₂ (278mg, in 5ml H₂O) making sure the temperature never rose above 5°C. The reaction mixture was stirred at 0°C for 30 minutes and then allowed to warm to room temperature before being heated at 85°C until the evolution of gas a finished. The reaction mixture was neutralised with sat. Na₂CO₃ solution and extracted with EtOAc. The organic extract was washed with brine and the solvent removed under reduced pressure. The residue was treated with TFA (4ml) and stirred at room temperature for 1 hour. The solvent was removed under reduced pressure and the residue azeotroped from toluene (x3) and DCM (x3) to afford the title compound as a foam (390mg, 50%).
¹H nmr: (CDCl₃) 0.82 (2H, m), 1.05-1.40 (8H, m), 1.55-1.85 (7H, m), 2.70 (1H, dd), 2.86 (1H, dd), 3.38 (1H, m), 4.58 (2H, brs).
MS : 304 (MNa⁺)

### Preparation 73:

### (3R)-6-cyclohexyl-3-{3-[(methylamino)methyl]-1,2,4-oxadiazol-5-yl}hexanoic acid

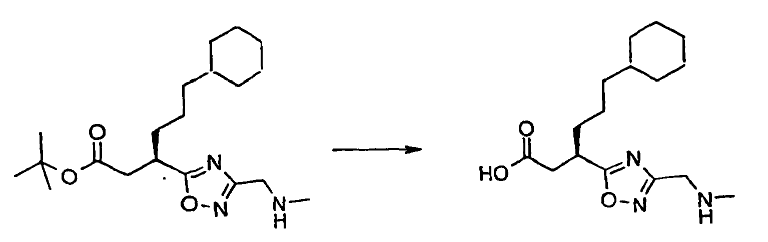

Method as for preparation 7 using *tert*-butyl (3*R*)-6-cyclohexyl-3-{3-[(methylamino)methyl]-1,2,4-oxadiazol-5-yl}hexanoate (preparation 5) (400mg, 1.09mmol) as starting material to afford the title compound as a yellow oil (389mg).
¹H nmr : (CD₃OD) 0.85 (2H, m), 1.10-1.40 (9H, m), 1.60-1.80 (6H, m), 2.75-2.95 (5H, s+m), 3.54 (1H, m), 4.40 (2H, s).
MS : 310 (MH⁺)
CHN : Found: C55.44%; H8.34%; N10.13%; C₁₆H₂₇N₃O₃. 0.1 H₂O. 0.6 dioxan. HCl requires C55.18%; H8.30%; N10.49%

### Preparation 74:

### tert-butyl (3R)-6-cyclohexyl-3-{3-[(ethylamino)methyl]-1,2,4-oxadiazol-5-yl}hexanoate

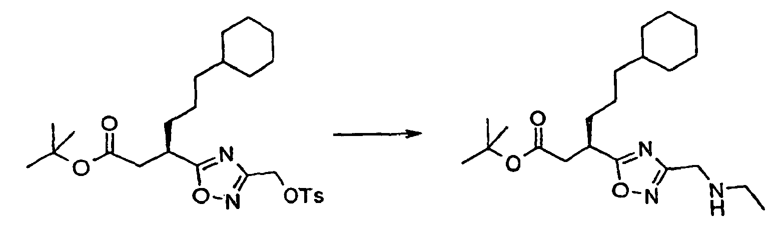

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (750mg, 1.48mmol) and ethylamine (2M in THF, 2.22ml, 4.4mmol) as starting materials. Purification: The crude material was purified on a silica column eluting with a solvent gradient of DCM : Et₂O (90 : 10) followed by (0 : 100) to afford the title compound as a runny oil (275mg, 49%).
¹H nmr : (CDCl₃) 0.82 (2H, m), 1.10-1.30 (11H, m), 1.39 (9H, s), 1.60-1.80 (7H, m), 2.55-2.70 (3H, m), 2.79 (1H, dd), 3.43 (1H, m), 3.91 (2H, s).
MS : 380 (MH⁺)

### Preparation 75:

### (3R)-6-cyclohexyl-3-{3-[(ethylamino)methyl]-1,2,4-oxadiazol-5-yl}hexanoic acid

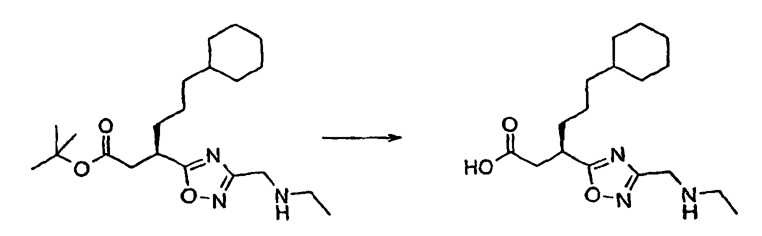

*tert*-butyl (3*R*)-6-cyclohexyl-3-{3-[(ethylamino)methyl]-1,2,4-oxadiazol-5-yl}hexanoate (preparation 74) (288mg, 0.76mmol) was treated with TFA (5ml) and stirred at room temperature for 1 hour. The reaction mixture was diluted with DCM and the solvents removed under reduced pressure. The residue was azeotroped from toluene (x3) and DCM (x3) to afford the title compound (373mg).
¹H nmr: (CDCl₃) 0.81 (2H, m), 1.10-1.30 (8H, m), 1.36 (3H, t), 1.60-1.80 (7H, m), 2.78 (1H, dd), 2.93 (1H, dd), 3.18 (2H, q), 3.43 (1H, m), 4.24 (2H, s).
MS : 324 (MH⁺)
CHN : Found: C49.84%; H6.55%; N8.65%; C₁₇H₂₉N₃O₃. 1.3 TFA requires C49.91%; H6.48%; N8.91%

### Preparation 76:

### tert-butyl (3R)-6-cyclohexyl-3-{3-[(propylamino)methyl]-1,2,4-oxadiazol-5-yl}hexanoate

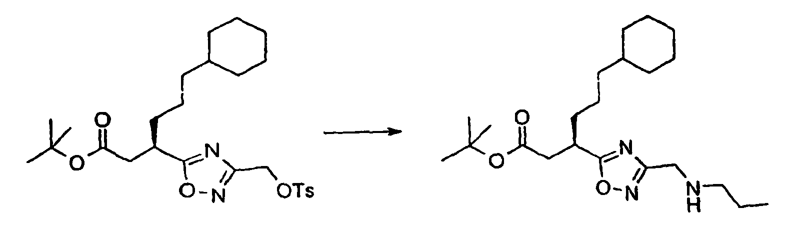

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 1.00mmol) and propylamine (175mg, 3.00mmol) as starting materials.
Purification: The crude material was purified on a silica column eluting with a solvent gradient of DCM : Et₂O (90 : 10) followed by (0 : 100) to afford the title compound as a colourless oil (257mg, 66%).
¹H nmr: (CDCl₃) 0.80 (2H, m), 0.91 (3H, t), 1.10-1.30 (8H, m), 1.39 (9H, s), 1.151 (2H, m), 1.60-1,80 (7H, m), 2.55-2.6 (3H, t+dd), 2.79 (1H, dd), 3.43 (1H, m), 3.91 (2H, s).
MS : 394 (MH⁺)
CHN : Found: C67.05%; H10.15%; N10.63%; C₂₂H₃₉N₃O₃ requires C67.14; H9.99%; N10.68%

### Preparation 77:

### (3R)-6-cyclohexyl-3-{3-[(propylamino)methyl]-1,2,4-oxadiazol-5-yl}hexanoic acid

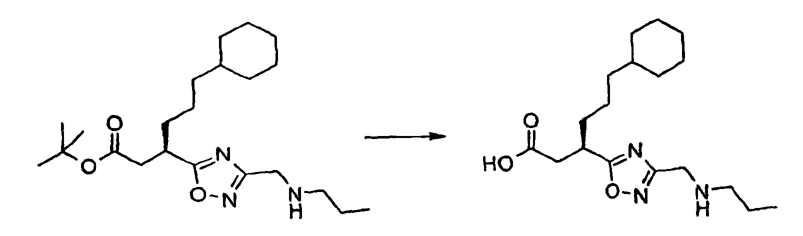

Method as for preparation 75 using *tert*-butyl (3*R*)-6-cyclohexyl-3-{3-[(propylamino)methyl]-1,2,4-oxadiazol-5-yl}hexanoate (preparation 76) (257mg, 0.65mmol) as starting material.
Purification: Crude material purified on a silica column eluting with DCM : MeOH (90 : 10) to afford the title compound (272mg).
¹H nmr: (CDCl₃) 0.81 (2H, m), 0.99 (3H, t), 1.05-1.30 (8H, m), 1.60-1.70 (7H, m), 1.77 (2H, m), 2.77 (1H. dd), 2.91 (1H, dd), 3.03 (2H, t), 3.41 (1H, m), 4.20 (2H, s).
MS : 338 (MH⁺)
CHN : Found: C50.22%; H6.75%; N8.50%; C₁₈H₃₁N₃O₃. 1.4 TFA requires C50.26; H6.57%; N8.45%

### Preparation 78:

### tert-butyl (3R)-6-cyclohexyl-3-{3-[(isopropylamino)methyl]-1,2,4-oxadiazol-5-yl}hexanoate

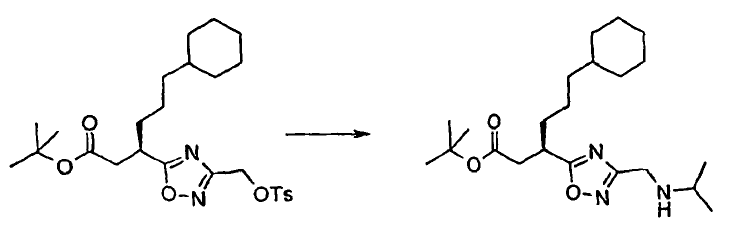

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (490mg, 0.97mmol) and isopropylamine (175mg, 3.00mmol) as starting materials.
Purification: Crude material was purified on a silica column eluting with DCM : MeOH (97 : 3) to afford the title compound as a yellow oil (323mg, 85%).
¹H nmr: (CDCl₃) 0.81 (2H, m), 1.06 (6H, d), 1.10-1.30 (8H, m), 1.39 (9H, s), 1.60-1.80 (7H, m), 2.61 (1H, dd), 2.80 (2H, m), 3.44 (1H, m), 3.91 (2H, s).
MS : 394 (MH⁺)

### Preparation 79:

### (3R)-6-cyclohexyl-3-{3-[(isopropylamino)methyl]-1,2,4-oxadiazol-5-yl}hexanoic acid

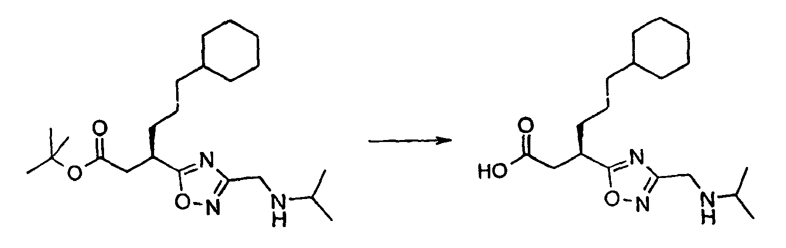

Method as for preparation 75 using *tert*-butyl (3*R*)-6-cyclohexyl-3-{3-[(isopropylamino)methyl]-1,2.4-oxadiazol-5-yl}hexanoate (preparation 78) (323mg, 0.82mmol) as starting material to afford the title compound as a yellow gum (390mg, 100%).
¹H nmr : (CDCl₃) 0.81 (2H, m), 1.10-1.30 (8H, m), 1.40 (6H, d), 1.60-1.80 (7H, m), 2.79 (1H, dd), 2.97 (2H, m), 3.42 (1H, m), 4.18 (2H, m).
MS : 338 (MH⁺)

### Preparation 80:

### tert-butyl (3R)-6-cyclohexyl-3-{3-[(isobutylamino)methyl]-1,2,4-oxadiazol-5-yl}hexanoate

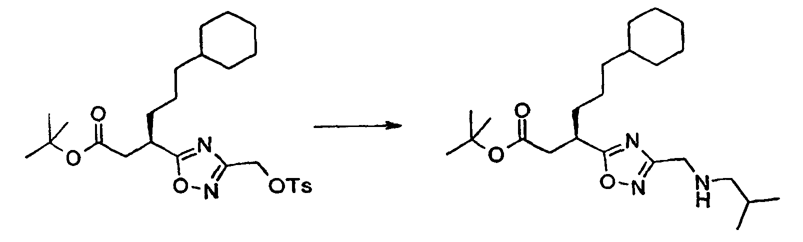

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (750mg, 1.48mmol) and isobutylamine (325mg, 4.44mmol) as starting materials.
Purification: The crude material was purified on a silica column eluting with a solvent gradient of DCM: Et₂O (90 : 10) followed by (0 : 100) to afford the title compound as a colourless oil (437mg, 72%).
¹H nmr : (CDCl₃) 0.81 (2H, m), 1.92 (6H, d), 1.10-1.30 (8H, m), 1.40 (9H, s), 1.48 (1H, brs), 1.60-1.80 (7H, m), 2.41 (2H, d), 2.61 (1H, dd), 2.80 (1H, dd), 3.43 (1H, m), 3.88 (2H, s).
MS : 408 (MH⁺)

### Preparation 81;

### (3R)-6-cyclohexyl-3-{3-[(isobutylamino)methyl]-1,2,4-oxadiazol-5-yl}hexanoic acid

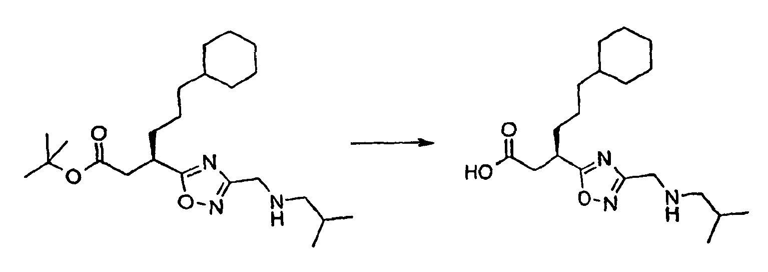

Method as for preparation 75 using *tert*-butyl (3*R*)-6-cyclohexyl-3-{3-[(isobutylamino)methyl]-1,2,4-oxadiazol-5-yl}hexanoate (preparation 80) (437mg, 1.1mmol) as starting material to afford the title compound (506mg).
¹H nmr : (CDCl₃) 0.82 (2H, m), 1.00 (6H, d), 1.10-1.35 (8H, m), 1.60-1.80 (7H, m), 2.09 (1H, m), 2.78 (1H, dd), 2.93 (3H, dd+d), 3.43 (1H, m), 4.22 (2H, s).
MS : 352 (MH⁺)
CHN : Found: C51.15%; H6.98%; N8.20%; C₁₉H₃₃N₃O₃. 1.4 TFA requires C51.23; H6.78%; N8.22%

### Preparation 82:

### tert-butyl (3R)-3-{3-[(tert-butylamino)methyl]-1,2,4-oxadiazol-5-yl}-6-cyclohexylhexanoate

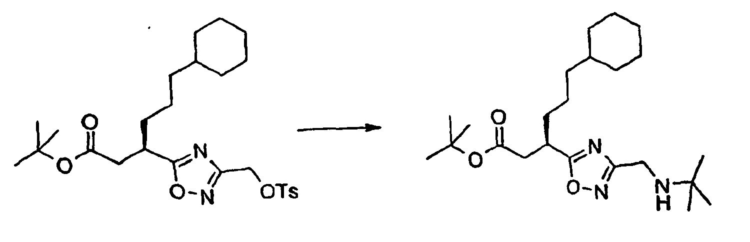

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and *tert*-butylamine (216mg, 2.96mmol) as starting materials.
Purification: The crude material was purified on a silica column eluting with a solvent gradient of DCM : Et₂O (90 : 10) followed by (0 : 100) to afford the title compound as a colourless oil (253mg, 63%).
¹H nmr : (CDCl₃) 0.82 (2H, m), 1.10-1.30 (17H, s+m), 1.39 (9H, s), 1.60-1.80 (7H, m), 2.60 (1H, dd), 2.78 (1H, dd), 3.43 (1H, m), 3.88 (2H, s).
MS : 408 (MH⁺)
CHN : Found: C67.48%; H10.20%; N10.29%; C₂₃H₄₁N₃O₃ requires C67.78; H10.14%; N10.31%

### Preparation 83:

### (3R)-3-{3-[(tert-butylamino)methyl]-1,2,4-oxadiazol-5-yl}-6-cyclohexythexanoic acid

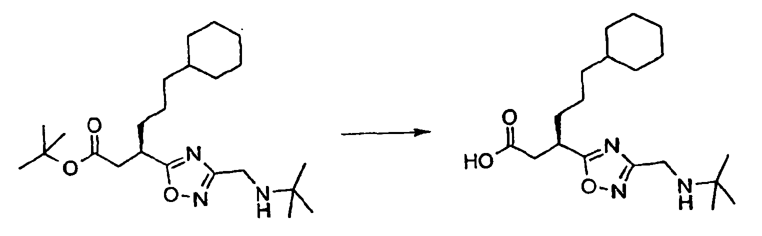

Method as for preparation 75 using *tert*-butyl (3*R*)-3-{3-[(*tert*-butylamino)methyl]-1,2,4-oxadiazol-5-yl}-6-cydobexylhexanoate (preparation 82) (240mg, 0.59mmol) as starting material to afford the title compound (300mg)
¹H nmr : (CDCl₃) 0.82 (2H, m), 1.10-1.30 (8H, s+m), 1.40 (9H, s), 1.50-1.70 (7H, m), 2.80 (1H, d), 3.05 (1H, dd), 3.40 (1H, m), 3.79 (1H, d), 4.12 (1H, d).
MS : 352 (MH⁺)
CHN : Found: C51.29%; H6.93%; N8.17%; C₁₉H₃₃N₃O₃. 1.4 TFA requires C51.23; H6.78%; N8.22%

### Preparation 84:

### tert-butyl (3R)-6-cyclohexyl-3-(3-{[(1-ethylpropyl)amino]methyl}-1,2,4-oxadiazol-5-yl)hexanoate

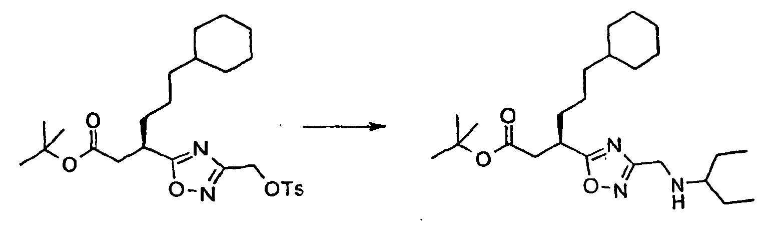

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (750mg, 1.48mmol) and 3-aminopentane (520µl, 4.44mmol) as starting materials. Purification: The crude material was purified on a silica column eluting with a solvent gradient of DCM : Et₂O (95 : 5) followed by (0 : 100) to afford the title compound as a colourless oil (132mg, 21%).
¹H nmr: (CDCl₃) 0.84 (8H, m+t), 1.10-1.30 (8H, m), 1.39 (9H, s), 1.42 (4H, m), 1.60-1.80 (7H, m), 2.40 (1H, t), 2.60 (1H, dd), 2.79 (1H, dd), 3.42 (1H, m), 3.88 (2H, s).
MS : 422 (MH⁺)
CHN : Found: C68.21 %; H10.57%; N9.86%; C₂₄H₄₃N₃O₃ requires C68.37; H10.28%; N9.97%

### Preparation 85:

### (3R)-6-cyclohexyl-3-(3-{[(1-ethylpropyl)amino]methyl}-1,2,4-oxadiazol-5-yl)hexanoic acid

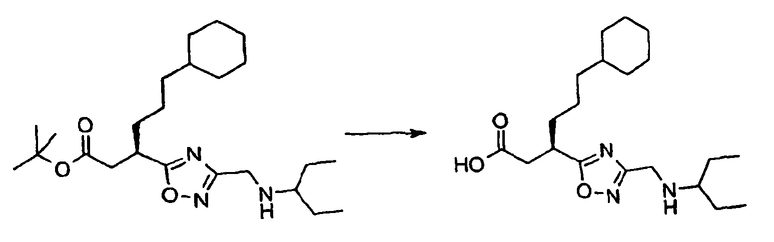

Method as for preparation 75 using *tert*-butyl (3*R*)-6-cyclohexyl-3-(3-{[(1-ethylpropyl)amino]methyl}-1,2,4-oxadiazol-5-yl)hexanoate (preparation 84) (682mg) as starting material.
Purification: The crude material was purified on a silica column eluting with DCM : MeOH (90 : 10) to afford the title compound (795mg)
¹H nmr : (CDCl₃) 0.82 (2H, m), 1.00 (6H, t), 1.10-1.35 (8H, m), 1.60-1.85 (11H, m), 2.75 (1H, dd), 2.93 (1H, dd), 3.08 (1H, t), 3.42 (1H, m), 4.21 (2H, q).
MS : 366 (MH⁺)
CHN : Found: C52.11%; H7.33%; N8.00%; C₂₀H₃₅N₃O₃. 1.4 TFA requires C52.15; H6.99%; N8.00%

### Preparation 86:

### tert-butyl (3R)-6-cyclohexyl-3-{3-[(cyclopropylamino)methyl]-1,2,4-oxadiazol-5-yl}hexanoate

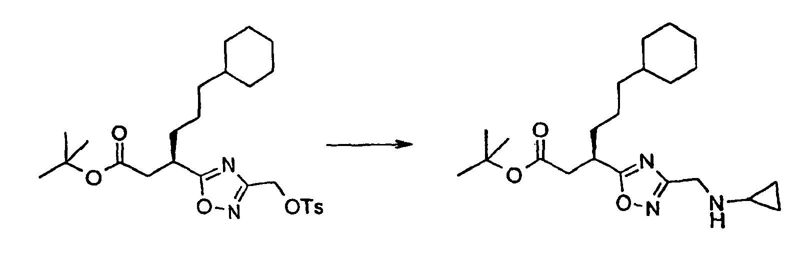

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and cyclopropylamine (210µl, 2.96mmol) as starting materials.
Purification: The crude material was purified on a silica column eluting with a solvent gradient of DCM : Et₂O (90 : 10) followed by (0 : 100) to afford the title compound (287mg, 74%).
¹H nmr : (CDCl₃) 0.40 (4H, m), 0.82 (2H, m), 1.10-1.30 (8H, m), 1.39 (9H, s), 1.60-1.80 (7H, m), 2.19 (1H, m), 2.60 (1 H, dd), 2.79 (1H, dd), 3.43 (1H, m), 3.94 (2H, s).
MS : 392 (MH⁺)
CHN : Found: C67.31%; H9.62%; N10.72%; C₂₂H₃₇N₃O₃ requires C67.49; H9.52%; N10.73%

### Preparation 87:

### (3R)-6-cyclohexyl-3-{3-[(cyclopropylamino)methyl]-1,2,4-oxadiazol-5-yl}hexanoic acid

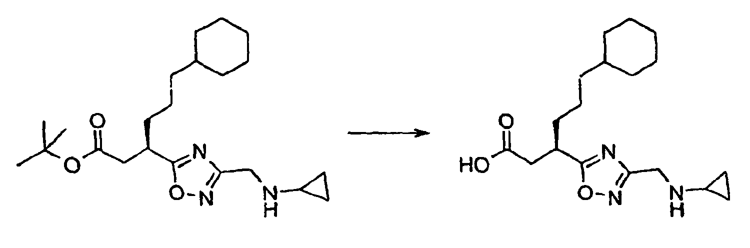

Method as for preparation 75 using *tert*-butyl (3*R*)-6-cyclohexyl-3-{3-[(cyclopropylamino)methyl]-1,2,4-oxadiazol-5-yl}hexanoate (preparation 86) (280mg, 0.72mmol) as starting material. Purification: Crude material purified on a silica column eluting with DCM : MeOH (90 : 10) to afford the title compound (300mg).
¹H nmr : (CDCl₃) 0.78 (2HM d), 0.82 (2H, m), 0.95 (2H, brs), 1.10-1.35 (8H, m), 1.60-1.80 (7H, m), 2.61 (1H, m), 2.75 (1H, dd), 2.89 (1H, dd), 3.40 (1H, m), 4.20 (2H, s).
MS : 336 (MH⁺)
CHN : Found: C51.59%; H6.73%; N8.74%; C₁₈H₂₉N₃O₃. 1.2 TFA requires C51.88; H6.45%; N8.90%

### Preparation 88:

### tert-butyl (3R)-3-{3-[(cyclobutylamino)methyl]-1,2,4-oxadiazol-5-yl}-6-cyclohexylhexanoate

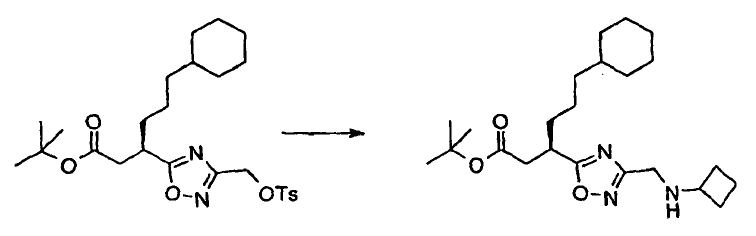

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and cyclobutylamine (250µl, 2.96mmol) as starting materials.
Purification: The crude material was purified on a silica column eluting with a solvent gradient of DCM : Et₂O (90 : 10) gradually changing to (50 : 50) to afford the title compound (180mg, 45%).
¹H nmr : (CDCl₃) 0.81 (2H, m), 1.10-1.35 (8H, m),1.39 (9H, s), 1.55-1.80 (11H, m), 2.17 (2H, m), 2.60 (1H, dd), 2.79 (1H, dd), 3.30 (1H, m), 3.43 (1H, m), 3.81 (2H, s).
MS : 406 (MH⁺)
CHN : Found: C67.98%; H9.82%; N10.36%; C₂₃H₃₉N₃O₃ requires C68.11; H9.69%; N10.36%

### Preparation 89:

### (3R)-3-{3-[(cyclobutylamino)methyl]-1,2,4-oxadiazol-5-yl}-6-cyclohexylhexanoic acid

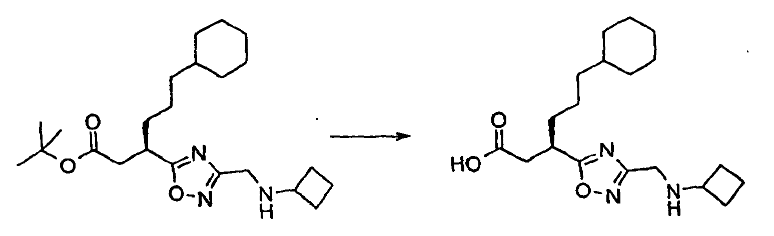

Method as for preparation 75 using *tert*-butyl (3*R*)-3-{3-[(cyclobutylamino)methyl]-1,2,4-oxadiazol-5-yl}-6-cyclohexylhexanoate (preparation 88) (180mg, 0.44mmol) as starting material to afford the title compound (197mg).
¹H nmr : (CDCl₃) 0.81 (2H, m), 1.10-1.35 (8H, m), 1.60-2.00 (9H, m), 2.30 (4H, m), 2.79 (1H, dd), 2.95 (1H, dd), 3.42 (1H, m), 3.74 (1H, m), 4.11 (2H, s).
MS : 350 (MH⁺)
CHN : Found: C51.68%; H6.78%; N8.40%; C₁₉H₃₁N₃O₃. 1.35 TFA requires C51.78; H6.48%; N8.35%

### Preparation 90:

### tert-butyl (3R)-6-cyclohexyl-3-{3-[(cyclopentylamino)methyl]-1,2,4-oxadiazol-5-yl}hexanoate

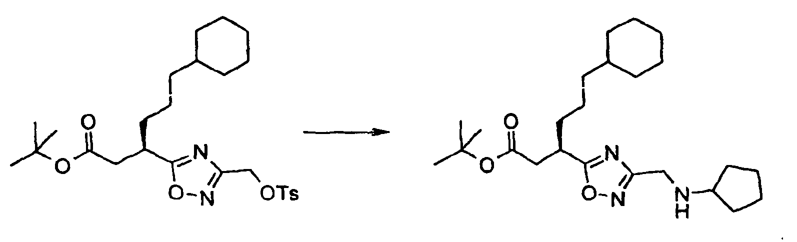

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and cyclopentylamine (170mg, 1.98mmol) as starting materials to afford the title compound as a colourless oil (220mg).
¹H nmr : (CDCl₃) 0.81 (2H, m), 1.05-1.30 (10H, m), 1.38 (11H, s+m), 1.51 (2H, m),1.55-1.75 (7H, m), 1.81 (2H, m), 2.60 (1H, dd), 2.79 (1H, dd), 3.06 (1H, m), 3.42 (1H, m), 3.87 (2H, s).

### Preparation 91:

### (3R)-6-cyclohexyl-3-{3-[(cyclopentylamino)methyl]-1,2,4-oxadiazol-5-yl}hexanoic acid

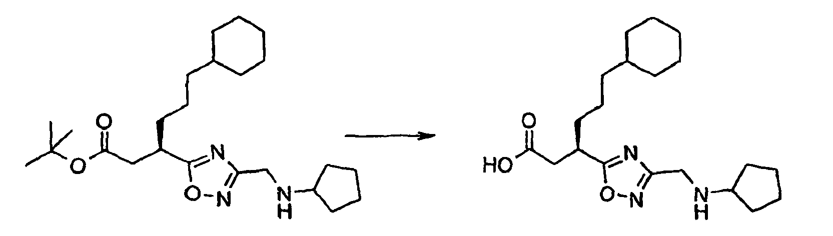

Method as for preparation 75 using *tert*-butyl (3*R*)-6-cyclohexyl-3-{3-[(cyclopentylamino)methyl]-1,2,4-oxadiazol-5-yl}hexanoate (preparation 90) (220mg, 0.53mmol) as starting material to afford the title compound as a white foam (240mg).
¹H nmr : (CDCl₃) 0.81 (2H, m), 1.10-1.35 (10H, m), 1.55-1.75 (7H, m), 1.81 (4H, m), 2.07 (2H, m), 2.78 (1H, dd), 2.95 (1H, dd), 3.40-3.60 (3H, m), 4.10-4,25 (2H, q).
MS : 364 (MH⁺)

### Preparation 92:

### tert-butyl (3R)-6-cyclohexyl-3-{3-[(cyclohexylamino)methyl]-1,2,4-oxadiazol-5-yl}hexanoate

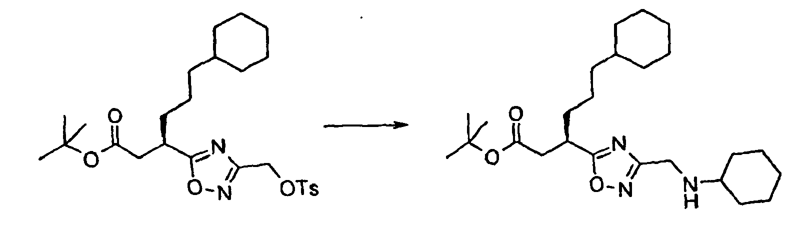

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and cyclohexylamine (198mg, 1.98mmol) as starting materials to afford the title compound as a colourless oil (250mg).
¹H nmr: (CDCl₃) 0.80 (2H, m), 1.00-1.30 (12H, m), 1.38 (9H, s), 1.55-1.75 (11H, m), 1.82 (2H, m), 2.39 (1H, m), 2.60 (1 H, dd), 2.79 (1H, dd), 3.41 (1H, m), 3.89 (2H, s).

### Preparation 93:

### (3R)-6-cyclohexyl-3-{3-[(cyclohexylamino)methyl]-1,2,4-oxadiazol-5-yl}hexanoic acid

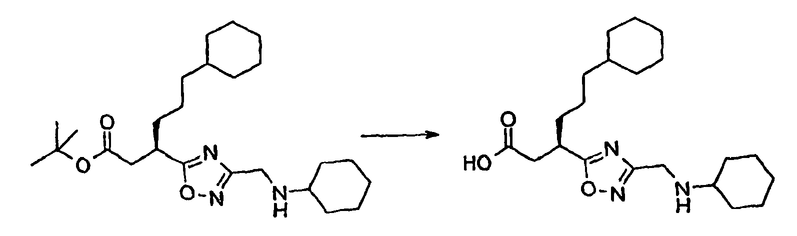

Method as for preparation 75 using *tert*-butyl (3*R*)-6-cyclohexyl-3-{3-[(cyclohexylamino)methyl]-1,2,4-oxadiazol-5-yl}hexanoate (preparation 92) (250mg, 0.58mmol) as starting material to afford the title compound as a white foam (262mg).
¹H nmr : (CDCl₃) 0.81 (2H, m), 1.10-1.35 (12H, m+Et₂O), 1.44 (2H, m),1.60-1.80 (7H, m), 1.85 (2H, m), 2.07 (2H, m), 2.78 (1H, dd), 2.96 (1H, dd), 3.05 (1H, m), 3.41 (1H, m), 4.08-4.25 (2H, q).
MS: 378 (MH⁺)

### Preparation 94:

### ten-butyl (3R)-6-cyclohexyl-3-(3-{[(2-hydroxyethyl)amino]methyl}-1,2,4-oxadiazol-5-yl)hexanoate

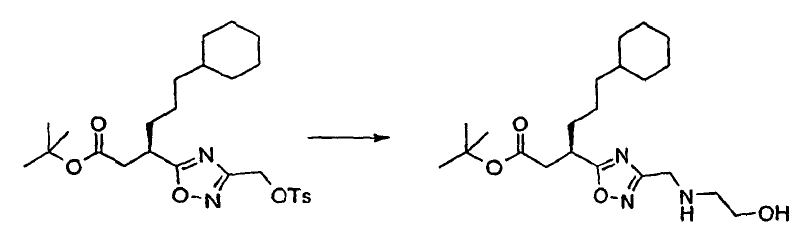

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (489mg, 0.97mmol) and ethanolamine (250µl, 3.87mmol) as starting materials.
Purification: The crude material was purified on a silica column eluting with DCM : MeOH (95 : 5) to afford the title compound as a colourless oil (322mg, 83%).
¹H nmr: (CDCl₃) 0.81 (2H, m), 1.10-1.35 (8H, m), 1.39 (9H, s), 1.50-1.80 (7H, m), 2.41 (1H, brs), 2.62 (1H, dd), 2.80 (3H, m), 3.43 (1H, m), 3.62 (2H, t), 3.94 (2H, s).
MS : 396 (MH⁺)

### Preparation 95:

### (3R)-6-cyclohexyl-3-(3-{[(2-hydroxyethyl)amino]methyl}-1,2,4-oxadiazol-5-yl)hexanoic acid

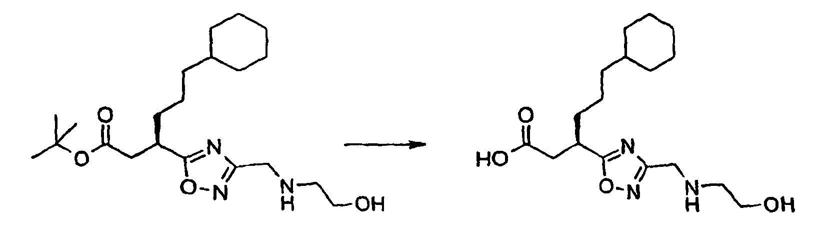

Method as for preparation 75 using *tert*-butyl (3*R*)-6-cyclohexyl-3-(3-{[(2-hydroxyethyl)amino]methyl}-1,2,4-oxadiazol-5-yl)hexanoate (preparation 94) (322mg, 0.82mmol) as starting material to afford the title compound as a sticky gum (398mg, 100%).
¹H nmr : (CDCl₃) 0.81 (2H, m), 1.10-1.35 (8H, m+Et₂O), 1.60-1.80 (7H, m), 2.79 (1H, dd), 2.89 (1H, dd), 3.20 (2H, m), 3.43 (1H, m+Et₂O), 3.87 (2H, t), 4.36 (2H, m).
MS : 340 (MH⁺)

### Preparation 96:

### tert-butyl (3R)-6-cyclohexyl-3-(3-{[(2-hydroxy-1,1-dimethylethyl)amino]methyl}-1,2,4-oxadiazol-5-yl)hexanoate

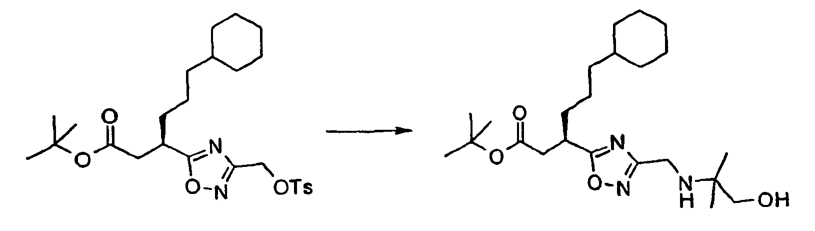

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (3.71g, 7.32mmol) and 2-amino-2-methyl-1-propanol (2.10ml, 22.0mmol) as starting materials. Purification: The crude material was purified on a silica column eluting with a solvent gradient of pentane : EtOAc (80 : 20) gradually changing to (0 : 100) to afford the title compound as a colourless oil (2.52g, 81%).
¹H nmr : (CDCl₃) 0.84 (2H, m), 1.10 (6H, s), 1.15-1.35 (9H, m), 1.40 (9H, s), 1.60-1.80 (6H, m), 2.60 (1H, dd), 2.70 (1H, brs), 2.79 (1H, dd), 3.29 (2H, s), 3.42 (1H, m), 3.83 (2H, s).
MS : 424 (MH⁺)

### Preparation 97:

### (3R)-6-cyclohexyl-3-(3-{[(2-hydroxy-1,1-dimethylethyl)amino]methyl}-1,2,4-oxadiazol-5-yl)hexanoic acid

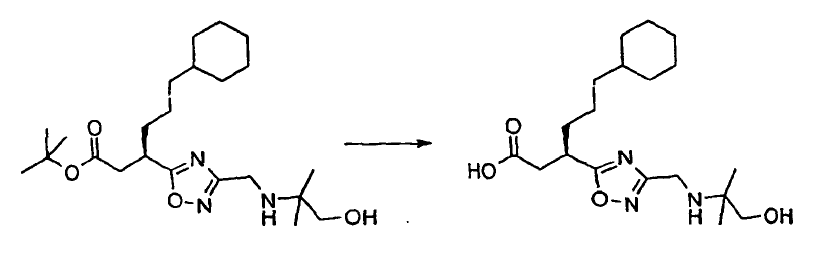

Method as for preparation 75 using *tert*-butyl (3*R*)-6-cyclohexyl-3-(3-{[(2-hydroxy-1,1-dimethylethyl)amino]methyl}-1,2,4-oxadiazol-5-yl)hexanoate (preparation 96) (2.5g, 5.90mmol) as starting material.
Purification: The crude material was purified on a silica column eluting with a solvent gradient of DCM: MeOH (100: 0) gradually changing to (90: 10) to afford the title compound as a colourless oil (2.95g)
¹H nmr : (CDCl₃) 0.82 (2H, m), 1.10-1.30 (8H, m), 1.39 (6H, d), 1.60-1.80 (7H, m), 2.78 (1H, dd), 2.92 (1H, dd), 3.42 (1H, m), 3.63 (2H, s), 4.24 (2H, m).
MS : 368 (MH⁺)

### Preparation 98:

### tert-butyl (3R)-6-cyclohexyl-3-(3-{[(2-methoxyethyl)amino]methyl}-1,2,4-oxadiazol-5-yl)hexanoate

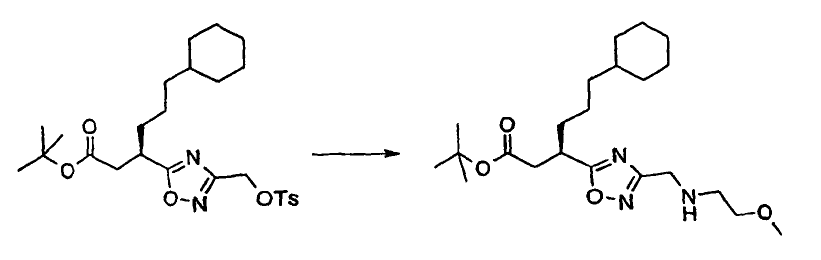

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and 2-methoxyethylamine (225mg, 2.97mmol) as starting materials to afford the title compound as a colouriess oil (372mg).
¹H nmr : (CDCl₃) 0.80 (2H, m), 1.05-1.30 (8H, m+EtOAC), 1.38 (9H, s), 1.55-1.75 (7H, m), 1.95 (1H, brs), 2.60 (1H, dd), 2.79 (1H, dd+t), 3.33 (3H, s), 3.42 (3H, m+t), 3.92 (2H, s).

### Preparation 99:

### (3R)-6-cyclohexyl-3-(3-{[(2-methoxyethyl)amino]methyl}-1,2,4-oxadiazol-5-yl)hexanoic acid

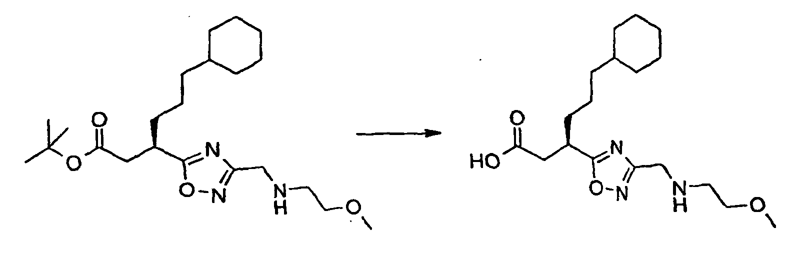

Method as for preparation 75 using *tert*-butyl (3R)-6-cyclohexyl-3-(3-{[(2-methoxyethyl)amino]methyl}-1,2,4-oxadiazol-5-yl)hexanoate (preparation 98) (372mg, 0.91mmol) as starting material to afford the title compound as a pale brown oil.
¹H nmr : (d₆DMSO) 0.80 (2H, m), 1.00-1.25 (8H, m), 1.55-1.70 (7H, m), 2.78 (2H, d), 3.21 (2H, t), 3.28 (3H, s), 3.44 (1H, m), 3.60 (2H, t), 4.42 (2H, s).

### Preparation 100:

### tert-butyl (3R)-6-cyclohexyl-3-(3-{[(3-methoxypropyl)amino]methyl}-1,2,4-oxadiazol-5-yl)hexanoate

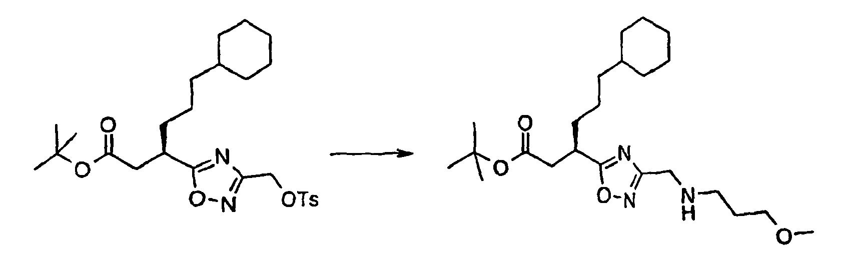

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (488mg, 0.96mmol) and 3-methoxypropylamine (325mg, 3.84mmol) as starting materials.
Purification: The crude material was purified on a silica column eluting with DCM : MeOH (95 : 5) to afford the title compound as a colourless oil (397mg, 97%).
¹H nmr : (CDCl₃) 0.81 (2H, m), 1.10-1.30 (8H, m), 1.39 (9H, s), 1.55-1.70 (7H, m), 1.79 (2H, quin), 2.61 (1H, dd), 2.72 (2H, t), 2.80 (1H, dd), 3.32 (3H, s), 3.42 (3H, t+m), 3.91 (2H, s).
MS : 425 (MH⁺)

### Preparation 101:

### (3R)-6-cyclohexyl-3-(3-{[(3-methoxypropyl)amino]methyl}-1,2,4-oxadiazol-5-yl)hexanoic acid

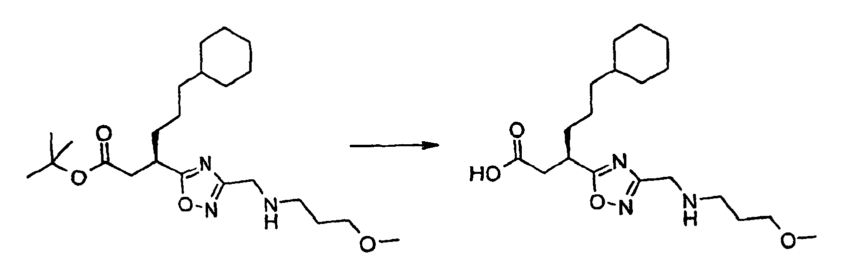

Method as for preparation 75 using *tert*-butyl (3*R*)-6-cyclohexyl-3-(3-{[(3-methoxypropyl)amino]methyl}-1,2,4-oxadiazol-5-yl)hexanoate (preparation 100) (390mg, 0.92mmol) as starting material to afford the title compound as a pale yellow oil (477mg).
¹H nmr : (CDCl₃) 0.81 (2H, m), 1.10-1.25 (8H, m), 1.60-1.70 (7H, m), 2.00 (2H, quin), 2.79 (1H, dd), 2.92 (1H, dd), 3.25 (2H, t), 3.38 (3H, s), 3.44 (1H, m), 3.58 (2H, t), 4.29 (2H, s).
MS : 369 (MH⁺)

### Preparation 102:

### tert-butyl (3R)-6-cyclohexyl-3-(3-{[(4-hydroxycyclohexyl)amino]methyl}-1,2,4-oxadiazol-5-yl)hexanoate

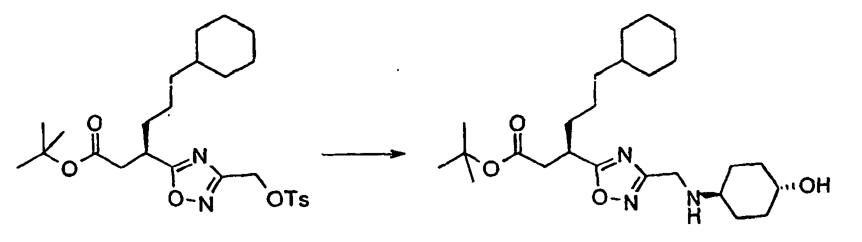

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (282mg, 0.96mmol) and trans-4-aminocyclohexanol.HCl (325mg, 3.84mmol) as starting materials. Dissolving the solid in 2M NaOH solution with Dowex 50Wx4-200R resin isolated the free base of trans-4-aminocyclohexanol.
Purification: The crude material was purified on a silica column eluting with a solvent gradient of DCM : MeOH (97.5 : 2.5) gradually changing to (95 : 5) to afford the title compound (180mg).
¹H nmr : (CDCl₃) 0.83 (2H, m), 1.10-1.35 (12H, m), 1.40 (9H, s), 1.60-1.80 (7H, m), 1.96 (4H, m), 2.48 (1H, m), 2.60 (1H, dd), 2.79 (1 H, dd), 3.43 (1H, m), 3.60 (1H, m), 3.92 (2H, s).
MS : 450 (MH⁺)
CHN : Found: C65.65%; H9.60%; N9.07%; C₂₅H₄₃N₃O₄. 0.5 H₂O requires C65.47; H9.67%; N9.16%

### Preparation 103:

### (3R)-6-cyclohexyl-3-(3-{[(4-hydroxycyclohexyl)amino]methyl}-1,2,4-oxadiazol-5-yl)hexanoic acid

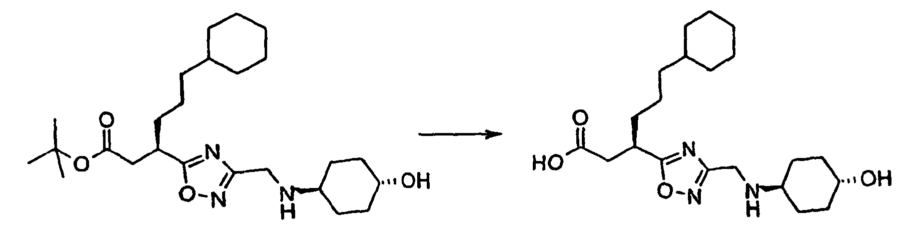

Method as for preparation 75 using *tert*-butyl (3*R*)-6-cyclohexyl-3-(3-{[(4-hydroxycyclohexyl)amino]methyl}-1,2,4-oxadiazol-5-yl)hexanoate (preparation 102) (250mg, 0.56mmol) as starting material.
Purification: The crude material was purified on a silica column eluting with a solvent gradient of DCM : MeOH (95 : 5) gradually changing to (85 : 15) to afford the title compound (190mg).
¹H nmr : (CD₃OD) 0.83 (2H, m), 1.10-1.40 (10H, m), 1.43 (2H, q), 1.60-1.80 (7H, m), 2.02 (2H, brd), 2.17 (2H, brd), 2.79 (1H, dd), 2.86 (1H, dd), 3.11 (1H, m), 3.54 (2H, m), 4.40 (2H, s).
MS : 394 (MH⁺)

### Preparation 104:

### tert-butyl (3R)-6-cyclohexyl-3-(3-{[(4-methoxycyclohexyl)amino]methyl}-1,2,4-oxadiazol-5-yl)hexanoate

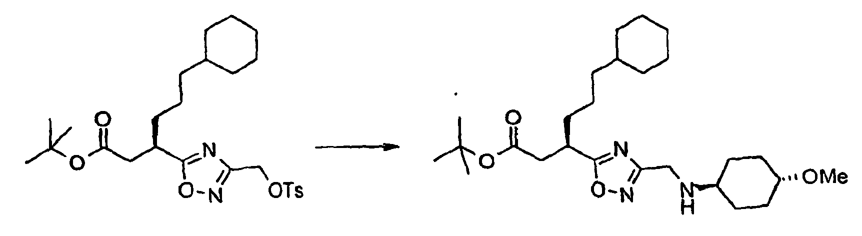

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and trans-4-methoxycyclohexanamine.HCl (490mg, 2.96mmol) as starting materials. Purification: A total of 3 silica columns were required to afford the title compound reasonably pure. First column eluted with DCM : MeOH (97 : 3), the second column eluted with DCM : MeOH (99 : 1) and the third column eluted with DCM : MeOH (98 : 2) to afford the title compound (160mg).
¹H nmr : (CDCl₃) 0.82 (2H, m), 1.10-1.50 (10H, m), 1.39 (9H, s), 1.40-1.80 (11H, m+H₂O), 1.85 (2H, m), 2.57 (1H, dd), 2.61 (2H, t), 2.79 (1H, dd), 3.23 (3H, s), 3.33 (1H, m), 3.42 (1H, m), 3.90 (2H, s). Evidence of the presence of the cis-isomer of the cyclohexanamine.
MS : 464 (MH⁺)

### Preparation 105:

### (3R)-6-cyclohexyl-3-(3-{[(4-methoxycyclohexyl)amino]methyl}-1,2,4-oxadiazol-5-yl)hexanoic acid

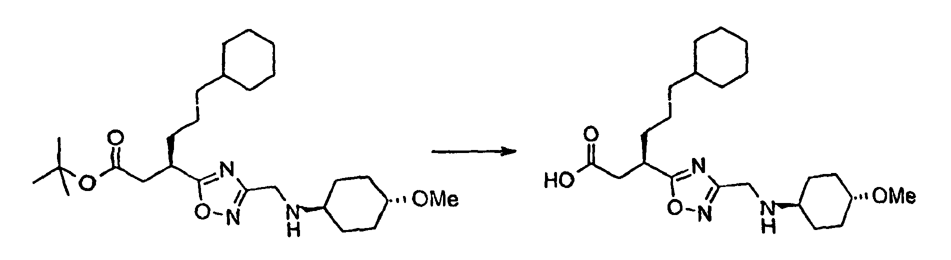

Method as for preparation 75 using *tert*-butyl (3*R*)-6-cyclohexyl-3-(3-{[(4-methoxycyclohexyl)amino]methyl}-1,2,4-oxadiazol-5-yl)hexanoate (preparation 104) (160mg) as starting material to afford the title compound (175mg).
¹H nmr : (CDCl₃) 0.82 (2H, m), 1.10-1.35 (8H, m), 1.40 (2H, m), 1.55-1.95 (11 H, m), 2.06 (2H, m), 2.78 (1H, dd), 2.95 (1H, dd), 3.19 (1H, m), 3.31 (3H, s), 3.42 (2H, m), 4.21 (2H, d). Evidence of the presence of the cis-isomer of the cyclohexanamine.
MS: 408 (MH⁺)

### Preparation 106:

### tert-butyl (3R)-6-cyclohexyl-3-(3-{[(2-methoxy-2-oxoethyl)amino]methyl}-1,2,4-oxadiazol-5-yl)hexanoate

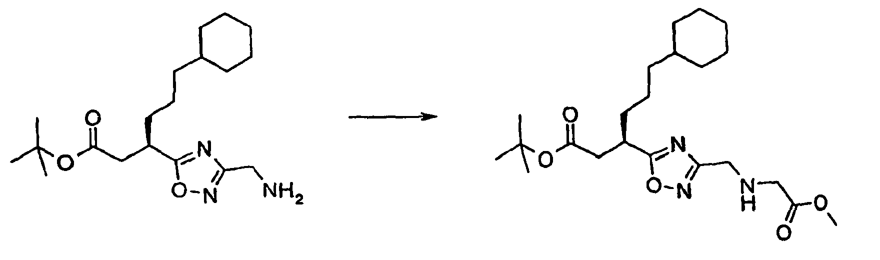

A solution of tert-butyl (3R)-3-[3-(aminomethyl)-1,2,4-oxadiazol-5-yl]-6-cyclohexylhexanoate (preparation 18) (200mg, 0.57mmol) in THF (2ml) was treated with methyl bromoacetate (55µl, 0.57mmol) and NMM (63µl, 0.57mmol) and stirred at room temperature, under a nitrogen atmosphere for 3 days. The reaction mixture was diluted with EtOAc and washed with H₂O followed by brine, dried over anhydrous MgSO₄ and filtered. The solvent was removed under reduced pressure. The crude material was purified on a silica column eluting with a solvent gradient of EtOAc : pentane (5 : 95) gradually changing to (50 : 50) to afford the title compound as a colourless oil (170mg, 70%).
¹H nmr: (CDCl₃) 0.83 (2H, m), 1.10-1.35 (9H, m), 1.39 (9H, s), 1.60-1.80 (6H, m), 2.61 (1H, dd), 2.79 (1H, dd), 3.40-3.50 (3H, m+s), 3.73 (3H, s), 3.95 (2H, s).
MS : 421 (M-H)

### Preparation 107:

### (3R)-6-cyclohexyl-3-(3-{[(2-methoxy-2-oxoethyl)amino]methyl}-1,2,4-oxadiazol-5-yl)hexanoic acid

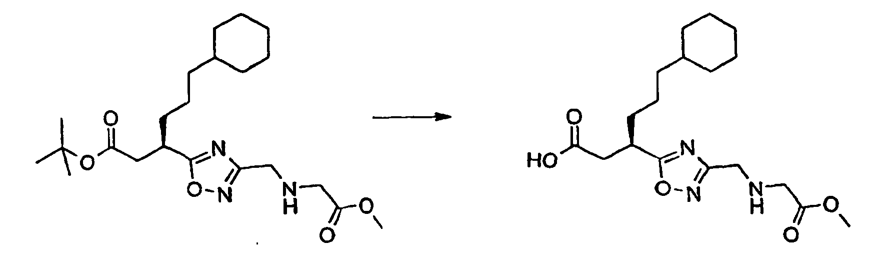

Method as for preparation 11 using *tert*-butyl (3*R*)-6-cyclohexyl-3-(3-{[(2-methoxy-2-oxoethyl)amino]methyl}-1,2,4-oxadiazol-5-yl)hexanoate (preparation 106) (198mg, 0.47mmol) as starting material.
Purification: The crude material was purified on a silica column eluting with a solvent gradient of DCM : MeOH : AcOH (100 : 0 : 0) gradually changing to (93 : 7 : 0.7) to afford the title compound as a pale yellow oil (137mg, 79%).
¹H nmr : (CDCl₃) 0.81 (2H, m), 1.10-1.35 (8H, m), 1.60-1.80 (7H, m), 2.79 (1H, brd), 2.90 (1H, m), 3.46 (1H, brs), 3.79 (3H, s), 3.86 (2H, brs), 4.26 (2H, brs).
MS: 368 (MH⁺)
Accurate mass : Found 368.2187 (MH⁺), Calculated C₁₈H₂₉N₃O₅, 368.2180

### Preparation 108:

### tert-butyl (3R)-3-(3-{[(2-amino-2-oxoethyl)amino]methyl}-1,2,4-oxadiazol-5-yl)-6-cyclohexylhexanoate

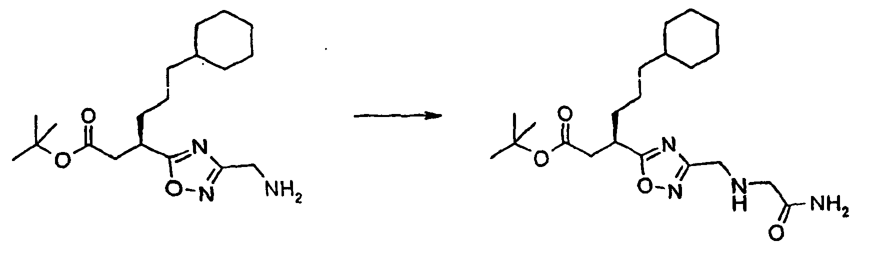

Method as for preparation 106 using tert-butyl (3R)-3-[3-(aminomethyl)-1,2,4-oxadiazol-5-yl]-6-cyclohexylhexanoate (preparation 18) (250mg, 0.71 mmol) and 2-bromoacetamide (98mg, 0.71mmol) as starting materials and Et₃N as the base.
Purification: The crude material was purified on a silica column eluting with DCM : MeOH : NH₃ (95 : 6 : 0.5) to afford the title compound as a colourless oil (150mg).
¹H nmr: (CDCl₃) 0.83 (2H, m), 1.10-1.35 (8H, m), 1.39 (9H, s), 1.50-1.80 (7H, m+H₂O), 2.60 (1H, dd), 2.79 (1H, dd), 3.35 (2H, s), 3.42 (1H, m), 3.92 (2H, s), 5.40 (1H, brs), 6.92 (1H, brs).
MS : 409 (MH⁺)

### Preparation 109:

### (3R)-3-(3-{[(2-amino-2-oxoethyl)amino]methyl}-1,2,4-oxadiazol-5-yl)-6-cyclohexylhexanoic acid

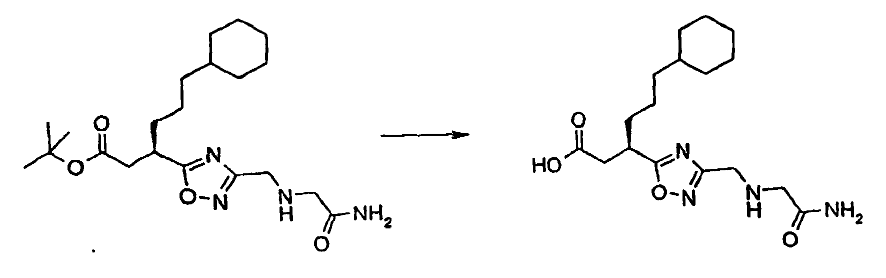

Method as for preparation 7 using *tert*-butyl (3*R*)-3-(3-{[(2-amino-2-oxoethyl)amino]methyl}-1,2,4-oxadiazol-5-yl)-6-cyclohexylhexanoate (preparation 108) (138mg, 0.34mmol) as starting material to afford the title compound as a yellow oil (147mg).
¹H nmr: (d₆DMSO) 0.81 (2H, m), 1.10-1.30 (8H, m), 1.55-1.70 (7H, m), 2.78 (2H, d), 3.43 (1H, m), 3.79 (2H, s), 4.40 (2H, s), 7.42 (1H, brs), 7.80 (1H, brs).
MS : 353 (MH⁺)

### Preparation 110:

### tert-butyl (3R)-6-cyclohexyl-3-{3-[(tetrahydro-2H-pyran-4-ylamino)methyl]-1,2,4-oxadiazol-5-yl}hexanoate

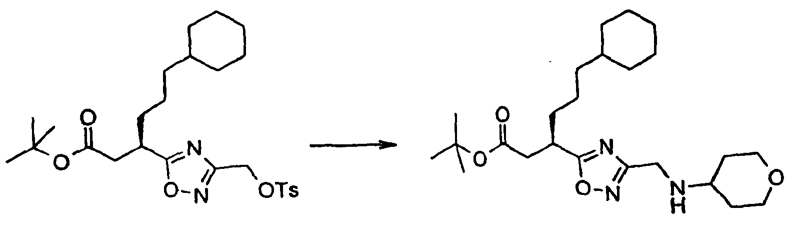

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and 4-aminotetrahydro-4H-pyran.HCl (J. Med. Chem.; 1971, *14*, 600-14) (400mg, 2.90mmol) as starting materials. Dissolving the solid in 2M NaOH solution with Dowex 50Wx4-200R resin isolated the free base of 4-aminotetrahydro-4H-pyran.
Purification: The crude material was purified on a silica column eluting with a solvent gradient of Et₂O : MeOH (100 : 0) gradually changing to (98 : 2) to afford the title compound (308mg)
¹H nmr : (CDCl₃) 0.83 (2H, m), 1.10-1.30 (8H, m), 1.35-1.55 (13H, s+m), 1.60-1.85 (7H, m), 2.60 (1H, dd), 2.72 (1H, m), 2.79 (1H, dd), 3.39 (2H, t), 3.42 (1H, m), 3.92 (4H, s+m).
MS : 436 (MH⁺)

### Preparation 111:

### (3R)-6-cyclohexyl-3-{3-[(tetrahydro-2H-pyran-4-ylamino)methyl]-1,2,4-oxadiazol-5-yl}hexanoic acid

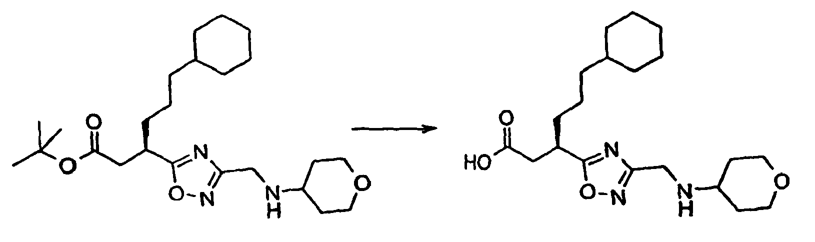

Method as for preparation 11 using *tert*-butyl (3*R*)-6-cyclohexyl-3-{3-[(tetrahydro-2*H*-pyran-4-ylamino)methyl]-1,2,4-oxadiazol-5-yl}hexanoate (preparation 110) (313mg) as starting material. Purified on a silica column eluting with a solvent gradient of DCM : MeOH (95 : 5) gradually changing to (93 : 7) to afford the title compound (231 mg).
¹H nmr : (CDCl₃) 0.84 (2H, m), 1.10-1.35 (8H, m), 1.60-1.75 (7H, m), 1.81 2H, m), 2.00 (2H, m), 2.73 (1H, brd), 2.84 (1H, m), 3.28 (1 H, m), 3.40 (3H, m), 4.05 (2H, d), 4.20 (2H, m).
MS : 380 (MH⁺)

### Preparation 112:

### tert-butyl (3R)-6-cyclohexyl-3-{3-[(1H-pyrazol-3-ylamino)methyl]-1,2,4-oxadiazol-5-yl}hexanoate

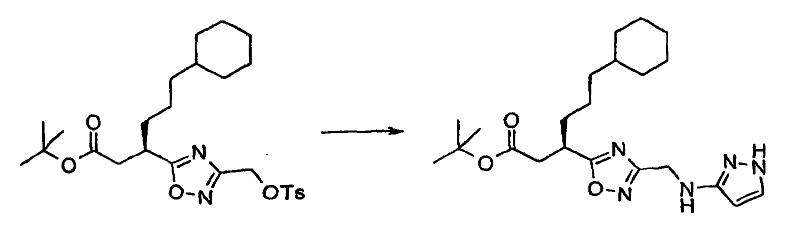

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and 3-aminopyrazole (249mg, 2.97mmol) as starting materials.
Purification: The crude material was purified on a silica column eluting with DCM : MeOH (95 : 5) to afford the title compound (132mg).
¹H nmr : (d₆DMSO) 0.79 (2H, m), 1.05-1.25 (8H, m), 1.28 (9H, s), 1.55-1.65 (7H, m), 2.66 (2H, d), 3.36 (1H, m), 5.19 (2H, s), 5.25 (3H, s), 7.01 (1H, s).
MS: 419 (MH⁺)

### Preparation 113:

### (3R)-6-cyclohexyl-3-{3-[(1H-pyrazol-3-ylamino)methyl]-1,2,4-oxadiazol-5-yl}hexanoic acid

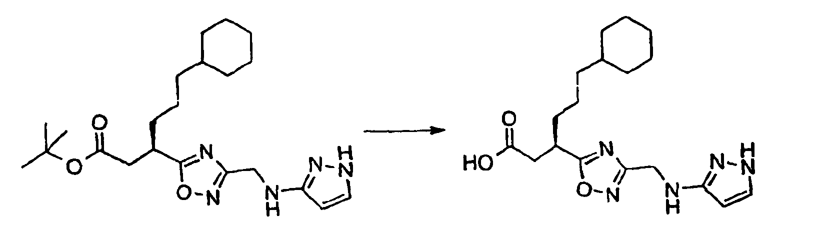

Method as for preparation 75 using *tert*-butyl (3*R*)-6-cyclohexyl-3-{3-[(1*H*-pyrazol-3-ylamino)methyl]-1,2,4-oxadiazol-5-yl}hexanoate (preparation 112) (139mg, 0.33mmol) as starting material to afford the title compound as a colourless oil (132mg)
¹H nmr : (CDCl₃) 0.81 (2H, m), 1.05-1.35 (8H, m), 1.55-1.80 (7H, m), 2.73 (1H, dd), 2.81 (1H, dd), 3.42 (1H, m), 5.50 (2H, m), 5.74 (1H, s), 7.18 (1H, d), 7.56 (1H, s).
MS : 362 (MH⁺)

### Preparation 114:

### tert-butyl (3R)-6-cyclohexyl-3-(3-{[(1-ethyl-1H-pyrazol-5-yl)amino]methyl}-1,2,4-oxadiazol-5-yl)hexanoate

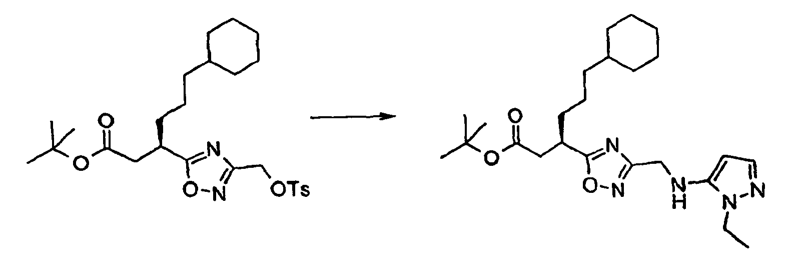

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (493mg, 0.97mmol) and 5-amino-3-ethylpyrazole (321 mg, 2.97mmol) as starting materials.
Purification: The crude material was purified on a silica column eluting with pentane : EtOAc (1 : 1) to afford the title compound as an oil (47mg).
¹H nmr : (CDCl₃) 0.80 (2H, m), 1.10-1.30 (8H, m), 1.39 (12H, s+t), 1.50-1.80 (7H, m), 2.60 (1H, dd), 2.79 (1H, dd), 3.42 (1H, m), 3.81 (1H, brs), 3.99 (2H, q), 4.38 (2H, d), 5.57 (1H, s), 7.24 (1H, s).
MS : 446 (MH⁺)

### Preparation 115:

### (3R)-6-cyclohexyl-3-(3-{[(1-ethyl-1H-pyrazol-5-yl)amino]methyl}-1,2,4-oxadiazol-5-yl)hexanoic acid

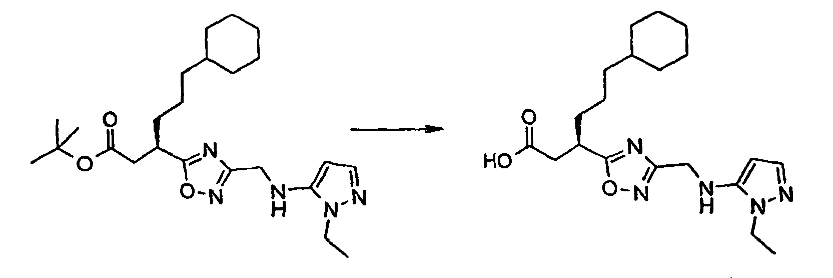

Method as for preparation 7 using *tert*-butyl (3*R*)-6-cyclohexyl-3-(3-{[(1-ethyl-1*H*-pyrazol-5-yl)amino]methyl}-1,2,4-oxadiazol-5-yl)hexanoate (preparation 114) (45mg, 0.10mmol) as starting material to afford the title compound as a gum (38mg).
¹H nmr : (CDCl₃) very broad.
MS : 390 (MH⁺)

### Preparation 116:

### tert-butyl (2S)-2-cyano-1-pyrrolidinecarboxylate

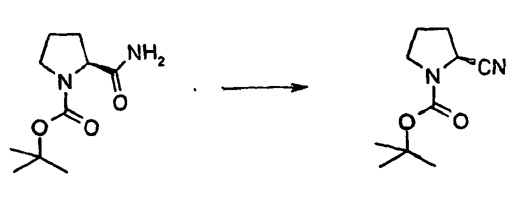

A solution of oxalyl chloride (11.0ml, 0.126mol) and DMF (11.4ml, 0.138mol) in MeCN (170ml) was treated dropwise with a solution of N-Boc-L-proline amide (12.3g, 0.57mol) and pyridine (20.4ml, 0.253mol) in MeCN (30ml) at 0°C and stirred at this temperature for 15 minutes. The reaction mixture was diluted with EtOAc and washed with H₂O (x3). The organic extract was dried over anhydrous MgSO₄ and filtered. The solvent was removed under reduced pressure. The residue was purified on a silica column eluting with a solvent gradient of pentane : EtOAc (80 : 20) gradually changing to (60 : 40) to afford the title compound (7.40g, 66%).
¹H nmr: (d₆DMSO) 1.40 (9H, s), 1.88 (2H, brs), 2.05-2.30 (2H, m), 3.21 (1H, m), 3.35 (1H, brs), 4.60 (1H, d).
MS : 214 (MNH₄⁺)

### Preparation 117:

### tert-butyl (2S)-2-[(Z)-amino(hydroxyimino)methyl]-1-pyrrolidinecarboxylate

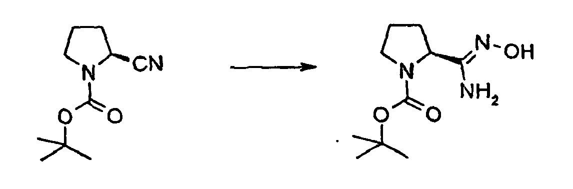

Method as for preparation 1 using *tert*-butyl (2S)-2-cyano-1-pyrrolidinecarboxylate (preparation 116) (1.96g, 10.0mmol) as starting material to afford the title compound as a white solid (1.63g)
¹H nmr: (d₆DMSO) 1.34 (9H, d), 1.65-2.00 (4H, m), 3.15-3.35 (2H, m+H₂O), 4.05 (0.5H, brs), 4.19 (0.5H, brs), 5.19 (2H, brd), 8.91 (1H, brs).

### Preparation 118:

### tert-butyl (2S)-2-{5-[(1R)-1-(2-tert-butoxy-2-oxoethyl)-4-cyclohexylbutyl]-1,2,4-oxadiazol-3-yl}-1-pyrrolidinecarboxylate

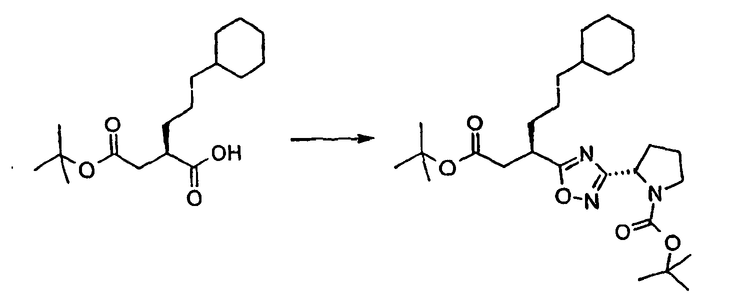

Method as for preparation 2 using *tert*-butyl (2*S*)-2-[(*Z*)-amino(hydroxyimino)methyl]-1-pyrrolidinecarboxylate (preparation 117) (1.60g, 7.00mmol) as starting material. The crude material was taken onto the cyclisation using the method as preparation 3.
Purification: The crude material was purified on a silica column eluting with DCM to afford the title compound as a colourless oil (1.42g).
¹H nmr : (CDCl₃) 0.81 (2H, m), 1.10-1.50 (26H, m), 1.60-1.80 (6H, m), 1.85-2.10 (4H, m), 2.22 (1H, m), 2.59 (1H, dd), 2.79 (1H, dd), 3.41 (1H, m), 3.50-3.65 (2H, m), 4.91 (0.5H. brs), 5.03 (0.5H, brs).
MS : 492 (MH⁺)

### Preparation 119:

### (3R)-6-cyclohexyl-3-{3-[(2S)-pyrrolidinyl]-1,2,4-oxadiazol-5-yl}hexanoic acid

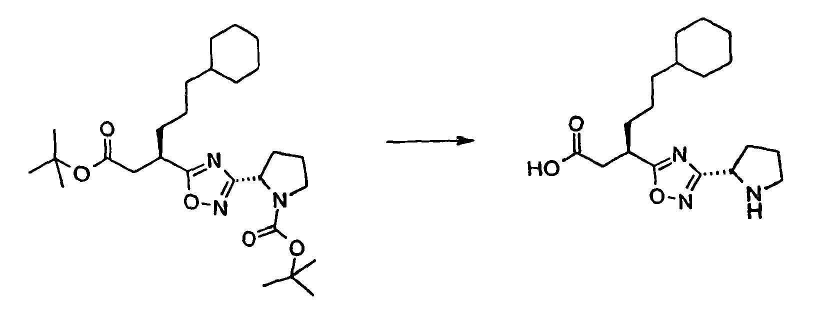

Method as for preparation 75 using *tert*-butyl (2*S*)-2-{5-[(1*R*)-1-(2-*tert*-butoxy-2-oxoethyl)-4-cyclohexylbutyl]-1,2,4-oxadiazol-3-yl}-1-pyrrolidinecarboxylate (preparation 118) (1.42g, 2.89mmol) as starting material to afford the title compound as a colourless oil.
¹H nmr : (d₆DMSO) 0.79 (2H, m), 1.05-1.30 (8H, m), 1.50-1.70 (7H, m), 2.01 (3H, m), 2.39 (1H, m), 2.78 (2H, d), 3.30 (2H, m), 3.42 (1H, m), 4.86 (1H, m).
MS : 336 (MH⁺)

### Preparation 120:

### (3R)-3-{3-[(2S)-1-(tert-butoxycarbonyl)pyrrolidinyl]-1,2,4-oxadiazol-5-yl}-6-cyclohexylhexanoic acid

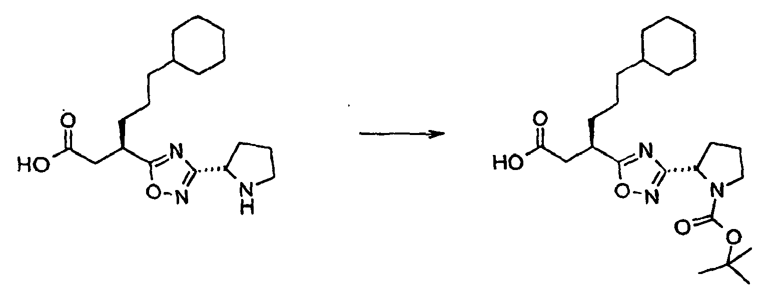

A solution of (3*R*)-6-cyclohexyl-3-{3-[(2*S*)-pyrrolidinyl]-1,2,4-oxadiazol-5-yl}hexanoic acid (preparation 119) (1.30g, 3.88mmol) in dioxan (10ml) was treated with sat. NaHCO₃ solution (1 0ml) followed by Boc anhydride (1.00g, 4.59mmol) and stirred at room temperature for 18 hours. The reaction mixture was partitioned between EtOAc and 5% citric acid solution. The organic extract was dried over anhydrous MgSO₄ and filtered. The solvent was removed under reduced pressure. The residue was purified on a silica column eluting with pentane : EtOAc (70 : 30) to afford the title compound as a colourless gum (1.18g)
¹H nmr : (d₆DMSO) 0.79 (2H, m), 1.10-1.25 (8H, m+^{t}BuOH+EtOAc), 1.42 (9H, s), 1.50-1.70 (7H, m), 1.70-1.95 (3H, m), 2.22 (1H, m), 2.69 (2H, m), 3.38 (3H, m), 4.81 (1H. brs).

### Preparation 121:

### tert-butyl (2S)-2-[(Z)-amino({[(2R)-2-(2-tert-butoxy-2-oxoethyl)-5-cyclohexylpentanoyl]oxy}imino)methyl]-1-piperidinecarboxylate

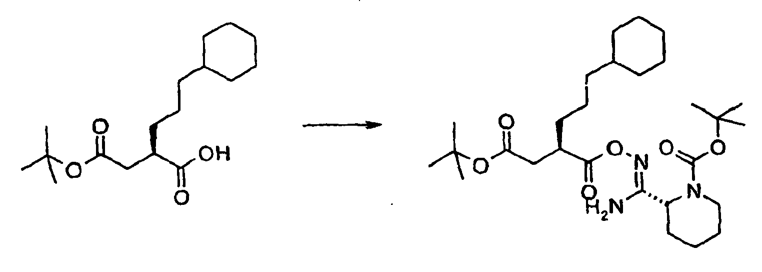

A solution of (2*R*)-2-(2-*tert*-butoxy-2-oxoethyl)-5-cyclohexylpentanoic acid (preparation 168) (500mg, 1.67mmol) in DCM (20ml) was treated with HOBt (227mg, 1.67mmol), NMM (203µl, 1.85mmol) and WSCDI (354mg, 1.85mmol) followed by *tert*-butyl (2*S*)-2-[(Z)-amino(hydroxyimino)methyl]-1-piperidinecarboxylate (WO 9945006) (406mg, 1.67mmol) and stiired at room temperature, under a nitrogen atmosphere for 18 hours. H₂O (10ml) was added and after 10 minutes the reaction mixture was separated in a 5 micron filter cartridge. The solvent was removed under reduced pressure to afford the title compound as a colourless foam.
¹H nmr : (COCl₃) 0.86 (2H, m), 1.10-1.30 (6H, m), 1.30-1.50 (20H, m+s+s), 1.50-1.70 (11H, m), 1.90 (1H, m), 2.30 (1H, brd), 2.41 (1H, dd), 2.68 (1H, dd), 2.80 (1H, brd), 2.89 (1H, m), 3.99 (1H, brd), 4.98 (1H, d), 5.17 (2H, brs).
MS : 547 (MNa⁺)

### Preparation 122:

### tert-butyl (2S)-2-{5-[(1R)-1-(2-tert-butoxy-2-oxoethyl)-4-cyclohexylbutyl]-1,2,4-oxadiazol-3-yl}-1-piperidinecarboxylate

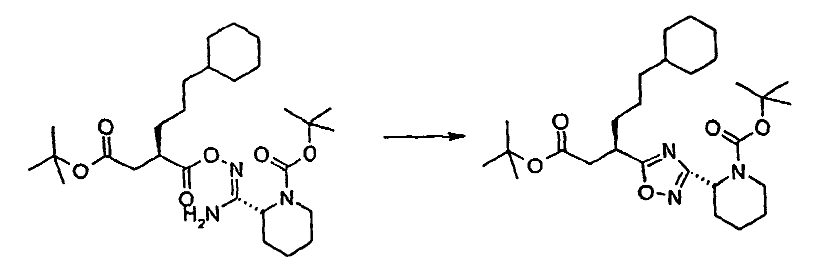

Method as for preparation 3 using *tert*-butyl (2*S*)-2-[(Z)-amino({[(2*R*)-2-(2-*tert*-butoxy-2-oxoethyl)-5-cyclohexylpentanoyl]oxy}imino)methyl]-1-piperidinecarboxylate (preparation 121) (870mg, 1.60mmol) as starting material.
Purification: The crude material was purified on a silica column eluting with DCM : MeOH (100: 0) gradually changing to (95 : 5) to afford the title compound as a pale yellow oil (472mg, 58%).
¹H nmr: (CDCl₃) 0.83 (2H, m), 1.10-1.30 (8H, m), 1.35-1.50 (21H, s+m+s), 1.60-1.75 (8H, m), 1.82 (1H, m), 2.21 (1H, brd), 2.59 (1H, dd), 2.78 (1H, dd), 1.98 (1H, brt), 3.41 (1H, m), 4.00 (1H, brd), 5.43 (1H, brs).
MS : 529 (MNa⁺)
CHN : Found: C66.36%; H9.37%; N8.22%; C₂₈H₄₇N₃O₅ requires C66.50; H9.37%; N8.31%

### Preparation 123:

### (3R)-6-cyclohexyl-3-{3-[(2S)-piperidinyl]-1,2,4-oxadiazol-5-yl}hexanoic acid

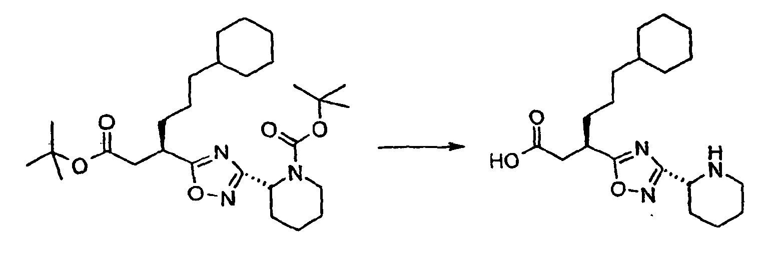

Method as for preparation 7 using *tert*-butyl (2*S*)-2-{5-[(1*R*)-1-(2-*tert*-butoxy-2-oxoethyl)-4-cyclohexylbutyl]-1,2,4-oxadiazol-3-yl}-1-piperidinecarboxylate (preparation 122) (437mg, 0.86mmol) as starting material.
¹H nmr : (d₆DMSO) 0.81 (2H, m), 1.05-1.30 (8H, m), 1.55-1.85 (12H, m), 2.12 (1H, d), 2.48 (2H, obs), 3.04 (1H, t), 3.23 (1H, t), 3.42 (1H, m), 4.61 (1 H, d).
MS : 348 (M-H)
CHN : Found: C57.81%; H8.42%; N10.13%; C₁₉H₃₁N₃O₃. HCl. 0.4H₂O. 0.1 dioxan requires C57.97; H8.43%; N10.45%

### Preparation 124:

### tert-butyl (3R)-6-cyclohexyl-3-{3-[(dimethylamino)methyl]-1,2,4-oxadiazol-5-yl}hexanoate

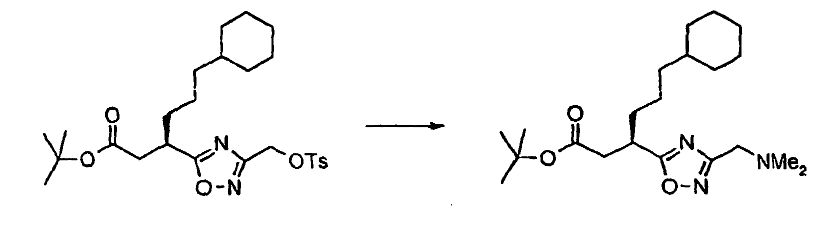

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (730mg, 1.44mmol) and *N,N*-dimethylamine (2M in THF) (800µl, 1.60mmol) as starting materials. Purification: The residue was purified on a silica column eluting with DCM : MeOH (95 : 5) to afford the title compound as a pale orange oil (430mg).
¹Hnmr (CDCl₃) : 0.82 (2H, m), 1.05-1.35 (8H, m), 1.39 (9H, s), 1.60-1.85 (7H, m), 2.35 (6H, s), 2.63 (1H, dd), 2.85 (1H, dd), 3.46 (1H, m), 3.60 (2H, s).
MS: 402 (MNa⁺)

### Preparation 125:

### (3R)-6-cyclohexyl-3-{3-[(dimethylamino)methyl]-1,2,4-oxadiazol-5-yl}hexanoic acid

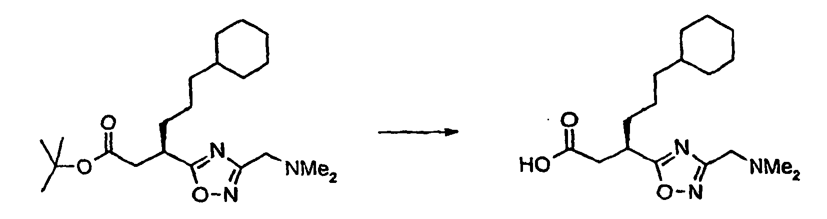

Method as for preparation 75 using *tert*-butyl (3*R*)-6-cyclohexyl-3-{3-[(dimethylamino)methyl]-1,2,4-oxadiazol-5-yl}hexanoate (preparation 124) (430mg, 1.14mmol) as starting material to afford the title compound as a yellow oil (495/550mg).
¹Hnmr (CDCl₃): 0.83 (2H, m), 1.05-1.40 (8H, m), 1.55-1.85 (7H, m), 2.74-2.99 (8H, m), 3.50 (1H, m), 4.32 (2H, s).
MS : 324 (MH⁺)

### Prepration 126:

### tert-butyl (3R)-6-cyclohexyl-3-(3-{[(2-methoxyethyl)(methyl)amino]methyl}-1,2,4-oxadiazol-5-yl)hexanoate

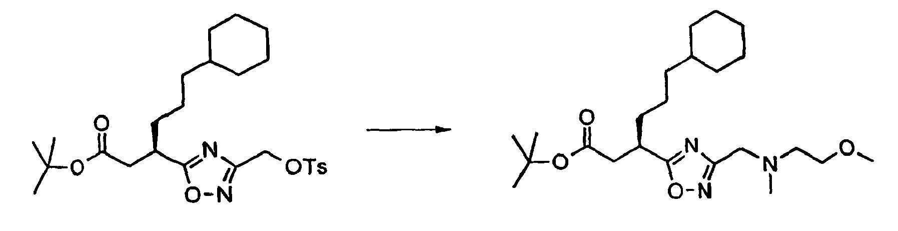

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (367mg, 0.72mmol) and 2-methoxyethylmethyl amine (130mg, 1.45mmol) as starting materials. Purification: The residue was purified on a silica column eluting with DCM : MeOH (96 : 4) to afford the title compound as a pale yellow oil (240mg).
¹Hnmr (CDCl₃) : 0.81 (2H, m), 1.10-1.30 (8H, m), 1.39 (9H, s), 1.55-1.80 (7H, m), 2.39 (3H, s), 2.61 (1H, dd), 2.74 (2H, t), 2.82 (1H, dd), 3.37 (3H, s), 3.45 (1H, m), 3.55 (2H, t), 3.82 (2H, s).
MS: 425 (MH⁺)

### Preparation 127:

### (3R)-6-cyclohexyl-3-(3-{[(2-methoxyethyl)(methyl)amino]methyl}-1,2,4-oxadiazol-5-yl)hexanoic acid

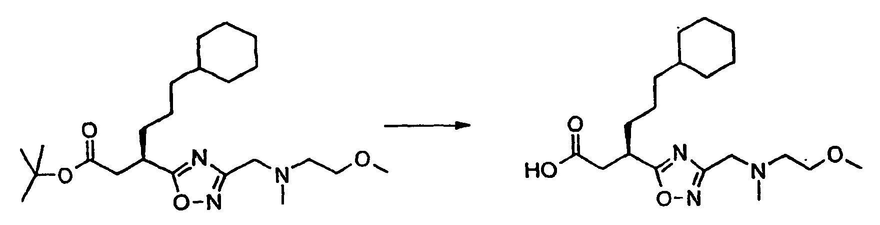

Method as for preparation 75 using *tert*-butyl (3*R*)-6-cyclohexyl-3-(3-{[(2-methoxyethyl)(methyl)amino]methyl}-1,2,4-oxadiazol-5-yl)hexanoate (preparation 126) (240mg, 0.57mmol) as starting material to afford the title compound as a pale orange oil (294mg).
¹Hnmr (CDCl₃) : 0.82 (2H, m), 1.10-1.35 (8H, m), 1.60-1.85 (7H, m), 2.80 (1H, dd), 2.90 (4H, s+dd), 3.38 (5H, s), 3.51 (1H, m), 3.79 (2H, t), 4.42 (2H, s).
MS: 368 (MH⁺)

### Preparation 128:

### tert-butyl (3R)-6-cyclohexyl-3-(3-{[[2-(dimethylamino)ethyl](methyl)amino]methyl}-1,2,4-oxadiazol-5-yl)hexanoate

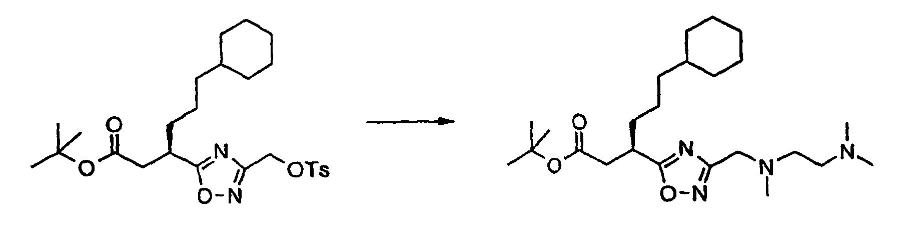

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and *N*,*N*,*N*'-trimethylethylenediamine (200mg, 1.98mmol) as starting materials to afford the title compound as a colourless oil (241 mg).
¹Hnmr (CDCl₃) : 0.80 (2H, m), 1.05-1.25 (8H, m), 1.38 (9H, s), 1.55-1.75 (7H, m), 2.22 (6H, s), 2.35 (3H, s), 2.45 (2H, t), 2.60 (3H, m), 2.79 (1H, dd), 3.41 (1H, m), 3.77 (2H, s).
MS : 437 (MH⁺)

### Preparation 129:

### (3R)-6-cyclohexyl-3-(3-{[[2-(dimethylamino)ethyl](methyl)amino]methyl}-1,2,4-oxadiazol-5-yl)hexanoic acid

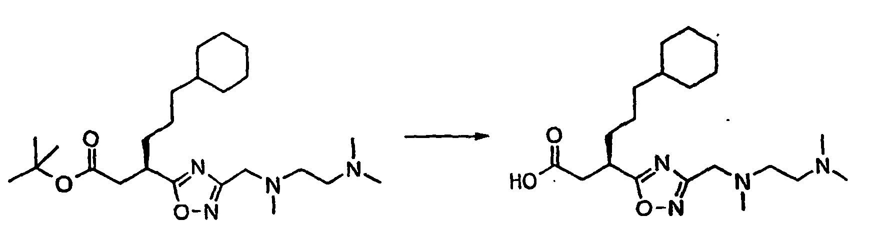

Method as for preparation 75 using *tert*-butyl (3*R*)-6-cyclohexyl-3-(3-{[[2-(dimethylamino)ethyl](methyl)amino]methyl}-1,2,4-oxadiazol-5-yl)hexanoate (preparation 128) (241mg, 0.55mmol) as starting material to afford the title compound as a pale brown oil.
¹Hnmr (d₆DMSO) : 0.79 (2H, m), 1.00-1.25 (8H, m), 1.50-1.70 (7H, m), 2.28 (3H, s), 2.75 (8H, m), 3.21 (3H, m), 3.39 (1H, m), 3.82 (2H, s).

### Preparation 130:

### tert-butyl (3R)-3-(3-{[(2-amino-2-oxoethyl)(methyl)amino]methyl}-1,2,4-oxadiazol-5-yl)-6-cyclohexylhexanoate

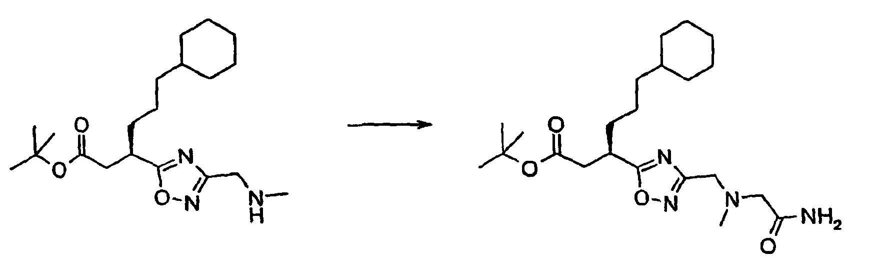

Method as for preparation 106 using *tert*-butyl (3*R*)-6-cyclohexyl-3-{3-[(methylamino)methyl]-1,2,4-oxadiazol-5-yl}hexanoate (preparation 5) (244mg, 0.67mmol) and 2-bromoacetamide (95mg, 0.69mmol) as starting materials and Et₃N as the base to afford the title compound as a colourless oil (296mg).
¹H nmr: (CDCl₃) 0.85 (2H, m), 1.10-1.35 (8H, m), 1.40 (9H, s), 1.55-1.80 (7H, m), 2.40 (3H, s), 2.61 (1H. dd), 2.79 (1H, dd), 3.19 (2H, s), 3.42 (1H, m), 3.75 (2H, s), 5.35 (1H, brs), 7.20 (1H, brs).
MS : 445 (MNa⁺)

### Preparation 131:

### (3R)-3-(3-{[(2-amino-2-oxoethyl)(methyl)amino]methyl}-1,2,4-oxadiazol-5-yl)-6-cyclohexylhexanoic acid

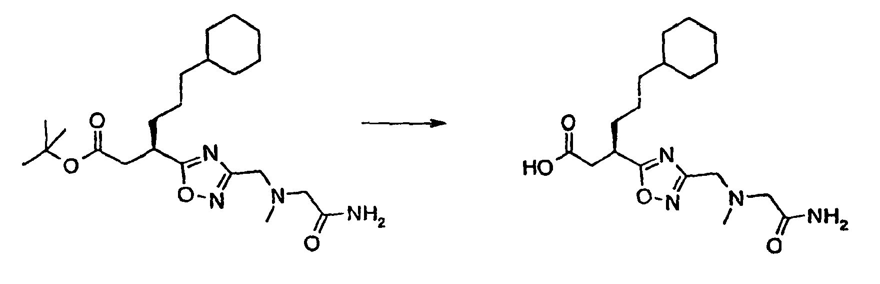

Method as for preparation 7 using *tert*-butyl (3*R*)-3-(3-{[(2-amino-2-oxoethyl)(methyl)amino]methyl}-1,2,4-oxadiazol-5-yl)-6-cyclohexylhexanoate (preparation 130) as starting material to afford the title compound as a yellow oil (250mg).
¹H nmr : (CD₃OD) 0.85 (2H, m), 1.10-1.40 (8H, m), 1.60-1.80 (7H, m), 2.81 (1H, dd), 2.90 (1H, dd), 3.02 (3H, s), 3.54 (1H, m), 4.08 (2H, s), 4.62 (2H, s).
MS : 389 (MNa⁺)
CHN : Found: C53.94%; H8.17%; N11.71%; C₁₈H₃₀N₄O₄. HCl. 0.7 dioxan requires C53.77; H7.94%; N12.06%

### Preparation 132:

### tert-butyl (3R)-6-cyclohexyl-3-(3-{[(2-ethoxy-2-oxoethyl)(methyl)amino]methyl}-1,2,4-oxadiazol-5-yl)hexanoate

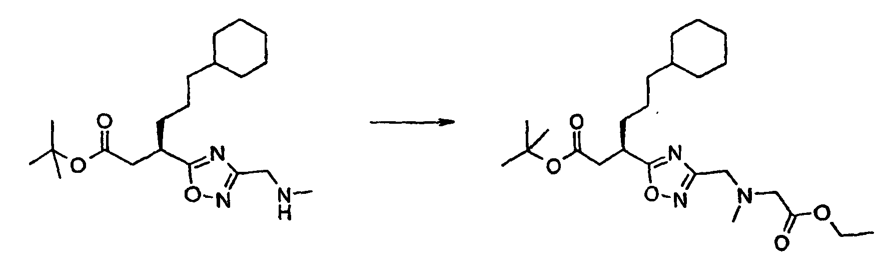

Method as for preparation 106 using *tert*-butyl (3*R*)-6-cyclohexyl-3-{3-[(methylamino)methyl]-1,2,4-oxadiazol-5-yl}hexanoate (preparation 5) (1.50g, 4.10mmol) and ethyl bromoacetate (460µl, 4.10mmol) as starting materials.
Purification: The crude material was purified on a silica column eluting with hexane: Et₂O (1 : 1) to afford the title compound as a colourless oil (1.04g, 55%).
¹H nmr: (CDCl₃) 0.84 (2H, m), 1.10-1.35 (11H, m), 1.40 (9H, s), 1.60-1.80 (7H, m), 2.48 (3H, s), 2.60 (1H, dd), 2.80 (1H, dd), 3.41 (3H, s+m), 3.97 (2H, s), 4.19 (2H, q).
MS: 474 (MNa⁺)
CHN : Found: C63.65%; H9.17%; N9.04%; C₂₄H₄₁N₃O₅ requires C63.83; H9.15%; N9.30%

### Preparation 133:

### [({5-[(1R)-1-(2-tert-butoxy-2-oxoethyl)-4-cyclohexylbutyl]-1,2,4-oxadiazol-3-yl}methyl)(methyl)amino]acetic acid

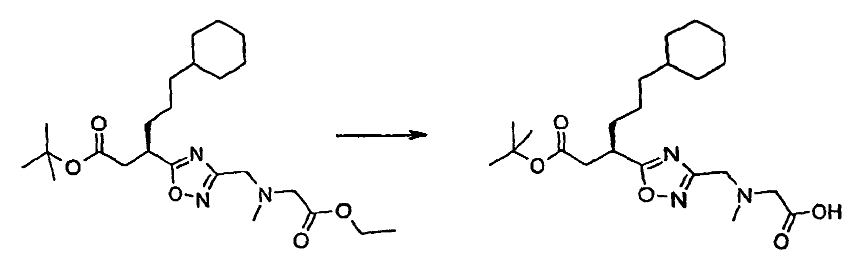

A solution of *tert*-butyl (3*R*)-6-cyclohexyl-3-(3-{[(2-ethoxy-2-oxoethyl)(methyl)amino]methyl}-1,2,4-oxadiazol-5-yl)hexanoate (preparation 132) (1.00g, 2.21 mmol) in dioxan (20ml) and H₂O (20ml) was treated with LiOH.H₂O (193mg, 2.21 mmol) and the reaction mixture stirred at room temperature for 19 hours. The solvent was removed under reduced pressure. The residue was dissolved in EtOAc and washed with brine. The aqueous was extracted with EtOAc (x3). The combined organic extracts were dried over anhydrous Na₂SO₄ and filtered. The solvent was removed under reduced pressure to afford the title compound as a white foam (1.13g).
¹H nmr : (CDCl₃) 0.82 (2H, m), 1.05-1.30 (8H, m), 1.39 (9H, s), 1.55-1.80 (7H, m), 2.23 (3H, s), 2.61 (1H, dd), 2.82 (1H, dd), 3.04 (2H, s), 3.41 (1H, m), 3.75 (2H, s).
MS : 446 (MNa⁺)

### Preparation 134:

### tert-butyl (3R)-6-cyclohexyl-3-[3-({methyl[2-(methylamino)-2-oxoethyl]amino}methyl)-1,2,4-oxadiazol-5-yl]hexanoate

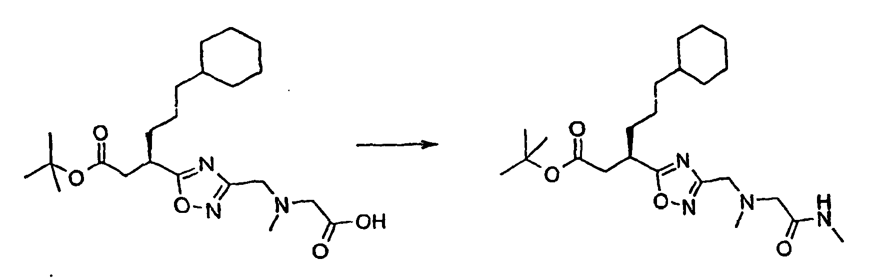

Method as for preparation 56 using [({5-[(1*R*)-1-(2-*tert*-butoxy-2-oxoethyl)-4-cyclohexylbutyl]-1,2,4-oxadiazol-3-yl}methyl)(methyl)amino]acetic acid (preparation 133) (618mg, 1.46mmol) and methylamine (2M in THF) (1.4ml, 2.80mmol) as starting materials.
Purification: The crude material was purified on a silica column eluting with a solvent gradient of DCM : MeOH (95 : 5) gradually changing to (90 : 10) to afford the title compound as a colourless oil (455mg).
¹H nmr : (CDCl₃) 0.82 (2H, m), 1.05-1.30 (8H, m), 1.39 (9H, s), 1.55-1.80 (7H, m), 2.39 (3H, s), 2.61 (1H, dd), 2.79 (1H, dd), 2.81 (3H, s), 3.18 (2H, s), 3.42 (1H, m), 3.71 (2H, s).
MS : 459 (MNa⁺)

### Preparation 135:

### (3R)-6-cyclohexyl-3-[3-({methyl[2-(methylamino)-2-oxoethyl]amino}methyl)-1,2,4-oxadiazol-5-yl]hexanoic acid

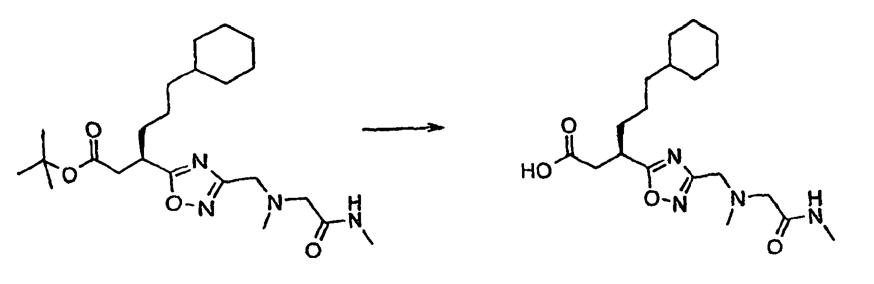

Method as for preparation 7 using *tert*-butyl (3*R*)-6-cyclohexyl-3-[3-({methyl[2-(methylamino)-2-oxoethyl]amino}methyt)-1,2,4-oxadiazol-5-yl]hexanoate (preparation 134) (435mg) as starting material to afford the title compound as a pale yellow solid.
¹H nmr : (CD₃OD) 0.86 (2H, m), 1.10-1.40 (8H, m), 1.60-1.80 (7H, m), 2.80 (3H, s), 2.82 (1H, dd), 2.89 (1H, dd), 3.00 (3H, s), 3.53 (1H, m), 4.00 (2H, s), 4.60 (2H, s).
MS : 403 (MNa⁺)

### Preparation 136:

### tert-butyl (3R)-6-cyclohexyl-3-(3-{[[2-(dimethylamino)-2-oxoethyl](methyl)amino]methyl}-1,2,4-oxadiazol-5-yl)hexanoate

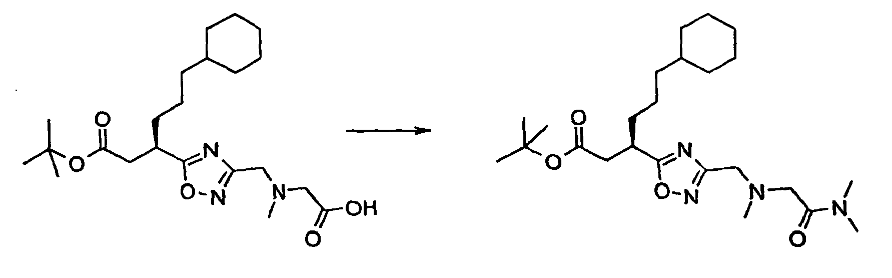

Method as for preparation 56 using [({5-[(1*R*)-1-(2-*tert*-butoxy-2-oxoethyl)-4-cyclohexylbutyl]-1,2,4-oxadiazol-3-yl}methyl)(methyl)amino]acetic acid (preparation 133) (725mg, 1.71mmol) and Me₂NH.HCl (106mg, 1.30mmol) as starting materials.
Purification: The crude material was purified on a silica column eluting with DCM : MeOH (19 : 1) to afford the title compound as a colourless oil (504mg).
¹H nmr : (CDCl₃) 0.82 (2H, m), 1.05-1.30 (8H, m), 1.39 (9H, s), 1.60-1.80 (7H, m), 2.41 (3H, s), 2.60 (1H, dd), 2.79 (1H, dd), 2.94 (3H, s), 3.00 (3H, s), 3.39 (2H, s), 3.42 (1H, m), 3.81 (2H, s).
MS : 473 (MNa⁺)

### Preparation 137:

### (3R)-6-cyclohexyl-3-(3-{[[2-(dimethylamino)-2-oxoethyl](methyl)amino]methyl}-1,2,4-oxadiazol-5-yl)hexanolc acid

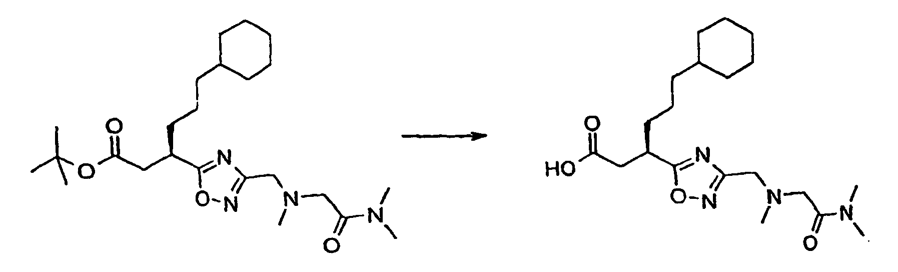

Method as for preparation 7 using *tert*-butyl (3*R*)-6-cyclohexyl-3-(3-{[[2-(dimethylamino)-2-oxoethyl](methyl)amino]methyl}-1,2,4-oxadiazol-5-yl)hexanoate (preparation 136) (489mg) as starting material to afford the title compound as pale yellow gum (439mg).
¹H nmr : (CD₃OD) 0.85 (2H, m), 1.10-1.40 (8H, m), 1.60-1.80 (7H, m), 2.82 (2H, m), 2.99 (6H, d), 3.01 (3H, s), 3.55 (1H, m), 4.31 (2H, s), 4.61 (2H, s).
MS : 395 (MH⁺)

### Preparation 138:

### tert-butyl (3R)-3-(3-{[bis(2-methoxyethyl)amino]methyl}-1,2,4-oxadiazol-5-yl)-6-cyclohexylhexanoate

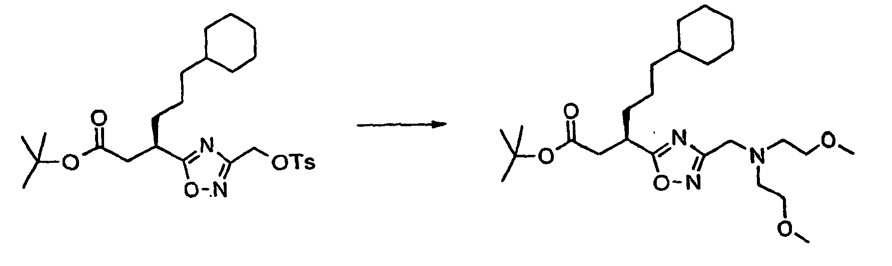

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and bis-(2-methoxyethyl)amine (280µl, 1.98mmol) as starting materials. Purification: The residue was purified on a silica column eluting with a solvent gradient of DCM : MeOH (99 : 1) gradually changing to (97 : 3) to afford the title compound as a colourless oil (220mg).
¹Hnmr (CDCl₃) δ: 0.81 (2H, m), 1.10-1.30 (8H, m), 1.39 (9H, s), 1.60-1.85 (7H, m+H₂O), 2.61 (1H, dd), 2.79 (1H, dd), 2.82 (4H, t), 3.35 (6H, s), 3.43 (1H, m), 3.50 (4H, t), 3.98 (2H, s).
MS : 468 (MH⁺)

### Preparation 139:

### (3R)-3-(3-{[bis(2-methoxyethyl)amino]methyl}-1,2,4-oxadiazol-5-yl)-6-cyclohexylhexanoic acid

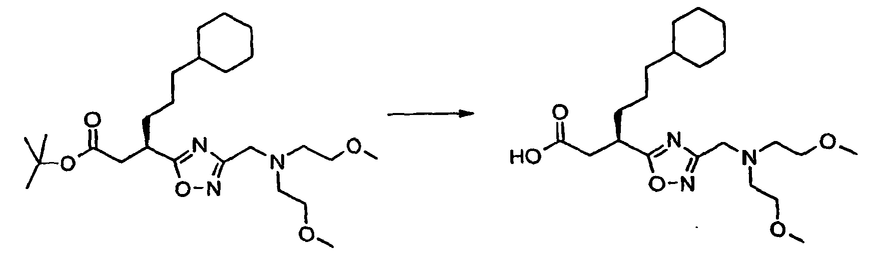

Method as for preparation 75 using *tert*-butyl (3*R*)-3-(3-{[bis(2-methoxyethyl)amino]methyl}-1,2,4-oxadiazol-5-yl)-6-cyclohexylhexanoate (preparation 138) (220mg, 0.46mmol) as staring material to afford the title compound as a yellow oil (176mg).
¹Hnmr (CDCl₃) δ : 0.80 (2H, m), 1.05-1.35 (8H, m), 1.55-1.85 (7H, m), 2.79 (1H, dd), 2.92 (1H, dd), 3.34 (6H, s), 3.42 (4H, t), 3.51 (1H, m), 3.79 (4H, t), 4.60 (2H, s).

### Preparation 140:

### tert-butyl (3R)-6-cyclohexyl-3-[3-(1-pyrrolidinylmethyl)-1,2,4-oxadiazol-5-yl]hexanoate

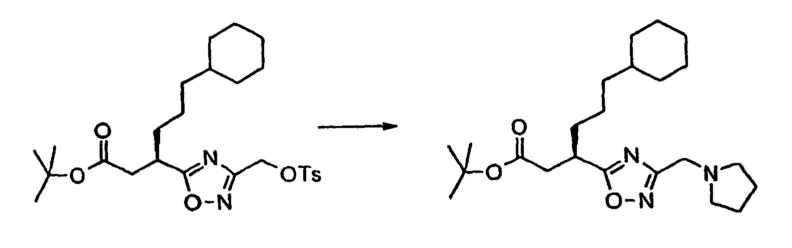

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (126mg, 0.25mmol) and pyrrolidine (100µl, 1.20mmol) as starting materials.
Purification: The crude material was purified on a silica column eluting with DCM : MeOH (98 : 2) to afford the title compound as a colourless oil (99mg).
¹Hnmr (CDCl₃) : 0.82 (2H, m),1.05-1.35 (8H, m), 1.39 (9H, s),1.50-1.85 (11H, m), 2.62 (5H, m), 2.83 (1H, dd), 3.46 (1H, m), 3.77 (2H, s).
MS : 428 (MNa⁺)

### Preparation 141:

### (3R)-6-cyclohexyl-3-[3-(1-pyrrolidinylmethyl)-1,2,4-oxadiazol-5-yl]hexanoic acid

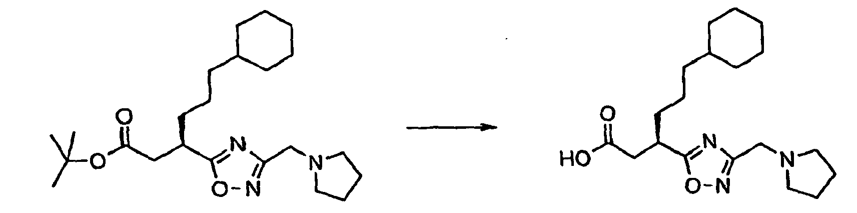

Method as for preparation 75 using *tert*-butyl (3*R*)-6-cyclohexyl-3-[3-(1-pyrrolidinylmethyl)-1,2,4-oxadiazol-5-yl]hexanoate (preparation 140) (99mg, 0.24mmol) as starting material to afford the title compound as a colourless oil (85mg).
¹Hmnr (CDCl₃) : 0.82 (2H, m), 1.00-1.35 (10H, m), 1.55-1.85 (7H, m), 2.00-2.20 (4H, m), 2.97-2.71 (2H, m), 3.51 (1H, m), 4.38 (2H, s).
MS: 349 (M⁺)

### Preparation 142:

### tert-butyl (3R)-6-cyclohexyl-3-{3-[(4-hydroxy-1-piperidinyl)methyl]-1,2,4-oxadiazol-5-yl}hexanoate

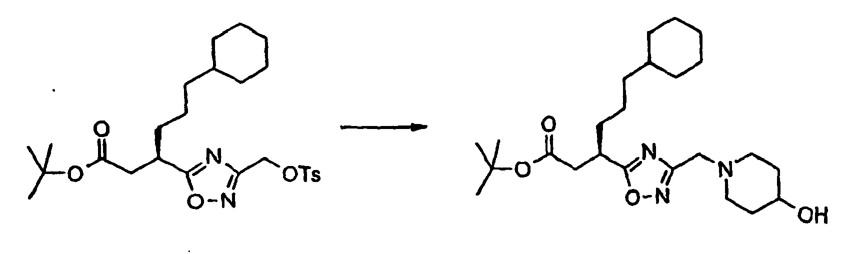

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 177) (500mg, 0.99mmol) and N-hydroxypiperidine (150mg, 1.48mmol) as starting materials. Purification: The crude material was purified on a silica column eluting with DCM : MeOH (95 : 5) to afford the title compound as a pale yellow oil (366mg).
¹Hnmr (CDCl₃) : 0.83 (2H, m), 1.05-1.35 (8H, m), 1.39 (9H, s), 1.60-1.80 (9H, m), 1.90 (2H, m), 2.31 (2H, m), 2.63 (1 H, dd), 2.83 (3H, m), 3.45 (1H, m), 3.67 (3H, m),
MS: 436 (MH⁺)

### Preparation 143:

### (3R)-6-cyclohexyl-3-{3-[(4-hydroxy-1-piperidinyl)methyl]-1,2,4-oxadiazol-5-yl}hexanoic acid trifluoroacetate

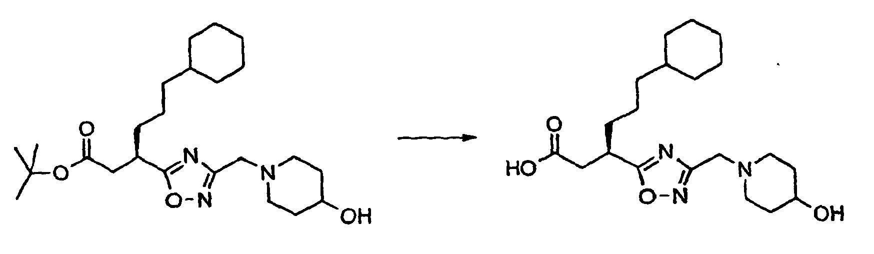

Method as for preparation 75 using *tert*-butyl (3*R*)-6-cyclohexyl-3-{3-[(4-hydroxy-1-piperidinyl)methyl]-1,2,4-oxadiazol-5-yl}hexanoate (Preparation 142) (366mg, 0.84mmol) as starting material to afford the title compound as a pale yellow oil (460mg).
¹Hnmr (CDCl₃) : 0.85 (2H, m), 1.05-1.40 (8H, m), 1.60-1.85 (7H, m), 1.92 (2H, m), 2.20 (2H, m), 2.79 (1H, dd), 2.90 (1H, dd), 3.59-3.12 (5H, m), 4.16 (1H, m), 4.34 (2H, m),
MS: 380 (MH⁺)

### Preparation 144:

### tert-butyl (3R)-6-cyclohexyl-3-[3-(4-morpholinylmethyl)-1,2,4-oxadiazol-5-yl]hexanoate

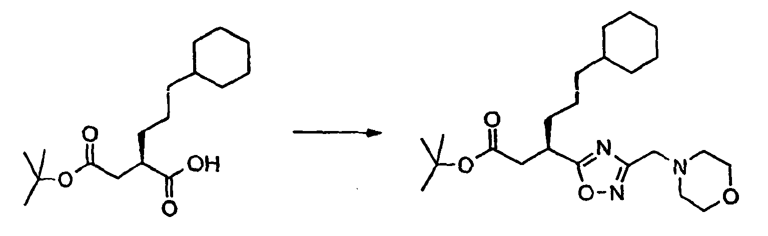

Method as for preparations 2 and 3 combined using 2-[2-(*tert*-butoxy)-2-oxoethyl]-5-cyclohexylpentanoic acid (preparation 168) (1.00g, 3.36mmol) and N-hydroxy-2-(4-morpholinyl)ethanimidamide (J. Org. Chem.; 1958, 23, 1112) (534mg, 3.36mmol) as starting material.
Purification: The crude material was purified on a silica column eluting with DCM : MeOH (95 : 5) to afford the title compound as a colourless oil (150mg)
¹Hnmr (CDCl₃) : 0.83 (2H, m), 1.05-1.35 (8H, m), 1.39 (9H, s), 1.40-1.80 (7H, m), 2.50-2.70 (5H, m), 2.83 (1H, dd), 3.46 (1H, m), 3.66 (2H, s), 3.73 (4H, m)
MS : 422 (MH⁺), 444 (MNa⁺)

### Preparation 145:

### (3R)-6-cyclohexyl-3-[3-(4-morpholinylmethyl)-1,2,4-oxadiazol-5-yl]hexanoic acid

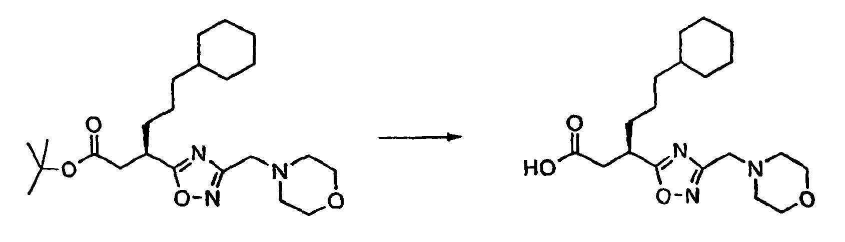

Method as for preparation 75 using *tert*-butyl (3*R*)-6-cyclohexyl-3-[3-(4-morpholinylmethyl)-1,2,4-oxadiazol-5-yl]hexanoate (Preparation 144) (150mg, 0.36mmol) as starting material to afford the title compound as a pale brown oil (120mg).
¹Hnmr (CDCl₃) δ: 0.84 (2H, m), 1.05-1.40 (8H, m), 1.55-1.85 (7H, m), 2.76 (1H, dd), 2.90 (1H, dd), 3.22 (4H, m), 3.47 (1H, m), 3.92 (4H, m), 4.28 (2H, m), 9.80 (1H, br s)
MS : 366 (MH⁺)

### Preparation 146:

### tert-butyl (3R)-6-cyclohexyl-3-{3-[(4-methyl-1-piperazinyl)methyl]-1,2,4-oxadiazol-5-yl}hexanoate

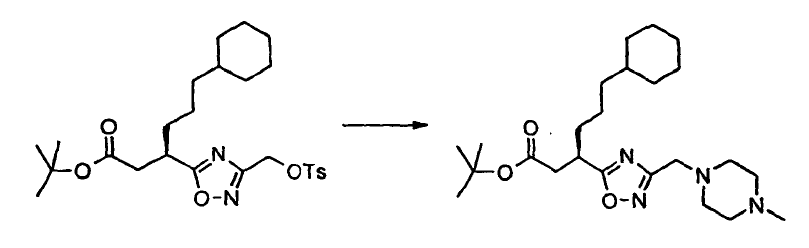

Method as for preparation 5 using *tert*-butyl (3*R*)-6-cyclohexyl-3-[3-({[(4-methylphenyl)sulfonyl]oxy}methyl)-1,2,4-oxadiazol-5-yl]hexanoate (preparation 168) (598mg, 1.18mmol) and *N*-methylpiperazine (120mg, 1.18mmol) as starting materials.
Purification: The crude material was purified on a silica column eluting with DCM : MeOH : NH₃ (95 : 5 : 0.5) to afford the title compound as a pale orange oil (395mg).
¹Hnmr (CDCl₃) : 0.83 (2H, m), 1.05-1.35 (8H, m), 1.40 (9H, s), 1.60-1.80 (7H, m), 2.29 (3H, s), 2.35-2.75 (9H, m), 2.83 (1H, dd), 3.45 (1H, m), 3.68 (2H, s)
MS : 436 (M2H⁺)

### Preparation 147:

### (3R)-6-cyclohexyl-3-{3-[(4-methyl-1-piperazinyl)methyl]-1,2,4-oxadiazol-5-yl}hexanoic acid trifluoroacetate

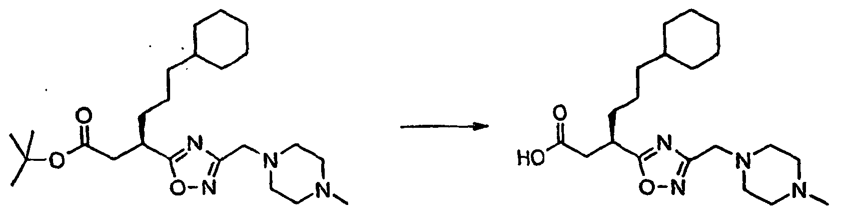

Method as for preparation 75 using *tert*-butyl (3*R*)-6-cyclohexyl-3-{3-[(4-methyl-1-piperazinyl)methyl]-1,2,4-oxadiazol-5-yl}hexanoate (Preparation 146) (395mg, 0.91mmol) as starting material to afford the title compound as a foam (585mg).
¹Hnmr (CDCl₃) : 0.84 (2H, m), 1.10-1.40 (8H, m), 1.60-1.85 (7H, m), 2.75-2.95 (5H, m), 3.00-3.25 (4H, m), 3.45-3.70 (3H, m), 4.05 (2H, m)
MS: 379 (MH⁺)

### Preparation 148:

### N-hydroxy-2-(1H-1,2,4-triazol-1-yl)ethanimidamide

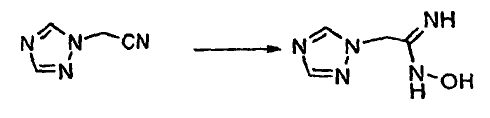

Method as for preparation 1 using 2-(1*H*-1,2,4-triazol-1-yl)acetonitrile (*J*.Org.Chem. USSR, 18; 2; 1982; 407) (2.16g, 20mmol) as starting material. The crystalline precipitate was filtered off and washed with cold MeOH and Et₂O to afford the title compound (2.37g)
¹Hnmr (d₆DMSO) : 4.72 (2H, s), 5.54 (2H, br s), 7.93 (1H, s), 8.46 (1H, s), 9.29 (1H, s),

### Preprartion 149:

### tert-butyl (3R)-6-cyclohexyl-3-[3-(1H-1,2,4-triazol-1-ylmethyl)-1,2,4-oxadiazol-5-yl]hexanoate

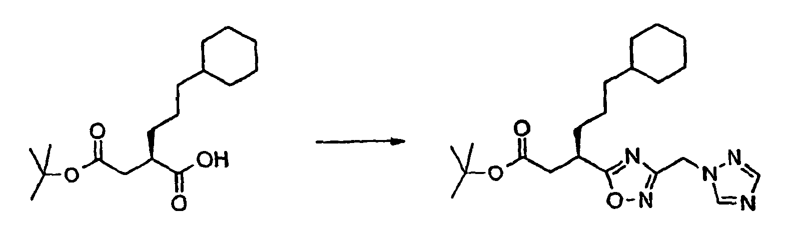

Method as for preparation 2 and 3 combined using 2-[2-(*tert*-butoxy)-2-oxoethyl]-5-cyclohexylpentanoic acid (preparation 168) (2.98g, 10mmol) and *N*-hydroxy-2-(1*H*-1,2,4-triazol-1-yl)ethanimidamide (preparation 148) (1.01g, 10mmol) as starting materials.
Purification: The crude material was purified on a silica column eluting with a solvent gradient EtOAc : pentane (100 : 0) gradually changing to (50 : 50) to afford the title compound as a golden oil (1.10g)
¹Hnmr (CDCl₃) : 0.82 (2H. m), 1.05-1.40 (17H, m), 1.60-1.80 (7H, m), 2.62 (1H, dd), 2.77 (1H. dd), 3.44 (1H, m), 5.46 (2H, s), 7.96 (1H, s), 8.25 (1H, s),
MS : 404 (MH⁺), 426 (MNa⁺)

### Preparation 150:

### (3R)-6-cyclohexyl-3-[3-(1H-1,2,4-triazol-1-ylmethyl)-1,2,4-oxadiazol-5-yl]hexanoic acid

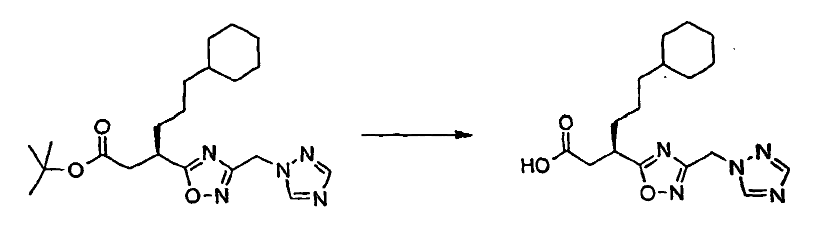

Nlethod as for preparation 75 using *tert*-butyl (3*R*)-6-cyclohexyl-3-[3-(1*H*-1,2,4-triazol-1-ylmethyl)-1,2,4-oxadiazol-5-yl]hexanoate (Preparation 149) (500mg, 1.24mmol) as starting material to afford the title compound as an oil which crystallised on standing (420mg).
¹Hnmr (CDCl₃) : 0.83 (2H, m), 1.05-1.40 (8H, m), 1.55-1.85 (7H, m), 2.78 (1H, dd), 2.92 (1H, dd), 3.51 (1H, m), 5.53 (2H, d), 8.06 (1H, s), 8.52 (1H, s)
MS: 346 (MH⁻)

### Preparation 151:

### tert-butyl (3R)-6-cyclohexyl-3-({[(1S)-2-ethoxy-1-(hydroxymethyl)-2-oxoethyl]amino}carbonyl)hexanoate

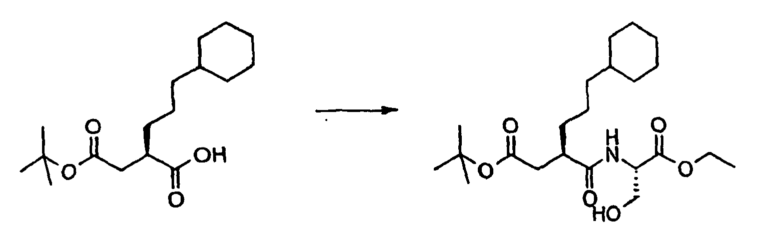

Method as for preparation 56 using 2-[2-(*tert*-butoxy)-2-oxoethyl]-5-cyclohexylpentanoic acid (preparation 168) (15.0g, 50.3mmol) and L-serine (9.38g, 55.3mmol) as starting materials. Purification: The crude material was combined with another batch and purified on a silica column eluting with a solvent gradient of pentane : EtOAc (100 : 0) gradually changing to (60:40) to afford the title compound as a pale yellow oil (21.6g, 78%).
¹Hnmr (CDCl₃) : 0.81 (2H, m), 1.10-1.35 (11H, m), 1.40 (9H, s), 1.55-1.70 (7H, m), 2.34 (1H, dd), 2.49 (1H, m), 2.65 (1H, dd), 3.81 (1H, d), 4.01 (1H, d), 4.21 (2H, q), 4.59 (1H, m), 6.56 (1H, d).
MS : 413 (M⁺)

### Preparation 152:

### ethyl (4S)-2-[(1R)-1-(2-tert-butoxy-2-oxoethyl)-4-cyclohexylbutyl]-4,5-dihydro-1,3-oxazole-4-carboxylate

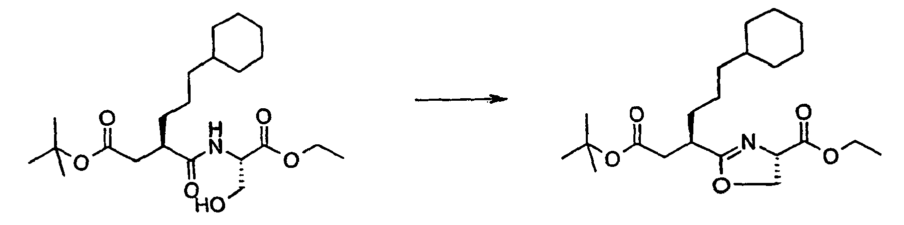

A solution of *tert*-butyl (3*R*)-6-cyclohexyl-3-({[(1*S*)-2-ethoxy-1-(hydroxymethyl)-2-oxoethyl]amino}carbonyl)hexanoate (preparation 151) (10.0g, 24.2mmol) in THF (80ml) was treated with Burgess reagent (6.34g, 26.6mmol) and heated at reflux for 1 hour. The reaction mixture was allowed to cool to room temperature and stored in a fridge for 3 days. The solvent was removed under reduced pressure. The crude material was ourififed on a silica column eluting with a solvent gradient of pentane : EtOAc (100 : 0) gradually changing to (80 : 20) to afford the title compound as a colourless oil (9.26g, 97%).
¹Hnmr (CDCl₃) : 0.81 (2H, m), 1.10-1.35 (11H, m), 1.40 (9H, s), 1.45-1.70 (7H, m), 2.39 (1H, dd), 2.60 (1H, dd), 2.85 (1H, m), 4.20 (2H, m), 4.30-4.45 (2H, m), 4.66 (1H, dd).
MS: 396 (MH⁺)

### Preparation 153:

### ethyl 2-[(1R)-1-(2-tert-butoxy-2-oxoethyl)-4-cyclohexylbutyl]-1,3-oxazole-4-carboxylate

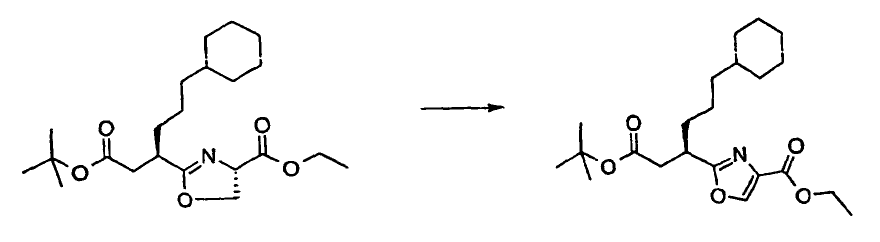

A suspension of CuBr₂ (20.88g, 93.5mmol) and HMTA (13.1g, 93.5mmol) in degassed DCM (250ml) was treated with DBU (14.0ml, 93.5mmol) and the dark solution formed was stirred in a water bath for 5 minutes. A solution of ethyl (4*S*)-2-[(1*R*)-1-(2-*tert*-butoxy-2-oxoethyl)-4-cyclohexylbutyl]4,5-dihydro-1,3-oxazole-4-carboxylate (preparation 152) (9.24g, 23.4mmol) In degassed DCM (30ml) was added dropwise to the reaction mixture and stirred at room temperature for a further 3 hours. The solvent was removed under reduced pressure. The resdue was dissolved in EtOAc and washed with a 1 : 1 solution of NH₃(aq) : NH₄Cl. The aqueous extract was washed with EtOAc (2x 380ml). The combined organic extracts were washed with 2M HCl (500ml), sat. NaHCO₃ (500ml) and brine (500ml). The organic extracts were dried over anhydrous MgSO₄ and filtered. The solvent was removed under reduced pressure. The crude material was purified on a silica column eluting with a solvent gradient of EtOAc : pentane (0 : 100) gradually changing to (25 : 75) to afford the title compound as colourless oil which formed needles on standing (7.39g, 80%).
¹Hnmr (CDCl₃) : 0.81 (2H, m), 1.10-1.30 (8H, m), 1.39 (12H, t+s), 1.55-1.80 (7H, m), 2.58 (1H. dd), 2.79 (1H, dd), 3.38 (1H, m), 4.39 (2H, q), 8.09 (1H, s).

### Preparation 154:

### (3R)-6-cyclohexyl-3-[4-(ethoxycarbonyl)-1,3-oxazol-2-yl]hexanoic acid

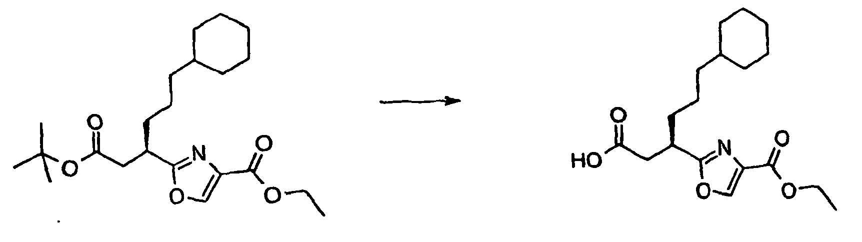

Method as for preparation 11 using ethyl 2-[(1*R*)-1-(2-*tert*-butoxy-2-oxoethyl)-4-cyclohexylbutyl]-1,3-oxazole-4-carboxylate (preparation 153) (7.39g, 18.8mmol) as starting material to afford the title compound as a pale yellow oil (6.34g, 99%).
¹Hnmr (CDCl₃) : 0.80 (2H, m), 1.05-1.30 (8H, m), 1.38 (3H, t), 1.55-1.80 (7H, m), 2.77 (1H, dd), 2.97 (1H, dd), 3.44 (1H, m), 4.38 (2H, q), 8.15 (1H, s).

### Preparation 155:

### ethyl 2-((1R)-1-{2-[(benzyloxy)amino]-2-oxoethyl}-4-cyclohexylbutyl)-1,3-oxazole-4-carboxylate

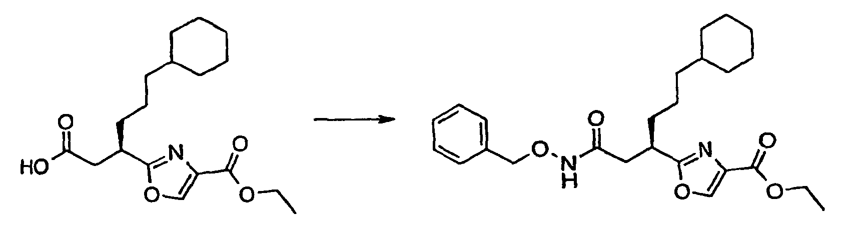

Method as for preparation 56 using (3*R*)-6-cyclohexyl-3-[4-(ethoxycarbonyl)-1,3-oxazol-2-yl]hexanoic acid (preparation 154) (2.21g, 6.55mmol) and *o*-benzylhydroxylamine (1.05g, (6.55mmol) as starting materials.
Purification: The crude material ws purified on a silica column eluting with a solvent gradient of pentane : EtOAc (100 : 0) gradually changing to (50 : 50) to afford the title compound as a colourless oil which solidified on standing (2.3g, 80%).
¹H nmr: (CDCl₃) 0.80 (2H, m), 1.10-1.30 (8H, m+EtOAc), 1.38 (3H, t), 1.55-1.75 (7H, m+H₂O), 2.41 (1H, brs), 2.65 (1H, brs), 3.45 (1H, brs), 4.39 (2H, q), 4.84 (2H, s), 7.37 (5H, s), 8.08 (1H, s), 8.50 (1H, brs).
MS : 443 (MH⁺)
CHN : Found: C67.62%; H7.78%; N6.30%; C₂₅H₃₄N₂O₅ requires C67.85; H7.74%; N6.33%

### Preparation 156:

### (3R)-N-(benzyloxy)-6-cyclohexyl-3-(4-formyl-1,3-oxazol-2-yl)hexanamide

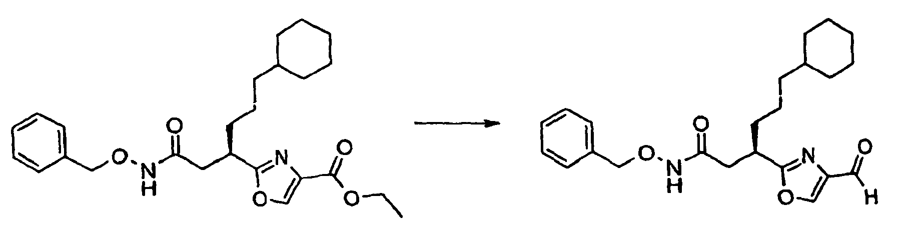

A solution of ethyl 2-((1*R*)-1-{2-[(benzyloxy)amino]-2-oxoethyl}-4-cyclohexylbutyl)-1,3-oxazole-4-carboxylate (preparation 155) (1.50g, 3.39mmol) in toluene (50ml) at -78°C was treated dropwise with DIBAL (1 M in hexanes) (11.86ml, 11.86mmol) and stirred at this temperature for 30 minutes. MeOH (12ml) was added to quench the reaction and the reaction mixture allowed to warm to room temperature. Alternate portions of NaHCO₃ and MgSO₄ were added to the reaction mixture to form a white paste. EtOAc was added and the solid filtered off. The solvent was removed under reduced pressure. The crude material was purified on a silica column eluting with a solvent gradient of EtOAc : pentane (1 : 1) gradually changing to (1 : 0) to afford the title compound as a colourless oil (605mg, 45%).
¹H nmr : (CDCl₃) 0.80 (2H, m), 1.10-1.30 (8H, m+EtOAc), 1.55-1.70 (7H, m+H₂O), 2.42 (1H, brs), 2.61 (1H, brs), 3.48 (1H, m+MeOH), 4.83 (2H, s), 7.35 (5H, s), 8.16 (1H, s), 8.43 (1H, brs), 9.81 (1H, brs).
MS : 399 (MH⁺)

### Preparation 157:

### (3R)-N-(benzyloxy)-6-cyclohexyl-3-{4-[(isopropylamino)methyl]-1,3-oxazol-2-yl}hexanamide

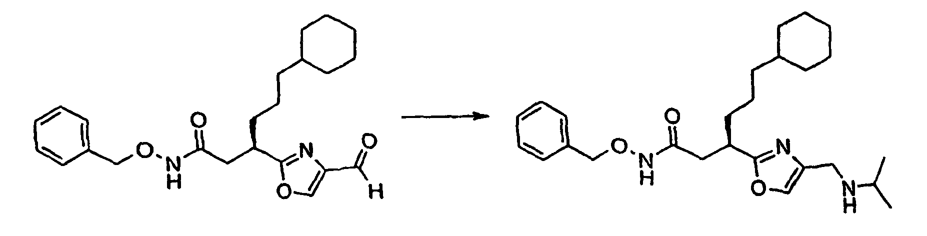

A solution of (3*R*)-*N*-(benzyloxy)-6-cyclohexyl-3-(4-formyl-1,3-oxazol-2-yl)hexanamide (preparation 156) (200mg, 0.50mmol) in DCM (10ml) was treated with isopropylamine (43µl, 0.50mmol) and stirred at room temperature for 15 minutes. NaHB(OAc)₃ (170mg, 0.80mmol) and AcOH (29µl, 0.50mmol) were added and the reaction mixture stirred at room temperature for 1 hour. The reaction mixture was diluted with DCM and washed with sat. NaHCO₃ solution followed by brine. The organic extract was dried over anhydrous MgSO₄ and filtered. The solvent was removed under reduced pressure. The crude material was purified on a silica column eluting with a solvent gradient of DCM : MeOH : NH₃ (100 : 0 : 0) gradually changing to (95 : 5 : 0.5) to afford the title compound asa colourless oil (75mg, 32%).
¹H nmr: (CDCl₃) 0.80 (2H, m), 1.00-1.30 (14H, d+m), 1.55-1.75 (7H, m), 2.40 (1H, brs), 2.59 (1H, brs), 2.80 (1H, m), 3.35 (1 H, brs), 3.59 (2H, s), 4.81 (2H, s), 7.37 (5H, s), 7.39 (1H, s), 8.50 (1H, s).
MS : 442 (MH⁺)

### Preparation 158:

### (3R)-N-(benzyloxy)-6-cyclohexyl-3-{4-[(cyclopentylamino)methyl]-1,3-oxazol-2-yl}hexanamide

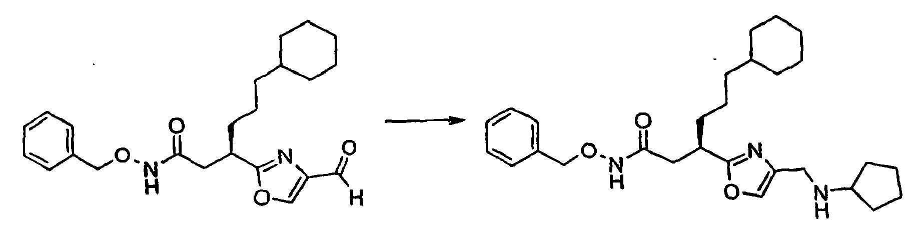

Method and purification as for preparation 157 using (3*R*)-*N*-(benzyloxy)-6-cyclohexyl-3-(4-formyl-1,3-oxazol-2-yl)hexanamide (preparation 156) (200mg, 0.50mmol) and cyclopentylamine (50µl, 0.50mmol) as starting materials to afford the title compound as a colourless oil (122mg, 48%).
¹H nmr: (CDCl₃) 0.80 (2H, m), 1.05-1.30 (8H, m), 1.34 (2H, m), 1.50 (2H, m), 1.55-1.75 (9H, m), 1.80 (2H, m), 2.40 (1H, brs), 2.59 (1H, brs), 3.03 (1H, m), 3.35 (1H, brs), 3.58 (2H, s), 4.82 (2H, s), 7.34 (5H, s), 7.38 (1H, s).
MS : 468 (MH⁺)

### Preparation 159:

### (3R)-N-(benzyloxy)-6-cyclohexyl-3-[4-(4-morpholinylmethyl)-1,3-oxazol-2-yl]hexanamide

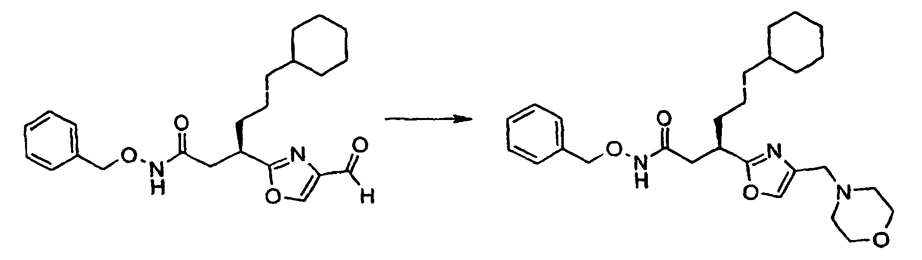

Method and purification as for preparation 157 using (3*R*)-*N*-(benzyloxy)-6-cyclohexyl-3-(4-formyl-1,3-oxazol-2-yl)hexanamide (preparation 156) (180mg, 0.45mmol) and morpholine (40µl, 0.45mmol) as starting materials to afford the title compound as a colourless oil (130mg, 62%).
¹H nmr : (CDCl₃) 0.80 (2H, m), 1.05-1.30 (8H, m), 1.55-1.75 (9H, m), 2.37 (1H, brs), 2.41 (4H, brs), 2.60 (1H, brs), 3.38 (2H, brs), 3.46 (1H, m), 3.35 (1H, brs), 3.66 (4H, brs), 4.82 (2H, s), 7.37 (5H, s), 7.40 (1H, s), 8.77 (1H, brs).
MS : 470 (MH⁺)

### Preparation 160:

### (3R)-N-(benzyloxy)-6-cyclohexyl-3-(4-formyl-5-methyl-1,3-oxazol-2-yl)hexanamide

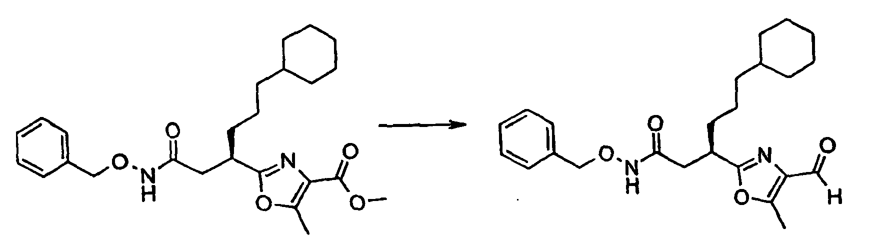

Method as for preparation 156 using methyl 2-((1*R*)-1-{2-[(benzyloxy)amino]-2-oxoethyl}-4-cyclohexylbutyl)-5-methyl-1,3-oxazole-4-carboxylate (preparation 175) (1.86g, 4.20mmol) as starting material.
Purification: The crude material was purified on a silica column eluting with EtOAc : pentane (1 : 1) to afford the title compound as a colourless oil (1.06g, 61%).
¹H nmr : (CDCl₃) 0.81 (2H, m), 1.05-1.30 (8H, m+EtOAc), 1.55-1.75 (7H, m), 2.46 (1H, brs), 2.55 (3H, s), 2.66 (1H, brs), 3.39 (1H, brs), 4.82 (2H, s), 7.33 (5H, brs), 9.81 (1H, brs).
MS: 413 (MH⁺)

### Preparation 161:

### (3R)-N-(benzyloxy)-6-cyclohexyl-3-{5-methyl-4-[(tetrahydro-2H-pyran-4-ylamino)methyl]-1,3-oxazol-2-yl}hexanamide

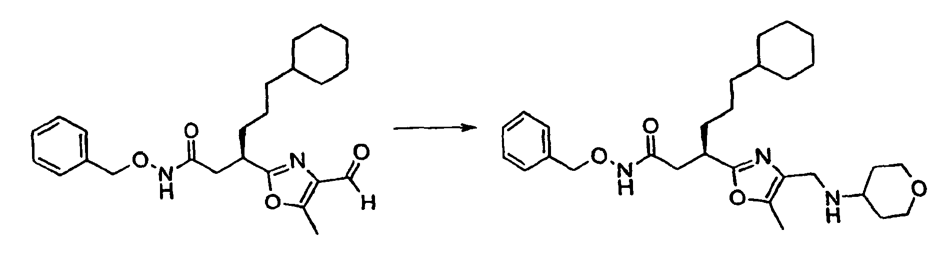

Method as for preparation 157 using (3*R*)-*N*-(benzyloxy)-6-cyclohexyl-3-(4-formyl-5-methyl-1,3-oxazol-2-yl)hexanamide (preparation 160) (300mg, 0.73mmol) and 4-aminotetrahydro-4H-pyran. HCl (J. Med. Chem.; 1971, *14*, 600-14) (110mg, 0.80mmol) as starting materials and DIPEA as base.
Purification: The crude material was purified on a silica column eluting with a solvent gradient of DCM : MeOH : NH₃ (98 : 2 : 0.2) gradually changing to (95 : 5 : 0.5) to afford the title compound as a colourless oil (222mg, 61 %).
¹H nmr : (CDCl₃) 0.82 (2H, m), 1.10-1.30 (8H, m), 1.38-1.70 (9H, m+H₂O), 1.79 (2H, brd), 2.21 (3H, s), 2.41 (1H, m), 2.65 (2H, m), 3.27 (1 H, brs), 3.38 (2H, t), 3.58 (2H, s), 3.95 (2H, m), 4.83 (2H, s), 7.35 (5H, s).
MS : 499 (M2H⁺)

### Preparation 162:

### (3R)-N-(benzyloxy)-6-cyclohexyl-3-[5-methyl-4-(4-morphotinylmethyl)-1,3-oxazol-2-yl]hexanamide

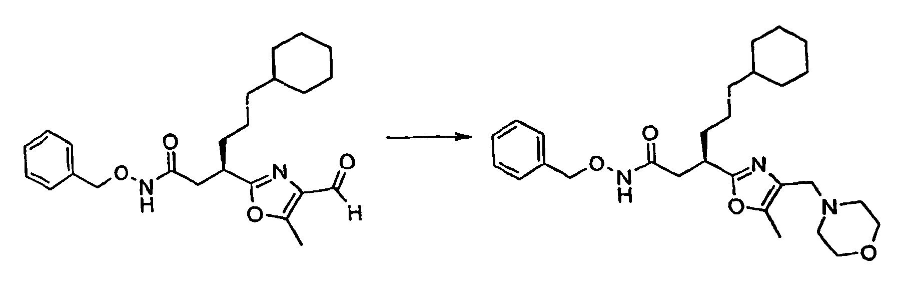

Method as for preparation 157 using (3*R*)-*N*-(benzyloxy)-6-cyclohexyl-3-(4-formyl-5-methyl-1,3-oxazol-2-yl)hexanamide (preparation 160) (300mg, 0.73mmol) and morpholine (64µl, 0.73mmol) as starting materials.
Purification: The crude material was purified on a silica column eluting with a solvent gradient of DCM: MeOH : NH₃ (100 : 0 : 0) gradually changing to (95 : 5 : 0.5) to afford the title compound as a colourless oil (156mg, 44%).
¹H nmr : (CDCl₃) 0.82 (2H, m), 1.05-1.35 (8H, m), 1.50-1.75 (7H, m), 2.21 (3H, s), 2.40 (5H, m), 2.62 (1H, brs), 3.23 (3H, m), 3.63 (4H, m), 4.83 (2H, s), 7.34 (5H, s).
MS : 484 (MH⁺)
Acc. Mass. : Found 484.3164 (MH⁺), Calculated C₂₈H₄₁N₃O₄ 484.3175 (MH⁺)

### Preparation 163:

### (3R)-3-[3-(aminomethyl)-1,2,4-oxadiazol-5-yl]-6-cyclohexylhexanoic acid

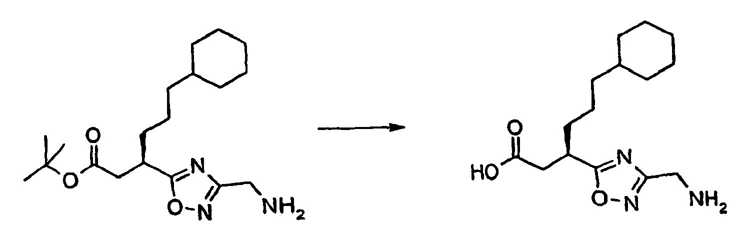

Method as for preparation 7 using (3R)-3-[3-(aminomethyl)-1,2,4-oxadiazol-5-yl]-6-cyclohexylhexanoate (preparation 18) (220mg, 0.63mmol) as starting material.
Purification: The crude material was azeotroped from EtOAc, DCM and Et₂O and dried under reduced pressure to afford the title compound (192mg, 92%).
¹H nmr: (d₆DMSO) 0.80 (2H, m), 1.05-1.30 (8H, m), 1.50-1.70 (7H, m), 2.75 (2H, d), 3.42 (1H, m), 4.20 (2H, s), 8.72 (2H, brs).
MS : 296 (MH⁺)
CHN : Found: C54.04%; H7.95%; N12.00%; C₁₅H₂₅N₃O₃. HCl. 0.1 EtOAc requires C54.30; H7.93%; N12.34%

### Preparation 164: 3-(diethoxyphosphoryl)succinic acid 1-tert-butyl ester

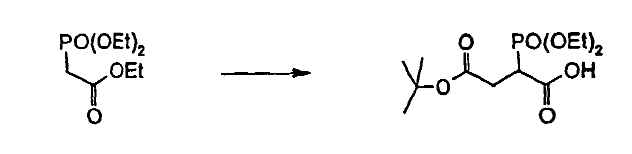

Triethylphosphonoacetate (12.0Kg, 53.5 mol) was added over 30 minutes to a stirred solution of potassium *tert*-butoxide (7.20Kg, 64.2 mol) in THF (118 litres), between 0 and 5°C, under nitrogen. The mixture was warmed to 25-30°C where it was stirred for 1 hour and then added over 45 minutes to a solution of *tert*-butyl bromoacetate (11.5Kg, 59.0 mol) in THF (28 litres), between 0 and 5°C, under nitrogen. The mixture was stirred at 0-5°C for 1 hour and then demineralised water (6.1 litres) and ethanol (30 litres) were added. A solution of potassium hydroxide (4.2Kg, 75.0 mol) in demineralised water (84 litres) was then added over 2 hours, between -5 and 0°C. The mixture was stirred at -10°C for 16 hours and then a solution of citric acid (16.5Kg, 85.8 mol) in demineralised water (32 litres) was added. The mixture was concentrated *in vacuo* to a volume of 180 litres and then ethyl acetate (90 litres) was added. The organic phase was separated and the aqueous phase was re-extracted with ethyl acetate (30 litres). The combined organic phases were washed with water (30 litres) and then stripped and replaced with cyclohexane by distillation at atmospheric pressure, at a constant volume of 72 litres. *tert*-Butylmethyl ether (18 litres) was added and the mixture was stirred at ambient temperature for 12 hours and then filtered. The residue was washed with a mixture of cyclohexane (16 litres) and *tert*-butylmethyl ether (3.6 litres) then dried *in vacuo* for 16 hours to give the title compound as a colourless solid (10.0Kg, 60% yield, 98% pure by HPLC).
¹H-NMR (CDCl₃) δ : 4.20-4.10 (4H, m), 3.49-3.36 (1H, m), 3.00-2.85 (1H, m), 2.72-2.60 (1H, m), 1.20 (9H, s), 1.37-1.27 (6H, m)

### preparation 165: (E)-2-[2-(tert-butoxy)-2-oxoethyl]-5-phenyl-2-pentenoic acid

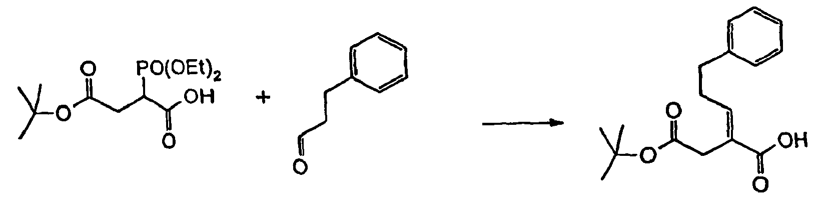

A solution of 3-(diethoxyphosphoryl)succinic acid 1-*tert*-butyl ester (Preparation 164,100g, 0.32mol) in THF (300ml) was added dropwise over 15 min to a stirred solution of potassium *tert-*butoxide (110g, 0.98mol) in THF (300ml), between -10 and -5°C, under nitrogen. The mixture was stirred at -10°C for 15 min and then a solution of hydrocinnamaldehyde (46.8g, 0.35mmol) in THF (100ml) was added dropwise over 15 min, between -13 and -8°C. The mixture was stirred at -10°C for 30 min and then a solution of citric acid (111g, 0.58mol) in demineralised water (500ml), and ethyl acetate (500ml), were added. The pH was adjusted to pH 4 with aqueous sodium hydroxide solution (50%) and the phases were separated. The aqueous fraction was washed with ethyl acetate (500ml) and the combined organic fractions were washed with saturated sodium bicarbonate solution (500ml), citric acid solution (10%, 500ml) and demineralised water (500ml) and then concentrated *in vacuo.* The resulting solid was slurried in cyclohexane (470ml) for 1 hour and then the mixture was filtered. The residue was washed with cyclohexane (2x50ml) and dried *in vacuo* to leave the title compound as a colourless solid (76g, 81% yield, 99% pure by HPLC).
MS : 289 [(M-H)]⁻
¹H-NMR (CDCl₃) δ : 7.33-7.16 (5H, m), 7.05 (1H, br t), 3.20 (2H, s), 2.89 (2H, br t), 2.50 (2H, br dd), 1.41 (9H, s)

### Preparation 166: (R)-2-[2-(tert-butoxy)-2-oxoethyl]-5-phenylpentanoic acid cyclohexylamine salt

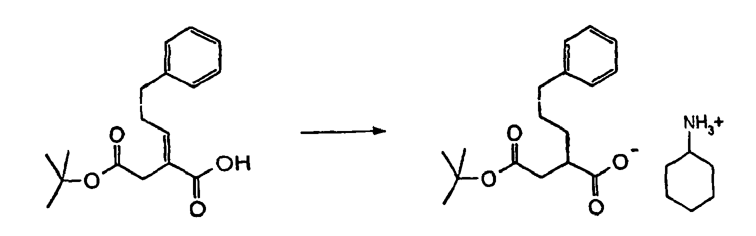

A stirred solution of cyclohexylamine (266ml, 2.32 mol), (*E*)-2-[2-(*tert*-butoxy)-2-oxoethyl]-5-phenyl-2-pentenoic acid (Preparation 165, 688g, 2.37 mol) and [(*S*)-2,2'-bis(diphenylphosphino-1,1'-binaphthyl]chloro(*p*-cymene)ruthenium chloride (4.4g, 4.7 mmol) in methanol (6.9 litres) was heated to 60°C, under hydrogen (60p.s.i.), for 47 hours and then allowed to cool to room temperature (enantiomeric excess= 88%). The mixture was filtered through celite and then the solvent was stripped and replaced with acetone by distillation at atmospheric pressure, at a constant volume of 4.2 litres. The resulting suspension was cooled to room temperature where it was stirred for 4 hours and then filtered. The residue was washed with acetone (2x 1 litre) and then dried *in vacuo* at 45°C for 16 hours to leave the title compound as a colourless solid (590g, 64% yield, enantiomeric excess=98.9%. 97% pure by HPLC).
¹H-NMR (CD₃OD) δ : 7.23-7.09 (5H, m), 3.05-2.98 (1H, m), 2.64-2.56 (3H, m), 2.53 (1H, dd, J 15.2, 7.2Hz), 2.23 (1H, dd, J 15.2, 7.2Hz), 2.00-1.97, (2H, m), 1.85-1.81 (2H, m), 1.72-1.20 (10H, m), 1.40 (9H, s)

### Preparation 167: (R)-2-[2-(tert-butoxy)-2-oxoethyl]-5-cyclohexylpentanoic acid sodium salt

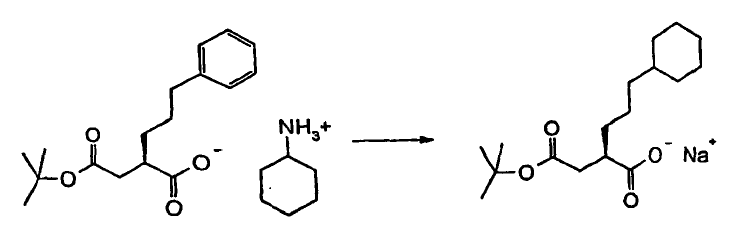

(*R*)-2-[2-(*tert*-Butoxy)-2-oxoethyl]-5-phenylpentanoic acid cyclohexylamine salt (Preparation 166, 6.8g, 17 mmol) and ethyl acetate (100ml) were added to an aqueous solution of citric acid (10%. 100ml) and the organic phase was separated and washed with water (100ml). /so-propyl alcohol (20ml) and 5% rhodium on alumina (51.6g) were added and the mixture was stirred at room temperature, under hydrogen (10 atmospheres, 150p.s.i.) for 48 hours and then filtered through celite. To the filtrate was added a solution of sodium hydroxide (0.67g, 17 mmol) in water and the mixture was stripped and replaced with acetonitrile by distillation at atmospheric pressure to a volume of 30ml. The mixture was allowed to cool to room temperature where it was stirred for 24 hours. The mixture was cooled to 0°C and then filtered. The residue was washed with acetonitrile (2x 10ml) and then dried *in vacuo* at 45°C for 2 hours to leave the title compound as a colourless solid (3.8g, 69% yield, 95% pure by NMR).
¹H-NMR (CD₃OD) δ: 2.62-2.57 (1H, m), 2.53 (1H, dd, J 14.8, 7.2Hz), 2.23 (1H, dd, J 14.8, 7.2Hz), 1.76-1.18 (15H, m),1.44 (9H, s), 0.93-0.82 (2H, m)

### Preparation 168 : (2R)-2-[2-(tert-Butoxy)-2-oxoethyl]-5-cyclohexylpentanoic acid

### Route A:

### (2R)-2-[2-(tert-butoxy)-2-oxoethyl]-5-cyclohexylpentanoic acid

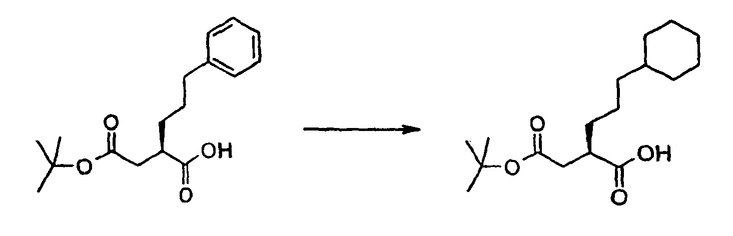

A solution of (2R)-2-[2-(tert-butoxy)-2-oxoethyl]-5-phenylpentanoic acid (Syn. Lett.; 1998; 637-639) (10.00g, 34.2mmol) in acetic acid (120ml) was treated with 5% Rhodium on alumina catalyst, pressurised to 60psi with hydrogen in a sealed vessel and stirred at room temperature for 17 hours. The mixture was filtered through a pad of Arbocel® and the solvent was removed from the filtrate under reduced pressure. The residue was azeotroped from toluene to afford the title compound (7.53g).
MS : 299 (MH⁺)
¹H-NMR (CDCl₃) δ : 2.80 (1H, m), 2.61 (1H, m), 2.38 (1 H, m), 1.75-1.56 (7H, m), 1.55-1.04 (17H, m), 0.84 (2H, m).

### Route B:

### (4S)-A-Benzyl-3-(5-cyclohexylpentanoyl)-1,3-oxazolidin-2-one

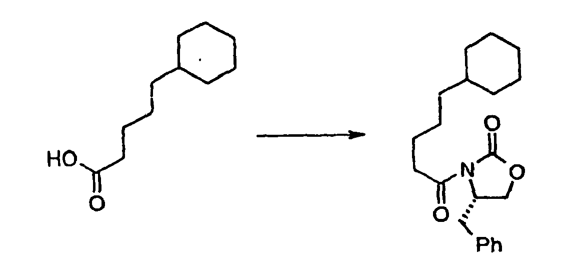

A solution of 5-cyclohexylpentanoic acid (63.50g, 345mmol) in N,N-dimethylformamide (0.5ml) and dichloromethane (350ml) was cooled to 5°C and treated dropwise with oxalyl chloride (31.6ml, 362mmol) over 30 minutes. The mixture was stirred at 0°C for 3 hours then the solvent was removed under reduced pressure to afford 5-cyclohexylpentanoyl chloride as a pale yellow solid (70.0g).
A solution of n-butyllithium (100ml, 250mmol, 2.5M in hexanes) was added via a cannula to a solution of (4S)-4-benzyl-1,3-oxazolidin-2-one (44.30g, 250mmol) in anhydrous tetrahydrofuran (400ml) at -78°C. The yellow solution was then stirred for 45 minutes. A solution of 5-cyclohexylpentanoyl chloride (55.5g, 275mmol) in tetrahydrofuran (100ml) was then added over 1 hour. The mixture was stirred at -78°C for 30 minutes then warmed to room temperature over 1 hour. The mixture was quenched with an aqueous solution of ammonium chloride (20% w/v, 400ml) and extracted with ethyl acetate. The layers were separated and the organic layer was washed with brine, dried over anhydrous sodium sulphate and the solvent removed under reduced pressure. The solid was recrystallised from hexane (500ml) to afford the title compound as a white solid (81.0g).
MS: 344 (MH⁺)
¹H-NMR (COCl₃) δ : 7.41-7.13 (5H, m), 4.68 (1H, m), 4.27-4.02 (2H, m), 3.31 (1H, dd, J=16, 4Hz), 3.06-2.70 (3H, m), 1.81-1.53 (7H, m), 1.49-1.04 (8H, m), 0.88 (2H, m)

### tert-Butyl 3-{[(4S)-4-benzyl-2-oxo-1,3-oxazolidin-3-yl]carbonyl}-6-cyclohexylhexanoate

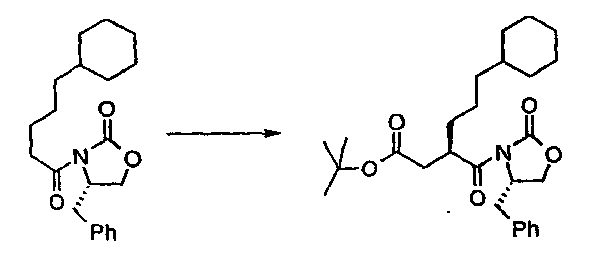

A solution of (4S)-4-benzyl-3-(5-cyclohexylpentanoyl)-1,3-oxazolidin-2-one (70.0g, 204mmol) in anhydrous tetrahydrofuran (650ml) was cooied to -70°C and treated dropwise with sodium hexamethyldisilazide (1M in tetrahydrofuran, 224ml, 224mmol) over 45 minutes. The mixture was stirred for a further 45 minutes before being treated with t-butylbromoacetate (31.6ml, 214mmol). This mixture was stirred at -70°C for 30 minutes then warmed to -30°C and quenched with an aqueous solution of ammonium chloride (20%w/v, 400ml) and warmed to room temperature. The mixture was extracted with ethyl acetate and the combined organic extracts were washed with brine, dried over anhydrous sodium sulphate and the solvent removed under reduced pressure. The solid was recrystallised from hexane to afford the title compound as a while solid (71.4g).
MS: 458(MH⁺)
¹H-NMR (CDCl₃) δ : 7.41-7.13 (5H, m), 4.66 (1H, m), 423-4.03 (3H, m), 3.35 (1H, dd, J=16, 4Hz), 2.95-2.68 (3H, m), 2.47 (1H, m), 1.80-1.07 (24H, m), 0.85 (2H, m)

### 2-[2-(tert-Butoxy)-2-oxoethyl]-5-cyclohexylpentanoic acid

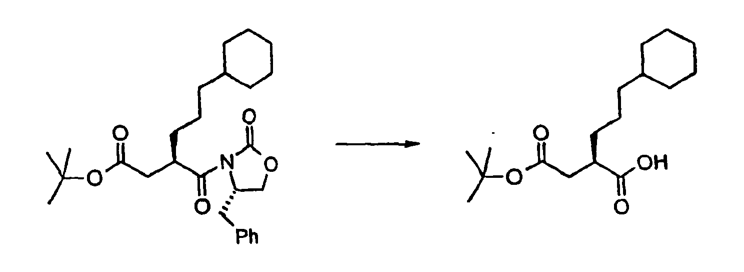

A solution of tert-butyl 3-{[(4S)-4-benzyl-2-oxo-1,3-oxazolidin-3-yl]carbonyl}-6-cyclohexylhexanoate (64.0g, 139.9mmol) in tetrahydrofuran : water (3:1, 800ml) was cooled to 5°C then treated sequentially with hydrogen peroxide (30%w/v water, 87ml, 769mmol) then lithium hydroxide hydrate (10.0g, 238mmol). The reaction was stirred for 1 hour then quenched by dropwise addition of an aqueous solution of sodium thiosulphate (500ml) keeping the temperature below 20°C. The mixture was extracted with ethyl acetate (discarded) and the aqueous phase was acidifed to pH 2 with solid citric acid and extracted with ethyl acetate. The combined organic phases were washed with brine, dried over anhydrous sodium sulphate and the solvent removed under reduced pressure. The residue was purified by column chromatography on silica gel eluting with a gradient system of hexane : ethyl acetate (2 : 1) gradually changing to hexane : ethyl acetate (1 : 1) to afford the title compound (40.7g)

### Route C:

### 3-(Diethoxyphosphoryl)succinic acid 1-tert-butyl ester

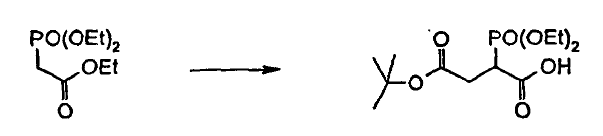

Triethylphosphonoacetate (102g, 0.45 mol) was added dropwise over 11 min to a stirred solution of potassium tert-butoxide (60g, 0.54 mol) in THF (500ml), at 0°C, under nitrogen. The mixture was stirred for 1 hour at 0°C and then dichloromethane (300ml) was added and the reaction mixture was warmed to 25-30°C. The mixture was stirred at 25-30°C for 1 hour and then added dropwise over 33 minutes to a solution of tert-butyl bromoacetate (96g, 0.49 mol) in THF (500ml), at 0°C, under nitrogen. The mixture was stirred at 0-5°C for 2 hours and then a solution of citric acid (174g, 0.91 mol) in demineralised water (250ml) was added. The mixture was concentrated in vacuo to remove most of the THF and then toluene (750ml) was added. The organic phase was separated, washed with brine (2x150ml) and concentrated in vacuo to leave a colourless oil. The oil was taken up in ethanol and a solution of potassium hydroxide (36.g, 0.64mol) in demineralised water (150ml) was added dropwise over 15 mins. The mixture was stirred at 0°C for 4 hours and then a solution of citric acid (158 g, 0.82 mol) in demineralised water (600ml), and toluene (600ml), were added. The organic phase was separated and the aqueous phase was re-extracted with toluene (600ml). The combined organic phases were washed with demineralised water (2x150ml) and concentrated in vacuo to leave a white solid. Toluene (150ml) was added and the slurry was re-concentrated in vacuo to leave a white solid. The product was purified by crystallisation from tert-butylmethyl ether (300ml) and cyclohexane (600ml) to give the title compound as a solid (79g).
¹H-NMR (CDCl₃) δ : 4.20-4.10 (4H, m), 3.49-3.36 (1H, m), 3.00-2.85 (1H, m), 2.72-2.60 (1H, m), 1.20 (9H, s), 1.37-1.27 (6H, m)

### Alternative preparation:

Triethylphosphonoacetate (12.0Kg, 53.5 mol) was added over 30 minutes to a stirred solution of potassium *tert*-butoxide (7.20Kg, 64.2 mol) in THF (118 litres), between 0 and 5°C, under nitrogen. The mixture was warmed to 25-30°C where it was stirred for 1 hour and then added over 45 minutes to a solution of *tert*-butyl bromoacetate (11.5Kg, 59.0 mol) In THF (28 litres), between 0 and 5°C, under nitrogen. The mixture was stirred at 0-5°C for 1 hour and then demineralised water (6.1 litres) and ethanol (30 litres) were added. A solution of potassium hydroxide (4.2Kg, 75.0 mol) in demineralised water (84 litres) was then added over 2 hours, between -5 and 0°C. The mixture was stirred at -10°C for 16 hours and then a solution of citric acid (16.5Kg, 85.8 mol) in demineralised water (32 litres) was added. The mixture was concentrated *in vacuo* to a volume of 180 litres and then ethyl acetate (90 litres) was added. The organic phase was separated and the aqueous phase was re-extracted with ethyl acetate (30 litres). The combined organic phases were washed with water (30 litres) and then stripped and replaced with cyclohexane by distillation at atmospheric pressure, at a constant volume of 72 litres. *tert*-Butylmethyl ether (18 litres) was added and the mixture was stirred at ambient temperature for 12 hours and then filtered. The residue was washed with a mixture of cyclohexane (16 litres) and *tert*-butylmethyl ether (3.6 litres) then dried *in vacuo* for 16 hours to give the title compound as a colourless solid (10.0Kg, 60%).
¹H-NMR (CDCl₃) δ : 4.20-4.10 (4H, m), 3.49-3.36 (1H, m), 3.00-2.85 (1H, m), 2.72-2.60 (1H, m), 1.20 (9H, s), 1.37-1.27 (6H, m)

### (E)-2-[2-(tert-Butoxy)-2-oxoethyl]-5-phenyl-2-pentenoic acid

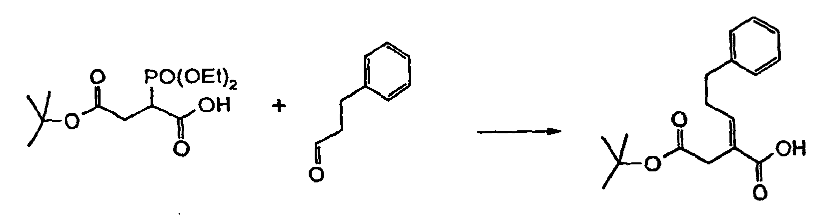

A solution of 3-(diethoxyphosphoryl)succinic acid 1-*tert*-butyl ester (100g, 0.32mol) in THF (300ml) was added dropwise over 15 min to a stirred solution of potassium *tert*-butoxide (110g, 0.98mol) in THF (300ml), between -10 and -5°C, under nitrogen. The mixture was stirred at -10°C for 15 min and then a solution of hydrocinnamaldehyde (46.8g, 0.35mmol) in THF (100ml) was added dropwise over 15 min, between -13 and -8°C. The mixture was stirred at -10°C for 30 min and then a solution of citric acid (111g, 0.58mol) in demineralised water (500ml), and ethyl acetate (500ml), were added. The pH was adjusted to pH 4 with aqueous sodium hydroxide solution (50%) and the phases were separated. The aqueous fraction was washed with ethyl acetate (500ml) and the combined organic fractions were washed with saturated sodium bicarbonate solution (500ml), citric acid solution (10%, 500ml) and demineralised water (500ml) and then concentrated *in vacuo.* The resulting solid was slurried in cyclohexane (470ml) for 1 hour and then the mixture was filtered. The residue was washed with cyclohexane (2x50ml) and dried *in vacuo* to leave the title compound as a colourless solid (76g, 81%).
MS : 289 [(M-H)]⁻
¹H-NMR (CDCl₃) δ : 7.33-7.16 (5H, m), 7.05 (1 H, br t), 3.20 (2H, s), 2.89 (2H, br t), 2.50 (2H, br dd), 1.41 (9H, s)

### (R)-2-[2-(tert-Butoxy)-2-oxoethyl]-5-phenylpentanoic acid

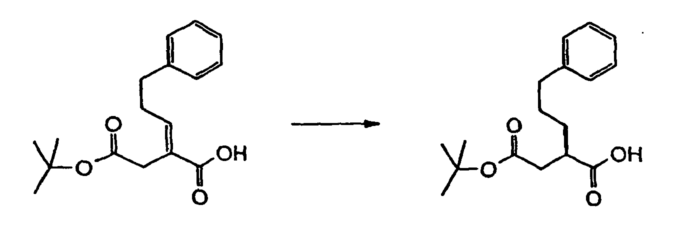

A stirred solution of (E)-2-[2-(tert-butoxy)-2-oxoethyl]-5-phenyl-2-pentenoic acid (100g, 0.34mol), cyclohexylamine (39ml, 0.34mol) and [(S)-2,2'-bis(diphenylphosphino-1,1'-binaphthyl]chloro(p-cymene)ruthenium chloride (0.64g, 0.69mmol) in methanol (1000ml) was heated to 60°C, under hydrogen (60p.s.i.), for 42 hours and then allowed to cool to room temperature. The mixture was filtered through celite and then concentrated in vacuo to a yellow solid which was purified by re-crystallisation from acetone (850ml). The resulting solid was partitioned between ethyl acetate (1200ml) and citric acid solution (10%, 1200ml) and the organic phase was separated, washed with demineralised water (1200ml) and concentrated in vacuo to leave the title compound as an oil (80g).
¹H-NMR (CDCl₃) δ : 7.30-7.17 (5H, m), 2.85-2.78 (1 H, m), 2.66-2.58 (3H, m), 2.37 (1H, br dd), 1.75-1.51 (4H, m), 1.40 (9H, s)

### Preparation of cyclohexylamine salt:

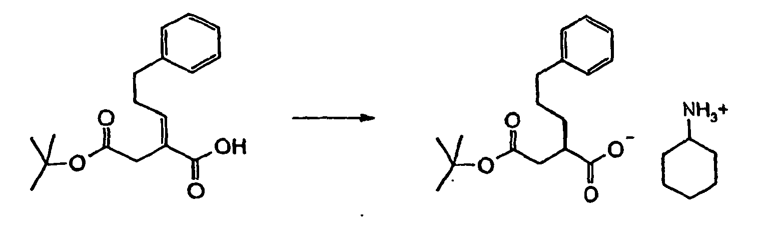

A stirred solution of cyclohexylamine (266ml, 2.32 mol), (*E*)-2-[2-(*tert*-butoxy)-2-oxoethyl]-5-phenyl-2-pentenoic acid (688g, 2.37 mol) and [(*S*)-2,2'-bis(diphenylphosphino-1,1'-binaphthyl]chloro(*p*-cymene)ruthenium chloride (4.4g, 4.7 mmol) in methanol (6.9 litres) was heated to 60°C, under hydrogen (60p.s.i.), for 47 hours and then allowed to cool to room temperature (enantiomeric excess= 88%). The mixture was filtered through celite and then the solvent was stripped and replaced with acetone by distillation at atmospheric pressure, at a constant volume of 4.2 litres. The resulting suspension was cooled to room temperature where it was stirred for 4 hours and then filtered. The residue was washed with acetone (2x 1 litre) and then dried *in vacuo* at 45°C for 16 hours to leave the title compound as a colouriess solid (590g, 64%, enantiomeric excess=98.9%).
¹H-NMR (CD₃OD) δ : 7.23-7.09 (5H, m), 3.05-2.98 (1H, m), 2.64-2.56 (3H, m), 2.53 (1H, dd, J 15.2, 7.2Hz), 2.23 (1H, dd, J 15.2, 7.2Hz), 2.00-1.97, (2H, m), 1.85-1.81 (2H, m), 1.72-1.20 (10H, m), 1.40 (9H, s)

### (R)-2-[2-(tert-Butoxy)-2-oxoethyl]-5-cyclohexylpentanoic acid cyclohexylamine salt

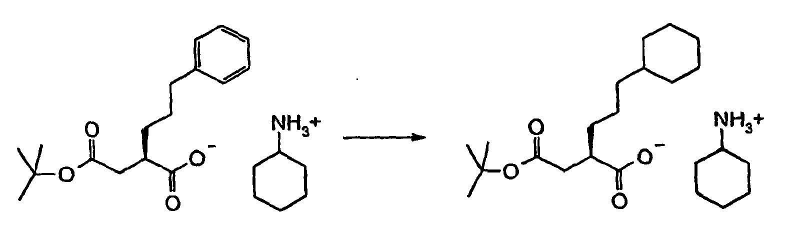

(*R*)-2-[2-(*tert*-Butoxy)-2-oxoethyl]-5-phenylpentanoic acid cyclohexylamine salt (691g, 1.77 mol) and ethyl acetate (7.0 litres) were added to an aqueous solution of citric acid (10%, 6.3 litres) and the organic phase was separated, washed with water (7.0 litres) and concentrated *in vacuo* to a yellow oil. A solution of the oil and 5% rhodium on carbon (51.6g) in methanol (7.0 litres) was stirred at ambient temperature, under hydrogen (150p.s.i.) for 48 hours and then filtered through celite. To the filtrate was added cyclohexylamine (202ml, 1.77 mol) and the methanol solution was stripped and replaced with methylethyl ketone by distillation at atmospheric pressure, to a volume of 5.5 litres. The mixture was allowed to cool to ambient temperature where it was stirred for 48 hours and then filtered. The residue was washed with methylethyl ketone (2x 500ml) and then dried *in vacuo* at 45°C for 4 hours to leave the title compound as a colourless solid (495g, 71 %).
¹H-NMR (CD₃OD) δ : 3.06-2.99 (1H, m), 2.63-2.56 (1H, m), 2.53 (1H, dd, J 15.2, 7.2Hz), 2.23 (1H, dd, J 15.2, 7.2Hz), 2.02-1.97 (2H, m), 1.77-1.15 (21H, m), 1.43 (9H, s), 0.93-0.82 (2H, m)

### (R)-2-[2-(tert-Butoxy)-2-oxoethyl]-5-cyclohexylpentanoic acid

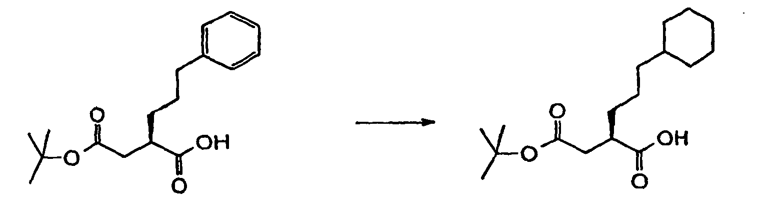

A solution of (R)-2-[2-(tert-butoxy)-2-oxoethyl]-5-phenylpentanoic acid (2.2g, 7.5mmol) and 5%Rh/C (0.22g) in methanol (220ml) was stirred at room temperature, under hydrogen (150p.s.i.) for 24 hours and then filtered through celite. The filtrate was concentrated in vacuo to leave the title compound as an oil (2.0g).
¹H-NMR (CDCl₃) δ : 2.82-2.76 (1H, m), 2.60 (1H, br dd), 2.37 (1H, br dd), 1.70-1.60 (6H, m), 1.51-1.30 (3H, m), 1.42 (9H, s), 1.23-1.11 (6H, m), 0.96-0.80 (2H, m)

### Preparation 169 :

### tert-Butyl (3R)-3-[({[(Z)-1-amino-2-ethoxy-2-oxoethylidene]amino}oxy)carbonyl]-6-cyclohexylhexanoate

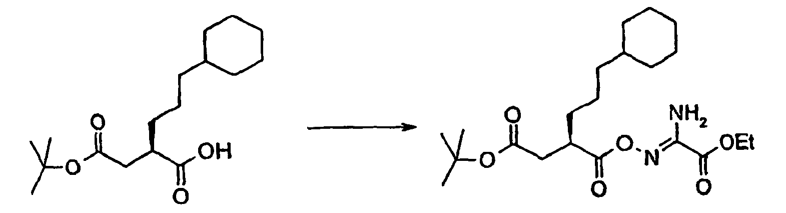

A solution of (2R)-2-[2-(tert-butoxy)-2-oxoethyl]-5-cyclohexylpentanoic acid (Preparation 168) (7.53g, 25.2mmol) in 1,4-dioxane (175ml) was treated with 1-hydroxybenzotriazole hydrate (3.75g, 27.8mmol) and the mixture cooled to 0°C. N,N'-Dicyclohexylcarbodiimide (5.47g, 26.5mmol) was then added and the mixture was stirred for 3 hours being allowed to warm to room temperature over this time. The mixture was then filtered and washed with 1,4-dioxane (2x50ml). The filtrate was then treated with sodium carbonate (4.01g, 37.8mmol) and ethyl 2-amino-2-(hydroxyimino)acetate (J.Org.Chem.;23; 1958; 1794) (3.33g, 25.2mmol). The resulting mixture was stirred at room temperature for 17 hours. The solvent was removed under reduced pressure and the residue was partitioned between ethyl acetate and water. The layers were separated and the aqueous phase was extracted with ethyl acetate (x2). The combined organic layers were dried over anhydrous magnesium sulphate, filtered and the solvent removed under reduced pressure. The residue was purified by column chromatography on silica gel eluting with a gradient system of ethyl acetate : pentane (30 : 70) gradually changing to ethyl acetate : pentane (50 : 50) to afford the title compound as a white solid (6.50g).
MS : 413 (MH⁺)
¹H-NMR (CDCl₃) δ : 5.71 (2H, br s), 4.39 (2H, q), 2.92 (1 H, m), 2.67 (1H, dd), 2.44 (1H, dd), 1.75-1.32 (22H, m), 1.26-1.04 (5H, m), 0.84 (2H, m).

### Preparation 170 :

### Ethyl 5-{(1R)-1-[2-(tert-buloxy)-2-oxoethyl]-4-cyclchexylbutyl}-1,2,4-oxadiazole-3-carboxylate

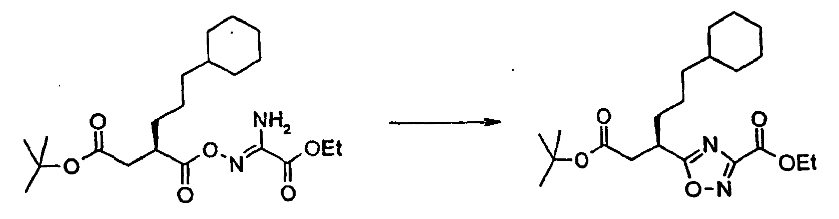

A solution of tert-Butyl (3R)-3-[({[(Z)-1-amino-2-ethoxy-2-oxoethylidene]amino}oxy)carbonyl]-6-cyclohexylhexanoate (Preparation 169) (21.0g, 50.82mmol) in xylene (400ml) was heated at 130°C for 17 hours, then allowed to cool to room temperature. The residue was purified by column chromatography on silica gel eluting with a gradient system of ethyl acetate : pentane (5 : 95) gradually changing to ethyl acetate : pentane (20 : 80) to afford the title compound as a colourless oil (20.0g).
MS: 395 (MH⁺), 412 (MNH₄⁺)
¹H-NMR (CDCl₃) δ : 4.51 (2H, m), 3.54 (1H, m), 2.86 (1H, dd), 2.65 (1H, dd), 1.86-1.57 (7H, m), 1.50-1.33 (12H, m), 1.30-1.03 (8H, m), 0.82 (2H, m).

### Preparation 171:

### tert-Butyl (3R)-6-cyclohexyl-3-({[2-hydroxy-1-(methoxycarbonyl)propyl]amino}carbonyl) hexanoate

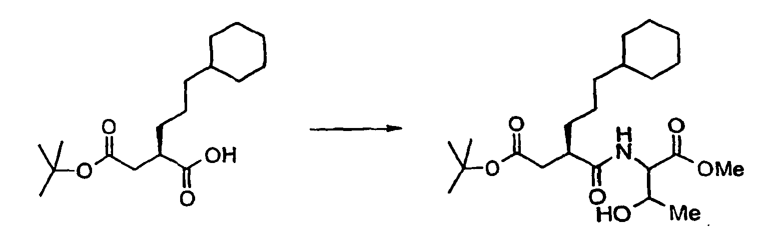

An ice-cooled solution of (2R)-2-[2-(tert-butoxy)-2-oxoethyl]-5-cyclohexylpentanoic acid (Preparation 168) (6.40g, 21.45mmol) in dichloromethane (75ml) was treated sequentially with 1-hydroxybenzotriazole hydrate (3.19g, 23.60mmol), threonine methyl ester hydrochloride (4.00g, 23.60mmol), N,N-diisopropylethylamine (7.85ml, 45.10mmol) and 1-[3-(dimethylamino)propyl]-3-ethylcarbodiimide hydrochloride (4.52g, 23.58mmol) and the mixture was stirred for 17 hours being allowed to warm to room temperature over this time. The solvent was removed under reduced pressure and the residue was dissolved in ethyl acetate then washed sequentially with water, aqueous citric acid solution (10% w/v), a saturated aqueous sodium hydrogen carbonate solution and brine, dried over anhydrous magnesium sulphate, filtered and the solvent removed under reduced pressure. The residue was dissolved in diethyl ether (100ml) and treated with pentane (150ml) to produce a white precipitate. This was filtered off and washed with pentane to afford the title compound as a white powder (6.48g).
MS : 436 (MNa⁺)
¹H-NMR (CDCl₃) δ : 6.35 (1H, br d), 4.63 (1H, m), 4.26 (1H, m), 3.76 (3H, s), 2.73-2.53 (2H, m), 2.34 (1H, m), 1.73-1.56 (7H, m), 1.45-1.09 (20H, m), 0.84 (2H, m)

### Preparation 172:

### tert-Butyl (3R)-6-cyclohexyl-3-({[1-(methoxycarbonyl)-2-oxopropyl]amino}carbonyl) hexanoate

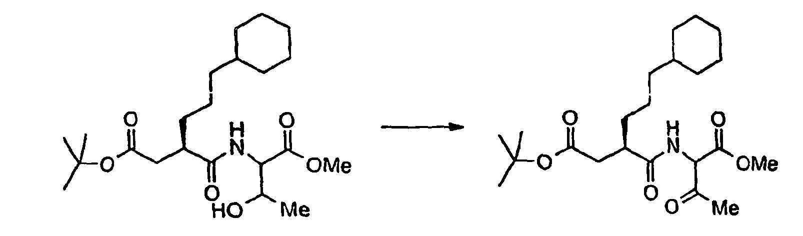

A solution of tert-butyl (3R)-6-cyclohexyl-3-({[2-hydroxy-1-(methoxycarbonyl)propyl]amino}carbonyl) hexanoate (Preparation 171) (6.48g, 15.69mmol) in dichloromethane (60ml) was treated with Dess-Martin periodinane [1.1,1-triacetoxy-1,1-dihydro-1,2-benziodoxol-3(1H)-one] (7.32g, 17.26mmol) and the resulting mixture was stirred at room temperature under a nitrogen atmosphere for 1 hour. A solution of sodium thiosulphate (6g in 50ml water) and a saturated aqueous sodium hydrogen carbonate solution (50ml) were then added to the mixture which was stirred for a further 10 minutes. The layers were separated and the aqueous layer was extracted with dichloromethane. The combined organic layers were sequentially washed with water and brine, dried over anhydrous magnesium sulphate, filtered and the solvent removed under reduced pressure. The residue was purified by column chromatography on silica gel eluting with a gradient system of pentane : ethyl acetate (100:0 to 90:10) to afford the title compound as a colourless oil (4.86g)
MS : 412 (MH⁺), 434 (MNa⁺)
¹H-NMR (CDCl₃) δ : 6.82 (1H, br d), 5.21 (1H, m), 3.78 (3H, s), 2.72-2.52 (2H, m), 2.40-2.25 (4H, m), 1.72-1.53 (7H, m), 1.45-1.04 (17H, m), 0.83 (2H, m)

### Preparation 173:

### Methyl 2-{(1R)-1-[2-(tert-butoxy)-2-oxoethyl]-4-cyclohexylbutyl}-5-methyl-1,3-oxazole-4-carboxylate

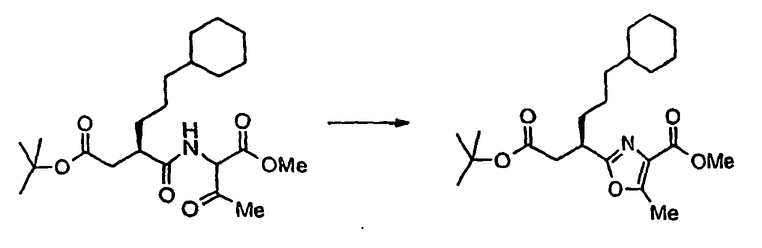

A suspension of triphenylphosphine (9.49g, 36.18mmol), iodine (7.98g, 31.44mmol) and triethylamine (8.40ml, 60.75mmol) in tetrahydrofuren was cooled to -78°C then treated with tert-butyl (3R)-6-cyclohexyl-3-({[1-(methoxycarbonyl)-2-oxopropyl]amino}carbonyl) hexanoate (Preparation 172) (4.86g, 11.80mmol) over 15 minutes. The mixture was stirred at -78°C for 30 minutes then at 0-5°C for 2 hours. The mixture was diluted with water and extracted with dichloromethane. The combined organic layers were dried over anhydrous magnesium sulphate, filtered and the solvent removed under reduced pressure. The residue was purified by column chromatography on silica gel eluting with a gradient system of ethyl acetate : pentane (0: 100 to 10:90) to afford the title compound as a colourless oil (2.92g).
MS : 394 (MH⁺), 416 (MNa⁺)
¹H-NMR (COCl₃) δ : 3.89 (3H, s), 3.29 (1H, m), 2.74 (1H, dd, J=14, 6Hz), 2.60-2.49 (4H, m), 1.79-1.54 (7H, m), 1.38 (9H, s), 1.31-1.05 (8H, m), 0.82 (2H, m)

### Preparation 174:

### (3R)-6-Cyclohexyl-3-[4-(methoxycarbonyl)-5-methyl-1,3-oxazol-2-yl]hexanoic acid

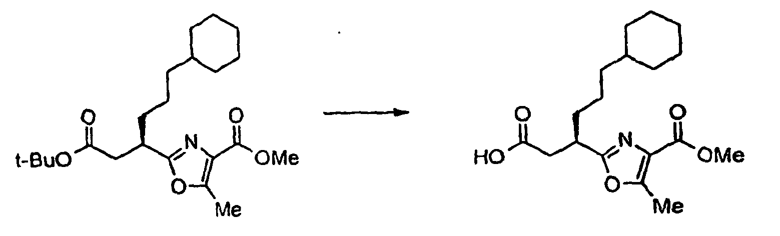

A solution of methyl 2-{(1R)-1-[2-(tert-butoxy)-2-oxoethyl]-4-cyclohexylbutyl}-5-methyl-1,3-oxazole-4-carboxylate (Preparation 173) (2.92g, 7.43mmol) in anhydrous dichloromethane (15ml) was treated with trifluoroacetic acid (7.5ml) and the resulting mixture was stirred at room temperature under a nitrogen atmosphere for 24 hours. The solvent was removed under reduced pressure and the residue was azeotroped with dichloromethane (x3). The residue was purified by column chromatography on silica gel eluting with a gradient system of ethyl acetate : pentane (0:100 to 40:60) to afford the title compound as a colourless oil (2.50g).
MS : 338 (MH⁺), 360 (MNa⁺)
Analysis : Found C, 62.90; H, 8.18; N, 3.93%; C₁₈H₂₇NO₅. 0.3 EtOAc requires C, 63.38;H, 8.14; N, 3.85%
¹H-NMR (CDCl₃) δ : 3.86 (3H, s), 3.33 (1H, m), 2.92 (1H, dd, J=17, 8Hz), 2.67 (1H, dd, J=17, 5Hz), 2.58 (3H, s), 1.81-1.56 (7H, m), 1.34-1.03 (8H, m), 0.81 (2H, m)

### Preparation 175:

### Methyl 2-((1R)-1-{2-[(benzyloxy)amino]-2-oxoethyl}-4-cyclohexylbutyl)-5-methyl-1,3-oxazole-4-carboxylate

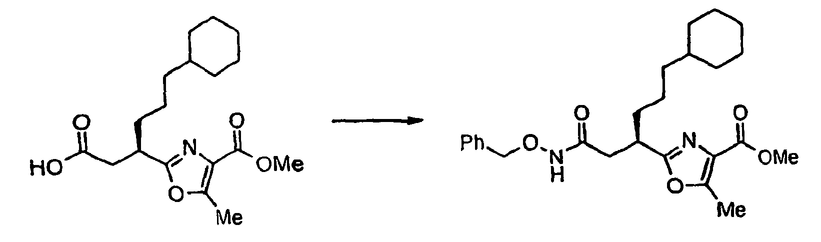

A solution of (3R)-6-cyclohexyl-3-[4-(methoxycarbonyl)-5-methyl-1,3-oxazol-2-yl]hexanoic acid (Preparation 174) (2.48g, 7.36mmol) was cooled to 0° and treated with 1-hydroxybenzotriazole hydrate (994mg, 7.36mmol), 1-[3-(dimethylamino)propyl]-3-ethylcarbodiimide hydrochloride (2.12g, 11.06mmol) and N-methylmorpholine (1.21ml, 11.04mmol). The mixture was stirred for 15 minutes then treated with O-benzylhydroxyamine (1.17g, 7.36mmol) and further N-methylmorpholine (0.81ml, 7.36mmol). The mixture was stirred for 1 hour being allowed to warm to room temperature over this time. The solvent was removed under reduced pressure and the residue was dissolved in ethyl acetate, washed sequentially with water, a saturated solution of sodium hydrogen carbonate and brine, dried over anhydrous magnesium sulphate, filtered and the solvent removed under reduced pressure to afford the title compound as a colourless oil (3.22g)
MS : 443 (MH⁺), 465 (MNa⁺)
Analysis : Found C, 66.78; H, 7.77; N, 6.19%; C₂₅H₃₄N₂O₅. 0.3 EtOAc requires C, 67.10;H, 7.82; N, 5.97%
¹H-NMR (CDCl₃) δ : 8.62 (1H, br s), 7.33 (5H, m), 4.84 (2H, s), 3.84 (3H, s), 3.36 (1H, m), 2.70-2.33 (5H, m), 1.78-1.54 (7H, m), 1.30-1.03 (8H, m), 0.82 (2H, m)

### Preparation 176:

### tert-Butyl (3R)-6-cyclohexyl-3-[3-(hydroxymethyl)-1,2,4-oxadiazol-5-yl]hexanoate

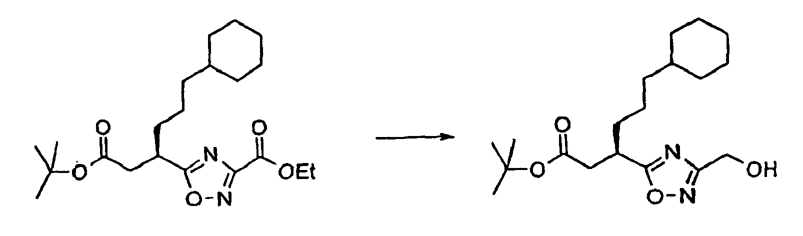

A solution of ethyl 5-{(1R)-1-[2-(*tert*-butoxy)-2-oxoethyl]-4-cyclohexylbutyl}-1,2,4-oxadiazole-3-carboxylate (Preparation 170) (15.2g, 38.50mmol) in ethanol (120ml) was treated with portions of sodium borohydride (1.46g, 38.50mmol) and the resulting mixture was was stirred at room temperature under a nitrogen atmosphere for 5 hours. Aqueous citric acid (5%^{w}/ᵥ solution) was added slowly and the mixture was stirred at room temperature for a further 30 minutes. The organic solvent was removed under reduced pressure. The aqueous layer was diluted with water and extracted with ethyl acetate giving an emulsion. Anhydrous sodium chloride was added to break up the emulsion. The combined organic layers were washed with saturated sodium hydrogen carbonate solution and brine, dried over anhydrous sodium sulphate, filtered and the solvent removed under reduced pressure to afford the title compound as a colourless oil (13.4g).
MS : 375 (MH⁺)
¹H-NMR (CDCl₃) δ : 4.77 (2H, s), 3.46 (1H, m), 2.80 (1H, dd), 2.62 (1H, dd), 1.58-1.80 (7H, m), 1.39 (9H, s), 1.07-1.33 (8H, m), 0.82 (2H, m).

### Preparation 177:

### tert-Butyl (3R)-6-cyclohexyl-3-[3-({[(4-methylphenyl)sulfonyl]oxy}methyl)-1,2,4-oxadiazol-5-yl]hexanoate

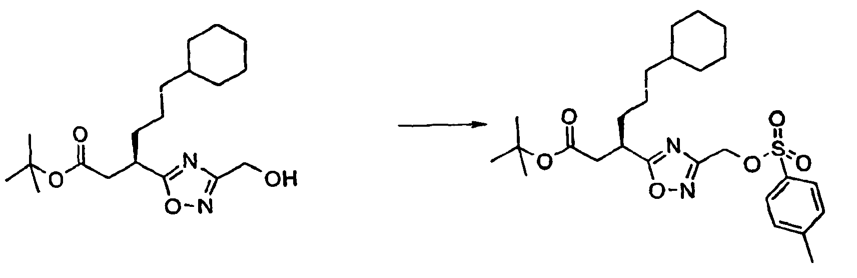

A suspension of sodium hydride 60% suspension in mineral oil (1.52g, 38.00mmol) in anhydrous tetrahydrofuran (30ml) was cooled to 0°C and treated with a solution of tert-butyl (3R)-6-cyclohexyl-3-[3-(hydroxymethyl)-1,2,4-oxadiazol-5-yl]hexanoate (Preparation 176) (13.40g, 38.00mmol) in anhydrous tetrahydrofuran (120ml) and stirred under a nitrogen atmosphere for 30 minutes. p-Toluene sulphonyl chloride (7.25g, 38.00mmol) was added portionwise and the mixture was allowed to warm to room temperature over 18 hours. The solvent was removed under reduced pressure The residue was dissolved in ethyl acetate and washed with water, saturated sodium hydrogen carbonate solution and brine, dried over anhydrous sodium sulphate, filtered and the solvent removed under reduced pressure. The oil was purified by column chromatography on silica gel eluting with dichloromethane to afford the title compound (10.75g).
MS : 529 (MNa⁺)
¹H-NMR (CDCl₃) δ : 7.80 (2H, d), 7.33 (2H, d), 5.12 (2H, s), 3.40 (1H, m), 2.72 (1H, dd), 2.58 (1H, dd), 2.43 (3H, s), 1.56-1.78 (7H, m), 1.35 (9H, s), 1.05-1.30 (8H, m), 0.82 (2H, m).

## Claims

1. A compound of formula (I): 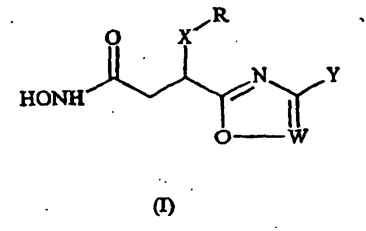 wherein:
X is C₁₋₆ alkylene or C₂₋₆ alkenylene, each of which is optionally substituted by one or more fluorine atoms;
R is aryl or C₃₋₆ cycloalkyl optionally substituted by one or more fluorine atoms;
W is N or CZ;
Y is
(a) NR¹R³,
(b) C₁₋₄ alkyl substituted by NR¹R² or by a 4- to 7-membered N-heterocycle attached via the N-atom, which heterocycle optionally contains 1 or 2 further ring hetero-atoms independently selected from N, O and S, and which heterocycle is optionally substituted by one or more substituents independently selected from R², =O and OH,
or (c) a 4- to 7-membered saturated or partially or fully unsaturated N-heterocycle, which heterocycle optionally contains 1 or 2 further ring hetero-atoms independently selected from N, O and S, and which heterocycle is optionally substituted by one or more substituents independently selected from R², =O and OH,
Z is H or C₁₋₄ alkyl,
or when W is CZ, Y is H or C₁₋₄ alkyl, and Z is
(a) NR¹R³,
(b) C₁₋₄ alkyl substituted by NR¹R² or by a 4- to 7-membered N-heterocycle attached via the N-atom, which heterocycle optionally contains 1 or 2 further ring hetero-atoms independently selected from N, O and S, and which heterocycle is optionally substituted by one or more substituents independently selected from R², =O and OH,
or (c) a 4- to 7-membered saturated of partially or fully unsaturated N-heterocycle attached via the N-atom, which heterocycle optionally contains 1 or 2 further ring hetero-atoms independently selected from N, O and S, and which heterocycle is optionally substituted by one or more substituents independently selected from R², =O and OH,
R² is H,
C₁₋₆ alkyl (optionally substituted by one or more substituents independently selected from OH,
C₁₋₄ alkoxy, C(O)ₚ(C₁₋₄ alkyl, aryl, heteroaryl, or NR¹R³), CONR¹R³ or NR¹R³),
SO₂(C₁₋₄ alkyl, aryl, heteroaryl or NR¹R³),
C(O)ₚ(C₁₋₄ alkyl optionally substituted by C₁₋₄ alkoxy or NR¹R³),
C(O)ₚ(C₃₋₇ cycloalkyl),
C(O)ₚ(aryl),
C(O)ₚ(heteroaryl),
CONR¹R³,
C₃₋₇ cycloalkyl optionally substituted by one or more substituents independently selected from OH and C₁₋₄ alkoxy,
a 4- to 7-membered saturated or partially or fully unsaturated heterocycle, which heterocycle ring contains up to 3 ring hetero-atoms independently selected from N, O and S, and which heterocycle is optionally substituted by one or more substituents independently selected from
R³, =O, OH, SO₂(C₁₋₄ alkyl) and/or C(O)ₚ(C₁₋₄ alkyl) groups, or aryl,
R¹ and R³ are each independently selected from H and (C₁₋₄ alkyl optionally substituted by OH, NR⁴R⁵ or by C₁₋₄ alkoxy),
R⁴ and R⁵ are each independently selected from H and C₁₋₄ alkyl,
p is 1 or 2,
"aryl" is phenyl optionally substituted by one or more substituents independently selected from R³, OH, SO₂(C₁₋₄ alkyl) and/or C(O)ₚ(C₁₋₄ alkyl) groups,
"heteroaryl" is a 5- to 7-membered aromatic heterocycle with 1, 2 or 3 ring hetero-atoms independently selected from N, O and S, and which ring is optionally substituted by one or more substituents independently selected from R³, =O, OH, SO₂(C₁₋₄ alkyl) and/or C(O)ₚ(C₁₋₄alkyl) groups,
and the pharmaceutically acceptable salts and solvates (including hydrates) thereof.

2. A compound, salt, or solvate according to claim 1 wherein the compound of formula (I) has the following stereochemistry (IA):

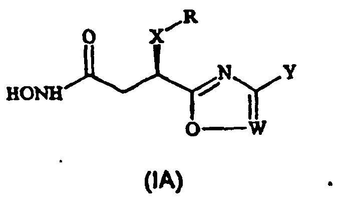

3. A compound, salt or solvate according to daim 1 or claim 2 wherein W is N.

4. A compound, salt or solvate according to any previous claim wherein X is a linear C₂₋₆ alkylene moiety optionally substituted by one or more fluorine atoms.

5. A compound, salt or solvate according to any previous claim wherein R is C₃₋₆ cycloalkyl optionally substituted by one or more fluorine atoms.

6. A compound, salt or solvate according to any previous claim wherein Y is C₁₋₄ alkyl substituted by NR¹R², or Y is a 4- to 7-membered saturated or partially or fully unsaturated N-heterocycle, which heterocycle optionally contains 1 or 2 further ring hetero-atoms independently selected from N, O and S, and which heterocycle is optionally substituted by one or more substituents independently selected from R², =O and OH.

7. A compound, salt or solvate according to any one of claims 1, 2, 4, 5 or 6 wherein W is CZ and Z is H or CH₃.

8. A compound, salt or solvate according to any previous claim wherein X is propylene.

9. A compound, salt or solvate according to any previous claim wherein R is cyclobutyl, cyclopentyl or cyclohexyl optimialty substituted by one or more fluorine atoms.

10. A compound, salt or solvate according to any previous claim wherein Y is CH₂ substituted by NR¹R², or Y is a 6-membered saturated or partially or fully unsaturated N-heterocycle, and which heterocyde is optionally substituted by one or more substituents independently selected from R², =O and OH.

11. A compound, salt or solvate according to any previous claim wherein R is cyclohexyl.

12. A compound, salt or solvate according to any previous claim wherein Y is CH₂N(H or CH₃)(SO₂(C₁₋₄ alkyl, aryl, heteroaryl or NR¹R³)), or Y is a 6-membered saturated or partially or fully unsaturated N-heterocycle, and which heterocycle is optionally substituted by SO₂(C₁₋₄ alkyl) and/or C(O)ₚ(C₁₋₄ alkyl) groups.

13. A compound, salt or solvate according to any previous claim wherein Y is CH₂NHSO₂(C₁₋₄ alkyl), or Y is a 6-membered saturated N-heterocyde, and which heterocycle is optionally substituted by SO₂(C₁₋₄ alkyl) or C(O)ₚ(C₁₋₄ alkyl).

14. A compound, salt or solvate according to any previous claim wherein Y is CH₂NHSO₂CH₃ or methylsulphonylpiperidinyl.

15. A pharmaceutical composition comprising a compound, salt or solvate according to any previous claim and a pharmaceutically acceptable adjuvant, diluent or carrier.

16. A kit comprising :
a compound, salt or solvate according to any one of claims 1 to 14, or a composition according to claim 15 ;
packaging; and
instructions for the use of the said compound, salt or solvate or composition in a manner suitable for treating or preventing a condition mediated by PCP.

17. A compound, salt or solvate according to any one of claims 1 to 14 for use in medicine.

18. A compound, salt or solvate according to any one of claims 1 to 14 for use in treating or preventing a condition mediated by PCP.

19. The use of a compound, salt or solvate according to any one of claims 1 to 14 in the manufacture of a medicament for the treatment or prevention of a condition mediated by PCP.

20. A process to make a compound of formula (I) where W is N and wherein the substituents X, R, and Y are as defined in claim 1,
which comprises any of the steps below: 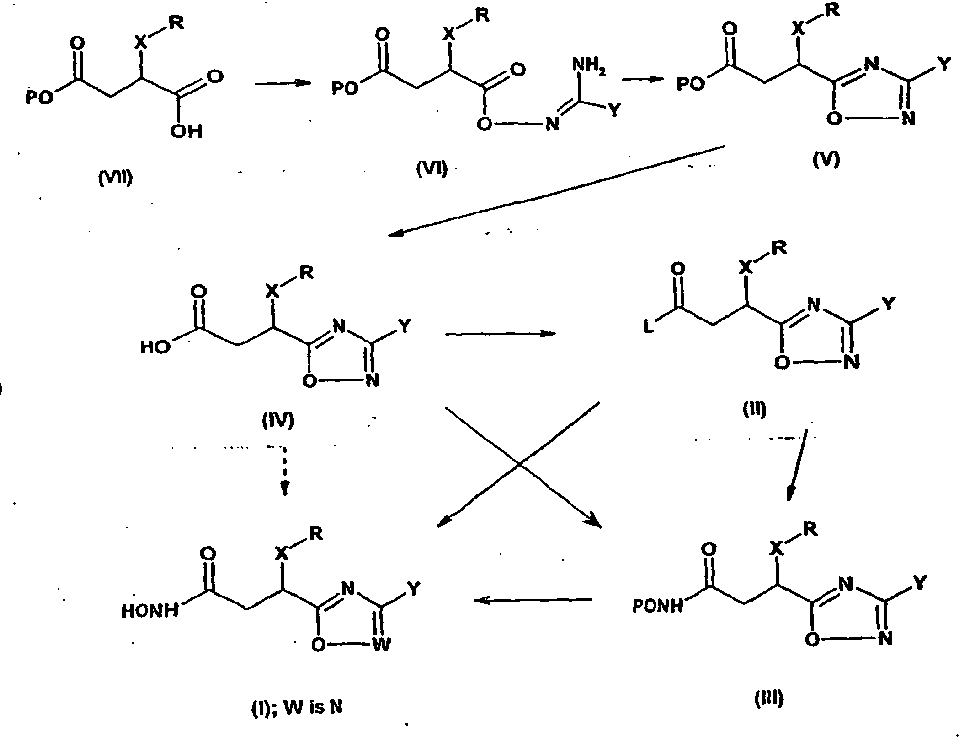 wherein
the hydroxamic acid of formula (I) where W is N is made by reaction of the corresponding activated acid derivative of formula (II), where L⁻ is a suitable leaving group, with hydroxylamine;
the compound of formula (II) is made from the corresponding acid (IV) by reaction with an acid-activating agent;
a coupling agent is used to convert compounds of formula (IV) to compounds of formula (I) for example via intermediates of formulae (II) and/or (III);
the compound of formula (I) is made from a NHO-protected compound of formula (III), where P is a suitable O-protecting group, by suitable deprotection;
the compound of formula (111) is made from a compound of formula (II), using a protected hydroxylamine PONH₂ or a suitable salt thereof;
the compound of formula (IV) is made by deprotection of the O-protected species of formula (V); the compound of formula (V) is made by condensation reaction of a compound of formula (VI);
the compound of formula (VI) is made by coupling an acid of formula (VII) with a reagent of formula C(NH₂)(Y)=NOH; and
the Y moiety of the compound of formula (I) where W is CZ undergoes a functional group transformation to give another compound of formula (I) where W is N and the Y group is different.

21. A process to make a compound of formula (I) where W is CZ and wherein the substituents X, R, and Y are as defined in claim 1, which comprises any of the steps below: 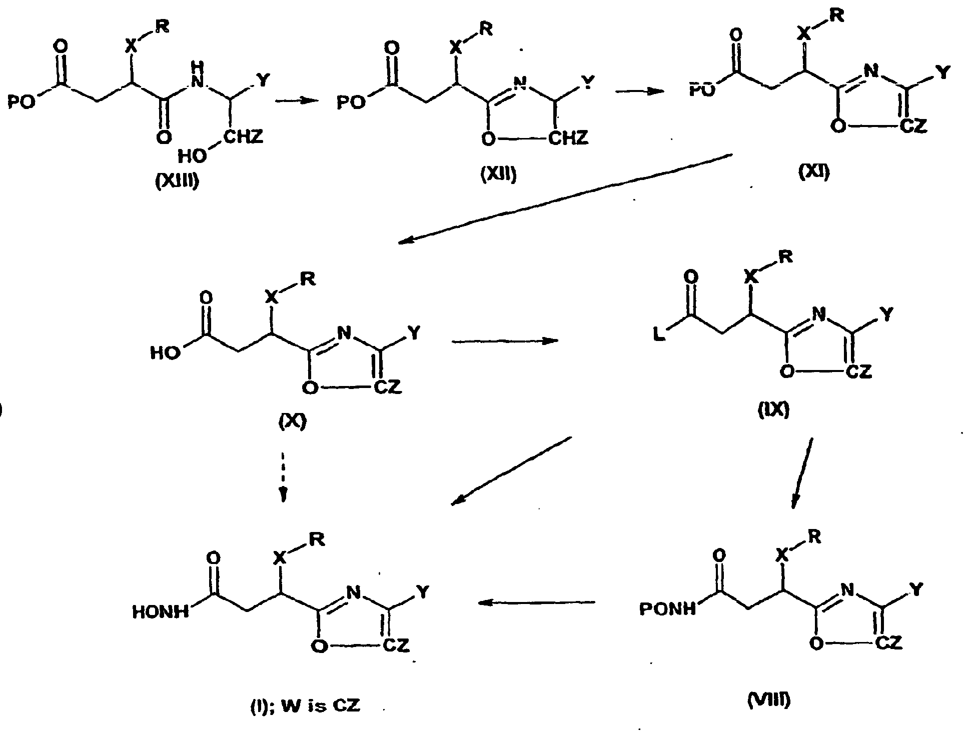 wherein
the hydroxamic acid compound of formula (I) where W is CZ is made by reaction of the corresponding activated add derivative of formula (IX), where L⁻ is a suitable leaving group, with hydroxylamine;
the compound of formula (IX) is made from the corresponding add (X) by reaction with an acid-activating agent;
the compound of formula (I) is made from a NHO-protected compound of formula (VIII), where P is a suitable O-protecting group, by suitable deprotection;
the compound of formula (VIII) is made a compound of formula (IX), using a protected hydroxylamine PONH₂ or a suitable salt thereof;
the acid of formula (X) is made by deprotection of the O-protected species of formula (XI);
the compound of formula (XI) is made by oxidation of a compound of formula (XII);
the compound of formula (XII) is made by condensation of a compound of formula (XIII);
a coupling agent is used to convert compounds of formula (X) to compounds of formula (I) for example via intermediates of formulae (IX) and/or (VIII);
the compound of formula (XIII) is made by condensation of the acid of formula (II) as defined in claim 20, with an agent of formula NH₂CH(Y)CH(Z)OH;
the compound of formula (XI) is made by condensation of compounds of formula (XIV) 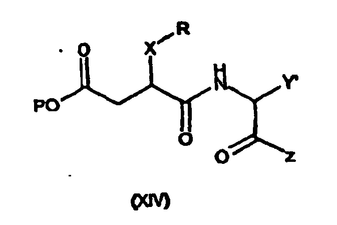 where Y' is a precursor molety which can undergo functional group transformation to give Y; and the Y molety of the compound of formula (I) where W is CZ undergoes a functional group transformation to give another compound of formula (I) where W is CZ and the Y group is different.

## Revendications

1. Composé de formule (I) : 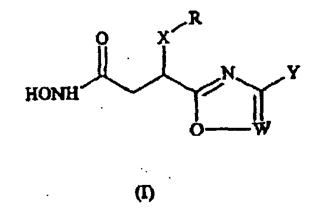 dans laquelle :
X représente un groupe alkylène en C₁ à C₆ ou alcénylène en C₂ à C₆, chacun de ces groupes étant facultativement substitué avec un ou plusieurs atomes de fluor ;
R représente un groupe aryle ou cycloalkyle en C₃ à C₈, facultativement substitué avec un ou plusieurs atomes de fluor ;
W représente N ou un groupe CZ ;
Y représente
(a) un groupe NR¹R³
(b) un groupe alkyle en C₁ à C₄ substitué avec un groupe NR¹R² ou avec un N-hétérocycle tétra- à heptagonal fixé par l'atome de N, hétérocycle qui contient facultativement 1 ou 2 hétéroatomes supplémentaires dans le noyau, choisis indépendamment entre N, O et S, et hétérocycle qui est facultativement substitué avec un ou plusieurs substituants choisis indépendamment entre des substituants R², =O et OH,
ou (c) un N-hétérocycle tétra- à heptagonal saturé ou bien partiellement ou totalement insaturé, hétérocycle qui contient facultativement 1 ou 2 hétéroatomes supplémentaires dans le noyau, choisis indépendamment entre N, O et S, et hétérocycle qui est facultativement substitué avec un ou plusieurs substituants choisis indépendamment entre des substituants R², =O et OH,
Z représente H ou un groupe alkyle en C₁ à C₄,
ou bien, lorsque W représente un groupe CZ, Y représente H ou un groupe alkyle en C₁ à C₄ et Z représente
(a) un groupe NR¹R³
(b) un groupe alkyle en C₁ à C₄ substitué avec un groupe NR¹R² ou avec un N-hétérocycle tétra- à heptagonal fixé par l'atome de N, hétérocycle qui contient facultativement 1 ou 2 hétéroatomes supplémentaires dans le noyau, choisis indépendamment entre N, O et S, et hétérocycle qui est facultativement substitué avec un ou plusieurs substituants choisis indépendamment entre des substituants R², =O et OH,
ou (c) un N-hétérocycle tétra- à heptagonal saturé ou bien partiellement ou totalement insaturé, fixé par l'atome de N, hétérocycle qui contient facultativement 1 ou 2 hétéroatomes supplémentaires dans le noyau, choisis indépendamment entre N, O et S, et hétérocycle qui est facultativement substitué avec un ou plusieurs substituants choisis indépendamment entre des substituants R², =O et OH,
R² représente H
un groupe alkyle en C₁ à C₆ (facultativement substitué avec un ou plusieurs substituants choisis indépendamment entre des substituants OH, alkoxy en C₁ à C₄, C(O)p(alkyle en C₁ à C₄, aryle, hétéroaryle, ou NR¹R³), CONR¹R³ ou NR¹R³),
un groupe SO₂(alkyle en C₁ à C₄, aryle, hétéroaryle ou NR¹R³),
un groupe C(O)ₚ(alkyle en C₁ à C₄ facultativement substitué avec un substituant alkoxy en C₁ à C₄ ou NR¹R³),
un groupe C(O)ₚ(cycloalkyle en C₁ à C₇),
un groupe C(O)paryle,
un groupe C(O)ₚhétéroaryle,
un groupe CONR¹R³,
un groupe cycloalkyle en C₃ à C₇ facultativement substitué avec un ou plusieurs substituants choisis indépendamment entre des substituants OH et alkoxy en C₁ à C₄,
un hétérocycle tétra- à heptagonal saturé ou bien partiellement ou totalement insaturé, le noyau de cet hétérocycle contenant jusqu'à 3 hétéroatomes choisis indépendamment entre N, O et S, et hétérocycle qui est facultativement substitué avec un ou plusieurs substituants choisis indépendamment entre des groupes R³, =O, OH, SO₂(alkyle en C₁ à C₄) et/ou C(O)ₚ(alkyle en C₁ à C₄),
ou un groupe aryle,
R¹ et R³ sont choisis chacun indépendamment entre H, et un groupe (alkyle en C₁ à C₄ facultativement substitué avec un substituant OH, NR⁴R⁵ ou avec un substituant alkoxy en C₁ à C₄),
R⁴ et R⁵ sont choisis chacun indépendamment entre H et un groupe alkyle en C₁ à C₄,
p est égal à 1 ou 2,
le terme "aryle" désigne un groupe phényle facultativement substitué avec un ou plusieurs substituants choisis indépendamment entre des groupes R³, OH, SO₂ (alkyle en C₁ à C₄) et/ou C(O)ₚ(alkyle en C₁ à C₄),
le terme "hétéroaryle" désigne un hétérocycle aromatique penta- à heptagonal avec 1, 2 ou 3 hétéroatomes dans le noyau choisis indépendamment entre N, O et S, noyau qui est facultativement substitué avec un ou plusieurs substituants choisis indépendamment entre des groupes R³, =O, OH, SO₂ (alkyle en C₁ à C₄) et/ou C(O)ₚ(alkyle en C₁ à C₄)
et ses sels et produits de solvatation (y compris hydrates) pharmaceutiquement acceptables.

2. Composé, sel, ou produit de solvatation suivant la revendication 1, dans lequel le composé de formule (I) a la stéréochimie (IA) suivante :

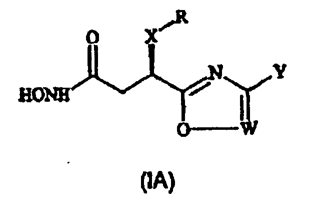

3. Composé, sel, ou produit de solvatation suivant la revendication 1 ou la revendication 2, dans lequel W représente N.

4. Composé, sel, ou produit de solvatation suivant l'une quelconque des revendications précédentes, dans lequel X représente un groupement alkylène en C₂ à C₄ linéaire facultativement substitué avec un ou plusieurs atomes de fluor.

5. Composé, sel, ou produit de solvatation suivant l'une quelconque des revendications précédentes, dans lequel R représente un groupe cycloalkyle en C₃ à C₈ facultativement substitué avec un ou plusieurs atomes de fluor.

6. Composé, sel, ou produit de solvatation suivant l'une quelconque des revendications précédentes, dans lequel Y représente un groupe alkyle en C₁ à C₄ substitué avec un groupe NR¹R², ou bien Y représente un N-hétérocycle tétra- à heptagonal saturé ou bien partiellement ou totalement insaturé, hétérocycle qui contient facultativement 1 ou 2 hétéroatomes supplémentaires dans le noyau, choisis indépendamment entre N, O et S, et hétérocycle qui est facultativement substitué avec un ou plusieurs substituants choisis indépendamment entre des substituants R², =O et OH.

7. Composé, sel, ou produit de solvatation suivant l'une quelconque des revendications 1, 2, 4, 5 et 6, dans lequel W représente un groupe CZ et Z représente H ou un groupe CH₃.

8. Composé, sel, ou produit de solvatation suivant l'une quelconque des revendications précédentes, dans lequel X représente un groupe propylène.

9. Composé, sel, ou produit de solvatation suivant l'une quelconque des revendications précédentes, dans lequel R représente un groupe cyclobutyle, cyclopentyle ou cyclohexyle facultativement substitué avec un ou plusieurs atomes de fluor.

10. Composé, sel, ou produit de solvatation suivant l'une quelconque des revendications précédentes, dans lequel Y représente un groupe CH₂ substitué avec un substituant NR¹R², ou bien Y représente un N-hétérocycle hexagonal saturé ou bien partiellement ou totalement insaturé, hétérocycle qui est facultativement substitué avec un ou plusieurs substituants choisis indépendamment entre des substituants R², =O et OH.

11. Composé, sel, ou produit de solvatation suivant l'une quelconque des revendications précédentes, dans lequel R représente un groupe cyclohexyle.

12. Composé, sel, ou produit de solvatation suivant l'une quelconque des revendications précédentes, dans lequel Y représente un groupe CH₂N(H ou CH₃)(SO₂(alkyle en C₁ à C₄, aryle, hétéroaryle ou NR¹R³)), ou bien Y représente un N-hétérocycle hexagonal saturé ou bien partiellement ou totalement insaturé, hétérocycle qui est facultativement substitué avec des groupes SO₂(alkyle en C₁ à C₄) et/ou C(O)ₚ(alkyle en C₁ à C₄).

13. Composé, sel, ou produit de solvatation suivant l'une quelconque des revendications précédentes, dans lequel Y représente un groupe CH₂NHSO₂(alkyle en C₁ à C₄), ou bien Y représente un N-hétérocycle hexagonal saturé, hétérocycle qui est facultativement substitué avec des groupes SO₂(alkyle en C₁ à C₄) ou C(O)ₚ(alkyle en C₁ à C₄).

14. Composé, sel, ou produit de solvatation suivant l'une quelconque des revendications précédentes, dans lequel Y représente un groupe CH₂NHSO₂CH₃ ou méthylsulfonyl-pipéridinyle.

15. Composition pharmaceutique comprenant un composé, sel, ou produit de solvatation suivant l'une quelconque des revendications précédentes et un adjuvant, diluant ou support pharmaceutiquement acceptable.

16. Kit comprenant :
un composé, sel, ou produit de solvatation suivant l'une quelconque des revendications 1 à 14, ou une composition suivant la revendication 15 ;
un emballage ; et
des instructions pour l'utilisation dudit composé, sel, ou produit de solvatation ou de ladite composition d'une manière convenable pour le traitement ou la prévention d'une affection à médiation par PCP.

17. Composé, sel, ou produit de solvatation suivant l'une quelconque des revendications 1 à 14, destiné à être utilisé en médecine.

18. Composé, sel, ou produit de solvatation suivant l'une quelconque des revendications 1 à 14, destiné à être utilisé dans le traitement ou la prévention d'une affection à médiation par PCP.

19. Utilisation d'un composé, sel, ou produit de solvatation suivant l'une quelconque des revendications 1 à 14, dans la production d'un médicament destiné au traitement ou à la prévention d'une affection à médiation par PCP.

20. Procédé pour la préparation d'un composé de formule (I) dans laquelle W représente N et dans laquelle les substituants X, R et Y sont tels que définis dans la revendication 1,
qui comprend n'importe laquelle des étapes ci-dessous : 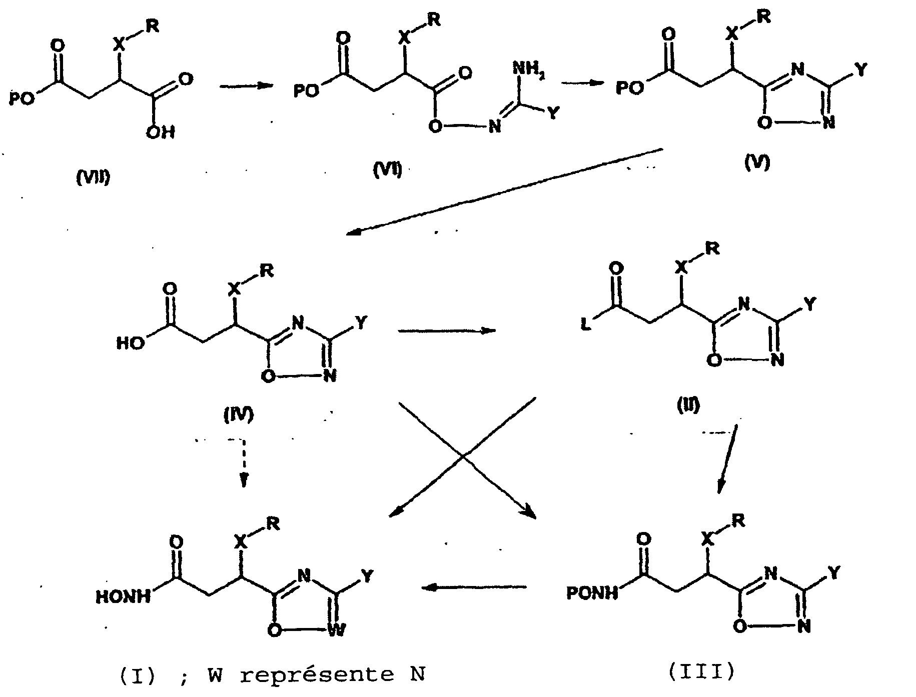 dans lesquelles
l'acide hydroxamique de formule (I) dans laquelle W représente N est préparé par réaction du dérivé d'acide activé correspondant de formule (II), dans laquelle L représente un groupe partant convenable, avec l'hydroxylamine ;
le composé de formule (II) est préparé à partir de l'acide (IV) correspondant par réaction avec un agent activateur d'acide ;
un agent de couplage est utilisé pour convertir les composés de formule (IV) en des composés de formule (I), par exemple par les intermédiaires de formules (II) et/ou (III) ;
le composé de formule (I) est préparé à partir d'un composé à groupe NHO protégé de formule (III), dans laquelle P représente un groupe convenable protecteur de l'atome de O, par une suppression de protection convenable ;
le composé de formule (III) est préparé à partir d'un composé de formule (II), en utilisant une hydroxylamine protégée PONH₂ ou un de ses sels convenables ;
le composé de formule (IV) est préparé par suppression de protection de l'entité de formule (V) à atome de O protégé ;
le composé de formule (V) est préparé par une réaction de condensation d'un composé de formule (VI) ;
le composé de formule (VI) est préparé par couplage d'un acide de formule (VII) avec un réactif de formule C(NH₂)(Y)=NOH ; et
le groupement Y du composé de formule (I) dans laquelle W représente un groupe CZ subit une transformation de groupe fonctionnel, ce qui donne un autre composé de formule (I) dans laquelle W représente N et le groupe Y est différent.

21. Procédé pour la préparation d'un composé de formule (I) dans laquelle W représente un groupe CZ et dans laquelle les substituants X, R et Y sont tels que définis dans la revendication 1,
qui comprend n'importe laquelle des étapes ci-dessous : 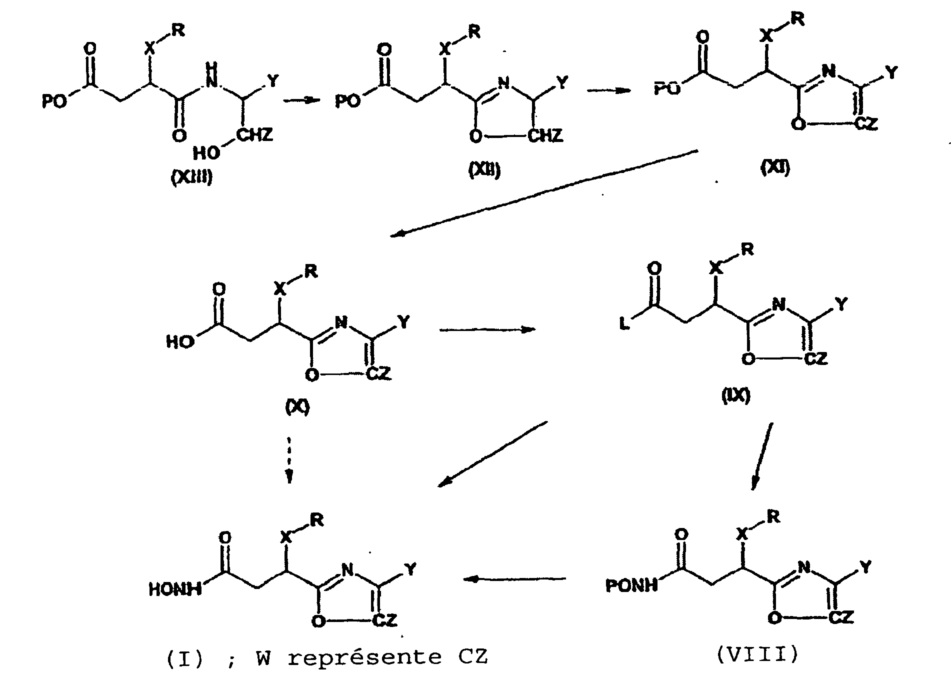 dans lesquelles
le composé du type acide hydroxamique de formule (I) dans laquelle W représente un groupe CZ est préparé par réaction du dérivé d'acide activé correspondant de formule (IX), dans laquelle L représente un groupe partant convenable, avec l'hydroxylamine ;
le composé de formule (IX) est préparé à partir de l'acide correspondant (X) par réaction avec un agent activateur d'acide ;
le composé de formule (I) est préparé à partir d'un composé à groupe NHO protégé de formule (VIII), dans laquelle P représente un groupe convenable protecteur de l'atome de O, par une suppression de protection convenable ;
le composé de formule (VIII) est préparé à partir d'un composé de formule (IX), en utilisant une hydroxylamine protégée PONH₂ ou un de ses sels convenables ;
l'acide de formule (X) est préparé par suppression de protection de l'entité à atome de O protégé de formule (XI)
le composé de formule (XI) est préparé par oxydation d'un composé de formule (XII);
le composé de formule (XII) est préparé par condensation d'un composé de formule (XIII) ;
un agent de couplage est utilisé pour convertir les composés de formule (X) en des composés de formule (I), par exemple par les intermédiaires des formules (IX) et/ou (VIII) ;
le composé de formule (XIII) est préparé par condensation de l'acide de formule (II) telle que définie dans la revendication 20, avec un agent de formule NH₂CH(Y)CH(Z)OH ;
le composé de formule (XI) est préparé par condensation de composés de formule (XIV): 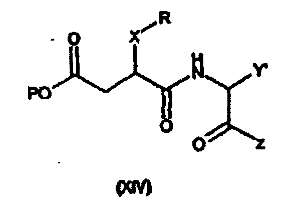 dans laquelle Y' représente un groupement précurseur qui peut subir une transformation de groupe fonctionnel pour obtenir Y ; et le groupement Y du composé de formule (I) dans laquelle W représente un groupe CZ subit une transformation de groupe fonctionnel, ce qui donne un autre composé de formule (I) dans laquelle W représente un groupe CZ et le groupe Y est différent.

## Patentansprüche

1. Verbindung der Formel (I) 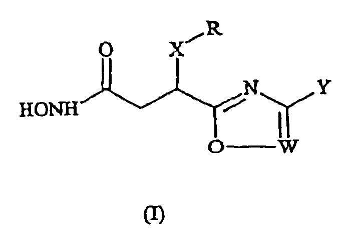 worin:
X C₁₋₆-Alkylen oder C₂₋₆-Alkenylen ist, von denen jedes gegebenenfalls mit einem Fluoratom oder mehreren Fluoratomen substituiert ist;
R Aryl oder C₃₋₈-Cycloalkyl, gegebenenfalls mit einem Fluoratom oder mehreren Fluoratomen substituiert, ist;
W für N oder CZ steht;
Y ist:
(a) NR¹R³,
(b) C₁₋₄-Alkyl, substituiert mit NR¹R² oder mit einem 4- bis 7-gliedrigen N-Heterocyclus, der über das N-Atom gebunden ist, wobei der Heterocyclus gegebenenfalls ein oder zwei weitere Ringheteroatome, unabhängig ausgewählt aus N, O und S, enthält und wobei der Heterocyclus gegebenenfalls mit einem Substituenten oder mehreren Substituenten substituiert ist, der/die unabhängig aus R², =O und OH ausgewählt sind,
oder (c) ein 4- bis 7-gliedriger gesättigter oder teilweise oder vollständig ungesättigter N-Heterocyclus ist, wobei der Heterocyclus gegebenenfalls ein weiteres Ringatom oder zwei weitere Ringatome, das/die unabhängig aus N, O und S ausgewählt sind, enthält und wobei der Heterocyclus gegebenenfalls mit einem Substituenten oder mehreren Substituenten substituiert ist, der/die unabhängig aus R², =O und OH ausgewählt ist/sind,
Z für H oder C₁₋₄-Alkyl steht, oder
wenn W für CZ steht, Y H oder C₁₋₄-Alkyl ist und Z ist:
(a) NR¹R³,
(b) C₁₋₄-Alkyl substituiert mit NR¹R² oder mit einem 4- bis 7-gliedrigen N-Heterocyclus, der über das N-Atom gebunden ist, wobei der Heterocyclus gegebenenfalls ein weiteres Ringheteroatom oder zwei weitere Ringheteroatome enthält, das/die unabhängig aus N, O und S ausgewählt ist/sind und wobei der Heterocyclus gegebenenfalls mit einem Substituenten oder mehreren Substituenten substituiert ist, der/die unabhängig aus R², =O und OH ausgewählt ist/sind,
oder (c) ein 4- bis 7-gliedriger gesättigter oder partiell oder vollständig ungesättigter N-Heterocyclus, der über das N-Atom gebunden ist, wobei der Heterocyclus gegebenenfalls ein weiteres Ringheteroatom oder zwei weitere Ringheteroatome enthält, das/die unabhängig aus N, O und S ausgewählt ist/sind, und wobei der Heterocyclus gegebenenfalls mit einem Substituenten oder mehreren Substituenten substituiert ist, der/die unabhängig aus R², =O und OH ausgewählt ist/sind,
R² für H,
C₁₋₆-Alkyl (gegebenenfalls substituiert mit einem Substituenten oder mehreren Substituenten, der/die unabhängig aus OH, C₁₋₄-Alkoxy, C (O)ₚ(C₁₋₄-Alkyl, Aryl, Heteroaryl oder NR¹R³), CONR¹R³ oder NR¹R³ ausgewählt ist/sind),
SO₂(C₁₋₄-Alkyl, Aryl, Heteroaryl oder NR¹R³),
C(O)ₚ(C₁₋₄-Alkyl, gegebenenfalls substituiert mit C₁₋₄-Alkoxy oder NR¹R³),
C(O)ₚ(C₃₋₇-Cycloalkyl),
C(O)ₚ(Aryl),
C(O)ₚ(Heteroaryl),
CONR¹R³,
C₃₋₇-Cycloalkyl, gegebenenfalls substituiert mit einem Substituenten oder mehreren Substituenten, der/die unabhängig aus OH und C₁₋₄-Alkoxy ausgewählt ist/sind,
einen 4- bis 7-gliedrigen gesättigten oder teilweise oder vollständig ungesättigten Heterocyclus, wobei der Heterocyclusring bis zu 2 Ringheteroatome enthält, die unabhängig aus N, O und S ausgewählt sind, und wobei der Heterocyclus gegebenenfalls mit einem Substituenten oder mehreren Substituenten substituiert ist, der/die unabhängig aus R³, =O, OH, SO₂(C₁₋₄-Alkyl)- und/oder C(O)ₚ(C₁₋₄-Alkyl)-Gruppen ausgewählt ist/sind,
oder Aryl steht,
R¹ und R³ jeweils unabhängig aus H und (C₁₋₄-Alkyl, gegebenenfalls substituiert mit OH, NR⁴R⁵ oder mit C₁₋₄-Alkoxy) ausgewählt sind,
R⁴ und R⁵ jeweils unabhängig aus H und C₁₋₄-Alkyl ausgewählt sind,
p 1 oder 2 ist,
"Aryl" Phenyl ist, gegebenenfalls substituiert mit einem Substituenten oder mehreren Substituenten, der/die unabhängig aus R³, OH, SO₂(C₁₋₄-Alkyl)- und/oder C(O)ₚ(C₁₋₄-Alkyl)-Gruppen ausgewählt ist/sind,
"Heteroaryl" ein 5- bis 7-gliedriger aromatischer Heterocyclus mit 1, 2 oder 3 Ringheteroatomen ist, die unabhängig aus N, O und S ausgewählt sind, und wobei der Ring gegebenenfalls mit einem Substituenten oder mehreren Substituenten substituiert ist, der/die unabhängig aus R³, =O, OH, SO₂(C₁₋₄-Alkyl)-und/oder C(O)ₚ(C₁₋₄-Alkyl)-Gruppen ausgewählt ist/sind,
und die pharmazeutisch annehmbaren Salze und Solvate (einschließlich Hydrate) davon.

2. Verbindung, Salz oder Solvat nach Anspruch 1, wobei die Verbindung der Formel (I) die folgende Stereochemie (IA) hat:

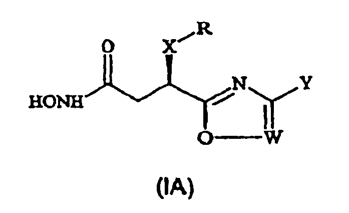

3. Verbindung, Salz oder Solvat nach Anspruch 1 oder Anspruch 2, wobei W für N steht.

4. Verbindung, Salz oder Solvat nach einem der vorangehenden Ansprüche, wobei X eine lineare C₂₋₆-Alkylengruppierung ist, die gegebenenfalls mit einem Fluoratom oder mehreren Fluoratomen substituiert ist.

5. Verbindung, Salz oder Solvat nach einem der vorangehenden Ansprüche, wobei R für C₃₋₈-Cycloalkyl steht, das gegebenenfalls mit einem Fluoratom oder mehreren Fluoratomen substituiert ist.

6. Verbindung, Salz oder Solvat nach einem der vorangehenden Ansprüche, wobei Y C₁₋₄-Alkyl ist, das mit NR¹R² substituiert ist, oder Y ein 4- bis 7-gliedriger gesättigter oder teilweise oder vollständig ungesättigter N-Heterocyclus ist, wobei der Heterocyclus gegebenenfalls ein oder zwei weitere Ringheteroatome enthält, die unabhängig aus N, O und S ausgewählt sind, und wobei der Heterocyclus gegebenenfalls mit einem Substituenten oder mehreren Substituenten substituiert ist, der/die unabhängig aus R², =O und OH ausgewählt ist/sind.

7. Verbindung, Salz oder Solvat nach einem der Ansprüche 1, 2, 4, 5 oder 6, wobei W für CZ steht und Z für H oder CH₃ steht.

8. Verbindung, Salz oder Solvat nach einem der vorangehenden Ansprüche, wobei X Propylen ist.

9. Verbindung, Salz oder Solvat nach einem der vorangehenden Ansprüche, wobei R Cyclobutyl, Cyclopentyl oder Cyclohexyl ist, das gegebenenfalls mit einem Fluoratom oder mehreren Fluoratomen substituiert ist.

10. Verbindung, Salz oder Solvat nach einem der vorangehenden Ansprüche, wobei Y für CH₂ steht, das mit NR¹R² substituiert ist, oder Y ein 6-gliedriger gesättigter oder teilweise oder vollständig ungesättigter N-Heterocyclus ist und wobei der Heterocyclus gegebenenfalls mit einem Substituenten oder mehreren Substituenten substituiert ist, der/die unabhängig aus R², =O und OH ausgewählt ist/sind.

11. Verbindung, Salz oder Solvat nach einem der vorangehenden Ansprüche, wobei R Cyclohexyl ist.

12. Verbindung, Salz oder Solvat nach einem der vorangehenden Ansprüche, wobei Y für CH₂N(H oder CH₃) (SO₂(C₁₋₄-Alkyl, Aryl, Heteroaryl oder NR¹R³)) steht oder Y ein 6-gliedriger gesättigter oder teilweise oder vollständig ungesättigter N-Heterocyclus ist und wobei der Heterocyclus gegebenenfalls mit SO₂(C₁₋₄-Alkyl)- und/oder C(O)ₚ(C₁₋₄-Alkyl)-Gruppen substituiert ist.

13. Verbindung, Salz oder Solvat nach einem der vorangehenden Ansprüche, wobei Y für CH₂NHSO₂(C₁₋₄-Alkyl) steht oder Y ein 6-gliedriger gesättigter N-Heterocyclus ist und wobei der Heterocyclus gegebenenfalls mit SO₂(C₁₋₄-Alkyl) oder C(O)ₚ(C₁₋₄₋Alkyl) substituiert ist.

14. Verbindung, Salz oder Solvat nach einem der vorangehenden Ansprüche, wobei Y für CH₂NHSO₂CH₃ oder Methylsulfonylpiperidinyl steht.

15. Pharmazeutische Zusammensetzung, die eine Verbindung, ein Salz oder ein Solvat nach einem der vorangehenden Ansprüche und ein pharmazeutisch annehmbares Adjuvans, Verdünnungsmittel oder einen pharmazeutisch annehmbaren Träger umfasst.

16. Kit, umfassend:
eine Verbindung, ein Salz oder ein Solvat nach einem der Ansprüche 1 bis 14 oder der Zusammensetzung nach Anspruch 15;
eine Verpackung und
Instruktionen zur Verwendung der Verbindung, des Salzes oder Solvats oder der Zusammensetzung in einer Weise, die für die Behandlung oder Prävention eines durch PCP vermittelten Zustands geeignet ist.

17. Verbindung, Salz oder Solvat nach einem der Ansprüche 1 bis 14 zur Verwendung in Medizin.

18. Verbindung, Salz oder Solvat nach einem der Ansprüche 1 bis 14 zur Verwendung bei der Behandlung oder Prävention eines durch PCP vermittelten Zustands.

19. Verwendung einer Verbindung, eines Salzes oder Solvats nach einem der Ansprüche 1 bis 14 bei der Herstellung eines Medikaments für die Behandlung oder Prävention eines durch PCP vermittelten Zustands.

20. Verfahren zur Herstellung einer Verbindung der Formel (I), wobei W für N steht und wobei die Substituenten X, R und Y wie in Anspruch 1 definiert sind,
wobei das Verfahren beliebige der folgenden Schritte umfasst: 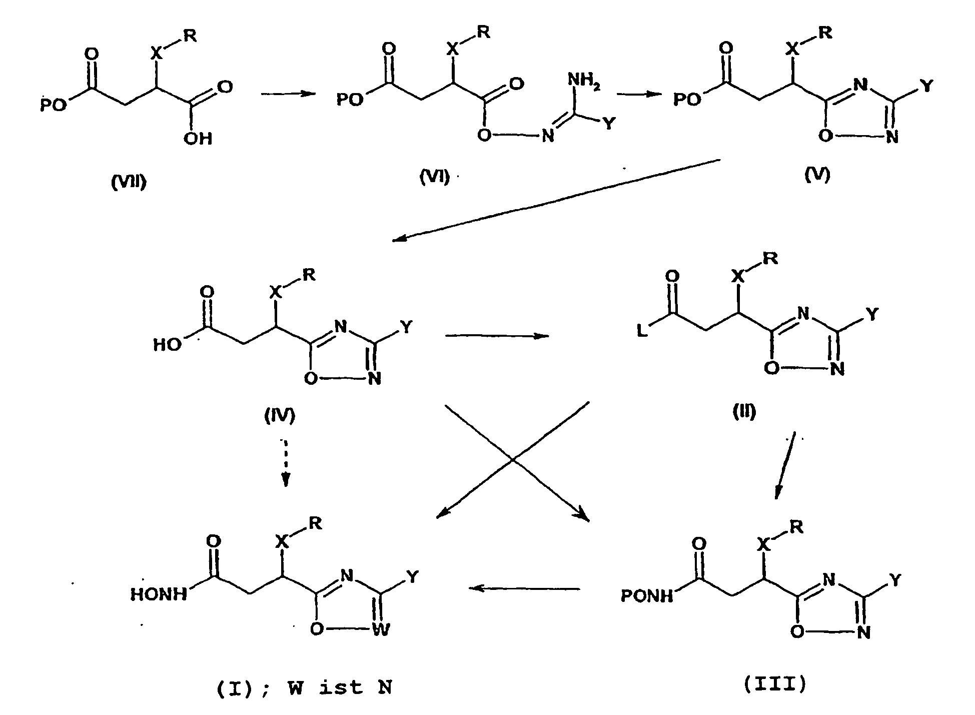 worin:
die Hydroxamsäure der Formel (I), worin W für N steht, durch Reaktion des entsprechenden aktivierten Säurederivats der Formel (II), worin L eine geeignete Austrittsgruppe ist, mit Hydroxylamin hergestellt wird;
die Verbindung der Formel (II) aus der entsprechenden Säure (IV) durch Reaktion mit einem Säure-aktivierenden Mittel hergestellt wird;
ein Kopplungsmittel verwendet wird, um Verbindungen der Formel (IV) in Verbindungen der Formel (I) beispielsweise über Zwischenprodukte der Formeln (II) und/oder (III) umzuwandeln; die Verbindung der Formel (I) aus einer NHO-geschützten Verbindung der Formel (III), worin P eine geeignete O-Schutzgruppe ist, durch geeignete Entschützung hergestellt wird;
die Verbindung der Formel (III) aus einer Verbindung der Formel (II) unter Verwendung eines geschützten Hydroxylamins PONH₂ oder eines geeigneten Salzes davon hergestellt wird;
die Verbindung der Formel (IV) durch Entschützen der O-geschützten Spezies der Formel (V) hergestellt wird;
die Verbindung der Formel (V) durch Kondensationsreaktion einer Verbindung der Formel (VI) hergestellt wird;
die Verbindung der Formel (VI) durch Koppeln einer Säure der Formel (VII) mit einem Reagens der Formel C(NH₂)(Y)=NOH hergestellt wird, und
die Y-Gruppierung der Verbindung der Formel (I), worin W für CZ steht, eine funktionelle Gruppen-Transformation durchmacht, um eine andere Verbindung der Formel (I) zu ergeben, in der W für N steht und die Y-Gruppe unterschiedlich ist.

21. Verfahren zur Herstellung einer Verbindung der Formel (I), wobei W für CZ steht und wobei die Substituenten X, R und Y wie in Anspruch 1 definiert sind,
wobei das Verfahren beliebige der folgenden Schritte umfasst: 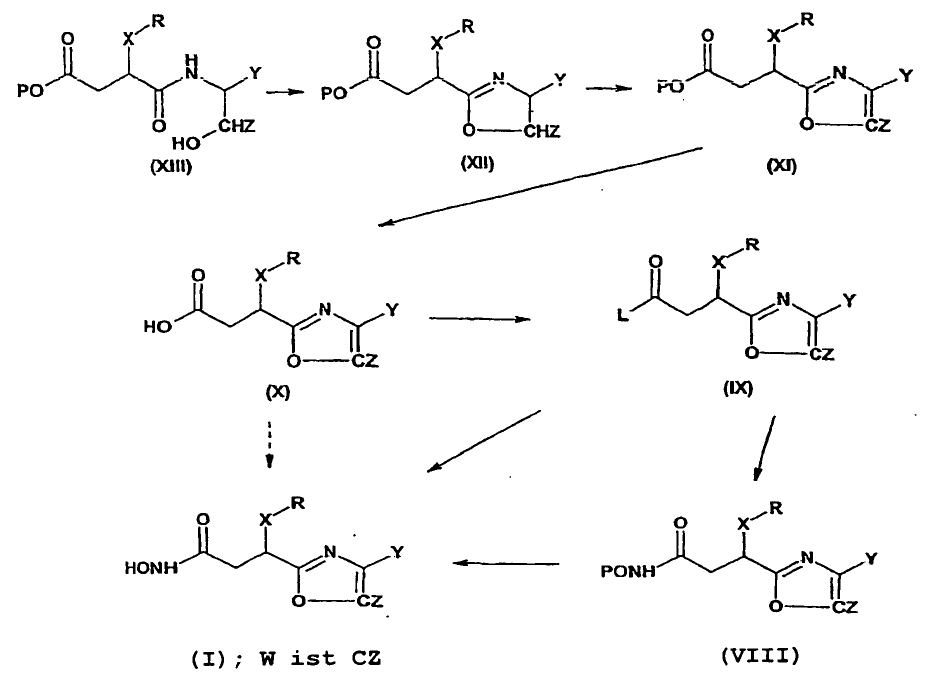 wobei
die Hydroxamsäureverbindung der Formel (I), worin W für CZ steht, durch Reaktion des entsprechenden aktivierten Säurederivats der Formel (IX), worin L eine geeignete Austrittsgruppe ist, mit Hydroxylamin hergestellt wird;
die Verbindung der Formel (IX) aus der entsprechenden Säure (X) durch Reaktion mit einem Säure-aktivierenden Agens hergestellt wird;
die Verbindung der Formel (I) aus einer NHO-geschützten Verbindung der Formel (VIII), worin P eine geeignete O-Schutzgruppe ist, durch geeignete Entschützung hergestellt wird;
die Verbindung der Formel (VIII) aus einer Verbindung der Formel (IX) unter Verwendung eines geschützten Hydroxylamins PONH₂ oder eines geeigneten Salzes davon hergestellt wird;
die Säure der Formel (X) durch Entschützen der O-geschützten Spezies der Formel (XI) hergestellt wird;
die Verbindung der Formel (XI) durch Oxidation einer Verbindung der Formel (XII) hergestellt wird;
die Verbindung der Formel (XII) durch Kondensation einer Verbindung der Formel (XIII) hergestellt wird;
ein Kopplungsmittel verwendet wird, um Verbindungen der Formel (X) in Verbindungen der Formel (I) zum Beispiel über Zwischenprodukte der Formel (IX) und/oder (VIII) umzuwandeln;
die Verbindung der Formel (XIII) durch Kondensation der Säure der Formel (II) wie in Anspruch 20 definiert mit einem Agens der Formel NH₂CH(Y)CH(Z)OH hergestellt wird;
die Verbindung der Formel (XI) durch Kondensation von Verbindungen der Formel (XIV) 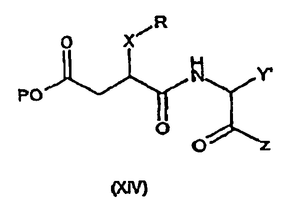 worin Y' eine Vorläufergruppierung ist, die eine funktionelle Gruppentransformation durchmachen kann, um Y zu ergeben, hergestellt wird; die Y-Gruppierung der Verbindung der Formel (I), worin W für CZ steht, einer funktionellen Gruppentransformation unter Erhalt einer anderen Verbindung der Formel (I), worin W für CZ steht und die Y-Gruppe unterschiedlich ist, durchmacht.
